# EUROPÄISCHE PATENTSCHRIFT

(11) **EP 0 607 206 B1**
(45) Veröffentlichungstag und Bekanntmachung des Hinweises auf die Patenterteilung: **10.04.2002**
(21) Anmeldenummer: 92920580.5
(22) Anmeldetag: 28.09.1992
(51) Int. Cl.: C12N 15/87, A61K 48/00, C12N 7/04, A61K 47/48, C07K 14/005, C12N 15/34, C12N 15/41, C12N 15/44

(54) **ZUSAMMENSETZUNG FÜR DAS EINBRINGEN VON NUKLEINSÄURE-KOMPLEXEN IN HÖHERE EUKARYOTISCHE ZELLEN**
COMPOSITION FOR INSERTING NUCLEIC ACID COMPLEXES INTO HIGHER EUCARYOTIC CELLS
COMPOSITION D'INSERTION DE COMPLEXES D'ACIDES NUCLEIQUES DANS DES CELLULES EUCARYOTES SUPERIEURES

(30) Priorität: 30.09.1991 US 767788; 30.09.1991 US 768039; 30.01.1992 US 827102; 30.01.1992 US 827103; 07.04.1992 US 864759; 02.09.1992 US 937788
(43) Veröffentlichungstag der Anmeldung: 27.07.1994
(73) Patentinhaber: Boehringer Ingelheim International GmbH, 55218 Ingelheim am Rhein (DE); UNIVERSITY OF NORTH CAROLINA AT CHAPEL HILL, Chapel Hill, N.C. 27599-7020 (US); GENENTECH, INC., South San Francisco California 94080 (US)
(72) Erfinder: CURIEL, David, Chapel Hill, NC 27516 (US); WAGNER, Ernst, A-2103 Langenzersdorf (AT); COTTEN, Matt, A-1130 Wien (AT); ZATLOUKAL, Kurt, A-1030 Wien (AT); PLANK, Christian, A-1030 Wien (AT); BIRNSTIEL, Max, L., A-1100 Wien (AT); OBERHAUSER, Bernd, A-1140 Wien (AT); SCHMIDT, Walter, G., M., A-1030 Wien (AT)
(74) Vertreter: Laudien, Dieter, Dr.
(86) Internationale Anmeldenummer: EP9202234
(87) Internationale Veröffentlichungsnummer: WO9307283

(56) Entgegenhaltungen:
- EP-A- 0 388 758
- EP-A- 0 422 543
- WO-A-91/17773
- PROCEEDINGS OF THE NATIONAL ACADEMY OF SCIENCES OF USA. Bd. 88, Nr. 19, 1. Oktober 1991, WASHINGTON US Seiten 8850 - 8854 CURIEL, D.T. ET AL. 'Adenovirus enhancement of transferrin-polylysine-mediated gene delivery'
- PROCEEDINGS OF THE NATIONAL ACADEMY OF SCIENCES OF USA. Bd. 89, Nr. 13, 1. Juli 1992, WASHINGTON US Seiten 6094 - 6098 COTTEN, M. ET AL. 'High-efficiency receptor-mediated delivery of small and large (48 kilobase gene constructs using the endosome-disruption activity of defective or chemically inactivated adenovirus particles'
- PROCEEDINGS OF THE NATIONAL ACADEMY OF SCIENCES OF USA. Bd. 89, Nr. 13, 1. Juli 1992, WASHINGTON US Seiten 6099 - 6103 WAGNER, E. ET AL. 'Coupling of adenovirus to transferrin-polylysine/DNA complexes greatly enhances receptor-mediated gene delivery and expression of transfected genes'
- PROCEEDINGS OF THE NATIONAL ACADEMY OF SCIENCES OF USA. Bd. 89, Nr. 17, 1. September 1992, WASHINGTON US Seiten 7934 - 7938 WAGNER, E. ET AL. 'Influenza virus hemagglutinin HA-2 N-terminal fusogenic peptides augment gene transfer by transferrin-polylysine-DNA complexes: toward a synthetic virus-like gene-transfer vehicle'
- MOLECULAR AND CELLULAR BIOLOGY Bd. 4, Nr. 8, August 1984, Seiten 1528 - 1533 SETH, P. ET AL. 'Evidence that the penton base of adenovirus is involved in potentiation of toxicity of Pseudomonas exotoxin conjugated to Epidermal Growth Factor'
- JOURNAL OF VIROLOGY Bd. 51, Nr. 3, September 1984, Seiten 650 - 655 SETH, P. ET AL. 'Role of a low-pH environment in adenovirus enhancement of the toxicity of a Pseudomonas exotoxin-Epidermal Growth Factor conjugate'
- GENE Bd. 63, Nr. 2, 1988, AMSTERDAM NL Seiten 321 - 330 TIKCHONENKO, T.I. ET AL. 'Transfer of condensed viral DNA into eukaryotic cells using proteoliposomes'
- GENE Bd. 84, Nr. 2, 14. Dezember 1989, AMSTERDAM NL Seiten 429 - 438 GOULD-FOGERITE, S. ET AL. 'Chimerasome-mediated gene transfer in vitro and in vivo'
- JOURNAL OF GENERAL VIROLOGY Bd. 69, Nr. 8, August 1988, UK Seiten 1847 - 1857 WHARTON, S.A. ET AL. 'Membrane fusion by peptide analogues of Influenza Virus haemagglutinin'

## Beschreibung

Die Erfindung bezieht sich auf das Einbringen von Nukleinsäuren in höhere eukaryotische Zellen.

Bedarf an einem effizienten System für das Einführen von Nukleinsäure in lebende Zellen besteht vor allem im Rahmen der Gentherapie. Dabei werden Gene in Zellen eingeschleust, um in vivo die Synthese therapeutisch wirksamer Genprodukte zu erzielen, z.B. um im Falle eines genetischen Defekts das fehlende Gen zu ersetzen. Die "klassische" Gentherapie beruht auf dem Prinzip, durch eine einmalige Behandlung eine dauernde Heilung zu erzielen. Daneben besteht jedoch Bedarf an Behandlungsmethoden, bei denen die therapeutisch wirksame DNA (oder auch mRNA) wie ein Medikament ("Gentherapeutikum") je nach Bedarf einmalig oder wiederholt verabreicht wird. Beispiele für genetisch bedingte Erkrankungen, bei denen die Gentherapie einen erfolgversprechenden Ansatz darstellt, sind Hämophilie, beta-Thalassämie und "Severe Combined Immune Deficiency"(SCID), ein Syndrom, das durch einen genetisch bedingten Mangel des Enzyms Adenosindeaminase hervorgerufen wird. Anwendungsmöglichkeiten bestehen weiters bei der Immunregulation, wobei durch Verabreichung funktioneller Nukleinsäure, die für ein sekretiertes Protein-Antigen oder für ein nichtsezerniertes Protein-Antigen kodiert, mittels einer Impfung eine humorale oder intrazelluläre Immunität erzielt wird. Weitere Beispiele für genetische Defekte, bei denen eine Verabreichung von Nukleinsäure, die für das defekte Gen kodiert, z.B. in individuell auf den Bedarf abgestimmter Form verabreicht werden kann, sind Muskeldystrophie (Dystrophin-Gen), Cystische Fibrose ("Cystic fibrosis transmembrane conductance regulator gene"), Hypercholesterolämie (LDL-Rezeptor-Gen). Gentherapeutische Behandlungsmethoden sind weiters potentiell dann von Bedeutung, wenn Hormone, Wachstumsfaktoren oder cytotoxisch oder immunmodulierend wirkende Proteine im Organismus synthetisiert werden sollen.

Die Gentherapie stellt auch einen erfolgversprechenden Ansatz für die Behandlung von Krebs dar, wobei sog. "Krebsvaccine" verabreicht werden. Um die Immunogenizität von Tumorzellen zu erhöhen, werden diese verändert, um sie entweder stärker antigenisch zu machen, oder um sie zu veranlassen, bestimmte immunmodulierende Substanzen zu erzeugen, z.B. Zytokine, die dann eine Immunantwort auslösen. Um dies zu bewirken, werden die Zellen mit DNA transfiziert, die für ein Zytokin, z.B. IL-2, IL-4, IFN-gamma, TNF-α, kodiert. Bisher wurde Gentransfer in autologe Tumorzellen hauptsächlich mittels retroviraler Vektoren durchgeführt.

Die bisher am weitesten fortgeschrittenen Technologien für die Anwendung von Nukleinsäuren im Rahmen der Gentherapie benutzen retrovirale Systeme für den Transfer von Genen in die Zelle (Wilson et al., 1990, Kasid et al., 1990). Die Verwendung von Retroviren ist jedoch problematisch, weil sie, zumindest zu einem geringen Prozentsatz, die Gefahr von Nebenwirkungen wie Infektion mit dem Virus (durch Rekombination mit endogenen Viren oder Kontamination mit Helferviren und mögliche anschließende Mutation zur pathogenen Form) oder Entstehung von Krebs in sich birgt. Außerdem ist die stabile Transformation der somatischen Zellen des Patienten, wie sie mit Hilfe von Retroviren erzielt wird, nicht in jedem Fall wünschenswert, weil dadurch die Behandlung, z.B. bei Auftreten von Nebeneffekten, nur mehr schwer rückgängig gemacht werden kann. Außerdem ist es bei dieser Therapieform schwierig, einen genügend hohen Titer zu erhalten, um genügend Zellen zu infizieren.

Nukleinsäuren als therapeutisch wirksame Substanzen kommen außerdem zur Anwendung, um bestimmte Zellfunktionen zu inhibieren, z.B. haben sich Antisense RNAs und -DNAs als wirksame Mittel für die selektive Inhibierung bestimmter Gensequenzen erwiesen. Ihre Wirkungsweise ermöglicht ihre Anwendung als Therapeutika zur Blockierung der Expression bestimmter Gene (wie deregulierter Onkogene oder viraler Gene) in vivo. Es wurde bereits gezeigt, daß kurze Antisense-Oligonukleotide in Zellen importiert werden und dort ihre inhibierende Wirkung ausüben können (Zamecnik et al., 1986), wenngleich ihre intrazelluläre Konzentration, u.a. wegen ihrer beschränkten Aufnahme durch die Zellmembran auf Grund der starken negativen Ladung der Nukleinsäuren, gering ist.

Ein weiterer Ansatz zur selektiven Inhibierung von Genen besteht in der Anwendung von Ribozymen. Auch hier besteht das Bedürfnis, eine möglichst hohe Konzentration von aktiven Ribozymen in der Zelle zu gewährleisten, wofür der Transport in die Zelle einer der limitierenden Faktoren ist.

Die Anwendung der Gentherapie zur Erzielung einer intrazellulären Immunität umfaßt die Transduktion von Genen, die eine Schutzwirkung gegen Viren haben (sog. "protective genes"), z.B. transdominante Mutationen von Genen, die für virale Proteine kodieren, oder DNA-Moleküle, die für sog. "RNA decoys" kodieren.

Es besteht daher Bedarf an Methoden, um die Expression von DNA in der Zelle zu ermöglichen.

Es wurden bereits mehrere Lösungen vorgeschlagen, den Transport von Nukleinsäuren in lebende Zellen, der bei deren therapeutischer Anwendung einen der limitierenden Faktoren darstellt, zu verbessern.

Für die Gentransformation von Säugetierzellen in vitro sind verschiedene Techniken bekannt, deren Anwendbarkeit in vivo jedoch beschränkt ist (dazu zählen das Einbringen von DNA mittels Liposomen, Elektroporation, Mikroinjektion, Zellfusion, DEAE-Dextran oder die Calciumphosphat-Präzipitationsmethode).

In jüngster Zeit wurden rekombinante virale Vektoren entwickelt, die den Transfer von Genen bewerkstelligen, indem sie sich der effizienten Eintrittsmechanismen ihrer Ausgangsviren bedienen. Diese Strategie wurde bei der Konstruktion rekombinanter retroviraler und adenoviraler Vektoren angewendet, um einen hoch wirksamen Gentransfer *in vitro* und *in vivo* zu erzielen (Berkner, 1988). Bei all ihrer Wirksamkeit sind diese Vektoren Beschränkungen, und zwar hinsichtlich der Größe und Konstruktion der zu transferierenden DNA, unterworfen. Außerdem bringen diese Mittel Sicherheitsrisken aufgrund des Co-Transfers von lebensfähigen viralen Gen-Elementen des Ursprungsvirus mit sich.

Um diese Beschränkungen zu umgehen, wurden alternative Strategien für den Gentransfer entwickelt, denen Mechanismen zugrundeliegen, deren sich die Zelle für den Transport von Makromolekülen bedient. Ein Beispiel dafür ist der Import von Genen in die Zelle über den äußerst leistungsfähigen Weg der Rezeptor-vermittelten Endozytose (Wu und Wu, 1987, Wagner et al., 1990, und EP-A1 0388 758). Dieser Ansatz bedient sich bifunktioneller molekularer Konjugate, die eine DNA-Bindungsdomäne und eine Domäne mit Spezifität für einen Zelloberflächen-Rezeptor aufweisen (Wu und Wu, 1987, Wagner et al., 1990). Wenn die Erkennungsdomäne vom Zelloberflächen-Rezeptor erkannt wird, wird das Konjugat über den Weg der Rezeptor-vermittelten Endozytose internalisiert, wobei die an das Konjugat gebundene DNA mittransportiert wird. Mit Hilfe dieser Methode konnten Gentransfer-Raten erzielt werden, die den herkömmlichen Methoden zumindest ebenbürtig waren (Zenke et al., 1990).

Es konnte gezeigt werden, daß mittels dieses Systems in die Zelle.transportierte DNA exprimiert wird und daß im Falle der Verwendung inhibierend wirkender Nukleinsäure die inhibierende Wirkung durch das Transportsystem nicht beeinträchtigt wird.

Die PCT-Anmeldung WO91/17773 bezieht sich auf ein System für den Transport von Nukleinsäuren mit spezifischer Wirkung für T-Zellen. Dieses System macht Gebrauch von Zelloberflächenproteinen der T-Zell-Abstammungslinie, z.B. CD4, dem vom HIV-Virus benutzten Rezeptor. Die zu importierende Nukleinsäure wird mit einem Protein-Polykation-Konjugat komplexiert, dessen Proteinanteil ein Protein mit der Fähigkeit ist, an das T-Zell-Oberflächenprotein, z.B. CD4, zu binden und Zellen, die dieses Oberflächenprotein exprimieren, mit den erhaltenen Protein-Polykation/Nukleinsäure-Komplexen in Berührung gebracht. Es konnte nachgewiesen werden, daß mit Hilfe dieses Systems in die Zelle transportierte DNA in der Zelle exprimiert wird.

Diesen beiden Erfindungen gemeinsam ist das Merkmal, daß sie spezifische Zellfunktionen benutzen, um den Import der Nukleinsäure in die Zelle zu ermöglichen oder zu erleichtern.
In beiden Fällen laufen die Aufnahmemechanismen unter Beteiligung von Faktoren ab, die im Rahmen der vorliegenden Erfindung als "Internalisierungsfaktoren" bezeichnet werden. Darunter sind Faktoren zu verstehen, die, im engeren oder weiteren Sinn zelltypspezifisch, gegebenenfalls unter Mitwirkung weiterer Faktoren (z.B. Zelloberflächenproteinen), an die Zelloberfläche binden und internalisiert werden. (Im Falle der beiden oben erwähnten Erfindungen ist der Internalisierungsfaktor Transferrin bzw. ein an ein T-Zell-Oberflächenantigen bindendes Protein, z.B. ein anti-CD4-Antikörper.) Der Internalisierungsfaktor ist mit einer Substanz polykationischen Charakters konjugiert, die aufgrund ihrer Affinität zu Nukleinsäuren eine Verbindung zwischen dem Internalisierungsfaktor und der Nukleinsäure herstellt. (Derartige Substanzen werden im folgenden als "Nukleinsäure-affine Substanzen" oder, im Zusammenhang mit DNA "DNA-Bindungsdomäne" bezeichnet. Wenn eine solche Substanz als Bestandteil des Konjugats zwischen der Nukleinsäure und einem Internalisierungsfaktor eine Verbindung herstellt, wird sie im folgenden als "Verbindungsfaktor" bezeichnet.)

Im Zuge dieser beiden Erfindungen war festgestellt worden, daß ein Optimum für die Aufnahme von Nukleinsäure in die Zelle erzielt werden kann, wenn das Verhältnis Konjugat:Nukleinsäure so gewählt wurde, daß die Internalisierungsfaktor-Polykation/Nukleinsäure-Komplexe weitgehend elektroneutral waren. Ausgehend von dieser Beobachtung wurden die Methoden verbessert, die für den Import von Nukleinsäuren in höhere eukaryotische Zellen Internalisierungsfaktor-Verbindungsfaktor/Nukleinsäure-Komplexe benutzen.

Von Wagner et al., 1991a, wurde eine Methode beschrieben, mit Hilfe derer die Effizienz von Systemen, bei denen die Aufnahme von Nukleinsäuren mittels Internalisierungsfaktoren erfolgt, verbessert werden kann. Die Menge der in die Zelle aufgenommenen Nukleinsäure-Menge wird nicht verringert, wenn ein Teil der Transferrin-Polykation-Konjugate durch nicht-kovalent gebundenes Polykation ersetzt wird; in bestimmten Fällen kann sogar eine beträchtliche Steigerung der DNA-Aufnahme erreicht werden. Untersuchungen zum molekularen Zustand von Transferrin-Polykation-Plasmid-DNA-Komplexen, die mit als optimal ermittelten DNA/Konjugat-Verhältnissen hergestellt worden waren, hatten ergeben, daß die Plasmid-DNA in Gegenwart der Konjugate zu toroiden Strukturen (ähnlich "doughnuts") mit einem Durchmesser von ca. 80 - 100 nm verdichtet vorliegt.)

Die mit an T-Zellen bindenden Proteinen als Internalisierungsfaktor durchgeführten Versuche brachten ähnliche Ergebnisse.

Der Zusatz freier Nukleinsäure-affiner Substanz(en) bewirkt auch dann eine Steigerung der Effizienz des Importsystems, wenn eine andere Nukleinsäure-affine Substanz als Verbindungsfaktor verwendet wird.

Die von Wagner et al., 1991a, beschriebenen Komplexe, die mittels Internalisierungsfaktor über Endozytose in höhere eukaryotische Zellen aufgenommen werden, enthalten Nukleinsäure, komplexiert mit einem Internalisierungsfaktor-Verbindungsfaktor-Konjugat. Zusätzlich enthalten die Komplexe eine oder mehrere Nukleinsäure-affine Substanzen, die gegebenenfalls identisch sind mit dem Verbindungsfaktor, in nicht-kovalent gebundener Form derart, daß die durch das Konjugat erzielte Internalisierung und/oder Expression der Nukleinsäure gesteigert wird, was in erster Linie auf eine kondensierende Wirkung, möglicherweise aber auch auf andere Mechanismen zurückzuführen sein dürfte.

Wenn auch mit Hilfe dieser Methode die Expressionsraten der importierten Nukleinsäure gesteigert werden konnten, so ist sie dennoch Beschränkungen unterworfen. Die Brauchbarkeit dieses Systems in einem gegebenen Zusammenhang wird nicht ausschließlich durch das Vorhandensein des jeweils für das System relevanten Zelloberflächenrezeptors bestimmt; die Beschränkungen, denen die Anwendung dieses Systems unterliegt, sind vermutlich eine Folge davon, daß die in Endosomen internalisierten Konjugat-DNA-Komplexe in die Lysosomen gelangen, wo sie enzymatisch abgebaut werden. Um den Anteil von Nukleinsäure, der in den Zellkern gelangt und dort seiner Bestimmung gemäß exprimiert wird, zu steigern, wurde in Experimenten, die der vorliegenden Erfindung vorangegangen sind, versucht, die Transfektion der Zellen in Gegenwart von Substanzen durchzuführen, die die enzymatische Aktivität in den Lysosomen inhibieren, sog. lysosomatroper Substanzen. Mit Hilfe dieser Maßnahme konnte eine verstärkte Expression der importierten DNA erzielt werden; die erzielten Reaktionen waren jedoch in Abhängigkeit der verwendeten Substanz stark unterschiedlich - ausgewählte lysosomatrope Substanzen bewirkten eine Verstärkung des Gentransfers, während andere diesen sogar inhibierten. So wurde z.B. festgestellt, daß der effiziente Import von DNA von der Anwesenheit der schwachen Base Chloroquin abhängt (Zenke et al., 1990, Cotten et al., 1990). Dieser durch Chloroquin erzielte Effekt dürfte jedoch nicht bzw. nicht ausschließlich darauf zurückzuführen sein, daß Chloroquin den pH-Wert in den Lysosomen erhöht; anhand verschiedener Experimente wurde festgestellt, daß andere Substanzen, die ebenso wie Chloroquin die Fähigkeit zur Modulation des pH-Werts aufweisen, wie Monensin, Ammoniumchlorid oder Methylamin, Chloroquin nicht ersetzen konnten, in verschiedenen Experimenten zeigten einige dieser Substanzen sogar eine inhibierende Wirkung. Weiters wurde festgestellt, daß verschiedene Zielzellen unterschiedliche Reaktionen auf dieselbe lysosomatrop wirkende Substanz zeigen.

Da der Gentransfer auf physiologischem Weg, wie ihn die Rezeptor-vermittelte Endozytose mittels Nukleinsäure-Komplexen darstellt, große Vorteile aufweist (nichttoxischer Mechanismus des Durchtritts durch die Zellmembran; Möglichkeit der Verabreichung biologisch aktiver Nukleinsäuren, wie Gene, Genabschnitte oder Zellfunktionen spezifisch inhibierende Nukleinsäuren, auf repetitiver oder kontinuierlicher Basis; Möglichkeit des zellspezifischen Targeting; Herstellbarkeit der Konjugate in großen Mengen), besteht das Bedürfnis, dieses System effizienter zu machen.

Der vorliegenden Erfindung lag die Aufgabe zugrunde, den Import von Nukleinsäure in höhere eukaryotische Zellen zu verbessern. (Unter "Import" bzw. "Transfer" wird im Rahmen der vorliegenden Erfindung außer dem Eintritt der Nukleinsäure-Komplexe in die Zelle durch die Zellmembran auch die Lokalisierung der Komplexe bzw. der daraus freigesetzten Nukleinsäure innerhalb der Zelle bis zum Erreichen eines für deren Expression geeigneten Ortes verstanden.) Die höheren eukaryotischen Zellen sind dem Fachmann geläufig; Hefen werden nicht dazugezählt (Watson et al., 1987).

Eine Vielzahl von Viren bewerkstelligen ihr Eindringen in den eukaryotischen Wirt über Mechanismen, die im Prinzip denen der Rezeptor-vermittelten Endozytose entsprechen. Eine Virusinfektion auf Basis dieses Mechanismus beginnt im allgemeinen mit der Bindung von Viruspartikeln an Rezeptoren auf der Zellmembran. Im Anschluß daran erfolgt die Internalisierung des Virus in die Zelle. Dieser Internalisierungsvorgang folgt einer gemeinsamen Route, entsprechend dem Eintritt von physiologischen Liganden oder von Makromolekülen in die Zelle: Die Rezeptoren auf der Zelloberfläche ordnen sich zunächst in Gruppen an, um ein sog. "coated pit" zu bilden, daraufhin stülpt sich die Membran nach innen und bildet ein von einer Hülle umgebenes Vesikel. Nachdem sich dieses Vesikel seiner Clathrin-Hülle entledigt hat, erfolgt in seinem Inneren mit Hilfe einer in der Membran lokalisierten Protonenpumpe eine Ansäuerung. Dadurch wird die Freisetzung des Virus aus dem Endosom hervorgerufen. In Abhängigkeit davon, ob das Virus eine Lipidhülle aufweist oder nicht, wurden zwei Arten der Freisetzung des Virus aus dem Endosom in Betracht gezogen: Im Fall von sog. "nackten" Viren (z.B. Adenovirus, Poliovirus, Rhinovirus) wurde vorgeschlagen, daß der niedrige pH-Wert Konformationsänderungen in Virusproteinen hervorruft. Dadurch werden hydrophobe Domänen freigelegt, die bei physiologischem pH-Wert nicht zugänglich sind. Diese Domänen erlangen dadurch die Fähigkeit, mit der Endosomenmembran in Wechselwirkung zu treten und damit die Freisetzung des Virusgenoms aus dem Endosom ins Zytoplasma zu bewirken. Von Viren mit Lipidhülle (z.B. Vesicular Stomatitis Virus, Semliki Forest Virus, Influenza Virus) wird vermutet, daß der niedrige pH-Wert die Struktur oder Konformation einiger Virusproteine modifiziert, wodurch die Fusion der Virusmembran mit der Endosomenmembran gefördert wird. Viren, die mit Hilfe dieses Mechanismus in die Zelle eindringen, weisen bestimmte molekulare Besonderheiten auf, die es ihnen ermöglichen, die Endosomenmembran aufzubrechen, um sich Eintritt ins Zytoplasma zu verschaffen.

Andere Viren, z.B. die eine Hülle aufweisenden Viren Sendai, HIV und einige Moloney Leukämie-Virusstämme, oder die keine Hülle aufweisenden Viren SV40 und Polyoma benötigen für ihr Eindringen in die Zelle kein niedriges pH-Milieu, können entweder direkt an der Zelloberfläche eine Fusion mit der Membran herbeiführen (Sendai Virus, möglicherweise HIV) oder aber sie vermögen Mechanismen auszulösen, um die Zellmembran aufzubrechen oder sie zu passieren. Es wird angenommen, daß auch die pH-Wert-unabhängigen Viren den Endozytose-Weg benutzen können (McClure et al., 1990).

Bei der Lösung der gestellten Aufgabe wurde von der Überlegung ausgegangen, den von bestimmten Viren bei deren Eindringen in eukaryotische Zellen benutzten Mechanismus auszunutzen, um den Import von Nukleinsäure-Komplexen in die Zelle zu verbessern und dadurch die Expression zu steigern.

Es wurde bereits versucht, Proteine gemeinsam mit Viren in die Zelle zu internalisieren (Otero und Carrasco, 1987). Dabei wurde festgestellt, daß die Permeabilisierung der Zelle durch das Virus für das Einschleusen von Makromolekülen ausgenutzt wird. Bei den dabei ablaufenden Vorgängen dürfte es sich um Flüssigphasen-Mechnismen handeln.

Anhand des Epidermalen Wachstumsfaktors (Epidermal Growth Factor, EGF), konjugiert an ein Toxin, wurde festgestellt, daß dieser natürliche Ligand, der nach Bindung an seinen Rezeptor durch Endozytose in die Zelle aufgenommen wird, gemeinsam mit dem Adenovirus, das ebenfalls über Rezeptor-vermittelte Endozytose in die Zelle aufgenommen wird, in demselben Endosom landet und aus diesem, ebenfalls gemeinsam mit dem Virus, ins Zytosol freigesetzt wird (FitzGerald et al., 1983).

Es wurde überraschend festgestellt, daß die Gegenwart bestimmter Agenzien (z.B. Viren, Viruskomponenten oder andere Wirkstoffe), die hinsichtlich ihres Eintrittsmechanismus in eukaryotische Zellen die Eigenschaften bestimmter Viren aufweisen, eine erhebliche Steigerung der Expressionsrate der als Teil eines Komplexes in die Zelle importierten Nukleinsäure bewirkt. Dieser Befund war vor allem deshalb überraschend, weil die in die Zelle aufgenommenen Nukleinsäure-Komplexe sehr groß sind.

Die vorliegende Erfindung betrifft somit eine Zusammensetzung für die Transfektion von höheren eukaryotischen Zellen mit einem Komplex aus Nukleinsäure und Nukleinsäure-affiner Substanz, die gegebenenfalls mit einem Internalisierungsfaktor für diese Zellen gekoppelt ist. Die Zusammensetzung ist dadurch gekennzeichnet, daß sie ein Mittel enthält, das die Fähigkeit hat, per se oder als Bestandteil des Nukleinsäure-Komplexes, in die zu transfizierenden Zellen aufgenommen zu werden und den Inhalt der Endosomomen, in denen der Komplex nach Eintritt in die Zelle lokalisiert ist, ins Zytoplasma freizusetzen.

Dieses Mittel wird im folgenden als "endosomolytisches Mittel" bezeichnet.

Die Fähigkeit der endosomolytischen Mittel, in die zu transfizierenden Zellen aufgenommen zu werden und den Inhalt der Endosomomen, in denen sie nach Eintritt in die Zelle lokalisiert sind, ins Zytoplasma freizusetzen, wird im folgenden als "Aufnahmefunktion" bezeichnet. Diese Aufnahmefunktion setzt sich zusammen aus der Fähigkeit, aktiv, über Rezeptor-abhängige Endozytosemechanismen, oder passiv, über die Flüssigphase oder als Bestandteil des Nukleinsäure-Komplexes, in die Zelle internalisiert zu werden, und der Fähigkeit, Endosomen aufzubrechen, die im allgemeinen als "endosomolytische Aktivität" oder "Endosomolyse" bezeichnet wird.

In einer Ausführungsform der Erfindung ist das endosomolytische Mittel ein Virus. In einer anderen Ausführungsform ist das endosomolytische Mittel eine Viruskomponente. Das Virus bzw. die Viruskomponente, die in diesen Ausführungsformen der Erfindung eingesetzt werden, werden im folgenden als "freie(s) Virus(komponente)" bezeichnet.

Im Rahmen der vorliegenden Erfindung wurde die Wirkung einer zunehmenden Dosis an Adenoviren auf die Gentransfer-Kapazität einer konstanten Menge Transferrin-Polylysin-Konjugat in HeLa-Zellen untersucht, wobei als Reportergen das Luciferase-Gen verwendet wurde. Die durch das Adenovirus bewirkte Verstärkung des Genstransfers erreichte ein Maximum bei 1x10⁴ Viruspartikeln pro Zelle, einer Zahl, die der ungefähren Anzahl von Adenovirus-Rezeptoren pro HeLa-Zelle entspricht. Die bis zu 2000fache Verstärkung der Luciferase-Expression gegenüber der Expression, die mit den Transferrin-Polylysin-Konjugaten allein erzielt wird, entsprach der höheren Virus-Dosis. In einer weiteren Versuchsreihe wurde die Kapazität limitierender Mengen von Konjugat-DNA-Komplexen in Gegenwart einer konstanten Adenovirus-Dosis untersucht. Es wurde festgestellt, daß die Aufnahme von Adenoviren in die Zellen den durch die Transferrin-Polylysin vermittelten Gentransfer über einen weiten Bereich von DNA-Dosierungen verstärkte. Die maximale Stärke der Genexpression, die durch die Konjugat-DNA-Komplexe erzielt wurde, entsprach der Stärke, die mit 100mal weniger DNA erzielt wurde, wenn Adenoviren zur Verstärkung der Transfektionseffizienz verwendet wurden.

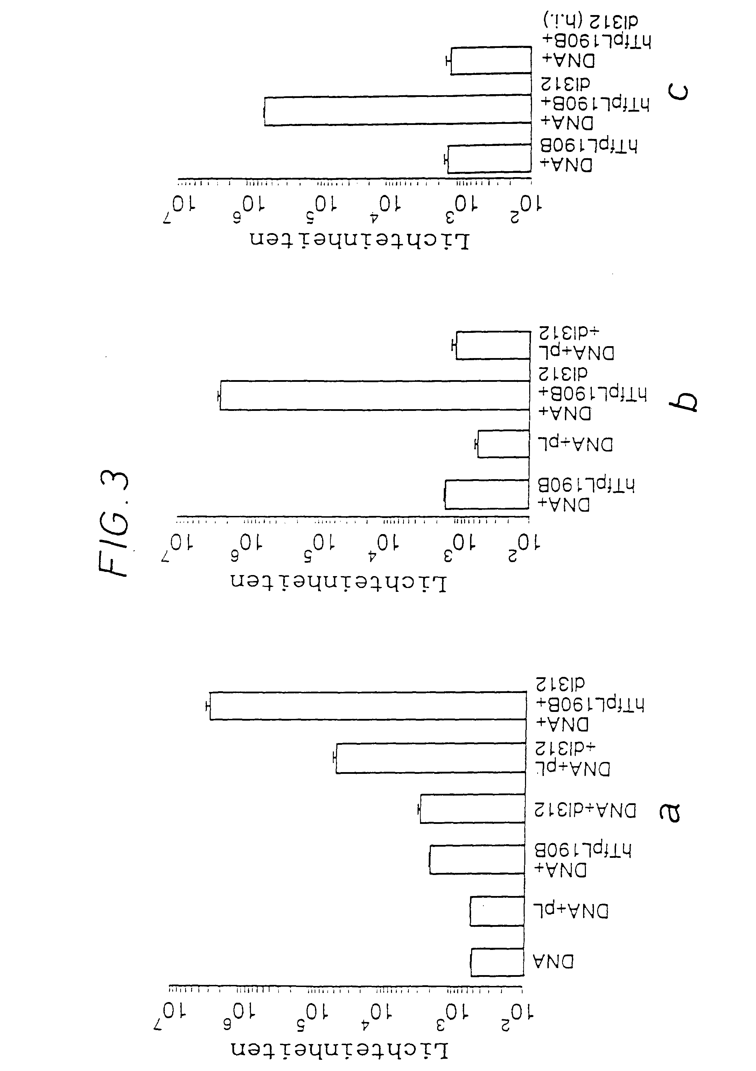

Die Wirkung der von Adenovirus auf den Gentransfer wurde sowohl für nicht-komplexierte als auch für DNA untersucht, die mit Polylysin oder mit Transferrin-Polylysin-Konjugaten komplexiert worden waren (Fig. 3A). Gemäß dieser Analyse verstärkte die Anwesenheit von Adenovirus während der Transfektion den Transfer von nackter, nicht-komplexierter DNA nur geringfügig. In starkem Gegensatz dazu wurde der Transfer von DNA, komplexiert mit Polylysin oder mit Transferrin-Polylysin-Konjugaten, durch Adenoviruszugabe verstärkt, wobei dieser Effekt bei den Transferrin-Polylysin-Konjugaten wesentlich stärker auftrat. Da der Polykationenanteil im Konjugat nicht nur dazu dient, die Verbindung zwischen DNA und Transferrin herzustellen, sondern auch deutliche strukturelle Änderungen in der Struktur der DNA bewirkt (Kompaktierung zu toroiden Strukturen; Wagner et al., 1991a), konnte aufgrund der Experimente zunächst keine Unterscheidung getroffen werden, ob der beobachtete Effekt auf einen verstärkten Transport der durch das Polykation kondensierten DNA in der flüssigen Phase oder auf eine durch das Virus bewirkte Steigerung des Transports von Rezeptor-gebundenem Konjugat-DNA-Komplex zurückzuführen ist. Um zwischen diesen beiden Möglichkeiten zu unterscheiden, wurden sequentielle Bindungsversuche durchgeführt (Fig. 3B). Die Bindung von Transferrin-Polylysin-DNA oder Polylysin-DNA-Komplexen bei niedriger Temperatur ohne Internalisierung ermöglicht es, vor der Behandlung mit dem Adenovirus überschüssigen Komplex in der flüssigen Phase zu entfernen (FitzGerald et al., 1983). Wenn so vorgegangen wurde, wurde der Transport der Rezeptorgebundenen Transferrin-Polylysin-DNA-Komplexe durch Zusatz von Adenovirus-Partikeln signifikant erhöht, was für die Polylysin-DNA-Komplexe nicht zutraf. Es ist daher der Eintritt der DNA in die Zelle über Rezeptorvermittelte Endozytose, der spezifisch verstärkt wird.

Weiters wurde untersucht, welche spezifische Funktion des Adenovirus es ist, die eine Verstärkung des Rezeptor-vermittelten Gentransfers bewirkt (Fig. 3C). Milde Hitzebehandlung der Virus-Partikel ändert nichts an ihrer Fähigkeit, an die Zellmembran der Zielzellen zu binden, beeinträchtigt jedoch ihre Fähigkeit, nach ihrem Eintritt in die Zelle die Endosomen aufzubrechen (Defer et al., 1990). Aufgrund dieser Gegebenheit konnten die unterschiedlichen Effekte der Virusbindung und des Eintritts des Virus in die Zelle getrennt untersucht werden. Bei den im Rahmen der vorliegenden Erfindung durchgeführten Versuche wurde festgestellt, daß eine Hitzeinaktivierung die Fähigkeit der Viren, den über Rezeptor-vermittelte Endozytose ablaufenden Gentransfer zu verstärken, komplett beseitigte. Daraus wurde die Schlußfolgerung gezogen, daß die Verstärkung des Gentransfers mittels Transferrin-Polylysin-Konjugaten spezifisch auf die Fähigkeit des Adenovirus, als Teil ihrer Aufnahmefunktionen Endosomen aufbrechen zu können, zurückzuführen ist. Die Tatsache, daß ein replikationsdefekter Virusstamm eine Verstärkung der Genexpression herbeiführen konnte, bestätigt die Annahme, daß dieses Phänomen nicht auf die Replikationsfunktion, sondern auf die Aufnahmefunktion des Virions zurückzuführen ist.

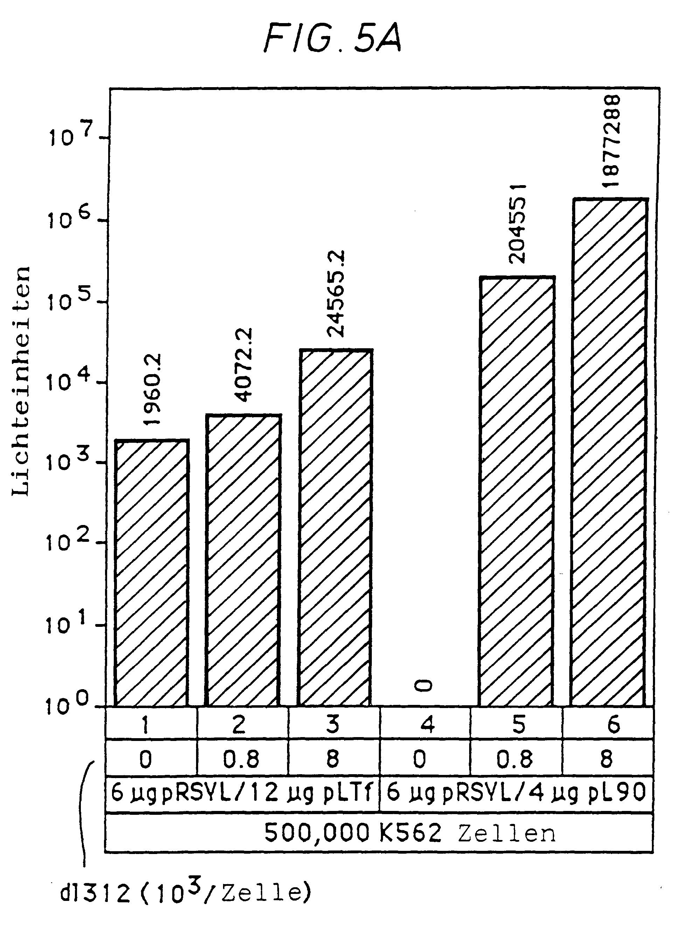

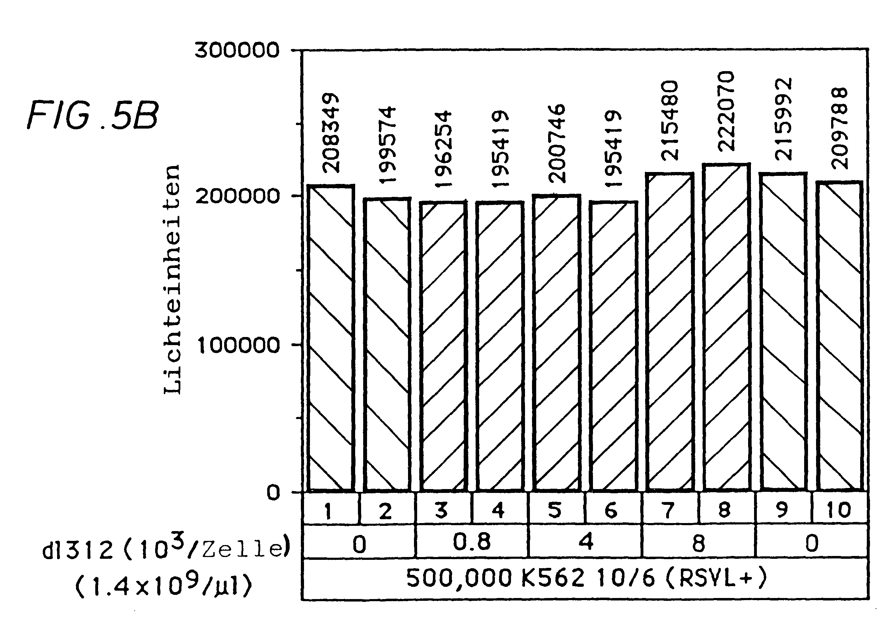

Um die Möglichkeit auszuschließen, daß die Verstärkung der Genexpression auf eine eventuelle Transaktivierung des importierten Gens durch das Virus zurückzuführen ist, wurden Versuche mit einer Zellinie durchgeführt, die das RSV-LTR-Luciferase-Gen konstitutiv exprimiert:
Adenoviren zeigten in dieser Zellinie keinen Effekt, während sie in der parentalen Zellinie, in die das Gen mittels Transferrin-Polylysin-Konjugaten eingeführt worden war, eine deutliche Verstärkung der Genexpression hervorriefen. Dieser Befund machte deutlich, daß das Adenovirus auf Ereignisse Einfluß nimmt, die vor der Transkription stattfinden, daß ihre verstärkende Wirkung auf den Genimport somit auf Ebene des Genimports und nicht auf Ebene der Genexpression wirkt (Fig. 5).

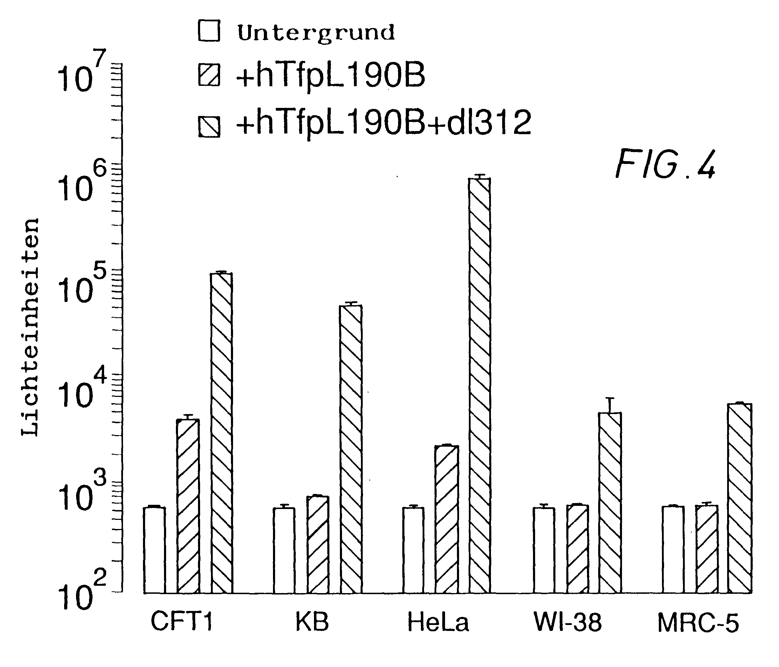

Weiters wurde im Rahmen der vorliegenden Erfindung untersucht, welche Auswirkung Adenoviren auf den Gentransfer mittels Transferrin-Polylysin-Konjugaten in ausgewählten Zellinien zeigen. Es wurde festgestellt, daß das Vorhandensein von Transferrin-Rezeptoren auf den Zielzellen notwendig, aber nicht in allen Fällen ausreichend ist, um den Gentransfer mittels Transferrin-Polylysin-Konjugaten zu ermöglichen. Zellspezifische Faktoren, die im Zusammenhang mit dem Schicksal der in den Endosomen internalisierten Konjugat-DNA-Komplexe stehen, scheinen eine wesentliche Rolle für das Ausmaß des Gentransfers, der über diese Route erzielt werden kann, zu spielen. Im Hinblick darauf wurden einige ausgewählte Zellinien sowohl auf Gentransfer mittels Transferrin-Polylysin-Konjugaten als auch auf eine Verstärkung des Gentransfers durch Adenoviren untersucht (Fig. 4). Zellen einer zystischen Fibrose-Zellinie (CFT1) zeigten mäßige Luciferase-Genexpression nach Behandlung mit Transferrin-Polylysin-DNA-Komplexen allein. Das Ausmaß der Expression wurde durch Behandlung mit dem Adenovirus dl312 signifikant erhöht. In deutlichem Gegensatz dazu zeigten KB-Zellen, die mit den Transferrin-Polylysin-DNA-Komplexen behandelt wurden, trotz des Vorhandenseins von Transferrin-Rezeptoren eine Luciferase-Genexpression, die kaum über dem Background-Niveau lag. Eine Behandlung mit Adenovirus dl312 bewirkte jedoch bei diesen Zellen deutlich nachweisbare Luciferase-Aktivitäten. Ähnlich wirkte sich die Behandlung mit Adenoviren auf HeLa-Zellen aus, wobei dieser Effekt bei diesen Zellen jedoch noch wesentlich stärker war. Da HeLa-Zellen und KB-Zellen ungefähr dieselbe Anzahl von Rezeptoren für das Adenovirus besitzen, könnte der Unterschied in der Verstärkung des Gentransfers die Anzahl von Transferrin-Rezeptoren, die für einen Zelltyp charakteristisch sind, widerspiegeln. In deutlichem Unterschied zu diesen Ergebnissen zeigten jedoch die Zellinien WI-38 und MRC-5, von denen bekannt ist, daß in ihnen die Infektion mit Adenovirus nur schwach vor sich geht (Precious und Russell, 1985), mit dl312 nur eine sehr geringe Verstärkung der durch die Konjugat-DNA-Komplexe allein erzielten Genexpression. Die Behandlung mit Adenovirus dürfte daher den Gentransfer mittels Konjugat-DNA-Komplexen in denjenigen Fällen verstärken, in denen der Gentransfer über Rezeptor-vermittelte Endozytose möglich ist, wie im Fall der CFT1-Zellen, sowie in einigen Fällen, in denen der Gentransfer über diese Route ineffizient abläuft, wie bei HeLa und KB-Zellen. Daß das Ausmaß der Verstärkung zwischen verschiedenen Zielzellen deutlich variiert, könnte darin seine Ursache haben, daß dieser Effekt sowohl eine Funktion der Anzahl der Virus-Rezeptoren, z.B. der Adenovirus-Rezeptoren, eines bestimmten Zelltyps als auch der Anzahl der Transferrin-Rezeptoren ist.

Im Falle der Verwendung von freiem Virus ist die Nukleinsäure-affine Substanz vorzugsweise ein organisches Polykation, die vorzugsweise mit einem Internalisierungsfaktor konjugiert ist. Es wurde jedoch im Rahmen der vorliegenden Erfindung festgestellt, daß unter bestimmten Umständen DNA, die nur mit Nukleinsäure-affiner Substanz komplexiert ist, also ohne Internalisierungsfaktor, in Gegenwart von freiem Virus in die Zelle eingebracht werden kann. Weiters wurde gefunden, daß bei einigen Zellinien die Aufnahme der Komplexe, bestehend aus Nukleinsäure und Nukleinsäure-affiner Substanz, über die Flüssigphase erfolgen kann, wenn die Konzentration der Komplexe entsprechend hoch ist. Aus den Versuchen, die im Rahmen der vorliegenden und der vorangegengenen Erfindungen durchgeführt wurden, wurde abgeleitet, daß ein wesentliches Element für die Aufnahmefähigkeit der Nukleinsäure-Komplexe deren Kompaktheit ist, die auf die Kondensierung der Nukleinsäure durch die Nukleinsäure-affine Substanz zurückzuführen ist. Wenn die Nukleinsäure-affine Substanz neben ihrer Fähigkeit, die weitgehende Elektroneutralität des Komplexes herzustellen und die Nukleinsäure zu einer kompakten Struktur zu verdichten, eine ausreichende Fähigkeit zur Bindung an die Zelloberfläche besitzt, um gemeinsam mit dem Virus in die Zelle einzudringen, kann darauf verzichtet werden, die Aufnahmekapazität dadurch zu erhöhen, daß ein Internalisierungsfaktor kovalent an die Nukleinsäure-affine Substanz gebunden wird, um den Komplex über Rezeptor-vermittelte Endozytose in die Zelle zu befördern. Manche Zellen weisen eine relativ hohe Affinität zu bestimmten Nukleinsäure-affinen Substanzen auf, sodaß die Konjugate aus Nukleinsäure und Verbindungsfaktor in die Zelle aufgenommen werden, ohne daß die Mitwirkung eines Internalisierungsfaktors erforderlich ist. Dies trifft z.B. auf Hepatozyten zu, von denen im Rahmen der vorliegenden Erfindung festgestellt wurde, daß sie DNA-Polylysin-Komplexe aufnehmen.

In einer bevorzugten Ausführungsform der Erfindung ist das endosomolytische Mittel ein Virus, das an die Nukleinsäure-affine Substanz gebunden ist und das die Fähigkeit hat, in die Zelle als Teil des Konjugat/Nukleinsäure-Komplexes einzudringen und den Inhalt der Endosomen, in denen der Komplex nach dem Eintritt in die Zelle lokalisiert ist, ins Zytoplasma freizusetzen.

In einer weiteren bevorzugten Ausführungsform ist das endosomolytische Mittel eine Viruskomponente, die an eine Nukleinsäure-affine Substanz gebunden ist und die die Fähigkeit hat, in die Zelle als Teil des Konjugat/Nukleinsäure-Komplexes einzudringen und den Inhalt der Endosomen, in denen der Komplex nach dem Eintritt in die Zelle lokalisiert ist, ins Zytoplasma freizusetzen.

Viren oder Viruskomponenten, die an eine Nukleinsäure-Bindungsdomäne gebunden sind, werden im folgenden ungeachtet der Art der Bindung als "virale Konjugate" bezeichnet.

Die viralen Konjugate, die ebenfalls Gegenstand der vorliegenden Erfindung sind, enthalten das Virus oder die Viruskomponente als integralen Bestandteil ihres funktionellen Konstrukts und vereinigen die Vorteile von Vektorsystemen auf Basis von Internalisierungsfaktor-Konjugaten mit den Vorteilen, die Viren in das System einbringen.

Darüberhinaus haben die viralen Konjugate gemäß diesen Ausführungsformen der Erfindung den Vorteil, daß sie die grundsätzliche Beschränkung umgehen, die dem bekannten bifunktionellen Konjugatsystem für den Gentransfer über Rezeptor-vermittelte Endozytose inhärent ist, indem sie über einen spezifischen Mechanismus verfügen, der ihre Freisetzung aus dem Zellvesikel-System ermöglicht. Die erfindungsgemäßen viralen Konjugate stellen eine grundsätzliche konzeptuelle Abkehr von den rekombinanten viralen Vektoren dar, indem die zu transportierende Fremd-DNA an der Außenseite des Virions getragen wird. Dadurch können mit Hilfe der erfindungsgemäßen Konjugate auch sehr große Genkonstrukte in die Zelle transportiert werden, wobei hinsichtlich der Sequenz keine Beschränkungen bestehen.

Die Eignung eines Virus, das als freies oder gebundenes Virus(teil) als Viruskomponente im Rahmen der vorliegenden Erfindung eingesetzt werden soll, wird durch seine Aufnahmefunktion definiert. Geeignete Viren sind einerseits solche, die die Fähigkeit besitzen, während der Transfektion der Zellen mit dem Nukleinsäure-Komplex, über Rezeptor-vermittelte Endozytose in die Zelle einzudringen und ihre Freisetzung - und damit die Freisetzung der Nukleinsäure - aus dem Endosom ins Zytoplasma herbeizuführen. Ohne auf diese Theorie festgelegt sein zu wollen, könnte dieser Mechanismus den in die Zelle importierten Nukleinsäure-Komplexen insofern zugute kommen, als diese, sofern sie bei der Internalisierung in dieselben Endosomen gelangen wie die Viren, zusammen mit den Viren aus den Endosomen ins Zytoplasma befördert werden. Wenn die Nukleinsäurekomplexe das Virus in gebundener Form enthalten, profitieren sie von der endosomolytischen Aktivität des Virus und werden aus den Endosomen ins Zytoplasma befördert. Dabei dürfte die Fusion zwischen Endosomen und Lysosomen und damit der in diesen Zellorganellen normalerweise stattfindende enzymatische Abbau vermieden werden.

Beispiele für Viren und höhere eukaryotische Zellen, in die sie eindringen können, sind z.B. Fields und Knipe, 1990, entnehmbar. Die Empfänglichkeit einer gegebenen Zellinie für die Transformation durch ein Virus, das in Form eines freien Virus zur Erleichterung des Eintrittes von Nukleinsäurekomplexen in Zellen eingesetzt wird, hängt vom Vorhandensein und der Anzahl von Oberflächenrezeptoren für das Virus auf der Zielzelle ab. Bezüglich des Zelloberflächenrezeptors für Adenovirus wurden Methoden zur Bestimmung seiner Anzahl auf HeLa- und KB-Zellen von Svensson, 1990, und Defer, 1990, beschrieben.

Zu Viren, die für die erfindungsgemäße Zusammensetzung geeignet sind und deren am Beginn der Infektion ablaufende Aufnahmefunktion über Rezeptor-vermittelte Endozytose erfolgt, zählen einerseits Viren ohne Lipid-Hülle wie Adenovirus, Poliovirus, Rhinovirus, andererseits die Hüllenviren Vesicular Stomatitis Virus, Semliki Forest Virus, Influenza-Virus; geeignet sind außerdem pH-Wert-abhängige Stämme von Moloney-Virus. Zu besonders bevorzugten Viren für die Anwendung der vorliegenden Erfindung zählen Adenovirus, Untergruppe C, Typ 5, Semliki Forest Virus, Vesicular Stomatitis Virus, Poliovirus, Rhinoviren und Moloney Leukämie-Virusstämme.

Die Verwendung von RNA-Viren, die keine reverse Transkriptase haben, für die vorliegende Erfindung hat den Vorteil, daß eine Transfektion in Gegenwart eines solchen Virus nicht die Bildung von viraler DNA in der Zelle zur Folge hat. Im Rahmen der vorliegenden Erfindung wurde gezeigt, daß Rhinovirus HRV2, ein Vertreter der Picornavirus-Gruppe, die Expression eines Reportergens erhöht. Die Leistungsfähigkeit des Rhinovirus wurde sowohl in freier Form als auch in Form von Viruskonjugaten gezeigt.

Im Rahmen der vorliegenden Erfindung werden unter Viren - vorausgesetzt, daß sie in die Zelle aufgenommen werden und den Inhalt der Endosomen, in die sie gelangen, freisetzen - außer den Wildtypen auch Mutanten verstanden, denen aufgrund einer oder mehrerer Mutationen Funktionen des Wildtyps, die von der Aufnahmefunktion verschieden sind, insbesondere die Replikationsfähigkeit, abhanden gekommen sind.

Derartige Mutanten werden durch Mutationen bzw. Deletionen in Virus-Protein-Regionen, die für Replikationsfunktionen verantwortlich und für die Aufnahmefunktion entbehrlich sind und die durch die Verpackungslinie komplementiert werden können, mittels herkömmlicher Mutageneseverfahren hergestellt. Dazu zählen, z.B.im Fall von Adenovirus, ts-Mutanten (temperatursensitive Mutanten), E1A- und E1B-Mutanten, Mutanten, die Mutationen in MLP-getriebenen Genen aufweisen (Berkner, 1988) und Mutanten, die Mutationen in Bereichen bestimmter Kapsidproteine aufweisen. Geeignet sind weiters Virusstämme, die entsprechende natürliche Mutationen aufweisen. Die Replikationsfähigkeit der Viren kann z.B. mit Hilfe literaturbekannter Plaque-Assays untersucht werden, bei denen Zellkulturen mit Suspensionen unterschiedlicher Viruskonzentration beschichtet werden und die Zahl der lysierten Zellen, die an Hand von Plaques sichtbar ist, festgestellt wird (Dulbecco, 1980).

Für die Anwendung im Rahmen der vorliegenden Erfindung geeignet sind außerdem sog. defekte Viren, das sind Viren, denen in einem oder mehreren Genen die für die autonome Virusreplikation erforderliche Funktion fehlt, für die sie Helferviren benötigen. Vertreter dieser Gruppe sind DI-Partikel ("defective interfering particles"), die sich vom infektiösen Standard-Virus ableiten, dieselben Strukturproteine wie das Standardvirus besitzen, Mutationen aufweisen und das Standardvirus als Helfervirus für ihre Replikation benötigen (Huang, 1987; Holland, 1990). Vertreter dieser Gruppe sind außerdem die Satellitenviren (Holland, 1990). Eine andere Gruppe ist die Klasse der Parvoviren, die als "adeno-associated virus" (Berns, 1990) bezeichnet werden. Da die Aufnahmezyklen vieler Viren in die Zelle noch nicht zur Gänze aufgeklärt sind, ist anzunehmen, daß es noch andere Viren gibt, die die endosomolytische Aktivität aufweisen, die für ihre Eignung zur Anwendung in der vorliegenden Erfindung erforderlich ist.

Weiters im Rahmen der vorliegenden Erfindung geeignet sind attenuierte Lebendvaccine (Ginsberg, 1980) oder Impfstämme.

Weiters fallen unter den Begriff Viren im Rahmen der vorliegenden Erfindung inaktivierte Viren, z.B. durch chemische Behandlung, wie Formaldehydbehandlung, durch UV-Bestrahlung, durch chemische Behandlung, kombiniert mit UV-Bestrahlung, z.B. Psoralen/UV- oder Bromdesoxyuridin/UV-Behandlung, durch gamma-Strahlung oder durch Neutronenbeschuß inaktivierte Viren.

Inaktivierte Viren, wie sie z.B. auch für Vaccine verwendet werden, können mittels literaturbekannter Standardmethoden (Davis und Dulbecco, 1980, Hearst und Thiry, 1977) hergestellt und für den Einsatz zur Erhöhung des Imports von DNA-Komplexen getestet werden. In den im Rahmen der vorliegenden Erfindung durchgeführten Versuchen wurden Adenovirus-Präparationen mittels einer herkömmlichen UV-Sterilisationslampe bzw. mit Formaldehyd inaktiviert und überraschend festgestellt, daß das Ausmaß der Inaktivierung der Viren wesentlich größer war als die Abnahme des Gentransfer-Effekts, der erreicht wurde, wenn Adenovirus dem Transfektionsmedium beigegeben wurde. Auch Versuche, die mit Präparationen von Psoralen/UV-inaktiviertem biotinyliertem Adenovirus, das mit Streptavidin-gekoppeltem Polylysin konjugiert war, durchgeführt wurden, zeigten, daß als Folge der Inaktivierung der Virustiter beträchtlich stärker absank als die Gentransferkapazität. Dies ist ein deutlicher Hinweis darauf, daß Mechanismen, die beim aktiven Virus mit dem normalen Infektionsmechanismus in Zusammenhang stehen, zerstört werden können, ohne daß der für den Gentransfer wesentliche Effekt zerstört wird.

Unter dem Begriff "Viruskomponenten" werden Teile von Viren verstanden, z.B. der von Nukleinsäure befreite Proteinanteil (das leere Viruskapsid, das mit Hilfe rekombinanter Methoden hergestellt werden kann, siehe z.B. Ansardi et al., 1991, Urakawa et al., 1989), Proteine , die bei der Fraktionierung erhalten werden, oder Peptide, die die für die Aufnahmefunktion wesentliche endosomolytische Fähigkeit des intakten Virus besitzen. Diese Viruskomponenten können auch synthetisch hergestellt werden, in Abhängigkeit von ihrer Größe entweder mittels Peptidsynthese oder mittels rekombinanter Methoden. Im Rahmen der vorliegenden Erfindung konnte gezeigt werden, daß Adenovirus-Proteine, die über Biotin/Streptavidin mit Polylysin konjugiert sind, den Gentransfer verstärken können. Beispiele für Proteinfragmente von anderen Viren als Adenoviren, die wesentlich für die Internalisierung des Virus sind, umfassen das Influenzavirus-Hämagglutinin (HA). Die N-terminale Sequenz der Influenzavirus-Hämagglutinin HA2-Untereinheit ist verantwortlich für die Freisetzung des Virus aus dem Endosom. Es wurde gezeigt, daß Peptide, die aus 20 Aminosäuren dieser Sequenz bestehen, Lipidmembranen fusionieren und teilweise aufbrechen/zerstören können (Wharton et al., 1988). In der vorliegenden Erfindung wurden authentische und modifizierte Influenzapeptide erfolgreich in verschiedenen Ausführungsformen eingesetzt. Ein weiteres Bespiel sind Hüllproteine von Retroviren, z.B. HIV gp41 (Rafalski et al., 1990) bzw. Teile dieser Virusproteine.

Die Verwendung von Viren, die von sich aus die Fähigkeit haben, in Zellen einzudringen, ist nur ein Aspekt der vorliegenden Erfindung.

Viren oder Viruskomponenten, die von sich aus nicht die Fähigkeit mitbringen, an die Zelle zu binden und in sie einzudringen, werden vorzugsweise in Form der oben definierten viralen Konjugate verwendet. Die Kopplung an eine DNA-Bindungsdomäne, z.B. ein Polykation, gewährleistet, daß das Virus bzw. die Viruskomponente eine starke Affinität zu DNA-Molekülen erhält, damit komplexiert und als Bestandteil des DNA-Komplexes, der auch ein Internalisierungsfaktor/DNA-Bindungsdomäne-Konjugat enthält, in die Zelle transportiert wird. Zusätzlich zum damit erreichten Transporteffekt kann die Bindung des Virus bzw. der Viruskomponente an eine Nukleinsäure-Bindungsdomäne auch eine Verbesserung seiner endosomolytischen Eigenschaften zur Folge haben.

Durch die Auswahl anderer Internalisierungsfaktoren kann praktisch jede höhere eukaryotische Zelle mit den erfindungsgemäßen Zusammensetzungen transfiziert werden.

Ob ein gegebenes Virus bzw. eine Viruskomponente eine Aufnahmefunktion im Sinne der vorliegenden Erfindung und somit zur Verwendung bei der Verstärkung des Gentransfers geeignet ist, kann mittels eines einfachen Screeningassays festgestellt werden. In einem solchen Assay, z.B. um ein Virus auf seine Anwendbarkeit als freies Virus zu testen, werden die Zielzellen mit einem DNA-Komplex in Gegenwart oder in Abwesenheit des Virus in Berührung gebracht. Die Menge an DNA-Komplex, die ins Zytoplasma freigesetzt wird, kann dann einfach durch Nachweis eines Markergen-Produkts, z.B. Luciferase, bestimmt werden. Wenn die Gegenwart des Virus zur Folge hat, daß der DNA-Komplex in größerem Ausmaß als ohne Virus in die Zelle aufgenommen und ins Zytoplasma freigesetzt wird, kann dies auf die Aufnahmefunktion des Virus zurückgeführt werden. Es ist auch möglich, das Testvirus hinsichtlich seiner Aufnahmefunktion mit einem Virus zu vergleichen, von dem bekannt ist, daß es eine geeignete Aufnahmefunktion aufweist, z.B. mit Adenovirus, Untergruppe C, Typ 5. Tests dieser Art können auch auf virale Konjugate angewendet werden, wobei zusätzliche Parameter, wie verschiedene Internalisierungsfaktor-Konjugate, in verschiedenen Mengen solchen Tests unterworfen werden können. Darüberhinaus kann der Durchschnittsfachmann Assays dieser Art, gegebenenfalls in Verbindung mit anderen Tests, wie z.B. Liposomendurchlässigkeits-Assays ("Liposomen Leakage Assays"), auf einfache Weise auf Viruskomponenten oder andere Mittel mit potentieller endosomolytischer Aktivität anwenden, um sie auf ihre Fähigkeit zur Steigerung der Genexpression zu untersuchen.

Im Falle der Verwendung intakter Viren wird, zweckmäßigerweise parallel zu den Vorversuchen, in denen das Virus auf seine Fähigkeit zur Verstärkung des Gentransfers untersucht wird, geprüft, ob das Virus replikationsfähig ist. Die Untersuchung auf Replikationsfähigkeit wird im Fall von zythopathischen Viren oder im Fall von Viren, die das Wachstum der Wirtszellen merklich beeinträchtigen, mit Hilfe von Plaque-Assays durchgeführt (vgl. oben). Bei anderen Viren werden Nachweismethoden speziell für das jeweilige Virus angewendet, z.B. der Hämagglutionationstest oder chemisch-physikalische Methoden (elektronenmikroskopisch).

Es werden im Rahmen der vorliegenden Erfindung, insbesondere bei Verwendung von freien Viren, solche Viren bevorzugt, die in hohem Titer herstellbar sind, die stabil sind, die als Wildtyp geringe Pathogenität aufweisen und bei denen eine gezielte Ausschaltung der Replikationsfunktionen möglich ist, insbesondere Adenoviren. Wenn eine bestimmte Zellpopulation transfiziert werden soll, sind Viren bevorzugt, die diese Zellpopulation spezifisch infizieren. Für den Fall, daß von der Transfektion verschiedene Zelltypen erfaßt werden sollen, können Viren eingesetzt werden, die für einen weiten Bereich von Zelltypen infektiös sind.

Die Anforderungen an die Viruspräparation sind im wesentlichen größtmögliche Reinheit sowie ein auf das jeweilige Virus abgestimmter stabilisierender Puffer.

In jedem Fall kommen für die therapeutische Anwendung der vorliegenden Erfindung in vivo nur solche Viren bzw. Viruskomponenten in Betracht, bei denen Sicherheitsrisken, insbesondere bzgl. der Replikation des Virus in der Zelle sowie der Rekombination von Virus-DNA mit Wirts-DNA, weitestgehend minimiert sind.

Vorteilhafterweise kann der Eintrittsmechanismus von Viren, die andere Tiere als Menschen infizieren, verwendet werden, um die Aufnahme und die Freisetzung von DNA in höheren eukaryotischen Zellen, insbesondere menschlichen Zellen, zu verstärken, soferne das Virus in den Zellen die Fähigkeit zum Aufbrechen von Endosomen aufweist. Mitglieder der Adenovirusfamilie wurden aus Vogelarten, aus Amphibien und verschiedenen anderen Tieren isoliert (siehe z.B. Laver et al., 1971; Bragg et al., 1991; Akopian et al., 1991; Takase et al., 1990; Khang und Nagaraji, 1989, und Reece et al., 1987). Amphibien-, Vogel-, Rinder-, Hunde-, Maus-, Schaf-, Schweine- und Affenadenoviren, wie auch humane Adenoviren, sind beziehbar von der American Type Culture Collection, Rockville, Maryland (siehe American Type Culture Collection Catalogue of Animal Viruses and Antisera, Chlamydae and Rickettsiae, 6. Aufl., 1990, C. Buck und G. Paulino Hsg., S. 1-17).

Mögliche Vorteile der Verwendung eines Virus, z.B. eines Adenovirus, einer entfernten Spezies dürfte eine verringerte Toxizität in den Zielzellen sein (z.B. würde vom Hühner- oder Froschadenovirus nicht erwartet werden, daß es in Säugetierzellen repliziert oder die Expression früher Gene initiiert), ein im Vergleich zum humanen Adenovirus verringertes Risiko für den Forscher, der dieses Virus einer entfernten Spezies herstellt, und eine verminderte Störung durch Antikörper gegen das humane oder das Mausadenovirus. Das Fehlen einer Störung durch die humanen oder Mausantikörper ist besonders wichtig, wenn die Viren für die Gentherapie im Menschen oder in der Maus verwendet werden.

Das Hühneradenovirus CELO (Chick Embryo Lethal Orphan Virus) zeigt keine Reaktivität mit Antikörpern, die die Hauptgruppenepitope der Adenoviren erkennen, die Säugetierzellen infizieren. Außerdem können CELO-Viren in Eiern mit Embryoentwicklung gezüchtet werden, um große Mengen von Virus zu erhalten (0.5 mg/Ei; Laver et al., 1971). Wie aus den Beispielen hervorgeht, verstärken CELO-Polylysin-Konjugate den DNA-Transport in HeLa-Zellen in einem Ausmaß, das mit dem humanen Adenovirus dl312 vergleichbar ist. Die Verwendung von CELO-Konjugaten zur Verstärkung des DNA-Transports ist daher für die humane Gentherapie sehr vielversprechend.

Viren von entfernten Spezies werden bevorzugt als Bestandteile von viralen Konjugaten in Kombinationskomplexen (gemäß der Definition im Rahmen der vorliegenden Erfindung) eingesetzt.

In den erfindungsgemäßen Konjugaten, die ein Virus enthalten, kann die Bindung des Virus an die Nukleinsäure-Bindungsdomäne kovalent oder nichtkovalent sein, letzteres z.B. über eine Biotin-Streptavidin-Brücke oder, für den Fall, daß das Virus auf seinen Oberflächenproteinen Regionen aufweist, die sauer sind und daher an ein Polykation binden können, über eine ionische Bindung.

In Versuchen im Rahmen der vorliegenden Erfindung wurden Komplexe unter Bedingungen, die die ionische Wechselwirkung zwischen Adenovirus und Polylysin vor der Komplexierung mit DNA erlauben, gebildet. Als Kontrolle wurden Versuche unter Bedingungen, unter denen Polylysin zuerst mit DNA neutralisiert wurde und daher nicht frei ist, an das Adenovirus zu binden, durchgeführt. In diesen Versuchen erwiesen sich die Komplexe mit ionisch gebundenem Adenovirus als überlegen.

Beispiele für Viruskomponenten in den erfindungsgemäßen Konjugaten mit endosomolytischer Aktivität sind die leeren Viruskapside oder virale Peptide. Die Bindung der Viruskomponente an die Nukleinsäure-Bindungsdomäne kann kovalent sein, z.B. durch chemische Kopplung des viralen Peptids mit Polylysin, oder nicht-kovalent, z.B. ionisch im Fall, daß die Viruskomponente saure Reste hat, um an ein Polykation zu binden.

Das Verhältnis von Virus oder Viruskomponente zur Nukleinsäure-affinen Substanz kann variiert werden. Im Falle des Influenza-Hämagglutininpeptid-Polylysin-Konjugats wurde im Rahmen der vorliegenden Erfindung gefunden, daß der Gentransfer in größerem Ausmaß verstärkt werden kann, wenn der Gehalt an viralem Peptid in den Konjugaten höher war.

Die vorliegende Erfindung betrifft in einem weiteren Aspekt Methoden zur Herstellung der erfindungsgemäßen Konjugate aus Virus(komponente) und Nukleinsäure-affiner Substanz.

Die viralen Konjugate können (wie die Internalisierungsfaktor-Polykation-Konjugate) durch Kopplung der Komponenten oder, falls die Viruskomponente und das Polykation Polypeptide sind, auf rekombinantem Weg hergestellt werden, bezüglich der Herstellungsmethoden wird auf die Offenbarung der EP 388 758 Bezug genommen.

Die Kopplung von Virus, Virusproteinen oder -peptiden mit Polyaminverbindungen auf chemischem Weg kann in für die Kopplung von Peptiden an sich bekannter Weise erfolgen, wobei, falls erforderlich, die Einzelkomponenten vor der Kopplungsreaktion mit Linkersubstanzen versehen werden (diese Maßnahme ist dann erforderlich, wenn von vornherein keine für die Kopplung geeignete funktionelle Gruppe, z.B. eine Mercapto- oder Alkoholgruppe, verfügbar ist. Bei den Linkersubstanzen handelt es sich um bifunktionelle Verbindungen, die zunächst mit funktionellen Gruppen der Einzelkomponenten zur Reaktion gebracht werden, worauf die Kopplung der modifizierten Einzelkomponenten durchgeführt wird.

Die Kopplung kann erfolgen über
a) Disulfidbrücken, die unter reduzierenden Bedingungen wieder gespalten werden können (z.B. bei Verwendung von Succinimidylpyridyldithiopropionat (Jung et al., 1981).
b) Unter biologischen Bedingungen weitgehend stabile Verbindungen (z.B. Thioether durch Reaktion von Maleimido-Linkern mit Sulfhydrylgruppen des an die zweite Komponente gebundenen Linkers).
c) Unter biologischen Bedingungen labile Brücken, z.B. Esterbindungen, oder unter schwach sauren Bedingungen instabile Acetal- oder Ketalbindungen.

In den im Rahmen der vorliegenden Erfindung durchgeführten Versuche wurden endosomolytische Influenza-Hämagglutinin HA2-Peptide mit Polylysin auf chemischen Weg mittels Succinimidylpyridyldithiopropionat (SPDP) gekoppelt. Es wurde gezeigt, daß die Modifikation des Peptids mit Polylysin die endosomolytische Aktivität erhöht. Transfektionsexperimente zeigten, daß die Effizienz des durch Transferrin-Polylysin vermittelten Gentransfers erheblich gesteigert wird, wenn die Influenzapeptid-Polylysin-Konjugate zusammen mit Transferrin-Polylysin im DNA-Komplex vorliegen.

Weiters wurde im Rahmen der vorliegenden Erfindung Adenovirus mit Hilfe verschiedener Methoden an Polylysin gekoppelt.
Eine Art der Kopplung an Polylysin erfolgte in ähnlicher Weise wie bei der Herstellung von Transferrin-Polylysinkonjugaten (Wagner et al., 1990) nach Modifizierung des defekten Adenovirus dl312 mittels eines heterobifunktionellen Reagens. Ungebundenes Polylysin wurde durch Zentrifugation abgetrennt. Die DNA Bindungsfähigkeit wurde in einem Bindungsexperiment mit radioaktiv markierter DNA nachgewiesen.
In K562 Zellen in Abwesenheit von Chloroquin konnte mit Komplexen, bestehend aus DNA, Adenovirus-Polylysin und Transferrin-Polylysin ein wesentlich höherer Gentransfer gefunden werden als mit unmodifiziertem Adenovirus, das nicht an die DNA gebunden vorliegt. Weiters wurde gefunden, daß in HeLa Zellen mittels Polylysin-modifiziertem Adenovirus eine deutliche Genexpression mit nur 0.0003 µg DNA in 5x10⁵ HeLa-Zellen stattfand.

Falls das Virus bzw. die Viruskomponente geeignete Kohlenhydratketten aufweisen, können sie mit der Nukleinsäure-affinen Substanz über eine oder mehrere Kohlenhydratketten des Glykoproteins miteinander verbunden werden.

Eine geeignete Methode zur Herstellung von Glykoprotein-Polykation-Konjugaten ist in der deutschen Patentanmeldung P 41 15 038.4 geoffenbart; sie wurde kürzlich von Wagner et al., 1991b, beschrieben.

Ein weiteres bevorzugtes Verfahren zur Herstellung der erfindungsgemäßen Konjugate ist die enzymatische Kopplung des Virus bzw. der Viruskomponente an eine Nukleinsäure-affine Substanz, insbesondere ein Polyamin, durch eine Transglutaminase.

Die Gruppe der Transglutaminasen umfaßt mehrere verschiedene Enzyme, die unter anderem in der Epidermis (epidermale Transglutaminase), im Blut (Faktor XIII) und in den Zellen der unterschiedlichen Gewebe (Gewebs-Transglutaminase) vorkommen (Folk, 1985).
Transglutaminasen katalysieren in Gegenwart von Ca++ und unter Abspaltung von NH3 die Bildung von ε-(γ-glutamyl)Lysin Bindungen. Vorrausetzung hierfür ist, daß an Proteinen entsprechende Glutamine und Lysine vorhanden sind, die von dem Enzym umgesetzt werden können. Neben der ε-Amino Gruppe von Lysin, können auch (poly)Amine wie Ethanolamin, Putreszin, Spermin oder Spermidin als Substrat dienen (Clarke et al., 1959). Zur Zeit ist noch ungeklärt, von welchen Faktoren es abhängt, ob ein Glutamin oder Lysin eines Proteins oder ein Polyamin von dem Enzym umgesetzt werden können. Bekannt ist, daß an zahlreiche Zellproteine wie Zytokeratine (Zatloukal et al., 1989), Tubulin, Zellmembranproteine und auch Oberflächenproteine von Influenzaviren (Iwanij, 1977) Polyamine mittels Transglutaminase gebunden werden können.

Im Rahmen der vorliegenden Erfindung konnte gezeigt werden, daß Polylysin mittels Transglutaminase an Adenoviren gekoppelt werden kann. Es wurde festgestellt, daß die Kopplung in Gegenwart von Glycerin durchgeführt werden kann. Dies bietet den Vorteil, daß eine Viruspräparation, z.B. eine Adenoviruspräparation, die im Puffer als stabilisierendes Agens Glycerin enthält, direkt für die Kopplung verwendet werden kann.
Mit Hilfe von Adenovirus-Polylysin-Konjugaten, die gemeinsam mit Transferrin-Polylysin-Konjugaten mit Plasmid-DNA komplexiert wurden, konnte eine um ein Vielfaches höhere Genexpression erzielt werden als mit Transferrin-Polylysin-Konjugaten in Gegenwart von nicht-Polylysin-gekoppelten Adenovirus.

Eine weitere im Rahmen der vorliegenden Erfindung bevorzugte Methode zur Herstellung der erfindungsgemäßen Konjugate besteht darin, das Virus oder die Viruskomponente über eine Biotin-Protein-Brücke, vorzugsweise eine Biotin-Streptavidin Brücke, an das Polykation zu koppeln.

Die bekannt starke Assoziation von Biotin mit Streptavidin bzw. Avidin (Wilchek et al., 1988) wurde zur Kopplung von Adenovirus an Polylysin ausgenützt, indem Adenovirus mit Biotin modifiziert wurde und Streptavidin in ähnlicher Weise wie bei der Herstellung von Transferrin-Polylysinkonjugaten (Wagner et al., 1990) chemisch mit Polylysin konjugiert wurde. Komplexe, bestehend aus DNA und Streptavidin-Polylysin, an das Biotin-modifiziertes Virus gebunden ist, und gegebenenfalls noch nicht-kovalent gebundenem Polylysin, weisen eine sehr hohe Transfektionseffizienz auf, auch bei niederen DNA-Konzentrationen. Besonders effiziente Komplexe werden gebildet, wenn das Biotin-modifizierte Virus zuerst an Streptavidin-Polylysin gebunden wird und erst in zweiter Stufe die Bindung an DNA erfolgt.

Gegebenenfalls kann die Bindung an Biotin auch über Avidin erfolgen.

Weiters ist es möglich, die Bindung zwischen Virus(komponente) und Polylysin herzustellen, indem einerseits das Virus biotinyliert wird und andererseits ein anti-Biotin-Antikörper mit Polylysin konjugiert und die Bindung zwischen Virus und Polylysin über die Biotin/Antikörper-Bindung hergestellt wird, wobei handelsübliche polyklonale oder monoklonale Antikörper gegen Biotin eingesetzt werden können.

Die Bindung zwischen dem Virus und Polylysin kann auch hergestellt werden, indem Polylysin mit einem Lectin gekoppelt wird, das Affinität zu einem Virus-Oberflächenglykoprotein hat, wobei die Bindung in einem solchen Konjugat über die Bindung zwischen dem Lectin und dem Glykoprotein erfolgt. Weist das Virus von sich aus keine geeigneten Kohlenhydratseitenketten auf, kann es entsprechend modifiziert werden.

Ein Virus kann auch an eine Nukleinsäure-affine Substanz gebunden werden, indem es zunächst an der Oberfläche mit einem virusfremden Antigen (z.B. Digoxigenin DIG, erhältlich von Boehringer Mannheim; oder mit Biotin) modifiziert wird und die Verbindung zwischen dem modifizierten Virus und der Nukleinsäure-affinen Substanz über einen Antikörper, der an dieses Antigen bindet, hergestellt wird.
Welche Methode zur Herstellung der erfindungsgemäßen Konjugate eingesetzt wird, richtet sich nach verschiedenen Kriterien. So ist z.B. die Kopplung über Biotin die unspezifischste und daher am breitesten einsetzbare Methode, die Biotin-vermittelte Bindung stellt eine sehr starke nicht-kovalent Bindung dar.
Die enzymatische Reaktion mit Transglutaminase weist den Vorteil auf, daß sie auch in kleinstem Maßstab durchführbar ist. Die chemische Kopplung kommt im allgemeinen zum Einsatz, wenn größere Konjugatmengen synthetisiert werden sollen, diese Methode ist im allgemeinen auch am zweckmäßigsten, wenn Virusproteine oder -peptide gekoppelt werden sollen.
Im Falle der Verwendung inaktivierter Viren wird im allgemeinen die Inaktivierung vor der Kopplung vorgenommen, sofern die Kopplung durch die Inaktivierung nicht beeinträchtigt wird.

Wenn ein Virus, z.B. Adenovirus oder eine endosomolytische Komponente davon, zugängliche Bindungsdomänen aufweist, z.B. saure Domänen zur Bindung an ein Polykation, kann die Bindung des Virus bzw. der Viruskomponente an das Polykation auch ionisch sein. In diesem Fall sind die positiven Ladungen des Polykations, das gegebenenfalls mit einem Internalisierungsfaktor konjugiert ist, teilweise durch die saure Domäne von Virus(komponente) neutralisiert, der Rest an positiven Ladungen wird im wesentlichen von der Nukleinsäure neutralisiert.

Falls als Nukleinsäure-affine Substanz eine interkalierende Substanz eingesetzt wird, ist sie mit einem für die jeweilige Kopplung von Virus(komponente) geeigneten Linker modifiziert, z.B. für die Kopplung mit Transglutaminase mit Spermin, oder mit einer bifunktionellen, für die chemische Kopplung kompetenten Gruppe, z.B. einem aktiven Ester.

Das Verhältnis von Virus(komponente):Nukleinsäure-affiner Substanz kann variieren, es wird für gewöhnlich empirisch ermittelt, z.B. indem eine konstante Menge Virus(komponente) mit verschiedenen Mengen Polylysin konjugiert und das für die Transfektion optimale Konjugat ausgewählt wird.

In einer weiteren Ausführungsform der Erfindung kann die Viruskomponente, z.B. ein endosomolytisches virales Peptid, modifiziert werden, um direkt an DNA zu binden. Zu diesem Zweck kann das Peptid selbst eine DNA-Bindungsdomäne aufweisen, die erhalten werden kann, indem das Peptid mittels Peptidsynthese hergestellt wird, wobei ein Abschnitt von positiv geladenen Aminosäuren vorgesehen wird, vorzugsweise durch Verlängerung des Peptids, insbesondere bevorzugt am C-Terminus.

In einer weiteren Ausführungsform der Erfindung ist das endosomolytische Mittel ein nicht-virales, gegebenenfalls synthetisches Peptid. Ein Peptid dieses Typs ist vorzugsweise in der erfindungsgemäßen Zusammensetzung derart enthalten, daß es ionisch an die Nukleinsäure-affine Substanz, z.B. an Polylysin im Fall von DNA/Internalisierungsfaktor-Polylysin-Komplexen, gebunden ist. Auf diese Weise wird der Einbau des endosomolytischen Peptids in die Nukleinsäure-Komplexe bewirkt, indem das Peptid über seine sauren Aminosäurereste an die positiv geladene Nukleinsäure-Bindungsdomäne, vorzugsweise Polylysin, gebunden wird. In Abhängigkeit von der chemischen Struktur des Peptids, insbesondere bezüglich seiner Endgruppe, kann die Bindung an Polylysin auch durch die hier für die Bindung von Peptiden an Polylysin beschriebenen Methoden erfolgen. Zu diesem Zweck kann, wenn ein in der Natur vorkommendes Peptid eingesetzt wird, dieses Peptid mit einer geeigneten terminalen Aminosäure als "Stiel" für die Konjugation modifiziert werden.

Eine andere Art, nicht-virale endosomolytische Peptide in die Nukleinsäure-Komplexe einzubauen, besteht darin, sie mit Sequenzen zu versehen, die an DNA binden. Die Lage solch einer Sequenz muß so sein, daß sie die endosomolytische Aktivität des Peptids nicht stört. Daher werden z.B. Peptide, deren N-Terminus für diese Aktivität verantwortlich ist, am C-Terminus mit DNAbindenden Sequenzen verlängert. Verlängerungen dieser Art können homologe oder heterologe kationische Oligopeptide sein, z.B. ein Oligolysin-Schwanz, oder eine natürliche DNA-Bindungsdomäne, z.B. ein von einem Histon abgeleitetes Peptid. Vorzugsweise umfassen diese einen integralen Teil des endosomolytischen Peptids bildenden DNA-Bindungssequenzen ca. 10 - 40 Aminosäuren. Diese Ausführungsform der Erfindung bietet die Möglichkeit eines höheren Verhältnisses endosomolytische Sequenz:DNA-Bindungssequenz als in Peptidkonjugaten, die größere Anteile von Polykationen enthalten, um eine größere Leistungsfähigkeit der Komplexe zu erreichen.

Die nicht-viralen endosomolytischen Peptide sollten die folgenden Anforderungen erfüllen:

Im Hinblick auf die endosomolytische Aktivität sollte die durch das Peptid bewirkte Durchlässigkeit von Lipidmembranen vorzugsweise bei niedrigem pH-Wert (5-6) höher sein als bei pH-Wert 7. Ferner sollten die aufgebrochenen Membranbereiche groß genug sein, um den Durchtritt großer DNA-Komplexe zu erlauben (kleine Poren sind nicht ausreichend). Um festzustellen, ob ein Peptid diese Anforderungen erfüllt, können in vitro Tests durchgeführt werden, in denen die Peptide in freier oder gebundener Form und/oder eingebaut in einen DNA-Komplex angewendet werden. Solche Tests können Liposomen- oder Erythrozyten-Durchlässigkeitstests ("leakage assays") und Zellkulturexperimente, in denen die Verstärkung der Genexpression bestimmt wird, umfassen. Tests dieser Art sind in den Beispielen beschrieben. Die optimale Peptidmenge kann in vorausgehenden Titrationen festgelegt werden, indem die Leistungsfähigkeit des resultierenden Gentransfers bestimmt wird. Dabei ist zu beachten, daß die Leistungsfähigkeit verschiedener Peptide und die optimale Zusammensetzung des Komplexes vom Zelltyp abhängig sein können.

Peptide, die Membranen aufbrechen, enthalten im allgemeinen amphipathische Sequenzen, nämlich eine hydrophobe Seite, die mit der Lipidmembran in Wechselwirkung treten kann, und eine hydrophile Seite, die die wässrige Phase an der Stelle, an der die Membran aufbricht, stabilisiert.

Es gibt in der Natur einige Beispiele für Peptide, die Membranen aufbrechen, für gewöhnlich kleine Peptide oder Peptiddomänen großer Polypeptide. Solche Peptide können nach ihrer Funktion im natürlichen Kontext klassifiziert werden, nämlich entweder in Membranen aufbrechende Peptide (z.B. Peptide von nackten Viren) und/oder Membranen fusionierende Peptide (z.B. Peptide von Viren mit Hülle). Für das Aufbrechen von Endosomen im Zusammenhang mit synthetischen Peptiden können beide Klassen von Peptidsequenzen von Nutzen sein. Die meisten natürlichen Peptide können amphipathische α-Helices bilden.

Durch Einbau saurer Reste auf der hydrophilen Seite einer mutmaßlichen amphipathischen α-Helix derart, daß sich die Helix nur bei saurem pH-Wert bilden kann, nicht jedoch bei neutralem pH-Wert, bei dem die Ladungsabstoßung zwischen den negativ geladenen sauren Resten die Helixbildung verhindert, kann pH-Spezifität erreicht werden. Diese Eigenschaft findet sich auch bei natürlich vorkommenden Sequenzen (z.B. der N-Terminus des Influenza-HA2-Peptids).

Ein zur Gänze synthetisches amphipathisches Peptid mit pH-Wert-spezifischen Eigenschaften zum Aufbrechen von Membranen wurde von Subbarao et al., 1987, und von Parente et al., 1990, beschrieben. Von diesem Peptid (in freier Form) wurde gezeigt, daß es in Membranen nur kleine Poren bildet, die nur die Freisetzung kleiner Verbindungen erlauben (Parente et al., 1990).

Im Rahmen der Ausführungsform der vorliegenden Erfindung, die sich nicht-viraler, gegebenenfalls synthetischer Peptide bedient, werden für gewöhnlich die folgenden Schritte unternommen: Aus der Gruppe natürlich vorkommender oder künstlicher Peptide wird eine amphipathische Peptidsequenz ausgewählt. Peptide dieser Art gehören zum Stand der Technik; eine Übersicht über Beispiele wird in Tabelle 2 gegeben. Wenn notwendig, werden saure Reste (Glu, Asp) eingeführt, um die Aktivität des Peptids, Membranen aufzubrechen, mehr pH-Wert-spezifisch zu machen (z.B. die doppelsaure Mutante des Influenza-Hämagglutininpeptids der Bezeichnung P50 entsprechend Beispiel 37). Wenn erforderlich, können saure Reste auch eingeführt werden, um die Bindung des Peptids an Polylysin zu erleichtern. Eine Möglichkeit, solch eine Polykationen-Bindungsdomäne vorzusehen, kann darin bestehen, C-terminale saure Verlängerungen, z.B. einen oligo-Glu-Schwanz, vorzusehen.

Für die vorliegende Erfindung geeignete endosomolytische Peptide können auch erhalten werden, indem natürlich vorkommende und künstliche Sequenzen vereinigt werden. Im Zuge der vorliegenden Erfindung wurden Beispiele mit verschiedenen Peptiden, abgeleitet von dem von Parente et al., 1990, beschriebenen synthetischen Peptid GALA, durchgeführt. Einige der in den Beispielen der vorliegenden Erfindung eingesetzten Derivate wurden erhalten, indem das Peptid GALA oder Modifikationen davon mit Sequenzen des Influenzapeptids oder Modifikationen davon kombiniert wurde, z.B. die Peptide der Bezeichnung EALA-Inf und EALA-P50 entsprechend Beispiel 37.

Die Länge der Peptidsequenz kann im Hinblick auf die amphipathische Helix kritisch sein; eine Erhöhung der Stabilität von kurzen Domänen, die von natürlichen Proteinen abgeleitet sind und denen der Kontext des stabilisierenden Proteins fehlt, kann durch Verlängerung der Helix erzielt werden.

Um die endosomolytische Aktivität der Peptide zu verstärken, können Homodimere, Heterodimere oder Oligomere gebildet werden; in den Beispielen der vorliegenden Erfindung wurde gezeigt, daß ein P50-Dimeres eine viel höhere Aktivität als das Monomer aufweist.

Die Erfinder haben die Wirkung synthetischer Peptide auf die DNA-Aufnahme mittels Transferrin-Polylysin-Konjugaten gezeigt. Es wurden mehrere verschiedene Peptide synthetisiert, ihre Fähigkeit, Liposomen und Erythrozyten durchlässig zu machen, bestimmt und ihre Wirkung auf die Luciferase-Expression in TIB 73-Zellen und in NIH 3T3-Zellen getestet.

In einer weiteren Ausführungsform der Erfindung ist das endosomolytische Mittel eine nicht-peptidische amphipathische Substanz. Die Anforderungen, die eine solche Substanz erfüllen muß, um für die Anwendung im Rahmen der vorliegenden Erfindung geeignet zu sein, sind im wesentlichen dieselben wie für die amphipathischen Peptide, nämlich die Fähigkeit, in den Nukleinsäure-Komplex integriert zu werden, pH-Spezifität, etc.

Die Erfindung betrifft in einem weiteren Aspekt Komplexe, die in höhere eukaryotische Zellen aufgenommen werden, enthaltend Nukleinsäure und ein Konjugat, das die Fähigkeit besitzt, mit Nukleinsäure einen Komplex zu bilden, zum Einführen von Nukleinsäure in höhere eukaryotische Zellen. Die Komplexe sind dadurch gekennzeichnet, daß sie ein Konjugat enthalten, das aus einer Nukleinsäure-affinen Substanz und einem endosomolytischen Mittel besteht, das an die Nukleinsäure-affine Substanz gebunden ist und die Fähigkeit besitzt, als Bestandteil eines Konjugat/Nukleinsäure-Komplexes in die Zelle aufgenommen zu werden und den Inhalt der Endosomen, in denen der Komplex nach Eintritt in die Zelle lokalisiert ist, ins Zytoplasma freizusetzen.

Die Nukleinsäure-Komplexe, die im Rahmen der vorliegenden Erfindung zur Anwendung kommen, sind vorzugsweise solche, in denen die Nukleinsäure mit einer Nukleinsäure-affinen Substanz derart komplexiert ist, daß die Komplexe im wesentlichen elektroneutral sind.

In einer bevorzugten Ausführungsform der Erfindung ist das endosomolytische Mittel ein Virus oder eine Viruskomponente, das bzw. die kovalent an ein Polykation gebunden ist.

Im Rahmen der vorliegenden Erfindung umfassen die endosomolytischen Konjugate - zusätzlich zu Konjugaten, in denen endosomolytische Mittel ionisch an eine DNA-Bindungsdomäne gebunden sind - definitionsgemäß auch endosomolytische Mittel, die direkt an DNA binden, z.B. über ihre basische Verlängerung, obwohl "Konjugate" dieser Art streng genommen nicht durch Konjugation, d.h. durch Verbinden zweier Komponenten miteinander, erhalten wurden. Die Funktion von endosomolytischen Mitteln dieses Typs als Bestandteile der erfindungsgemäßen Zusammensetzung ist unabhängig davon, ob sie durch Konjugation eines endosomolytischen Mittels und einer DNA-Bindungsdomäne synthetisiert wurden oder ob eine DNA-Bindungsdomäne ursprünglich im endosomolytischen Mittel vorhanden war.

In einer bevorzugten Ausführungsform der Erfindung enthalten die Komplexe zusätzlich zum endosomolytischen Konjugat ein weiteres Konjugat, in dem eine Nukleinsäure-affine Substanz, im Falle eines endosomolytischen Polykation-Konjugats im allgemeinen dieselbe wie die des Konjugats, mit einem Internalisierungsfaktor mit Affinität für die Zielzelle gekoppelt ist. Diese Ausführungsform der vorliegenden Erfindung wird vor allem dann angewendet, wenn die Zielzelle keine oder nur wenige Rezeptoren für das Virus aufweist, das als Bestandteil des endosomolytischen Konjugats zur Anwendung kommt. Eine weitere Anwendung dieser Ausführungsform ist die, bei der eine Viruskomponente, z.B. ein natürlich vorkommendes, gegebenenfalls modifiziertes Peptid, ein nicht-virales, gegebenenfalls synthetisches endosomolytisches Peptid oder ein Virus einer entfernten Spezies eingesetzt werden, die nicht die Fähigkeit aufweisen, von sich aus in die zu transfizierenden Zellen einzudringen. In Gegenwart eines weiteren Internalisierungsfaktor-Verbindungsfaktor-Konjugats profitieren diese endosomolytischen Konjugate von der Internalisierungsfähigkeit des zweiten Konjugats, indem sie gemeinsam mit diesem an die Nukleinsäure komplexiert werden und als Bestandteil des so entstandenen Komplexes, im folgenden als "Kombinations-Komplex" oder "ternärer Komplex" bezeichnet, in die Zelle aufgenommen werden. Ohne auf diese Theorie festgelegt sein zu wollen, werden die Kombinations-Komplexe von Zellen entweder durch Bindung an den für den Internalisierungsfaktor spezifischen Oberflächenrezeptor oder, im Falle der Verwendung eines Virus oder einer Viruskomponente, durch Bindung an den Virusrezeptor oder durch Bindung an beide Rezeptoren über den Weg der Rezeptor-vermittelten Endozytose aufgenommen. Bei der Freisetzung der endosomolytische Mittel aus den Endosomen wird auch die in den Komplexen enthaltene DNA in das Zytoplasma freigesetzt und entkommt dadurch dem lysosomalen Abbau.

In den Versuchen der vorliegenden Erfindung mit HeLa-Zellen konnten beinahe alle Zellen mit freiem Adenovirus transfiziert werden. Die Leistungsfähigkeit für Hepatozyten konnte noch weiter gesteigert werden, wenn ternäre DNA-Komplexe verwendet wurden, in denen die Reporter-DNA mit Polylysin-Transferrin-Konjugaten komplexiert ist und an Adenovirus gebunden wird. Hier wird die gemeinsame Lokalisierung des endosomolytischen Virus und des Ligand/Rezeptor-Komplexes in den Endosom gewährleistet, wobei für verschiedene Zellen, wie BNL.CL2- und HepG2-Zellen, eine Transfektion in praktisch allen Zellen erreicht wurde. Solch eine Situation kann für die Experimente angenähert werden, in denen ternäre, Transferrin enthaltende Komplexe eher über den Transferrin-Rezeptor als über den Adenovirus-Rezeptor Eintritt in K562-Zellen fanden.

Überraschenderweise transportierten ternäre Komplexe DNA sogar in sehr niedrigen Mengen. So wurden bei einem Einsatz von 30 pg DNA pro 3 x 10⁵ Zellen 1.8 x 10⁴ Lichteinheiten (resultierend aus der Expression einer auf einem Plasmid enthaltenen, für Luciferase kodierenden Sequenz) erhalten. Bei einem solchen Input kommen auf eine Zelle nur 60 DNA-Moleküle und 1 PFU an Virus ("Plaque Forming Unit"). Zum Vergleich: die weniger effiziente Kalziumpräzipitationsvorschrift verwendet 2 x 10⁵ DNA-Moleküle pro Zelle (Sambrook et al., 1989). Die vorliegende Erfindung stellt daher einen beträchtlichen Fortschritt dar, weil sie die effiziente Transformation von höheren eukaryotischen Zellen mit sehr kleinen DNA-Mengen erlaubt.

Die Gegenwart von Viren, Viruskomponenten oder nichtviralen endosomolytischen Mitteln als Bestandteile von endosomolytischen Konjugaten in den DNA-Komplexen hat folgende Vorteile:
1) Breitere Anwendbarkeit der Gentransfertechnik mit Nukleinsäure-Komplexen, weil das endosomolytische Mittel selbst, insbesondere für den Fall, daß ein Virus oder eine Viruskomponente eingesetzt wird, den Internalisierungsfaktor darstellen kann oder auch in Kombination mit einem weiteren Internalisierungsfaktor (z.B. Transferrin oder Asialofetuin etc.) an die DNA komplexiert werden kann. Dadurch ist es möglich, den positiven Effekt der Viren auch für Zellen auszunützen, die keinen Rezeptor für die jeweiligen Viren haben.
2) Verbesserung der Gentransfer Effizienz, da durch die Bindung der endosomolytischen Konjugate an die DNA eine gemeinsame Aufnahme in die Zellen gewährleistet ist. Durch die koordinierte Aufnahme und Freisetzung von Viren und DNA, ergibt sich weiters die Möglichkeit einer Reduktion der für einen effizienten Gentransfer benötigten Menge an DNA und Viren, was besonders für Anwendungen *in vivo* von essentieller Bedeutung ist.

Unter Internalisierungsfaktor sind im Rahmen der vorliegenden Erfindung Liganden oder Fragmente davon zu verstehen, die nach Bindung an die Zelle über Endozytose, vorzugsweise Rezeptor-vermittelte Endozytose, internalisiert werden, oder Faktoren, deren Bindung/Internalisierung über Fusion mit Zellmembranelementen erfolgt.
Zu geeigneten Internalisierungsfaktoren zählen die Liganden Transferrin (Klausner et al., 1983), Conalbumin (Sennett et al., 1981), Asialoglykoproteine (wie Asialotransferrin, Asialorosomucoid oder Asialofetuin), (Ashwell et al., 1982), Lectine (Goldstein et al., 1980 und Shardon, 1987), bzw. Substanzen, die Galaktose enthalten und über den Asialoglykoproteinrezeptor internalisiert werden; mannosylierte Glykoproteine (Stahl et al., 1987), lysosomale Enzyme (Sly et al., 1982), LDL (Goldstein et al., 1982), modifizierter LDL (Goldstein et al., 1979), Lipoproteine, die über Rezeptoren in die Zelle aufgenommen werden (apo B100/LDL); virale Proteine, wie das HIV-Protein gp120; Antikörper (Mellman et al., 1984, Kuhn et al., 1982, Abrahamson et al., 1982) bzw. Fragmente davon gegen Zelloberflächenantigene, z.B. anti-CD4, anti-CD7; Zytokine wie Interleukin-l (Mizel et al., 1987), Interleukin-2 (Smith et al., 1985), TNF (Imamure et al., 1987), Interferone (Anderson et al., 1982); CSF ("Colony-stimulating Factor"), (Walker et al., 1987), Faktoren und Wachstumsfaktoren wie Insulin (Marshall, 1985), EGF ("Epidermal Growth Factor"), (Carpenter, 1984); PDGF ("Platelet-derived Growth Factor"), (Heldin et al., 1982), TGFβ (Transforming Growth Factor β"), (Massague et al., 1986), Nervenwachstumsfaktor (Hosang et al., 1987), Insulinähnlicher Wachstumsfaktor I ("Insulin-like Growth Factor"), (Schalch et al., 1986), LH, FSH (Ascoli et al., 1978), Wachstumshormon (Hizuka et al., 1981), Prolactin (Posner et al., 1982), Glucagon (Asada-Kubota et al., 1983), Thyroidhormone (Cheng et al., 1980), α-2-Makroglobulin-Protease (Kaplan et al., 1979), sowie "entwaffnete" Toxine. Weitere Beispiele sind Immunglobuline oder Fragmente davon als Liganden für den Fc-Rezeptor oder Anti-Immunglobulin-Antikörper, die an SIgs ("Surface Immunoglobulins") binden. Die Liganden können natürlichen oder synthetischen Ursprungs sein (siehe Trends Pharmacol. Sci., 1989, und die darin zitierten Referenzen).

Wesentlich für die Eignung solcher Internalisierungsfaktoren im Rahmen der vorliegenden Erfindung ist,
a) daß sie vom spezifischen Zelltyp, in den die Nukleinsäure eingebracht werden soll, internalisiert werden können und ihre Internalisierungsfähigkeit nicht oder nicht wesentlich beeinträchtigt wird, wenn sie mit dem Verbindungsfaktor konjugiert werden, und
b) daß sie im Rahmen dieser Eigenschaft in der Lage sind, Nukleinsäure auf dem von ihnen benutzten Weg in die Zelle "huckepack" mitzunehmen.

In den im Rahmen der vorliegenden Erfindung durchgeführten Versuchen wurde die breite Anwendbarkeit der Erfindung hinsichtlich des Internalisierungsfaktors bzw. eines zusätzlichen Internalisierungsfaktors in den Kombinations-Komplexen anhand von human- und Maus-Transferrin-Polylysin-Konjugaten, Asialofetuin-Polylysin-Konjugaten, Galaktose-Polylysin-Konjugaten, Weizenkeimagglutinin-pL-Konjugaten, der T-Zellspezifischen gp120-pL und antiCD7-pL-Konjugate, von LDL-pL-Konjugaten, Ig-pL- und anti-Ig-pL-Konjugate sowie anhand von DNA-Polylysin-Komplexen, die keinen Internalisierungsfaktor enthalten, gezeigt. Außerdem wurde anhand von Komplexen aus DNA und Polylysinkonjugiertem Virus (bzw. Viruskomponente), die kein zusätzliches Internalisierungsfaktor-Verbindungsfaktor-Konjugat enthielten, die Leistungsfähigkeit der erfindungsgemäßen viralen Konjugate gezeigt.

Im einzelnen kann in Vorversuchen bestimmt werden, ob im Fall der Verwendung von freiem Virus als endosomolytisches Mittel die Anwendung eines Internalisierungsfaktors, oder ob für den Fall, daß das endosomolytische Mittel ein Virus oder eine Viruskomponente oder ein nicht-virales Peptid als Teil eines endosomolytischen Konjugats ist, ein "zusätzlicher" Verbindungsfaktor die Aufnahme von Nukleinsäure-Komplexen ermöglicht oder verbessert. Solche Test umfassen Parallel-Transfektionen mit Nukleinsäure-Komplexen einmal ohne (zusätzlichen) Internalisierungsfaktor, z.B. im Fall von viralen Konjugaten mit Komplexen, bestehend aus Nukleinsäure und viralem Konjugat, und einmal mit Komplexen, in denen die Nukleinsäure mit einem weiteren Konjugat, enthaltend einen weiteren Internalisierungsfaktor, für den die Zielzellen einen Rezeptor haben.

Bei Anwendung eines Internalisierungsfaktors oder eines zusätzlichen Internalisierungsfaktors, d.h. wenn ein Kombinations-Komplex zur Anwendung kommt, wird dieser vor allem durch die Zielzellen definiert, z.B. durch bestimmte Oberflächenantigene oder Rezeptoren, die für einen Zelltyp spezifisch sind und somit einen gerichteten Import der Nukleinsäure in diesen Zelltyp ermöglichen.

Im Rahmen der vorliegenden Erfindung geeignete Nukleinsäure-affine Substanzen sind z.B. homologe organische Polykationen wie Polylysin, Polyarginin, Polyornithin oder heterologe Polykationen mit zwei oder mehr unterschiedlichen positiv geladenen Aminosäuren, wobei diese Polykationen verschiedene Kettenlänge aufweisen können, weiters nicht-peptidische synthetische Polykationen wie Polyethylenimin. Geeignete Nukleinsäure-affine Substanzen sind ferner natürliche DNA bindende Proteine polykationischen Charakters wie Histone oder Protamine bzw. Analoge oder Fragmente davon, sowie Spermin oder Spermidine.

Die Länge des Polykations ist nicht kritisch, sofern die Komplexe im Hinblick auf die bevorzugte Ausführungsform im wesentlichen elektroneutral sind. Der bevorzugte Bereich der Polylysin-Kettenlänge liegt im Bereich von ca. 20 bis ca. 1.000 Lysinmonomere. Es besteht jedoch für eine vorgegebene DNA-Länge keine kritische Länge für das Polykation. Wenn die DNA aus 6.000 bp und 12.000 negativen Ladungen besteht, kann die Menge an Polykation pro Mol DNA z.B. sein:
60 Mol Polylysin 200 oder
30 Mol Polylysin 400 oder
120 Mol Polylysin 100, etc.
Der Durchschnittsfachmann kann mittels einfacher Routineversuche andere Kombinationen von Polykationlänge und molarer Menge auswählen.

Weitere geeignete Nukleinsäure-affine Substanzen als Bestandteil der Konjugate sind interkalierende Substanzen wie Ethidiumdimere, Acridin oder interkalierende Peptide, enthaltend Tryptophan und/oder Tyrosin und/oder Phenylalanin.

Hinsichtlich der qualitativen Zusammensetzung der Nukleinsäure-Komplexe wird im allgemeinen zunächst die in die Zelle zu -importierende Nukleinsäure festgelegt. Die Nukleinsäure wird vor allem durch den in der Zelle zu erzielenden biologischen Effekt definiert, im Falle der Anwendung im Rahmen der Gentherapie durch das zur Expression zu bringende Gen bzw. den Genabschnitt, z.B. zwecks Substitution eines defekten Gens, oder durch die Zielsequenz eines zu inhibierenden Gens. Bei den in die Zelle zu transportierenden Nukleinsäuren kann es sich um DNAs oder RNAs handeln, wobei hinsichtlich der Nukleotidsequenz keine Beschränkungen bestehen.

Wenn die Erfindung auf Tumorzellen angewendet wird, um diese als Krebsvaccine einzusetzen, kodiert die in die DNA einzuführende Zelle vorzugsweise für eine immunmodulierende Substanz, z.B. ein Zytokin wie IL-2, IL-4, IFN-gamma, TNF-α. Von besonderem Nutzen können Kombinationen von DNAs sein, die für Zytokine kodieren, z.B. IL-2 und IFN-gamma. Ein weiteres Gen, das für das Einbringen in Tumorzellen nützlich ist, ist das "Multi Drug Restistance Gene" (mdr). In der vorliegenden Erfindung wurden mit Erfolg Transferrin-Polylysin- und Low Density-Lipoprotein-Konjugate zusammen mit Adenovirus-Konjugaten für die Transfektion von Tumorzellen (Melanomzellen) eingesetzt. In Abhängigkeit von der spezifischen Anwendung kann in Vorversuchen ermittelt werden, welcher Ligand für den speziellen Anwendungsfall für den jeweiligen Tumorzelltyp geeignet ist.

Es ist auch möglich, zwei oder mehr verschiedene Nukleinsäuresequenzen in die Zelle einzubringen, z.B. ein Plasmid, enthaltend cDNAs, kodierend für zwei verschiedene Proteine, unter der Kontrolle von geeigneten regulatorischen Sequenzen, oder zwei verschiedene Plasmidkonstrukte, enthaltend verschiedene cDNAs.

Zu therapeutisch wirksamen inhibierenden Nukleinsäuren, die zum Zweck der Inhibierung spezifischer Gensequenzen in die Zelle eingeführt werden, zählen Genkonstrukte, von denen Antisense-RNA oder Ribozyme transkribiert werden. Ferner ist es auch möglich, Oligonukleotide, z.B. Antisenseoligonukleotide, in die Zelle einzubringen. Antisenseoligonukleotide umfassen vorzugsweise 15 Nukleotide oder mehr. Gegebenenfalls können die Oligonukleotide multimerisiert sein. Ribozyme werden vorzugsweise als Teil eines Genkonstrukts, das stabilisierende Genelemente, z.B. tRNA-Genelemente, enthält, in die Zelle eingeführt. Genkonstrukte dieses Typs sind in der EP A 0 387 775 geoffenbart. Inhibierende Nukleinsäuren und deren Wirkmechanismen sind dem Durchschnittsfachmann geläufig; diesbezüglich wird auf die Übersichtsartikel von Hèlène und Toulme, 1990, und Takayama und Inouye, 1990, sowie die darin zitierten Referenzen verwiesen.

Außer Nukleinsäuremolekülen, die Gene, z.B. virale Gene, aufgrund ihrer Komplementarität inhibieren, können auch Gene mit anderer inhibierender Wirkung verwendet werden. Beispiele dafür sind Gene, die für virale Proteine kodieren, die sog. transdominante Mutationen haben (Herskowitz, 1987). Expression der Gene in der Zelle liefert Proteine, die das entsprechende Wildtypprotein dominieren und auf diese Weise die Zelle, die zelluläre Immunität erwirbt, schützen, indem sie die Virusreplikation verhindern. Geeignet sind transdominante Mutationen viraler Proteine, die für die Replikation und Expression erforderlich sind, z.B. Gag-, Tat- und Rev-Mutanten, von denen gezeigt wurde, daß sie HIV-Replikation inhibieren (Trono et al., 1989; Green et al., 1989; Malim et al., 1989).

Ein anderer Mechanismus zur Erlangung intrazellulärer Immunität umfaßt die Expression von RNA-Molekülen, die die Bindungsstelle für ein essentielles virales Protein enthalten, z.B. sog. "TAR Decoys" (Sullenger et al., 1990).

Beispiele für im Rahmen der somatischen Gentherapie anwendbare Gene, die mit Hilfe der vorliegenden Erfindung als Bestandteil von Genkonstrukten in die Zelle eingeschleust werden können, sind Faktor VIII (Hämophilie A), (siehe z.B. Wood et al., 1984), Faktor IX (Hämophilie B), (siehe z.B. Kurachi et al., 1982), Adenosindeaminase (SCID), (siehe z.B. Valerio et al., 1984), α-1 Antitrypsin (Lungenemphysem), (siehe z.B. Ciliberto et al., 1985) oder das "Cystic fibrosis transmembrane conductance regulator gene" (siehe z.B. Riordan et al., 1989). Diese Beispiele stellen keinerlei Beschränkung dar.

Bezüglich der Größe der Nukleinsäuren sind Anwendungen in einem weiten Bereich möglich; mit Hilfe der vorliegenden Erfindung können Nukleinsäure-Moleküle einer Größenordnung von ca. 0.15 kb (im Falle eines ein Ribozym-Gen enthaltenden tRNA-Gens) bis ca. 50 kb und darüber in die Zelle befördert werden; kleinere Nukleinsäuremoleküle können als Oligonukleotide angewendet werden.

Es ist offensichtlich, daß gerade aufgrund der Tatsache, daß die vorliegende Erfindung keinerlei Beschränkungen hinsichtlich der Gensequenz unterliegt und daß mit Hilfe der Erfindung auch sehr große Genkonstrukte transportiert werden können, breiteste Anwendungsmöglichkeiten gegeben sind.

Ausgehend von der Nukleinsäure wird die Nukleinsäure-affine Substanz, vorzugsweise eine organische polykationische Substanz, bestimmt, die die Komplexierung der Nukleinsäure gewährleistet, die erhaltenen Komplexe sind vorzugsweise im wesentlichen elektroneutral. Wenn die Komplexe neben dem endosomolytischen Konjugat ein Konjugat aus einem Internalisierungsfaktor und einer Nukleinsäure-affinen Substanz enthalten, wird der Kationenanteil beider Konjugate hinsichtlich des Aspekts der Elektroneutralität in Betracht gezogen.

Im Zuge von früheren Erfindungen war festgestellt worden, daß ein Optimum für den Import von Nukleinsäure in die Zelle erzielt werden kann, wenn das Verhältnis Konjugat:Nukleinsäure so gewählt wurde, daß die Internalisierungsfaktor-Polykation/Nukleinsäure-Komplexe weitgehend elektroneutral waren. Es wurde festgestellt, daß die Menge der in die Zelle aufgenommenen Nukleinsäure-Menge nicht verringert wird, wenn ein Teil der Transferrin-Polykation-Konjugate durch nicht-kovalent gebundenes Polykation ersetzt wird; in bestimmten Fällen kann sogar eine beträchtliche Steigerung der DNA-Aufnahme erreicht werden (Wagner et al., 1991a). Dabei war beobachtet worden, daß die DNA innerhalb der Komplexe in zu toroiden Strukturen mit einem Durchmesser von 80 - 100 nm verdichteter Form vorliegt. Die Menge an Polykation wird somit im Hinblick auf die beiden Parameter Elektroneutralität und Erzielung einer kompakten Struktur gewählt, wobei die Menge an Polykation, die sich im Hinblick auf die Erzielung der Elektroneutralität aus der Ladung der Nukleinsäure ergibt, im allgemeinen auch eine Kompaktierung der DNA gewährleistet.

Gegebenenfalls enthalten somit in einer weiteren Ausführungsform der Erfindung die Komplexe zusätzlich Nukleinsäure-bindende Substanzen in nicht kovalentgebundener Form, die gleich oder verschieden vom Verbindungsfaktor, also der Nukleinsäure-affinen Substanz in den Konjugaten, sein können. Für den Fall, daß das endosomolytische Mittel freies Virus ist, umfassen die Komplexe Nukleinsäure und Internalisierungsfaktor-Konjugat. Für den Fall, daß ein endosomolytisches, z.B. ein virales Konjugat verwendet wird, ist die Nukleinsäure mit diesem Konjugat komplexiert, gegebenenfalls gemeinsam mit einem Konjugat eines zusätzlichen Internalisierungsfaktors. Die Auswahl von nicht-kovalent gebundenen "freien" Nukleinsäure-affinen Substanzen nach Art und Menge wird außerdem auf das Konjugat bzw. die Konjugate abgestimmt, wobei vor allem der im Konjugat enthaltene Verbindungsfaktor zu berücksichtigen ist: ist der Verbindungsfaktor z.B eine Substanz, die keine oder nur geringe Fähigkeit zur DNA-Kondensation aufweist, ist es im Hinblick auf eine effiziente Internalisierung der Komplexe im allgemeinen zweckmäßig, solche DNA-affine Substanzen einzusetzen, die diese Eigenschaft in stärker ausgeprägtem Maß besitzen. Ist der Verbindungsfaktor selbst eine Nukleinsäure kondensierende Substanz und wird bereits durch ihn eine im Hinblick auf effiziente Internalisierung ausreichende Kompaktierung der Nukleinsäure bewirkt, wird zweckmäßigerweise eine Nukleinsäure-affine Substanz verwendet, die aufgrund anderer Mechanismen eine Steigerung der Expression bewirkt.

Zu den im Rahmen der vorliegenden Erfindung geeigneten nicht-kovalent gebundenen Nukleinsäure-affinen Substanzen zählen Verbindungen mit der Fähigkeit, Nukleinsäure zu kondensieren und/oder diese vor unerwünschtem Abbau in der Zelle zu schützen, insbesondere die bereits angeführten Substanzen polykationischen Charakters. Eine weitere Gruppe geeigneter Substanzen sind solche, die durch Bindung an die Nukleinsäure eine Verbesserung deren Transkription/Expression bewirken, indem sie die Zugänglichkeit der Nukleinsäure für die Expressionsmaschinerie der Zelle verbessern. Ein Beispiel für eine solche Substanz ist das chromosomale Nicht-Histon-Protein HMG1, von dem festgestellt wurde, daß es die Fähigkeit aufweist, DNA zu kompaktieren und in der Zelle zur Expression zu bringen.

Bei der Bestimmung des molaren Verhältnisses von endosomolytischem Mittel und/oder Internalisierungsfaktor/Nukleinsäure-affine Substanz/Nukleinsäure(n) ist zu berücksichtigen, daß Komplexierung der Nukleinsäure(n) stattfindet und gewährleistet ist, daß der gebildete Komplex an die Zelle gebunden, in die Zelle befördert wird und daß er entweder von selbst oder mit Hilfe des endosomolytischen Mittels aus den Endosomen freigesetzt wird.

Das jeweils gewählte Verhältnis Internalisierungsfaktor/Verbindungsfaktor/Nukleinsäure richtet sich vor allem nach der Größe der Polykationmoleküle sowie nach der Anzahl und Verteilung der positiv geladenen Gruppierungen, Kriterien, die auf Größe und Struktur der zu transportierenden Nukleinsäure(n) abgestimmt werden. Vorzugsweise beträgt das molare Verhältnis Internalisierungsfaktor:Nukleinsäure-affine Substanz ca. 10:1 bis ca. 1:10.

Nach Konstruktion und Synthese der Konjugate und der Bestimmung des für die Effizienz der Transfektion optimalen Verhältnisses Konjugat(e):DNA kann mit Hilfe von Titrationen die Menge des Internalisierungsfaktor-Konjugat-Anteils bestimmt werden, der gegebenenfalls durch freie Nukleinsäure-affine Substanz ersetzbar ist. Im Falle der Verwendung von Polykationen sowohl als Verbindungsfaktor als auch als freie Nukleinsäure-affine Substanz können die Polykationen gleich oder verschieden sein.

Für die Ausführungsform der Erfindung, bei der virale Konjugate zur Anwendung kommen, besteht eine geeignete Methode zur Bestimmung des Verhältnisses der in den Komplexen enthaltenen Komponenten darin, zuerst das in die Zelle zu importierende Genkonstrukt zu definieren und, wie oben beschrieben, ein Virus oder eine Viruskomponente zu finden, das bzw. die für die spezielle Transfektion geeignet ist. Dann wird das Virus oder die Viruskomponente an das Polykation gebunden und mit dem Genkonstrukt komplexiert. Ausgehend von einer definierten Menge an viralem Konjugat können Titrationen durchgeführt werden, indem die Zielzellen mit dieser (konstanten) Konjugatmenge und abnehmenden DNA-Konzentrationen behandelt werden, oder umgekehrt. Auf diese Weise wird das optimale Verhältnis DNA:Virus-Konjugat ermittelt. Bei Verwendung eines zusätzlichen Internalisierungsfaktors wird z.B. so vorgegangen, daß ausgehend von einer konstanten DNA-Menge durch Titrationen das optimale Verhältnis zwischen Virus-Konjugat und Internalisierungsfaktor-Konjugat bestimmt wird.

Die Komplexe können hergestellt werden, indem die Komponenten i) Nukleinsäure, ii) virales Konjugat, gegebenenfalls iii) Internalisierungsfaktor/Verbindungsfaktor-Konjugat und gegebenenfalls iv) nicht-kovalent gebundene Nukleinsäure-affine Substanz, die jeweils in Form verdünnter Lösungen vorliegen, gemischt werden. Im Falle der Verwendung von Polykationen als Verbindungsfaktor und gleichzeitig als "freie" Polykationen ist es im allgemeinen zweckmäßig, zuerst eine Mischung von Konjugaten mit freien Polykationen herzustellen und diese Mischung mit DNA zu vereinigen. Dabei wird das optimale Verhältnis von DNA zu Konjugat(en) und Polykationen durch Titrationsexperimente, d.h. in einer Reihe von Transfektionsexperimenten mit konstanter DNA-Menge und zunehmender Menge an Konjugat(en)/Polykationenmischung, bestimmt. Das optimale Verhältnis von Konjugat(en):Polykationen in der Mischung kann mit Hilfe von Routineexperimenten bzw. aus dem Vergleich der optimalen Verhältnisse der in den Titrationsexperimenten eingesetzten Mischungen erhalten werden.

Die Herstellung der DNA-Komplexe kann bei physiologischen Salzkonzentrationen erfolgen. Eine weitere Möglichkeit besteht im Einsatz hoher Salzkonzentrationen (etwa 2 M NaCl) und anschließende Einstellung auf physiologische Bedingungen durch langsames Verdünnen bzw. Dialyse.

Die jeweils am besten geeignete Reihenfolge für die Mischung der Komponenten Nukleinsäure, Konjugat(e), gegebenenfalls nicht-kovalent gebundene freie Nukleinsäure-affine Substanz wird im einzelnen in Vorversuchen bestimmt. Fallweise kann es sich als zweckmäßig erweisen, zuerst die Nukleinsäure mit dem Konjugat zu komplexieren und erst dann die freie Nukleinsäure-affine Substanz, z.B. das Polykation zuzufügen, z.B. im Fall von Transferrin-Ethidiumdimer-Konjugaten und Polylysin.

In einer bevorzugten Ausführungsform der Erfindung ist der (zusätzliche) Internalisierungsfaktor Transferrin und der Verbindungsfaktor ein Polykation. Unter "Transferrin" sind sowohl die natürlichen Transferrine zu verstehen als auch diejenigen Transferrin-Modifikationen, die vom Rezeptor gebunden und in die Zelle transportiert werden.
Die Aufnahme der Nukleinsäure erfolgt in Form von Komplexen, in denen Internalisierungsfaktor-Polykation-Konjugate mit Nukleinsäure komplexiert sind. Im Falle des Gehalts an nicht-kovalent gebundener Nukleinsäure-affiner Substanz ist diese bevorzugt ein Polykation, das gleich oder verschieden ist von dem im Konjugat enthaltenen Polykation.

Im Fall von Kombinations-Komplexen wird die Nukleinsäure in Form von Komplexen internalisiert, in denen einerseits die Internalisierungsfaktor-Konjugate und andererseits die endosomolytischen mit Nukleinsäure komplexiert sind.

Die Internalisierungsfaktor-Polykation-Konjugate, die gemeinsam mit freiem Virus oder mit den viralen Konjugaten in den Kombinations-Komplexen zur Anwendung kommen, können auf chemischem oder, falls das Polykation ein Polypeptid ist, auf rekombinantem Weg hergestellt werden, bezüglich der Herstellungsmethoden wird auf die Offenbarung der EP 388 758 Bezug genommen.

Die Konjugate können auch hergestellt werden, indem ein Glykoprotein, z.B. Transferrin, und der Verbindungfaktor über eine oder mehrere Kohlenhydratketten des Glykoproteins miteinander verbunden sind. Solche Konjugate sind im Gegensatz zu den mittels herkömmlicher Kopplungsverfahren hergestellten Konjugaten frei von Modifikationen, die von den verwendeten Linkersubstanzen stammen.
Diese Konjugate weisen im Falle von Glykoproteinen, die nur eine oder wenige für die Kopplung geeignete Kohlenhydratgruppen aufweisen, z.B. Transferrin, außerdem den Vorteil auf, daß sie hinsichtlich ihrer Bindungsstelle Glykoprotein/Verbindungsfaktor exakt definiert sind.

Die Menge an eingesetztem endosomolytischem Mittel bzw. dessen Konzentration richtet sich nach der im einzelnen vorgenommenen Transfektion. Es ist zweckmäßig, die Mindestmenge an Virus bzw. Viruskomponente einzusetzen, die erforderlich ist, um die Internalisierung von Virus und Nukleinsäure-Komplex und die Freisetzung aus den Endosomen zu gewährleisten. Die Menge an Virus(konjugat) wird auf den jeweiligen Zelltyp abgestimmt, dabei ist vor allem die Infektiosität des Virus für diesen Zelltyp zu berücksichtigen. Ein weiteres Kriterium ist das jeweilige Internalisierungsfaktor-Verbindungsfaktor-Konjugat, insbesondere hinsichtlich des Internalisierungsfaktors, für den die Zielzelle eine definierte Anzahl von Rezeptoren aufweist. Außerdem richtet sich die Menge an Virus(konjugat) nach der Menge der zu importierenden DNA. Für eine stabile Transfektion, für die nur eine geringe DNA-Menge erforderlich ist, ist im allgemeinen eine kleine Menge an Virus ausreichend, während für eine transiente Transfektion, für die größere DNA-Mengen erforderlich sind, die eingesetzte Virusmenge größer ist. Für eine spezielle Anwendung wird in Vorversuchen mit den jeweils für die Transfektion vorgesehenen Zielzellen, gegebenenfalls mit einer gemischten Zellpopulation, und dem für die Transfektion vorgesehenen Vektorsystem die optimale Viruskonzentration durch Titrieren ermittelt, wobei zweckmäßigerweise als DNA ein Genkonstrukt eingesetzt wird, das hinsichtlich der Größe mit dem für die konkrete Anwendung vorgesehenen weitgehend übereinstimmt und das zwecks einfacherer Messung der Effizienz des Gentransfers ein Reportergen enthält. Im Rahmen der vorliegenden Erfindung wurde die Eignung des Luciferase- und des β-Galaktosidasegens als Reportergen für derartige Versuche gezeigt.

Die Erfindung betrifft in einem weiteren Aspekt ein Verfahren zum Einbringen von Komplexen aus Nukleinsäure, einer Nukleinsäure-bindenden Substanz und gegebenenfalls einem Internalisierungsfaktor, in höhere eukaryotische Zellen. Das Verfahren ist dadurch gekennzeichnet, daß die Zellen mit einem Mittel in Kontakt gebracht werden, das die Fähigkeit aufweist, entweder von sich aus oder als Bestandteil der Nukleinsäure-Komplexe in die Zellen aufgenommen zu werden und den Inhalt der Endosomomen, in denen die Nukleinsäure-Komplexe nach Eintritt in die Zelle lokalisiert sind, ins Zytoplasma freizusetzen.

Im allgemeinen wird eine gleichzeitige Verabreichung von endosomolytischem Mittel und Nukleinsäure-Komplex bevorzugt, die Verabreichung kann jedoch auch nacheinander erfolgen, wobei im Fall der getrennten Anwendung die Reihenfolge nicht kritisch ist, solange die Verabreichung kurz hintereinander erfolgt, um einen möglichst gleichzeitigen Kontakt der Zelle mit den Komponenten zu gewährleisten. Im Falle der Verwendung von freiem Virus in einer gesonderten Zubereitung, kann die gleichzeitige Verabreichung der Viruspräparation mit den Komplexen dadurch gewährleistet werden, daß die Viruspräparation Bestandteil des Transfektionsmediums ist, das den Nukleinsäure-Komplex enthält. Im Falle der gleichzeitigen Verabrechung von freiem Virus werden die Nukleinsäure-Komplexe und die Viruszubereitung vor der Anwendung gemischt.

In einer bevorzugten Ausführungsform des erfindungsgemäßen Verfahrens wird das endosomolytische Mittel als Bestandteil eines Kombinations-Komplexes eingesetzt.

Um die Genexpression zu verstärken, können die erfindungsgemäßen Zusammensetzungen auch wiederholt angewendet werden.

In einer bevorzugten Ausführungsform sind die Zellen primäre Tumorzellen. In einer besonders bevorzugten Ausführungsform ist die Nukleinsäure eine DNA, die eine oder mehrere Sequenzen enthält, die für eine immunmodulierende Substanz, vorzugsweise ein Zytokin, kodieren.

In einer weiteren Ausführungsform sind die Zellen Myoblasten, vorzugsweise primäre Myoblasten.

In einer weiteren Ausführungsform sind die Zellen Fibroblasten, vorzugsweise primäre Fibroblasten.

In einer weiteren Ausführungsform sind die Zellen Hepatozyten, vorzugsweise primäre Hepatozyten.

In einer weiteren Ausführungsform sind die Zellen primäre Endothelzellen.

In einer weiteren Ausführungsform sind die Zellen primäre Atemwegsepithelzellen.

In einer weiteren Ausführungsform sind die Zellen T-Zellen.

In einer weiteren Ausführungsform sind die Zellen B-Zellen.

Tabelle 1 zeigt die erfolgreiche Transfektion mit Hilfe der vorliegenden Erfindung am Beispiel verschiedener Zelltypen.

Die erfindungsgemäße Zusammensetzung wurde auch bei der Transfektion von Hunde-Hämophilie B-Fibroblasten untersucht. Sowohl Luciferase als auch β-Galaktosidase konnten in diesen Fällen erfolgreich exprimiert werden. Außerdem wurde das System verwendet, um die 1.4 Hunde-Faktor IX cDNA in diese Fibroblasten einzubringen. Mittels Sandwich-ELISA konnte Faktor IX 24 Stunden nach der Transfektion nachgewiesen werden.

In bestimmten Fällen ist es zweckmäßig, zusätzlich zum endosomolytischen Mittel eine lysosomatrope Substanz zu verwenden, z.B. wenn das endosomolytische Mittel ein Peptidkonjugat oder ein Retrovirus ist, deren endosomolytische Aktivität nicht strikt pH-Wertabhängig ist.

Es ist bekannt, daß lysosomatrope Substanzen die Aktivität von Proteasen und Nukleasen hemmen und somit den Abbau von Nukleinsäuren inhibieren können (Luthmann und Magnusson, 1983). Zu diesen Substanzen zählen Chloroquin, Monensin, Nigericin und Methylamin. Es konnte gezeigt werden, daß Monensin eine Steigerung der Reportergenexpression im Falle der Anwendung von Moloneyvirus hervorruft. Es konnte gezeigt werden, daß die Gegenwart von Chloroquin in praktisch 100 % von K562-Zellen zur Expression eines Reportergens führt, das durch Transferrin vermittelten DNA-Transfer in die Zellen eingebracht wurde. BNL.CL2 oder HepG2-Hepatozyten reagierten nicht so gut auf Chloroquin wie die K562-Zellen, konnten jedoch in einem Ausmaß von 5 bis 10 % transfiziert werden, wenn die endosomolytischen Eigenschaften von zugegebenem replikationsdefektem oder chemisch inaktiviertem Adenovirus ausgenutzt werden.

Mit Hilfe der vorliegenden Erfindung werden die Vorteile der biologischen Vektoren verstärkt. Aufgrund der Verteilung der Rezeptoren gibt es einen Tropismus sowohl für Internalisierungsfaktor als auch für das Virus. Durch Abstimmung dieser beiden Komponenten auf die jeweilige Zellpopulation ist eine erhöhte Selektivität erreichbar, die vor allem bei der therapeutischen Anwendung der vorliegenden Erfindung zum Tragen kommt. Diesem Aspekt kommt besondere Bedeutung bei der therapeutischen Anwendung der vorliegenden Erfindung in der Lunge zu, weil die unterschiedlichen Zellpopulationen in der Lunge unterschiedliche Rezeptoren aufweisen, was die Konstruktion von Vektoren mit hoher Bindungsaffinität für eine bestimmte Zellpopulation, z.B. für die bewimperten Zellen der Atemwege, erfordern kann. Als Liganden kommen z.B. Lectine in Betracht. Die Konstruktion solcher Konjugate erfordert u.a. die Bestätigung der Bindungseigenschaften eines Liganden-Kandidaten in der Konjugat-Konformation. Diese Bestätigung kann z.B. mit Antikörpern gegen den Liganden mit Hilfe von immunhistochemischen Färbemethoden in dem Gewebe, in dem die therapeutische Anwendung erfolgen soll, durchgeführt werden.

Die vorliegende Erfindung betrifft in einem weiteren Aspekt pharmazeutische Zusammensetzungen, die als aktiven Bestandteil einen Komplex therapeutisch wirksamer Nukleinsäure, vorzugsweise als Teil eines Genkonstruktes, sowie ein endosomolytisches Mittel, das gegebenenfalls konjugiert ist, und gegebenenfalls ein Internalisierungsfaktor-Konjugat enthalten. Jeder inerte pharmazeutisch annehmbarer Träger kann verwendet werden, z.B. Kochsalzlösung oder phosphatgepufferte Kochsalzlösung oder jeder Träger, in dem die DNA-Komplexe geeignete Löslichkeitseigenschaften haben, um im Rahmen der vorliegenden Erfindung angewendet zu werden. Bezüglich Methoden zur Formulierung pharmazeutischer Zubereitungen wird auf Remington's Pharmaceutical Sciences, 1980, Bezug genommen.

Die vorliegende Erfindung bietet den Vorteil, größtmöglicher Flexibilität bei der Anwendung, u.a. als pharmazeutische Zubereitung. Die erfindungsgemäße Zusammensetzung kann als Lyophilisat oder in einem geeigneten Puffer in tiefgefrorenem Zustand vorliegen. Sie kann auch als gebrauchsfertiges Reagens in Lösung, vorzugsweise gekühlt transportiert und gelagert, bereitgestellt werden. Gegebenenfalls liegen die für die Transfektion erforderlichen Komponenten, nämlich DNA, endosomolytisches Mittel, gegebenenfalls konjugiert oder bereit für die Konjugation mit einem getrennten Konjugationspartner, DNA-bindende Substanz, gegebenenfalls konjugiert mit einem Internalisierungsfaktor, gegebenenfalls freies Polykation, in einem geeigneten Puffer getrennt oder teilweise getrennt als Bestandteile eines Transfektionskits vor, der ebenfalls Gegenstand der vorliegenden Erfindung ist. Der Transfektionskit der vorliegenden Erfindung umfaßt einen Träger, der einen oder mehrere Behälter, wie Röhrchen, Fläschchen oder ähnliches enthält, die die für die Transfektion der höheren eukaryotischen Zelle entsprechend der vorliegenden Erfindung erforderlichen Materialien enthalten. In einem solchen Transfektionskit kann ein erster Behälter ein oder mehrere verschiedene DNAs, z.B. kodierend für verschiedene Antigene, enthalten. Ein zweiter Behälter kann ein oder mehrere verschiedene Internalisierungsfaktor-Konjugate enthalten, wobei die Anwendung des Transfektionskits in Form eines Baukastensystems ermöglicht wird. Ob die Bestandteile als gebrauchsfertige Zubereitung oder getrennt vorliegen, um unmittelbar vor der Verwendung gemischt zu werden, hängt neben der spezifischen Anwendung von der Stabilität der Komplexe ab, die routinemäßig in Stabilitätstests untersucht werden kann. In einer bevorzugten Ausführungsform befindet sich ein Transglutaminase-gekoppeltes Adenovirus-Polylysin-Konjugat, das sich als lagerstabil erwiesen hat, in einem der Behälter eines Kits. In einer weiteren bevorzugten Ausführungsform liegen biotinyliertes Adenovirus und Streptavidin-Polylysin in getrennten Behältern vor und werden vor der Anwendung gemischt. Unter Ausnutzung der Flexibilität der Erfindung kann der Durchschnittsfachmann zahlreiche verschiedene Transfektionskits entwickeln.

Die therapeutische Anwendung der erfindungsgemäßen Zusammensetzung als pharmazeutische Zubereitung kann systemisch erfolgen, dabei wird die intravenöse Route bevorzugt. Zielorgane für diese Anwendungsform sind z.B. Leber, Milz, Lunge, Knochenmark, sowie Tumore.

Beispiele für die lokale Anwendung sind das Lungengewebe (Anwendung der erfindungsgemäßen Zusammensetzung als Flüssigkeit zur Instillation oder als Aerosol zur Inhalation). Neben einer hohen Spezifität des Liganden für die differenzierten Lungenzellen kann es dabei als Begleitmaßnahme erforderlich sein, verschiedene Faktoren, die in der Umgebung des Lungengewebes vorhanden sind und den Gentransfer beeinträchtigen könnten, zu beeinflussen (z.B. Lähmung der Wimpernbewegung, Auflösung des Bronchialmucus, Anwendung von Proteaseinhibitoren). Weiters können die erfindungsgemäßen pharmazeutischen Zubereitungen durch direkte Injektion in die Leber, ins Muskelgewebe oder in einen Tumor oder durch lokale Anwendung im Gastrointestinaltakt verabreicht werden. Eine weitere Methode für die Verabreichung der pharmazeutischen Zusammensetzung ist die Applikation über das Gallengangsystem. Diese Anwendungsmethode erlaubt den direkten Zugang zu den Hepatozytenmembranen an den Gallenkanälchen, wobei die Wechselwirkung der Zusammensetzung mit Blutbestandteilen vermieden wird.

Kürzlich wurde die Möglichkeit aufgezeigt, Myoblasten (unreife Muskelzellen) zu verwenden, um Gene in die Muskelfasern von Mäusen einzubringen. Da von Myoblasten gezeigt wurde, daß sie das Genprodukt ins Blut sezernieren, kann diese Methode breitere Anwendung finden als zur Behandlung genetischer Defekte von Muskelzellen, wie z.B. der Defekt im Zusammenhang mit Muskeldystrophie. Es können somit die manipulierten Myoblasten verwendet werden, um Genprodukte zu liefern, die entweder im Blut wirken, oder vom Blut transportiert werden. Die Experimente der vorliegenden Erfindung haben gezeigt, daß sowohl Myoblasten- als auch Myotuben-Kulturen, sogar primäre, mit hoher Effizienz transfiziert werden können. Die Transfektionsmedien, die die besten Ergebnisse zeigten, enthielten Kombinations-Komplexe von biotinyliertem Adenovirus, Transferrin-Polylysin und Streptavidin-Polylysin. Außer den Reportergenprodukten Luciferase und β-Galaktosidase wurde Faktor VIII in den Muskelzellen exprimiert. Außerdem wurde das Hühneradenovirus CELO in Kombinations-Komplexen, die Weizenkeimagglutinin als zusätzlichen Internalisierungsfaktor enthielten, eingesetzt.

Die therapeutische Anwendung kann auch ex vivo erfolgen, wobei die behandelten Zellen, z.B. Knochenmarkszellen, Hepatozyten oder Myoblasten in den Körper rückgeführt werden, z.B. Ponder et al., 1991, Dhawan et al., 1991. Eine weitere ex vivo Anwendung der vorliegenden Erfindung betrifft sog. Krebsvaccine. Das Prinzip dieses therapeutischen Ansatzes besteht darin, Tumorzellen von einem Patienten zu isolieren und die Zellen mit einer für ein Zytokin kodierenden DNA zu transfizieren. In einem nächsten Schritt werden die Zellen gegebenenfalls inaktiviert, z.B. durch Strahlen, und zwar derart, daß sie sich nicht länger vermehren, aber noch immer das Zytokin exprimieren. Dann werden die genetisch veränderten Zellen dem Patienten, dem sie entnommen wurden, als Vaccineverabreicht. In der Umgebung der Impfstelle aktivieren die sezernierten Zytokine das Immunsystem, u.a. indem sie zytotoxische T-Zellen aktivieren. Diese aktivierten Zellen können ihre Wirkung in anderen Teilen des Körpers ausüben und greifen auch nichtbehandelte Tumorzellen an. Auf diese Weise wird das Risiko von Tumorrezidiven und Metastasenentwicklung verringert. Eine für die Anwendung von Krebsvaccinen für die Gentherapie geeignete Vorschrift wurde von Rosenberg et al., 1992, beschrieben. Statt der von Rosenberg vorgeschlagenen retroviralen Vektoren kann das Gentransfersystem der vorliegenden Erfindung verwendet werden. In den Versuchen der vorliegenden Erfindung wurden primäre Melanomzellen erfolgreich mit einem Reportergen transfiziert, das in Kombinations-Komplexen aus Polylysin-gekoppeltem Adenovirus und Transferrin-Polylysin oder LDL-Polylysin enthalten war.

Die vorliegende -Erfindung kann auch in Assays verwendet werden, um die Immunantwort auf ein gegebenes Antigen zu bestimmen. Antigenspezifische zytotoxische T-Lymphozyten (CTL), die infizierte Zellen töten, spielen eine wichtige Rolle bei der Abwehr gegen virale Infektionen oder Tumore durch den Wirt. Die Wechselwirkung zwischen T-Zellen und antigenpräsentierenden Zellen (APC) ist HLA-beschränkt ("Human Lyphocytic Antigens" = MHC, "Major Histocompatibility Molecules"). Um das Abtöten von Zellen, die ein Antigen exprimieren, durch CTLs in einem in vitro "CTL Killing Assay" festzustellen, muß das Antigen den CTLs im richtigen HLA-Kontext präsentiert werden, was für gewöhnlich bedeutet auf der autologen Zielzelle. Ein "CTL Killing Assay" kann wie folgt durchgeführt werden: APCs werden mit einem DNA-Konstrukt transfiziert, das eine für ein Antigen kodierende Sequenz enthält. Antigenepitope werden an MHC Klasse I Moleküle gebunden und an der Zelloberfläche als Ziel für eine spezifische CTL-Antwort präsentiert. Nach Inkubation mit einer Probe von Patientenserum werden in Abhängigkeit vom Vorhandensein spezifischer CTLs die APCs lysiert. Die Zellyse kann z.B. durch Verfolgung der Freisetzung von radioaktivem Chrom, das vor Zugabe des Serums in die APCs eingebracht wurde, gemessen werden. Etablierte Vorschriften (Walker et al., 1989) verwenden B-LCLs (B-lymphoblastoide Zellinien), die durch Transfektion mit rekombinanten Vacciniaviren zur Expression von Antigen-Genen induziert wurden. Jedoch sterben die Zellen, die das Antigen ca. einen Tag lang effizient exprimieren, aufgrund des lytischen Wirkung von Vaccinia. Diese Schwierigkeiten können mit Hilfe von "CTL Killing Assays" überwunden werden, die das Gentransfersystem der vorliegenden Erfindung verwenden, um für Antigene kodierende DNA in die Zelle einzuführen, z.B. um Konstrukte, kodierend für HIV- oder Tumorantigene, in Fibroblasten einzuführen, um diese das Antigen exprimieren zu lassen. Primäre Fibroblasten können leicht aus Biopsien gewonnen werden, sind leicht zu züchten und haben sich als besonders effizient (ca. 50 bis ca. 70 %) mit Hilfe der vorliegenden Erfindung transfizierbar erwiesen. Solche Assays sind nützlich, um, im Hinblick auf die Entwicklung von Vaccinen, Epitope zu identifizieren, die von Killerzellen erkannt werden. Außerdem können sie vorteilhafterweise angewendet werden, um die HLA-beschränkte Immunantwort einer Person gegen ein bestimmtes Antigen zu bestimmen.

Aufgrund der großen Menge, in der die mit Hilfe der vorliegenden Erfindung transportierten Gene in praktisch allen Zellen exprimiert werden, kann die Erfindung auch verwendet werden, um rekombinante Proteine zu produzieren. Auch in diesem Fall gibt es keine oder nur geringe Beschränkungen bezüglich der Sequenz bzw. der Größe der transferierten DNA, ferner gibt es ein breites Spektrum von Zelltypen, die mit der erfindungsgemäßen Zusammensetzung transfizierbar sind. Es kann daher beinahe jeder Zelltyp für die Herstellung rekombinanter Proteine verwendet werden, der gewährleistet, daß das rekombinante Protein in naturgetreuer und in vollständig modifizierter posttranslational prozessierter Form, die die hohe biologische Aktivität des Produktes sicherstellt, produziert wird.

Der Gentransfer in die Zellen kann erzielt werden, wie in den vorliegenden Beispielen anhand von Luciferase und von IFN-α gezeigt, es kann praktisch jedes Genkonstrukt, das zur Entstehung eines gewünschten Proteinproduktes führt, transportiert werden. Das gewünschte Proteinprodukt kann 24 Stunden bis 1 Woche oder länger nach der Transfektion von der Zellkultur gewonnen werden (entweder vom Zellüberstand oder von einem geeigneten Zellhomogenisat, entsprechend der Vorschrift für das jeweilige Proteinprodukt).

Die Anwendung des Gentransfersystems gemäß der vorliegenden Erfindung auf die Herstellung rekombinanter Proteine hat die folgenden Vorteile:
1) Aufgrund der hohen Transfektionseffizienz (mehr als 90 % der transfizierten Zellen können das Gen in großen Mengen exprimieren) ist keine Vorselektion von transfizierten Zellen erforderlich; ferner ist es nicht nötig, stabile Zellinien zu etablieren. Eine Zellkultur in kleinem Maßstab kann ausreichend sein, um brauchbare Mengen an Protein zu erhalten.
2) Große Genkonstrukte können transportiert werden; es konnten 48 kb erfolgreich transportiert werden.
3) Die Genexpression kann in Zellen stattfinden, die gewährleisten, daß die entsprechende posttranslationale Prozessierung und Modifikation stattfindet (z.B. Vitamin K-abhängige Carboxylierung von Gerinnungsfaktoren, siehe Armentano et al., 1990, oder zelltypspezifische Glykosylierung.
4) Durch das erfindungsgemäße System wird eine größere Auswahl von Zielzellen für die Genexpression verfügbar.

### Figurenübersicht:

- Fig. 1:: Wirkung der Adenovirus-Infektion auf den Gentransfer mittels Transferrin-PolylysinKonjugaten
- Fig. 2:: Konjugat-DNA-Komplex-Dosiseffekt
- Fig. 3:: Verstärkung des Transferrin-Polylysin-vermittelten Gentransfers durch Adenoviren erfolgt über Rezeptor-vermittelte Endozytose:
A) Wirkung auf komplexierte DNA
B) Wirkung auf Rezeptor-gebundene DNA
C) Wirkung auf Gentransfer mittels
Transferrin-Polylysin-Konjugaten
- Fig. 4:: Wirkung der Adenovirus-Infektion auf den Gentransfer mittels Transferrin-Polylysin-Konjugaten in ausgewählten Zellinien
- Fig. 5:: Untersuchung, ob die Verstärkung der Genexpression auf Ebene des Gentransports oder auf Transaktivierung beruht
- Fig. 6:: Tetra-Galaktosepeptid-Polylysin-Konjugat
- Fig. 7:: Transfektion von HepG2-Zellen mit pRSVL-DNA-Komplexen in Gegenwart von Adenovirus
- Fig. 8:: Transfektion von HepG2-Zellen mit pCMVL-DNA-Komplexen in Gegenwart von Adenovirus
- Fig. 9:: Transfektion von TIB73-Zellen mit pCMVL-DNA-Komplexen:
A) Vergleichswerte mit Chloroquin
B) in Gegenwart von Adenovirus
- Fig. 10:: Import von pCMVL-DNA in T-Zellen in Gegenwart von Adenovirus:
A) H9-Zellen
B) primäre Lymphozyten
- Fig. 11:: UV-Inaktivierung von Adenoviren:
A) Verstärkung des Gentransfer-Effekts in HeLa-Zellen durch UV-inaktivierte Viren
B) Vergleich der UV-Inaktivierung mit dem Gentransfer-Effekt
- Fig. 12:: Inaktivierung von Adenoviren durch Formaldehyd
- Fig. 13:: Transfektion von NIH3T3-Zellen mit Transferrin-Polylysin-DNA-Komplexen in Gegenwart von Moloney-Virus
- Fig. 14:: Untersuchung, ob der Gentransfer-Effekt bei der Transfektion von NIH3T3-Zellen mit Transferrin-Polylysin-DNA-Komplexen auf Moloney-Virus zurückzuführen ist
- Fig. 15:: Wechselwirkungen zwischen Transferrin und seinem Rezeptor spielen für den Gentransfer-Effekt von Moloney-Virus eine Rolle
- Fig. 16:: Einfluß des pH-Werts auf den Gentransfer-Effekt von Retroviren
- Fig. 17:: Influenza-Hämagglutinin-Peptid; Liposomen-Leakage-Assay
- Fig. 18:: Transfektion von K562-Zellen mit Transferrin-Polylysin-Konjugaten in Gegenwart von Influenzapeptid-Polylysin-Konjugat
- Fig. 19:: Transfektion von HeLa-Zellen mit Transferrin-Polylysin-Konjugaten in Gegenwart von Influenzapeptid-Polylysin-Konjugat
- Fig. 20:: *In situ* Nachweis der β-Galaktosidase-Expression nach Transfektion von HeLa-Zellen mit Transferrin-Polylysin-pCMV-β-gal-DNA in Gegenwart von Adenovirus
- Fig. 21:: In situ β-Galaktosidaseexpression in HeLa-Zellen in Gegenwart von Adenovirus
- Fig. 22:: Transfektion von Zellen mit einem 48 kb Cosmid in Gegenwart von Adenovirus. A: Hela-Zellen. B: Neuroblastomzellen
- Fig. 23:: Herstellung von Adenovirus-Polylysin durch chemische Kopplung
- Fig. 24:: Transfektion von K562-Zellen mit chemisch gekoppelten Adenovirus-Konjugaten
- Fig. 25:: Transfektion von HeLa-Zellen mit chemisch gekoppelten Adenovirus-Konjugaten
- Fig. 26:: Bindung von Polylysin an Adenovirus mittels Transglutaminase
- Fig. 27:: Transfektion von Maus-Hepatozyten mittels Transglutaminase-gekoppelten Adenovirus-Polylysin-Konjugaten
- Fig. 28:: Verstärkung der Transfektionseffizienz durch Transglutaminase-gekoppelte Adenovirus-Polylysin-Konjugate im Vergleich zu nichtgekoppeltem Virus
- Fig. 29:: Transfektion von HeLa-Zellen mit Biotin-Streptavidin-gekoppelten Adenovirus-Konjugaten
- Fig. 30:: Transfektion von K562-Zellen mit Biotin-Streptavidin-gekoppelten Adenovirus-Konjugaten
- Fig. 31:: Transfektion von Neuroblastom-Zellen mit einem 48 kb Cosmid mittels Biotin-Streptavidingekoppelten Adenovirus-Konjugaten
- Fig. 32:: Transfektion von Hepatozyten in Gegenwart von Chloroquin oder von Adenovirus
- Fig. 33:: Transfektion von K562-Zellen in Gegenwart von verschiedenen endosomolytischen Mitteln
- Fig. 34:: Vergleich der Transfektionsprotokolle auf zellulärer Ebene mit β-Galaktosidase als Reportergen in Gegenwart verschiedener endosomolytischer Mittel
- Fig. 35:: Langzeit-Luciferaseexpression in konfluenten, nicht-teilenden Hepatozyten
- Fig. 36:: Expression in HeLa-Zellen, transfiziert in Gegenwart von freiem und über Biotin-Streptavidin an Polylysin gebundenes CELO-Virus
- Fig. 37:: Transfektion von Myoblasten und Myotuben in Gegenwart von freiem und über Biotin-Streptavidin an Polylysin gebundenes Adenovirus
- Fig. 38:: Transfektion von primären Myoblasten- und Myotubenkulturen
- Fig. 39:: Vergleich von Adenovirus dl312 und CELO-Virus bei der Transfektion von HeLa-Zellen und C2C12-Myoblasten
- Fig. 40:: Verbesserung der Transfektion mit CELO-Virus durch Verwendung eines Lectin-Liganden
- Fig. 41:: Expression einer Faktor VIII cDNA in C2C12-Myoblasten- und Myotuben-Kulturen
- Fig. 42:: Verstärkung des DNA-Transports durch Adenovirus-Proteine. A: HeLa-Zellen.
B: Fibroblasten
- Fig. 43:: Galaktose-Influenzapeptid-Konjugate für den DNA Transport in Hepatozyten
- Fig. 44:: Galaktose-Adenovirus-Konjugate für den DNA Transport in Hepatozyten
- Fig. 45:: Gentransfer in B-lymphoblastoide B-Zellen
- Fig. 46:: DNA-Transfer mit Transferrin-Polylysin in Gegenwart von Rhinovirus. A: freies Rhinovirus. B: konjugiertes Rhinovirus
- Fig. 47:: Transfektion von primären humanen Melanomzellen mit Kombinations-Komplexen enthaltend Transferrin- und Adenovirus-Konjugate
- Fig. 48:: Transfektion von primären humanen Melanomzellen mit Kombinations-Komplexen enthaltend LDL- und Adenovirus-Konjugate
- Fig. 49:: Gentransfer in Atemwegsepithelzellen von Ratten in vivo
- Fig. 50:: Liposomen Leakage Assay mit amphipathischen Peptiden
- Fig. 51:: Erythrozyten Leakage Assay mit amphipathischen Peptiden
- Fig. 52:: Transfektion von BNL CL.2 Zellen in Gegenwart amphipathischer Peptide
- Fig. 53:: Transfektion von NIH3T-Zellen in Gegenwart von amphipathischen Peptiden
- Fig. 54:: Expression von IFN-α in HeLa-Zellen, transfiziert in Gegenwart verschiedener endosomolytischer Mittel

In den folgenden Beispielen, die die vorliegende Erfindung illustrieren, wurden, sofern nicht anders angegeben, die folgenden Materialien und Methoden verwendet:

### Herstellung von Transferrin-Polylysin/DNA-Komplexen

### a) Humantransferrin-Polylysin-Konjugate

Es wurde die von Wagner et al., 1991b, beschriebene Methode verwendet, bei der die Kopplung von Polylysin an die Kohlenhydratseitenketten des Transferrins erfolgt.

Eine Lösung von 280 mg (3.5 µmol) humanem Transferrin (eisenfrei, Sigma) in 6 ml 30 mM Natriumacetatpuffer, pH 5, wurde auf 0°C gekühlt und 750 µl 30 mM Natriumacetatpuffer pH 5, enthaltend 11 mg (51 µmol) Natriumperiodat hinzugefügt. Die Mischung wurde im Eisbad 90 min lang im Dunklen stehen gelassen. Zur Entfernung der niedermolekularen Produkte wurde eine Gelfiltration durchgeführt (Sephadex G-25, Pharmacia), die eine Lösung mit einem Gehalt von ca. 250 mg oxidiertem Transferrin (Messung durch Ninhydrinassay) ergab. (Um die oxidierte Form nachzuweisen, die Aldehyde enthält und bei Färbung mit Anisaldehyd eine Farbreaktion gibt, wurden die Proben auf eine Silikagel-Dünnschichtplatte getropft, getrocknet und die Platten in p-Anisaldehyd/Schwefelsäure/Ethanol (1/1/18) getaucht, getrocknet und erhitzt.) Die modifizierte Transferrin-Lösung wurde rasch (innerhalb 10 bis 15 min) einer Lösung, enthaltend 1.5 µmol fluoreszenzmarkiertes Poly(L)Lysin mit einer durchschnittlichen Kettenlänge von 190 Lysinmonomeren in 4.5 ml 100 mM Natriumacetat pH 5 hinzugefügt. Der pH-Wert der Lösung wurde durch Zusatz von 1 M Natriumbicarbonatpuffer auf pH 7.5 eingestellt. Zu der Mischung wurden in Abständen von je 1 h 4 Portionen von je 28.5 mg (450 µmol) Natriumcyanoborhydrid hinzugefügt. Nach 17 h wurden 2 ml 5 M Natriumchlorid zugefügt, um die Lösung auf eine Gesamtkonzentration von ca. 0.75 M zu bringen. Die Reaktionsmischung wurde auf eine Kationenaustauschsäule (Pharmacia Mono S HR 10/10) aufgebracht und mit einem Salzgradienten von 0.75 M bis 2.5 M Natriumchlorid mit einem konstanten Gehalt von 25 mM HEPES pH 7.3 fraktioniert. Die hohe Salzkonzentration beim Laden der Säule und ab Beginn des Gradienten war wesentlich für die Gewinnung der Polykation-Konjugate. Etwas Transferrin (ca. 30 %) zusammen mit einer schwachen Fluoreszenzaktivität eluierte im Durchfluß; die Hauptmenge von fluoreszenzmarkiertem Konjugat eluierte bei einer Salzkonzentration zwischen 1.35 M und 1.9 M und wurde in 3 Fraktionen gepoolt. Diese Fraktionen ergaben (in der Reihenfolge ihrer Elution) nach 2maliger Dialyse gegen 2 l 25 mM HEPES pH 7.3 eine Fraktion A (TfpL190A) mit einem Gehalt an 45 mg (0.56 µmol) Transferrin, modifiziert mit 366 nmol Polylysin, eine Fraktion B (TfpL190B) mit einem Gehalt von 72 mg (0.90 µmol) Transferrin, modifiziert mit 557 nmol Polylysin, und eine Fraktion C (TfpL190C), enthaltend 7 mg (85 nmol) Transferrin, modifiziert mit 225 nmol Polylysin. Die Transferrin-Konjugate wurden, sofern sie nicht sofort verwendet wurden, nach Schockgefrieren in flüssigem Stickstoff bei -20°C in eisenfreier Form gelagert. Vor dem Einbau von Eisen wurden Proben (0.5 bis 1 mg) mit Natriumchlorid auf eine physiologische Salzkonzentration (150 mM) gebracht.

Der Eiseneinbau wurde durch Hinzufügen von 4 µl 10 mM Eisen(III)citratpuffer (enthaltend 200 mM Citrat, durch Zugabe von Natriumbicarbonat auf einen pH-Wert von 7.8 eingestellt) pro mg Transferrin-Gehalt vorgenommen. Die eisenhältigen Konjugate wurden vor ihrer Verwendung für die DNA-Komplexbildung in kleine Aliquots aufgeteilt, schockgefroren in flüssigem Stickstoff oder Trockeneis/Ethanol und bei -20°C aufbewahrt. (Diese Maßnahme erwies sich als zweckmäßig, nachdem sich gezeigt hatte, daß mehrmaliges Auftauen und Einfrieren den Verderb der Konjugate zur Folge hat.)

### b) Maus-Transferrin-Polylysin-Konjugate

Es wurde analog der für Humantransferrin angegebenen Methode vorgegangen, indem die Kopplung über die Kohlenhydratseitenketten vorgenommen wurde. Aus 4.1 mg (51 nmol) Maustransferrin und 2.1 mg (34 nmol) pL290 wurden Konjugate aus 15.5 nmol Maustransferrin und 13 nmol pL290 erhalten.

### Plasmid-DNA

### a) pRSVL-DNA

6 µg des DNA-Plasmids pRSVL (enthaltend das Photinus pyralis Luciferasegen unter der Kontrolle des Rous Sarcoma Virus LTR Enhancer/Promoters (Uchida et al., 1977, De Wet et al., 1987), hergestellt mittels Triton-X Lyse Standard-Methode (Maniatis), gefolgt von CsCl/EtBr Gleichgewichtsdichtegradienten-Zentrifugation, Entfärbung mit Butanol-1 und Dialyse gegen 10 mM Tris/HCl pH 7,5, 1 mM EDTA) in 350 µl HBS (150 mM NaCl, 20 mM HEPES pH 7.3) wurden 30 min vor Zugabe zu den Zellen mit 12 µg Transferrin-Polylysin-Konjugat in 150 µl HBS gemischt.

### b) pCMV-DNA

Das Plasmid pCMV wurde hergestellt, indem das BamHI-Insert des Plasmids pSTCX556 (Severne et al., 1988) entfernt, das Plasmid mit Klenow-Fragment behandelt und das HindIII/SspI sowie Klenow-behandelte Fragment aus dem Plasmid pRSVL, das die für Luciferase kodierende Sequenz enthält, bzw. die für β-Galaktosidase (Macgregor und Caskey, 1989) eingesetzt wurde. Die Komplexbildung wurde analog pRSVL vorgenommen.

### Herstellung von Virus-Präparationen

### a) Adenovirus-Präparationen

Es wurde der von Jones und Shenk, 1979, beschriebene Adenovirus-Stamm d1312, der eine Deletion in der EIa-Region aufweist, verwendet. Die Vermehrung des Virus wurde in der EIa-trans-komplementierenden Zellinie 293 durchgeführt, wobei die Herstellung in großem Maßstab durchgeführt wurde, wie von Davidson und Hassell, 1987, beschrieben. Das gereinigte Virus wurde in Lagerpuffer (100 mM Tris, pH 8.0, 100 mM NaCl, 0.1 % BSA, 50 % Glyzerin) oder in HBS/40 % Glyzerin aufgenommen und Aliquots bei -70° C aufbewahrt. Die Bestimmung der Virion-Konzentration wurde mittels UVspektrophotometrischer Analyse der extrahierten genomischen Virus-DNA durchgeführt (Formel: eine optische Dichteeinheit (OD, A260) entspricht 10¹² Viruspartikeln/ml; (Chardonnet und Dales, 1970)).

### b) Retrovirus-Präparationen

Das Moloney-Mäuseleukämie-Retrovirus N2 wurde in eine ecotropische Verpackungslinie gepackt (Keller et al., 1985, Armentano et al., 1987). Überstände von Virus exprimierenden Zellinien wurden gesammelt, in flüssigem Stickstoff schockgefroren und bei -20°C gelagert. Die Überstände, die in den Beispielen verwendet wurden, hatten einen Titer von ca. 10⁶ cfu/ml, der mittels Neomycin-Resistenz-Kolonienbildung mit NIH3T3-Zellen gemessen wurde. Für die Viruskonzentrierungs-Experimente wurden die Überstände unter Stickstoffdruck durch ein 300 Kb Membranfilter (FILTRON) in einem AMICON-Rührzell-Konzentrator geschickt. Damit konnten normalerweise 10 - 30 ml Überstand auf das 10fache konzentriert werden.

### Zellen und Medien

HeLa-Zellen wurden in DMEM-Medium, ergänzt mit 5 % hitzeinaktiviertem fötalem Kälberserum (FCS), Penicillin zu 100 I.E./ml, Streptomycin zu 100 µg/ml und 2 mM Glutamin, gezüchtet. WI-38, MRC-5, und KB-Zellen wurden in EMEM-Medium (Eagle's modified essential medium), ergänzt mit 10 % hitzeinaktiviertem FCS, Antibiotika wie DMEM-Medium 10 mM nichtessentielle Aminosäuren und 2 mM Glutamin gezüchtet. CFT1, eine respiratorische zystische Fibrose Epithel-Zellinie (hergestellt nach der von Yankaskas et al., 1991, beschriebenen Methode; die CFT1-Zellinie ist dadurch charakterisiert, daß sie für die ΔF508-Deletion CF-Mutation homozygot ist) wurde in F12-7X-Medium gezüchtet (Willumsen et al., 1989). Für die Gentransfer-Versuche wurden die Zellen in 6 cm Zellkulturplatten gezüchtet, bis sie zu ca. 50 % konfluent waren (5 x 10⁵ Zellen). Das Medium wurde entfernt und 1 ml DMEM- oder EMEM/2 % FCS-Medium hinzugefügt. Daraufhin wurden die Konjugat-DNA-Komplexe zugegeben, unmittelbar danach das Adenovirus d1312 (0.05 - 3.2 x 10⁴ Partikel/Zelle) oder ein vergleichbares Volumen von Virus-Lagerpuffer (1 - 80 µl). Die Platten wurden für eine Stunde in den Brutschrank zurückgegeben (5 % CO₂, 37°C), danach wurden 3 ml Komplettmedium zugegeben. Nach weiteren 24 h Inkubation wurden die Zellen geerntet, um die Luciferase-Genexpression zu bestimmen. Im Falle der CFT1-Zellen wurden die Zellen vor den Gentransfer-Experimenten 4 h lang in F12-7X-Medium ohne Humantransferrin gezüchtet.

Die folgenden Zellinien wurden von ATCC, erhältlich unter den angegebenen Katalog-Nummern, bezogen:
HeLa-Zellen: CCL 2, K562-Zellen: CCL 243, HepG2-Zellen:
   HB 8065, TIB-73-Zellen: TIB 73 (BNL CL.2), NIH3T3-Zellen: CRL 1658, 293-Zellen: CRL 1573, KB-Zellen:
      CCL 17, WI-38-Zellen: CCL 75, MRC-5-Zellen: CCL 171. H9-Zellen: wurden bezogen vom AIDS Research and Reference Reagent Program, U.S. Department of Health and Human Services, Katalog-Nr. 87.

Primäre Lymphozyten wurden erhalten, indem eine 25 ml Probe von Nabelschnurblut in EDTA enthaltende Proberöhrchen aufgenommen wurde. Aliquots wurden mit 4.5 ml Ficoll-hypaque (Pharmacia) unterlegt und 15 min lang bei 2.500 Upm zentrifugiert. Die bräunliche Schicht zwischen der oberen Plasmaschicht und der klaren Ficollschicht wurde entnommen (ca. 10 ml). 40 ml IMDM plus 10 % FCS wurden beigegeben, die Probe 15 min lang bei 1.200 Upm zentrifugiert und das Zellpellet in 50 ml frischem IMDM plus 10 % FCS aufgenommen (die Zelldichte betrug ca. 2 x 10⁶ Zellen/ml). Ein 250 µl Aliquot Phytohämagglutinin (PHA P, DIFCO) wurde beigegeben, die Kultur 48 h lang bei 37°C und 5 % CO₂ inkubiert, anschließend wurde rekombinantes IL-2 (BMB) beigegeben (Konzentration: 20 Einheiten/ml). Dann wurden die Zellen mit IMDM/20 % FCS, 2 Einheiten/ml IL-2 1:3 geteilt. Aliquots der Zellen wurden in flüssigem Stickstoff in FCS plus 5 % DMSO tiefgefroren. Vor der Verwendung wurden die Zellen in IMDM plus 20 % FCS plus 2 Einheiten/ml IL-2 gezüchtet.

Für die sequentiellen Bindungsuntersuchungen wurden HeLa-Zellen bei 4°C in 1 ml DMEM, ergänzt mit 2 % FCS, equilibriert. Die Konjugat-DNA-Komplexe wurden zugegeben wie bei den anderen Versuchen und die Platten 2 h lang bei 4°C inkubiert. Dann wurden die Platten ausgiebig mit eiskaltem DMEM/2 % FCS gewaschen, anschließend 2 ml dieses Mediums zugegeben. Daraufhin wurde Adenovirus dl312 bzw. Viruspuffer aufgegeben, die Zellen langsam aufwärmen gelassen, bevor sie für weitere 24 h in den Brutschrank gegeben wurden. Nach dieser Inkubation wurden die Zellen geerntet und auf Luciferase-Genexpression untersucht.

### Luciferase-Assay

Die Herstellung von Zellextrakten, die Standardisierung des Proteingehalts sowie die Bestimmung der Luciferaseaktivität wurde durchgeführt, wie von Zenke et al., 1990, Cotten et al., 1990, bzw. in der EP 388 758 beschrieben.

### Beispiel 1

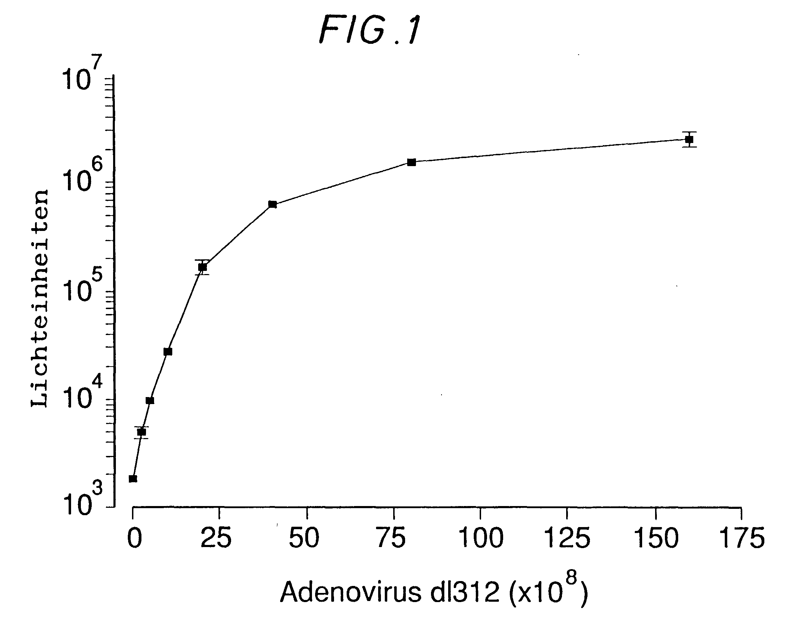

Bestimmung der Wirkung der Adenovirus-Behandlung auf den Gentransfer durch Transferrin-Polylysin-Konjugate Zunächst wurde die Wirkung einer steigenden Virus-Dosis auf die Fähigkeit einer definierten Menge Konjugat-DNA-Komplex, Gentransfer zu erzielen, untersucht. Dazu wurden für die Komplexbildung 6 µg des Plasmids pRSVL mit 12 µg Humantransferrin-Polylysin-Konjugat (hTfpL190B) gemischt. Der Konjugat-DNA-Komplex plus verschiedene Mengen des Adenovirus dl312 (0.05 - 3.2 x 10⁴ Viruspartikel/Zelle) wurden den HeLa-Zellen beigegeben. Das Ergebnis dieser Analyse ist in Fig. 1 dargestellt. Die Luciferaseaktivität ist in Lichteinheiten pro 50 µg Gesamtzellprotein ausgedrückt. Nach dieser Analyse ergaben steigende Mengen von beigefügtem Adenovirus entsprechende Zunahmen des Gentransfers. In der Figur sind die Durchschnittswerte von 2 - 4 getrennten Experimenten gezeigt; die Balken zeigen die Standardabweichung.

### Beispiel 2

### Konjugat-DNA-Komplex-Dosiseffekt

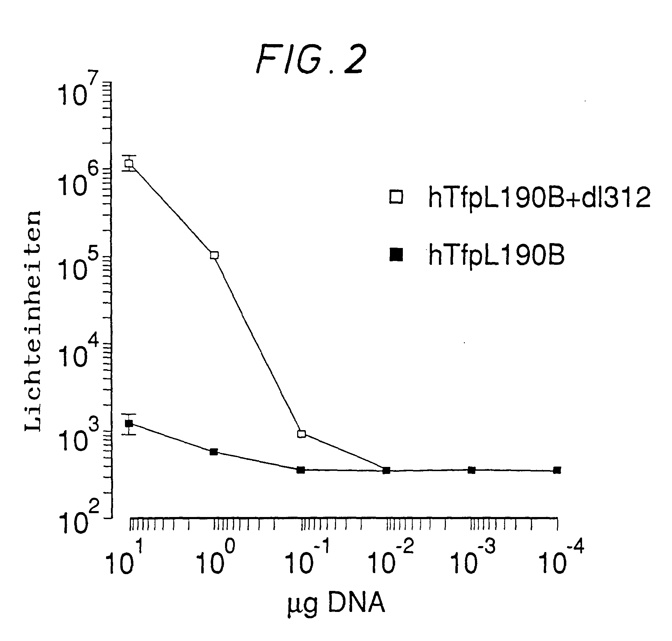

Logarithmische Verdünnungen von Konjugat-DNA-Komplexen, hergestellt wie in Beispiel 1, wurden zu HeLa-Zellen gegeben, und zwar mit oder ohne Zusatz einer konstanten Dosierung von Adenovirus d1312 (1 x 10⁴ Viruspartikel/Zelle). Die Luciferaseaktivität wurde bestimmt, wie in Beispiel 1 angegeben. Die Ergebnisse sind in Fig. 2 dargestellt.

### Beispiel 3

### Verstärkung des durch Transferrin-Polylysin bewirkten Gentransfers durch Adenoviren erfolgt über Rezeptor-vermittelte Endozytose

### a) Wirkung der Adenovirus-Behandlung auf den Transfer von komplexierter DNA

Für die Transfektion wurden folgende Komponenten eingesetzt:
6 µg pRSVL-DNA ohne Transferrin-Polylysin-Konjugat (DNA); 6 µg pRSVL-DNA plus 6 µg nicht-konjugiertes Polylysin270 (DNA + pL); 6 µg pRSVL-DNA plus 12 µg der in den vorigen Beispielen eingesetzten Transferrin-Polylysin-Konjugate (DNA + hTfpL190B). Diese Transfektionsmaterialien wurden den HeLa-Zellen mit oder ohne Adenovirus d1312 (d1312) (1 x 10⁴ Viruspartikel/Zelle) beigegeben. Die Herstellung der Zellextrakte, die Standardisierung nach Gesamtprotein und die Bestimmung der Luciferaseaktivität erfolgte wie in den vorangegangenen Beispielen. Das Ergebnis der durchgeführten Versuche ist in Fig. 3A dargestellt.

### b) Wirkung der Adenovirus-Behandlung auf den Transfer von Rezeptor-gebundener DNA

Konjugat-DNA-Komplexe (DNA + hTfpL190B) oder Polylysin-DNA-Komplexe (DNA + pL) wurden an HeLa-Zellen gebunden, ohne internalisiert zu werden, indem bei 4°C inkubiert wurde. Nicht-gebundener Komplex wurde vor der Beigabe von Adenovirus d1312 (1 x 10⁴ Viruspartikel/Zelle) oder eines vergleichbaren Puffervolumens entfernt. Die anschließende Inkubation wurde bei 37°C durchgeführt, um die Internalisierung der gebundenen DNA-Komplexe und der Adenoviren zu ermöglichen. Die Bestimmung der Luciferaseaktivität erfolgte wie angegeben (Fig. 3B).

### c) Wirkung der Adenovirus-Behandlung auf den Gentransfer durch Transferrin-Polylysin-Konjugate

Konjugat-DNA-Komplexe, enthaltend 6 µg pRSVL-DNA plus 12 µg Transferrin-Polylysin (DNA + hTfpL190B) wurden bei einer Konzentration von 1 x 10⁴ Adenovirus-Partikeln (dl312)/Zelle oder einer vergleichbaren Menge von hitzeinaktiviertem Adenovirus dl312 (d1312 h.i.) den HeLa-Zellen beigegeben. Hitzeinaktivierung wurde durch 30 min Inkubation bei 45°C vorgenommen (Defer et al., 1990).

### Beispiel 4

### Wirkung der Adenovirus-Behandlung auf den Gentransfer durch Transferrin-Polylysin-Konjugate in ausgewählten Zellinien

Konjugat-DNA-Komplexe (6 µg pRSVL + 12 µg hTfpL190B) wurden Zellen der Zellinien CFT1, KB, HeLa, WI38 und MRC5 mit oder ohne Adenovirus dl312 (1 x 10⁴ Viruspartikel/Zelle) beigegeben. Die Effizienz des Gentransfers für die verschiedenen Zellinien wurde wie in den vorangegangenen Beispielen mittels Luciferase-Assay bestimmt (Fig. 4).

### Beispiel 5

### Die Verstärkung der Luciferase-Genexpression funktioniert auf Ebene des Gen-Transports und nicht auf Transaktivierung

Es wurde zunächst eine Luciferase konstitutiv exprimierende Zellinie der Bezeichnung K562 10/6 hergestellt, indem Zellen mit einem Plasmid transfiziert wurden, das ein RSV-Luciferase-Genfragment (ein ApaI/PvuI-Fragment von pRSVL (De Wet et al., 1987)), kloniert in die ClaI-Stelle des pUCµ-Locus (Collis et al., 1990), enthielt. Dieses Plasmid wurde mit einem Transferrin-Polylysin-Konjugat komplexiert und K562-Zellen mit diesen Komplexen transfiziert, wobei nach der von Cotten et al., 1990, beschriebenen Methode vorgegangen wurde. Da das pUCµ-Locus-Plasmid ein Neomycin-Resistenzgen enthält, konnte aufgrund der Neomycin-Resistenz auf Luciferase exprimierende Klone selektioniert werden. Für die weiteren Versuche wurde ein Klon der Bezeichnung K562 10/6 ausgewählt.

Aliquots der parentalen Zellinie K562 (in 200 µl RPMI 1640 plus 2 % FCS; 500.000 Zellen/Probe) wurden entweder mit 12 µg TfpL plus 6 µg pRSVL oder mit 4 µg pL90 plus 6 µg pRSVL, jeweils in 500 µl HBS behandelt. Die angegebenen Mengen von Adenovirus dl312 (Fig. 5) wurden 1.5 h lang bei 37°C auf die Zellen einwirken gelassen, worauf 2 ml RPMI und 10 % FCS zugefügt wurden. Daraufhin wurde die Inkubation bei 37°C für weitere 24 h fortgesetzt und anschließend die Zellen für die Luciferase-Aktivitätsbestimmung vorbereitet. Es wurde gefunden, daß die Inkubation mit Adenovirus eine deutliche Zunahme in der Luciferase-Aktivität bewirkt (Fig. 5A). Dies gilt sowohl für die TfpL-Komplexe (200 Lichteinheiten gegenüber 25.000 Lichteinheiten) als auch für die pL90-Komplexe (0 gegenüber 1.9 x 10⁶ Lichteinheiten). Daraus ergibt sich, daß die K562-Zellinie die Fähigkeit aufweist, pRSVL/Polylysin-Komplexe zu internalisieren und daß diese Internalisierung, gemessen über die Luciferase-Expression, beträchtlich durch die Gegenwart von Adenovirus gesteigert wird.

Analoge Versuche wurden mit den das RSVL-Luciferase-Gen konstitutiv exprimierenden K562 10/6-Zellen durchgeführt, wobei ähnliche Mengen von Adenovirus dl312 eingesetzt wurden. Aliquots von 500.000 Zellen (in 200 µl RPMI plus 2 % FCS) wurden mit den in Fig. 5B angegebenen Mengen von Adenovirus dl312 1.5 h lang bei 37°C inkubiert. Daraufhin wurden wie bei der parentalen Zellinie RPMI plus 10 % FCS zugegeben, 24 h lang weiter inkubiert und die Luciferase-Aktivität bestimmt. Wie aus Fig. 5B ersichtlich ist, wirkte sich die Behandlung dieser Zellen mit dem Adenovirus nicht nachweisbar auf die Luciferase-Aktivität aus; die Kontrollwerte liegen im selben Bereich wie die Werte für die virusbehandelten Proben.

### Beispiel 6

### Transfektion von Leberzellen mit Asialofetuin-Polylysin-Konjugaten (AFpL) bzw. mit Tetra-Galaktosepeptid-pL-Konjugaten ((gal)4pL) in Gegenwart von Adenovirus

### a) Herstellung des lactosylierten Peptids

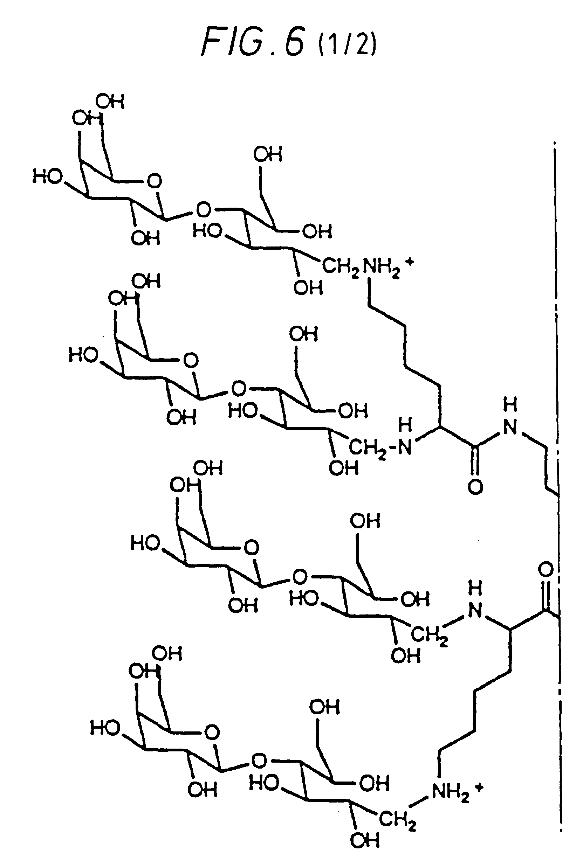

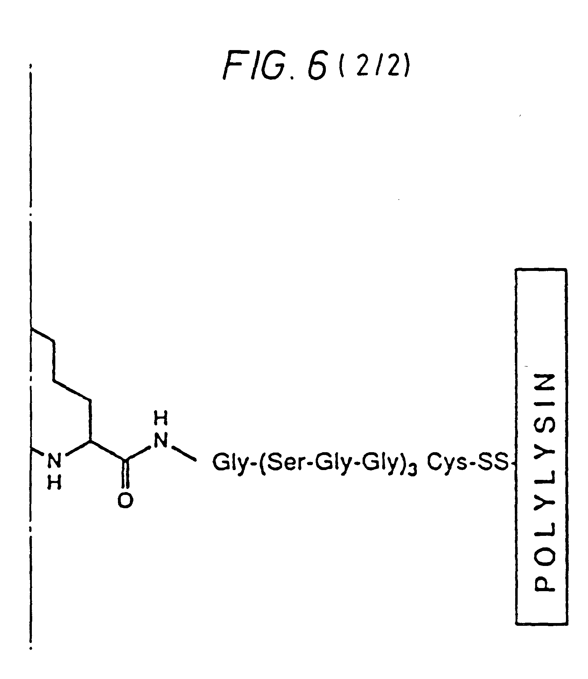

3.5 mg (1.92 µmol) des verzweigten Peptids Lys-(Nε-Lys)Lys-Gly-Ser-Gly-Gly-Ser-Gly-Gly-Ser-Gly-Gly-Cys, hergestellt mittels Fmoc-Methode unter Verwendung eines Applied Biosystems 431A Peptid-Synthesizers, enthaltend eine Dithiopyridingruppe für Cys, wurden mit einer Lösung von 7.85 mg Lactose in 40 µl 10 mM wässrigem Natriumacetat pH 5 bei 37°C behandelt. Zu der Lösung wurden 4 Portionen 0.6 mg (10 µmol) Natriumcyanoborhydrid in Intervallen von ca. 10 h gegeben. Nach insgesamt 64 h bei 37°C wurden 0.5 ml HEPES pH 7.3 und 15 mg Dithiothreitol (DTT) zugegeben. Fraktionierung mittels Gelfiltration (Sephadex G-10, 12 x 130 mm, Eluens: 20 mM NaCl) unter Argon ergab 3.6 ml Lösung von lactosyliertem Peptid in der freien Mercaptoform (1.73 µmol, entsprechend dem Ellmanns-Test; 84 % Ausbeute). Die Proben des modifizierten Peptids zeigten eine Farbreaktion mit Anisaldehyd, aber keine Farbreaktion mit Ninhydrin; dies stimmt überein mit der Annahme, daß alle 4 N-terminalen Aminogruppen lactosyliert sind. Das Tetra-Galaktosepeptid-Polylysin-Konjugat ist in Fig. 6 dargestellt.

### b) Herstellung von 3-Dithiopyridinepropionatmodifiziertem Polylysin

Zu einer gelfiltrierten Lösung von 0.60 µmol Poly-L-Lysin mit einer durchschnittlichen Kettenlänge von 290 Lysinmonomeren (pL290, Hydrobromid, Sigma) in 1.2 ml 100 mM HEPES pH 7.9 wurden unter intensivem Mischen 400 µl einer 15 mM ethanolischen Lösung von SPDP (6.0 µmol) gegeben. 1 h später wurden 500 µl 1 M Natriumacetat pH 5 zugegeben; nach Gelfiltration (Sephadex G-25) mit 100 mM Natriumacetat enthielt die Lösung 0.56 µmol pL290 mit 5.77 µmol Dithiopyridin-Linker.

### c) Konjugation des Peptids mit Polylysin

Konjugate wurden hergestellt, indem 1.5 µmol des in a) hergestellten lactosylierten Peptids in 3 ml 20 mM NaCl mit 0.146 µmol des aus b) erhaltenen modifizierten pL290 in 620 µl 100 mM Natriumacetatpuffer unter Argonatmosphäre gemischt wurden. Nach Zugabe von 100 µl 2 M HEPES pH 7.9 wurde die Reaktionsmischung 18 h lang bei Raumtemperatur stehen gelassen. Durch Zugabe von NaCl wurde die Salzkonzentration auf 0.66 M gebracht, und die Konjugate wurden durch Kationenaustauschchromatographie (Pharmacia Mono S Säule HR 5/5; Gradientenelution, Puffer A: 50 mM HEPES pH 7.3; Puffer B: Puffer A plus 3 M NaCl) isoliert. Die Produktfraktionen eluierten bei Salzkonzentrationen von ca. 1.2 M - 1.8 M und wurden in 2 Konjugatfraktionen gepoolt; die Konjugatfraktionen wurden mit (gal)4pL1 und (gal)4pL2 bezeichnet. Dialyse gegen 25 mM HEPES pH 7.3 ergab die Konjugatfraktionen (gal)4pL1, enthaltend 24 nmol modifiziertes pL290 und (gal)4pL2, enthaltend 24.5 nmol modifiziertes pL290.

### d) Herstellung von Asialofetuin-Konjugaten

Die Konjugate wurden nach demselben Prinzip hergestellt, wie die Transferrin-Konjugate; ein ähnliches Verfahren für die Herstellung von Asialoorosomucoid-Polylysin-Konjugaten wurde von Wu und Wu, 1988, beschrieben.

Die Kopplung von Asialofetuin an Polylysin wurde durch Verbindung über Disulfidbrücken nach Modifikation mit dem bifunktionellen Reagens SPDP (Pharmacia) durchgeführt. Eine Lösung von 100 mg (2.2 µmol) Asialofetuin (Sigma) in 2 ml 100 mM HEPES pH 7.9 wurde einer Gelfiltration auf einer Sephadex G-25-Säule unterworfen. Zu den daraus erhaltenen 4 ml Lösung wurden 330 µl einer 15 mM ethanolischen Lösung von SPDP (5.0 µmol) unter kräftigem Mischen beigegeben. Nach 1 h bei Raumtemperatur wurde durch eine weitere Gelfiltration (Sephadex G-25) gereinigt; daraus ergaben sich 5 ml einer Lösung von 1.4 µmol Asialofetuin, modifiziert mit 2.25 µmol Dithiopyridinlinker.

Konjugate wurden hergestellt, indem 1.4 µmol modifiziertes Asialofetuin in 5 ml 100 mM HEPES pH 7.9 mit 0.33 µmol modifiziertem pL190 (enthaltend 1.07 µmol Mercaptopropionatgruppen; dabei wurde genau wie für die Herstellung der Transferrin-Konjugate vorgegangen) in 6.5 ml 200 mM HEPES pH 7.6 unter Argonatmosphäre gemischt wurden. Die Reaktionsmischung wurde 24 h lang bei Raumtemperatur stehen gelassen. Die Konjugate wurden aus der Reaktionsmischung durch Kationenaustauschchromatographie (Pharmacia Mono S-Säule HR 10/10; Gradientenelution, Puffer A: 50 mM HEPES pH 7.9; Puffer B: Puffer A plus 3 M Natriumchlorid) isoliert und Natriumchlorid bis zu einer Endkonzentration von 0.6 M vor dem Beladen der Säule hinzugefügt. Die Produktfraktion eluierte bei einer Salzkonzentration von ca. 1.5 M. Dialyse gegen HBS ergab Konjugate, enthaltend 0.52 µmol Asialofetuin, modifiziert mit 0.24 µmol pL190.

### e) Transfektion von HepG2-Zellen mit pRSVL-DNA-Komplexen

HepG2-Zellen wurden in DMEM-Medium plus 10 % FCS 100 I.E./ml Penicillin, 100 µg/ml Streptomycin und 2 mM Glutamin in T25-Flaschen gezüchtet. Die Transfektionen wurden bei einer Dichte von 400.000 Zellen/Flasche durchgeführt. Vor der Transfektion wurden die Zellen mit 4 ml frischem Medium, enthaltend 10 % FCS, gewaschen. Unmittelbar vor der Transfektion wurde Chloroquin (Sigma) beigegeben, so daß die Endkonzentration in der Zellsuspension (plus DNA-Lösung) 100 µM betrug.

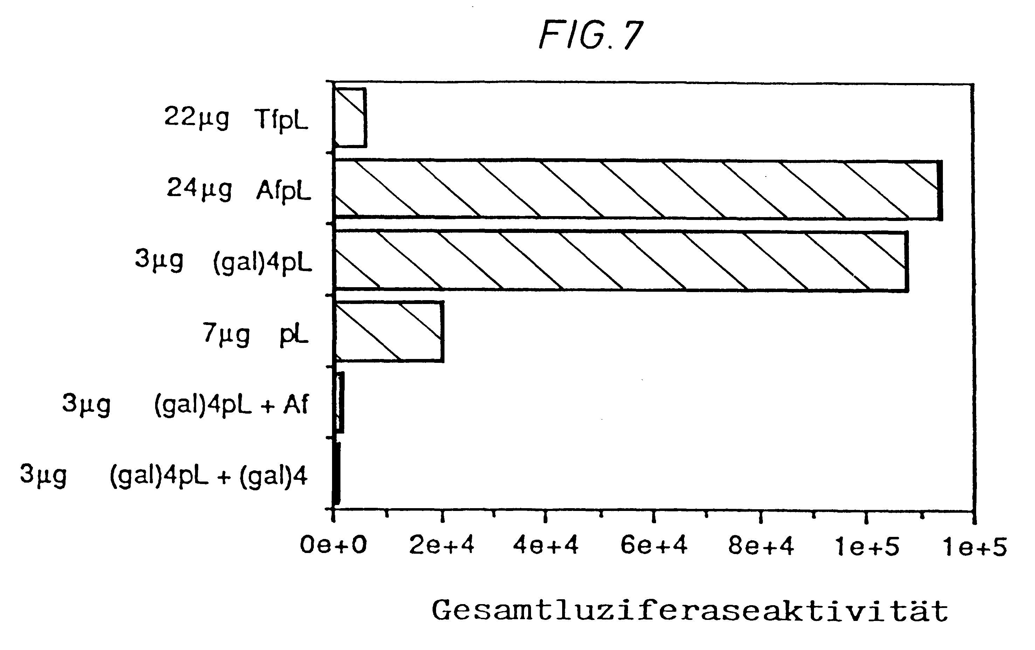

10 µg pRSVL-DNA in 330 µl HBS wurden mit den in Fig.7 angegebenen Mengen TfpL190B-Konjugat (TfpL), Asialofetuin-pL90-Konjugat (AfpL), Polylysin290 (pL) oder Tetra-Galaktose-Peptid-Polylysin-Konjugat (gal)4pL in 170 µl HBS gemischt. In den Kompetitionsexperimenten wurden nach 30 min 240 µg Asialofetuin ((gal)4pL + Af) oder 30 µg lactosyliertes Peptid ((gal)4pL + (gal)4) zugegeben. Die Mischung wurde zu den Zellen hinzugefügt; die Zellen wurden 4 h lang bei 37°C inkubiert, dann wurde das Transfektionsmedium durch 4 ml frisches DMEM-Medium plus 10 % FCS ersetzt. Nach 24 h wurden die Zellen für den Luciferase-Assay geerntet. Die in Fig. 7 dargestellten Werte stellen die Gesamtluciferaseaktivität der transfizierten Zellen dar. Wie sich aus der Figur ergibt, zeigen pL und TfpL geringe Luciferaseaktivitäten; (gal)4pL zeigt ähnlich hohe Werte wie AfpL; Zugabe von (gal)4 oder Af konkurrieren um den Asialoglykoproteinrezeptor und erniedrigen, wie erwartet, die Werte.

### f) Transfektion von HepG2-Zellen mit pCMVL-DNA-Komplexen

HepG2-Zellen wurden in 6 cm Platten bis zu einer Zelldichte von 300.000 Zellen/Platte gezüchtet, wie in e) angegeben. Vor der Transfektion wurden die Zellen mit 1 ml frischem Medium, enthaltend 2 % FCS, gewaschen.

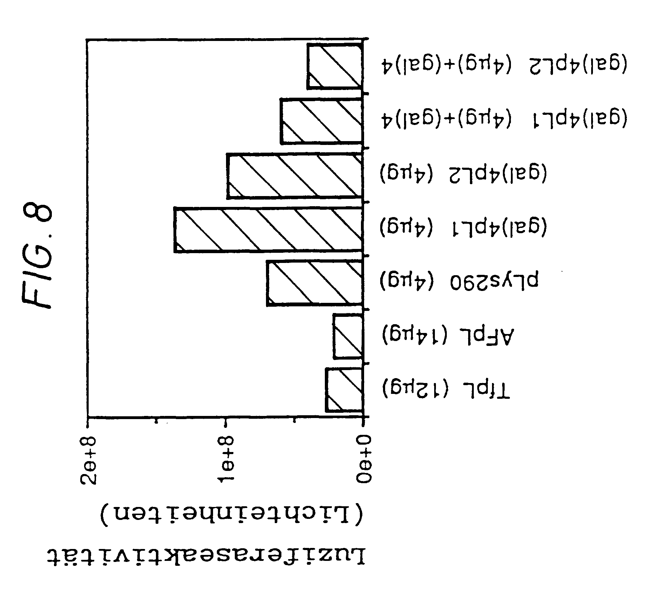

6 µg pCMVL-DNA in HBS wurden mit den in Fig. 8 angegebenen Mengen TfpL190B-Konjugat (TfpL), Asialofetuin-pL-Konjugat (AFpL), Polylysin290 (pLys290), (gal)4pL1 oder (gal)4pL2 in 170 µl HBS gemischt. Nach 30 min wurde jedem DNA-Konjugat-Komplex 1 ml DMEM, enthaltend 2 % FCS und 50 µl Adenovirus-Stammlösung d1312C, beigegeben. In den Kompetitionsexperimenten wurden, wie angegeben, 30 µg lactosyliertes Peptid (gal)4pL ((gal)4pL1 + (gal)4 bzw. (gal)4pL2 + (gal)4) beigegeben. Die Mischung wurde zu den Zellen gegeben; die Zellen wurden 2 h lang bei 37°C inkubiert, dann wurden 1.5 ml Medium, enthaltend 10 % FCS, zugegeben. 2 h später wurde das Transfektionsmedium durch 4 ml frisches DMEM-Medium plus 10 % FCS ersetzt. Nach 24 h wurden die Zellen für den Luciferase-Assay geerntet; die Werte in Fig. 8 stellen die Gesamtluciferaseaktivität der transfizierten Zellen dar. pLys290 zeigt einen Effekt, (gal)4pL zeigt einen stärkeren Effekt; Zugabe von (gal)4, das um den Asialoglykoproteinrezeptor konkurriert, reduziert die Werte auf den für Polylysin erhaltenen Wert.

### g) Transfektion von TIB73-Zellen mit pCMVL-DNA-Komplexen

Zellen der embryonischen Maus-Leberzellinie ATCC TIB73 (BNL CL.2; Patek et al., 1978) wurden bei 37°C in 5 % CO₂-Atmosphäre in "high glucose" DMEM (0.4 % Glucose), ergänzt mit 10 % hitzeinaktiviertem FCS, enthaltend 100 I.E./ml Penicillin, 100 µg/ml Streptomycin und 2 mM Glutamin, in 6 cm Platten gezüchtet.

Die Transfektionen wurden bei einer Zelldichte von 300.000 Zellen/Platte durchgeführt. Vor der Transfektion wurden die Zellen mit 1 ml frischem Medium plus 2 % FCS gewaschen.

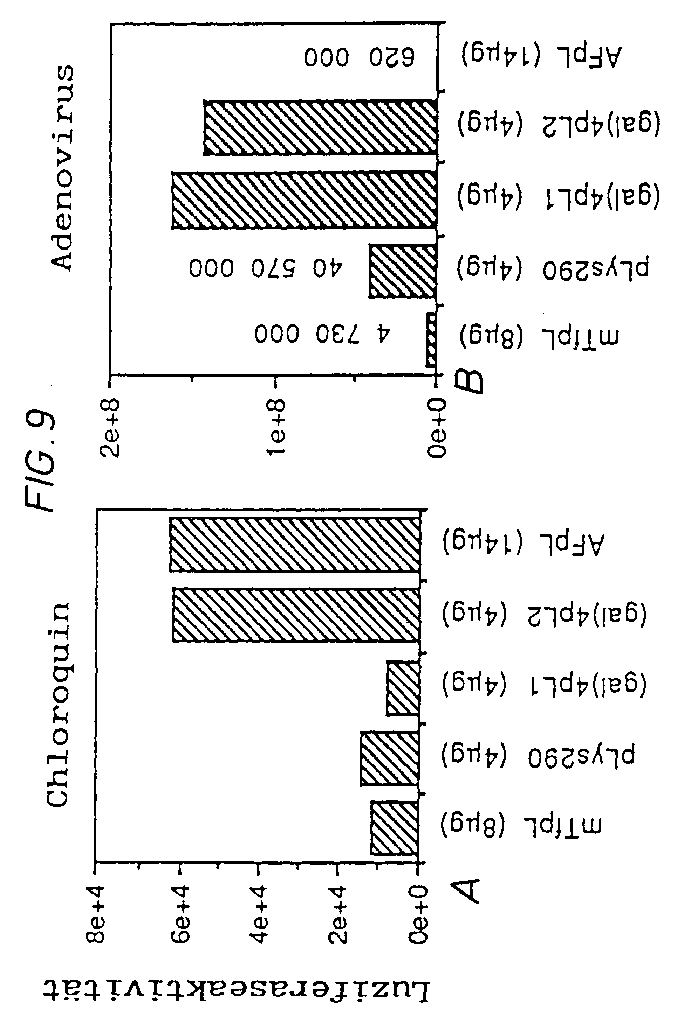

6 µg pCMVL-DNA in 300 µl HBS wurden mit den angegebenen Mengen Maustransferrin-Polylysin290-Konjugat (mTfpL), Asialofetuin-pL-Konjugaten (AFpL), Polylysin290 (pLys290), (gal)4pL1 oder (gal)4pL2 in 170 µl HBS gemischt. Nach 30 min wurde jedem DNA-Konjugat-Komplex 1 ml DMEM, enthaltend 2 % FCS und 50 µl Adenovirus-Stammlösung dl312C beigegeben. Die Mischung wurde zu den Zellen gegeben, die Zellen wurden bei 37°C 2 h inkubiert, dann wurden 1.5 ml Medium, enthaltend 10 % FCS, beigegeben. 2 h später wurde das Transfektionsmedium mit 4 ml frischem Medium ersetzt. Nach 24 h wurden die Zellen für den Luciferase-Assay geerntet; die in Fig. 9A gezeigten Werte stellen die Gesamtluciferaseaktivität der transfizierten Zellen dar.

Zum Vergleich wurde eine Transfektion ohne Adenovirus in Gegenwart von Chloroquin vorgenommen: Die Transfektionen wurden bei einer Zelldichte von 300.000 Zellen/Platte durchgeführt. Vor der Transfektion wurden die Zellen mit 1 ml frischem Medium, enthaltend 2 % FCS, gewaschen. Unmittelbar vor der Transfektion wurde Chloroquin (Sigma) beigefügt, so daß die Endkonzentrationen in der Zellsuspension (plus DNA-Lösung) 100 µM betrug. 6 µg pCMVL-DNA in 330 µl HBS wurden mit den angegebenen Mengen mTfpL, AFpL, pLys290, (gal)4pL1 oder (gal)4pL2 in 170 µl HBS gemischt. Nach 30 min wurden die DNA-Komplexe zu den Zellen gegeben. Die Zellen wurden bei 37°C 2 h lang inkubiert, dann wurden 1.5 ml Medium, enthaltend 10 % FCS und 100 µM Chloroquin, beigegeben. 2 h später wurde das Transfektionsmedium durch 4 ml frisches Medium ersetzt. Nach 24 h wurden die Zellen für die Luciferasebestimmung geerntet. Die für die Luciferase-Aktivität erhaltenen Werte sind in Fig. 9B dargestellt.

### Beispiel 7

### Import von DNA in T-Zellen

### a) Herstellung von AntiCD7-Polylysin190-Konjugaten

Eine Lösung von 1,3 mg antiCD7-Antikörper (Immunotech) in 50 mM HEPES pH 7,9 wurde mit 49 µl 1 mM ethanolischer Lösung von SPDP (Pharmacia) versetzt. Nach 1 h bei Raumtemperatur wurde über eine Gelsäule Sephadex G-25 filtriert (Eluens 50 mM HEPES-Puffer pH 7,9) wobei 1,19 mg (7,5 nmol) antiCD7, modifiziert mit 33 nmol Pyridyldithiopropionatresten, erhalten wurden. Poly(L)lysin190, Fluoreszenzmarkierung mittels FITC, wurde analog mit SPDP modifiziert und durch Behandlung mit Dithiothreitol und nachfolgender Gelfiltration in die mit freien Mercaptogruppen modifizierte Form gebracht.

Eine Lösung von 11 nmol Polylysin190, modifiziert mit 35 nmol Mercaptogruppen, in 0,2 ml 30 mM Natriumacetatpuffer wurde unter Sauerstoffausschluß mit modifiziertem antiCD7 (in 0.5 ml 300mM HEPES pH 7.9) gemischt und über Nacht bei Raumtemperatur stehen gelassen. Das Reaktionsgemisch wurde durch Zugabe von 5 M NaCl auf einen Gehalt von ungefähr 0.6 M gebracht. Die Isolierung der Konjugate erfolgte durch Ionenaustauschchromatographie (Mono S, Pharmacia, 50 mM HEPES pH 7.3, Salzgradient 0.6 M bis 3 M NaCl); nach Dialyse gegen 10 mM HEPES pH 7.3 wurden entsprechende Konjugate, bestehend aus 0.51 mg (3.2 nmol) antiCD7-Antikörper, modifiziert mit 6.2 nmol Polylysin190, erhalten.

### b) Herstellung von gp120-Polylysin 190-Konjugaten

Die Kopplung erfolgte analog literaturbekannten Methoden durch Thioether-Verknüpfung nach Modifizierung mit 6-Maleimidocapronsäure-N-hydroxysuccinimidester (EMCS, Sigma) (Fujiwara et al., 1981).

Thioether-verknüpfte gp120-Polylysin 190-Konjugate:
Eine Lösung von 2 mg rekombinantem gp120 in 0.45 ml 100 mM HEPES pH 7.9 wurde mit 17 µl einer 10 mM Lösung von EMCS in Dimethylformamid versetzt. Nach 1 h bei Raumtemperatur wurde über eine Gelsäule Sephadex G-25 filtriert (Eluens 100 mM HEPES-Puffer 7.9). Die Produktlösung (1.2 ml) wurde sogleich unter Sauerstoffausschluß umgesetzt mit einer Lösung von 9.3 nmol Polylysin 190, fluoreszenzmarkiert und modifiziert mit 30 nmol Mercaptogruppen (in 90 µl 30 mM Natriumacetat pH 5.0), und über Nacht bei Raumtemperatur stehen gelassen. Das Reaktionsgemisch wurde durch Zugabe von 5 M NaCl auf einen Gehalt von ca. 0.6 M gebracht. Die Isolierung der Konjugate erfolgte durch Ionenaustauschchromatographie (Mono S, Pharmacia, 50mM HEPES pH 7.3, Salzgradient 0.6 M bis 3 M NaCl); nach Fraktionierung und Dialyse gegen 25 mM HEPES pH 7.3 wurden drei Konjugatfraktionen A, B und C erhalten, bestehend aus 0.40 mg rgp120 modifiziert mit 1.9 nmol Polylysin 190 (im Falle der Fraktion A), bzw. 0.25 mg rgp 120 modifiziert mit 2.5 nmol Polylysin 190 (Fraktion B), bzw. 0.1 mg rgp 120 modifiziert mit 1.6 nmol Polylysin 190 (Fraktion C).

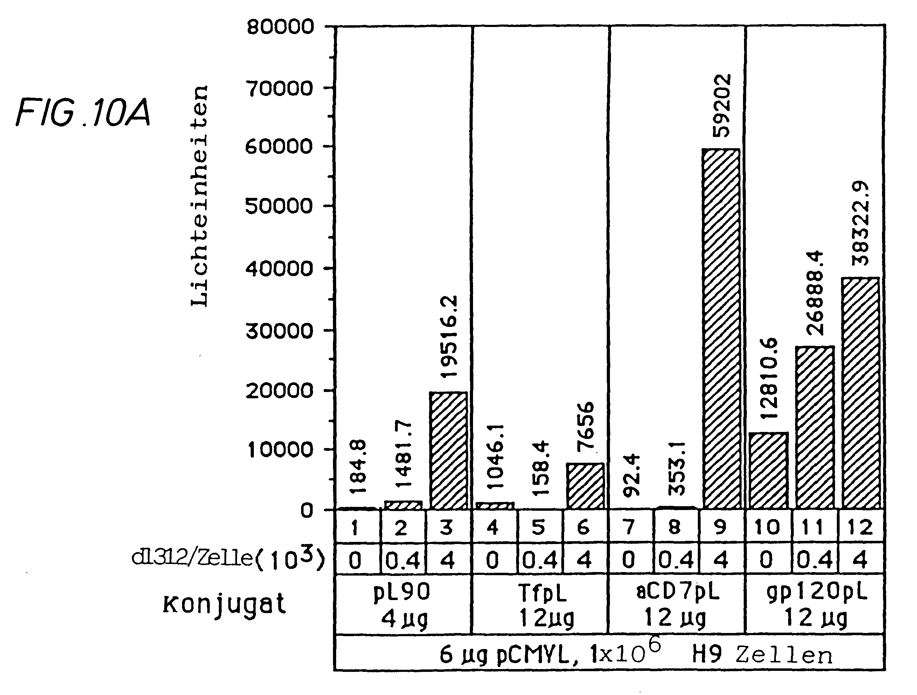

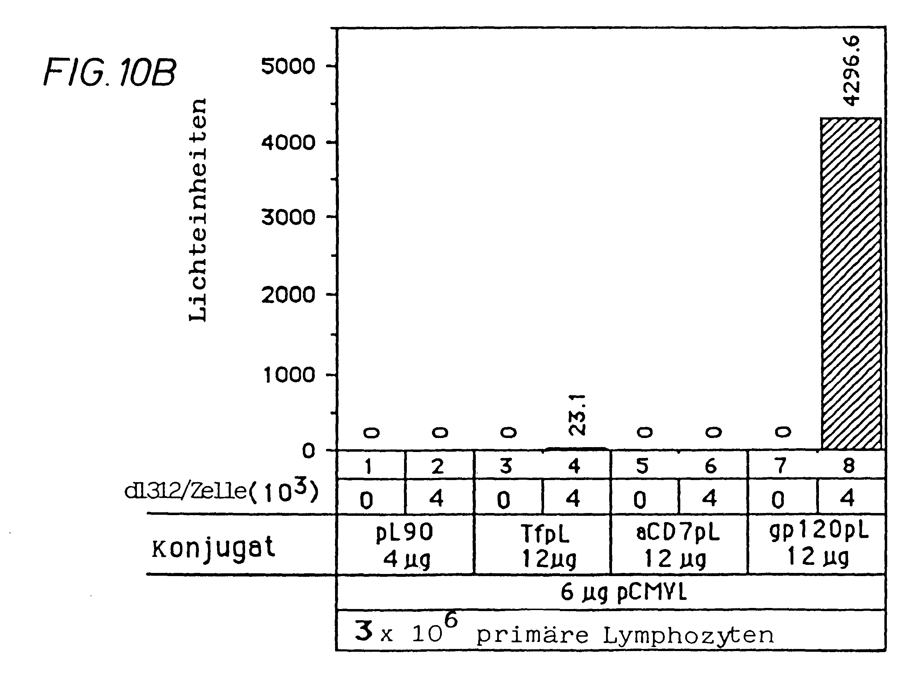

pCMVL-DNA (6 µg/Probe) wurde mit den angegebenen Mengen Polylysin90 oder den angegebenen Polylysin-Konjugaten in 500 µl HBS komplexiert. In der Zwischenzeit wurden Aliquots von H9-Zellen (10⁶ Zellen in 5 ml RPMI mit 2 % FCS) oder primären humanen Lymphozyten (3 x 10⁶-Zellen in Iscove's modifiziertem Dulbecco's Medium (IMDM) plus 2 % FCS) hergestellt. Die Polylysin-DNA-Komplexe wurden jeder Zellprobe zugefügt. 5 min später wurde die angegebene Menge Adenovirus dl312 zugegeben. Die Zellen wurden daraufhin bei 37°C 1.5 h lang inkubiert, dann wurden 15 ml RPMI (im Fall der H9-Zellen) oder IMDM (im Fall der primären Lymphozyten) plus 20 % FCS jeder Probe beigegeben. Die Zellen wurden bei 37°C 24 h lang bebrütet, geerntet und für die Bestimmung der Luciferase-Aktivität behandelt wie in den übrigen Beispielen. Das Ergebnis der durchgeführten Versuche ist in Fig. 10A (H9-Zellen) bzw. Fig.10B (primäre Lymphozyten) dargestellt: In H9-Zellen zeigten das antiCD7-Konjugat (Fig.10A, Spalten 7-9) und das gp120-Konjugat (Spalten 10-12) die besten Ergebnisse hinsichtlich des mit Adenovirus erzielten Gentransfers, wobei das gp120-Konjugat auch in Abwesenheit von Adenovirus bereits eine deutliche Expression des Luciferase-Gens erzielte. Es ist bemerkenswert, daß in den durchgeführten Versuchen nur das gp120-Konjugat in der Lage war, DNA in primäre Lymphozyten einzubringen, und dies nur in Gegenwart des defekten Adenovirus (Fig. 10B, Spalten 7 und 8).

### Beispiel 8

### Inaktivierung von Adenoviren

### a) UV-Inaktivierung

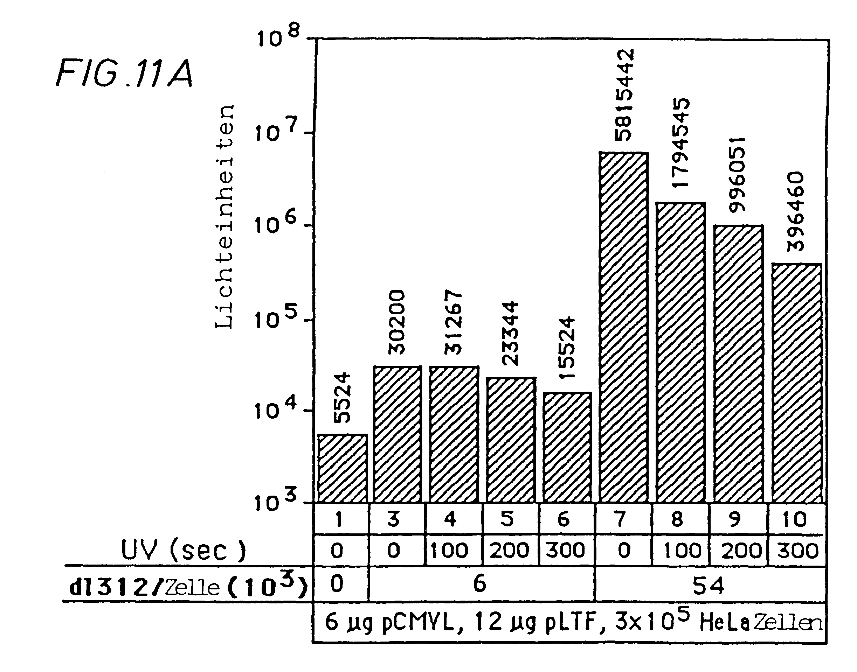

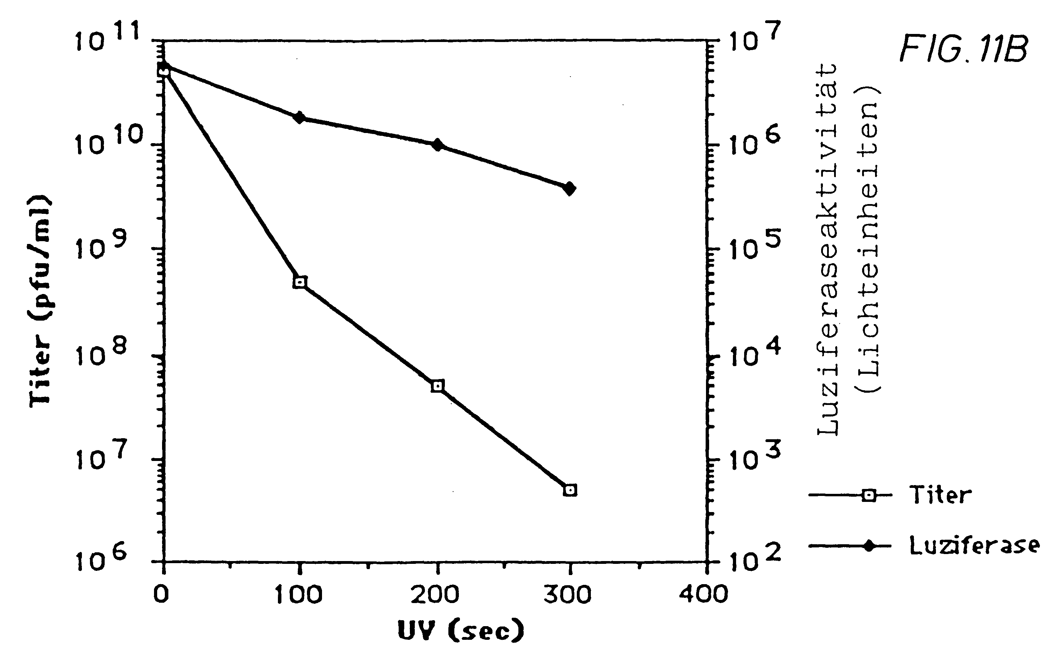

Eine Adenovirus dl312-Präparation, hergestellt und gelagert, wie in der Einleitung zu den Beispielen beschrieben, wurde in die 2 cm Vertiefungen einer Zellkulturplatte (300 pl/Vertiefung) auf Eis in 8 cm Abstand von zwei UV-Lampen (Philips TUV15 (G15 T8)-Lampen) gegeben. Das Virus wurde der UV-Bestrahlung für die Dauer der in Fig.11A angegebenen Zeiten ausgesetzt und Aliquots jeder Präparation auf ihren Virustiter sowie auf ihre Fähigkeit untersucht, ob und in welchem Umfang sie in der Lage sind, den Gentransfer mit Polylysin-Transferrin-Konjugaten in HeLa-Zellen zu verstärken.
Die Züchtung der Zellen und die Transfektion wurde im wesentlichen vorgenommen, wie oben unter "Zellen und Medien" angegeben; die für die Transfektion verwendeten Komponenten sind aus Fig. 11A ersichtlich. Die Komplexe aus pCMVL-DNA und 12 µg TfpL wurden in 500 µl HBS hergestellt und 3 x 10⁵ HeLa-Zellen (in 1 ml DMEM plus 2 % FCS) beigegeben. Ca. 5 min später wurden 54 µl jeder Viruspräparation jeder Kultur zugegeben und die Kultur bei 37°C eineinhalb bis zwei Stunden inkubiert. Daraufhin wurde ein 5 ml Aliquot DMEM plus 10 % FCS jeder Kultur beigegeben, die Inkubation bei 37°C 24 h lang fortgesetzt und die Kulturen geerntet und auf Luciferase-Aktivität untersucht. Die Menge von 54 µl von nichtbestrahltem Virus liegt nicht im Sättigungsbereich, d.h. der Test ist empfindlich auf eine mindestens 3 x höhere Virusmenge. Die erhaltenen Werte für die Luciferaseexpression sind in Fig. 11B dargestellt (geschlossene Vierecke).

Der Virustiter jeder Präparation wurde unter Verwendung der E1A komplementierenden Zellinie 293 bestimmt. Zunächst wurden Serienverdünnungen der unbestrahlten und der bestrahlten Virusproben in DMEM plus 2 % FCS hergestellt. Parallel dazu wurden Proben von 5 x 10⁴ 293-Zellen (in einer 2 cm Vertiefung) hergestellt und in 200 µl DMEM plus 2 % FCS gegeben. Ein 5 µl Aliquot jeder Verdünnung wurde zum Vergleich in jeweils eine zweite Vertiefung gegeben. Um die Bindung des Virus an die Zellen zu bewirken, wurde eineinhalb Stunden lang bei 37°C bebrütet, dann wurden 2 ml DMEM plus 10 % FCS in jede Vertiefung gegeben. 48 h später wurden die Kulturen gezählt, um den zytopathischen Effekt festzustellen. Diejenige Virusverdünnung, oberhalb derer weniger als 50 % der Zellen in der Kultur nach 48 h einen deutlichen zytopathischen Effekt zeigen, zeigt die relative Menge von infektiösem Virus in jeder Viruspräparation an. Die erhaltenen Werte sind in Fig. 11B (offene Vierecke) dargestellt. Das Ergebnis der in diesem Beispiel durchgeführten Versuche zeigt, daß ein Abfall um 4 Zehnerpotenzen im Virustiter durch UV-Bestrahlung nur eine 20fache Reduktion des Luciferase-Gentransfers bewirkt. Dies zeigt, daß Mechanismen, die für die Infektiosität des Virus maßgeblich sind, zerstört werden können, ohne die Fähigkeit des Virus zur Verstärkung des Gentransfers zu beeinträchtigen.

Es wurde beobachtet, daß bei niedrigen Virusdosen die Verstärkung des Gentransfers durch das Virus geringfügig abnahm (Fig. 11A Spalten 3-6) und daß dieser Effekt bei den hohen Dosen (Spalten 7-10) deutlicher auftrat.

### b) Inaktivierung von Adenoviren mit Formaldehyd

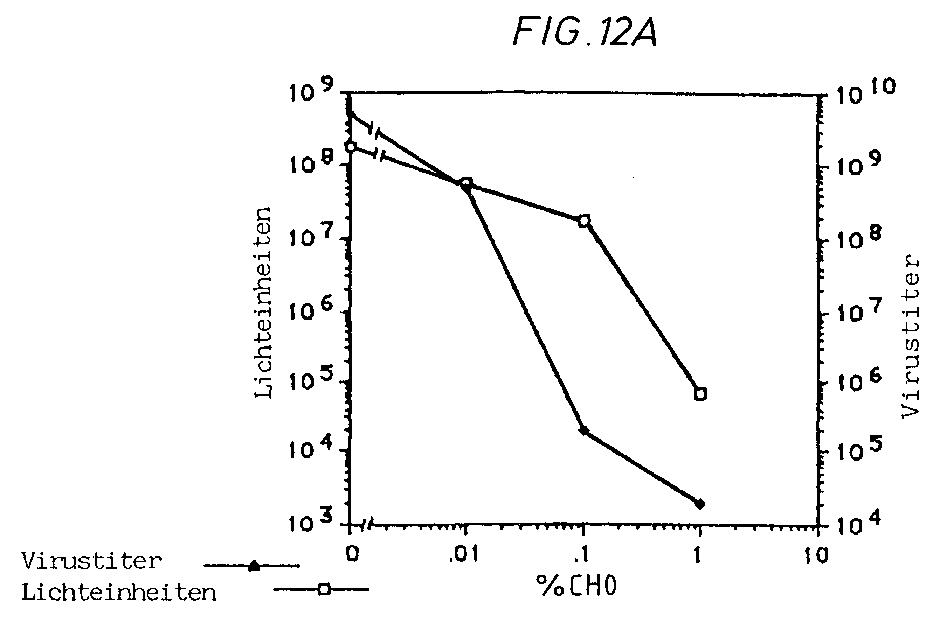

2 ml Adenovirus-Präparation wurden über eine 10 ml G25-Säule (Pharmacia PD 10 G, 25 M), vorequilibriert mit 150 mM NaCl, 25 mM HEPES pH 7.9, 10 % Glyzerin, geschickt und in einem Volumen von 2.5 ml aufgefangen. Aliquots der gelfiltrierten Viruspräparation wurden ohne (0), mit 0.01 %, 0.1 % oder 1 % Formaldehyd 20 h auf Eis inkubiert. Darauf wurde Tris pH 7.4 auf eine Konzentration von 100 mM zugegeben, dann wurden die Proben zuerst 2 h lang gegen 1 l 150 mM NaCl, 50 mM Tris pH 7.4 und 50 % Glyzerin und anschließend über Nacht gegen 2 x 1 l 150 mM NaCl, 20 mM HEPES pH 7.9 und 50 % Glyzerin dialysiert.
Aliquots des Virus wurden daraufhin auf 293-Zellen auf ihren Titer untersucht (CPE-Endpunkt-Assay oder Plaque-Assay, Precious und Russel, 1985). Dann wurde die Wirkung der Formaldehyd-behandelten Viren auf den Gentransfer in HeLa-Zellen (300.000) wie in den vorangegangenen Beispielen bestimmt, indem die Luciferase-Aktivität gemessen wurde. 90 µl der Viruspräparation bewirkten einen DNA-Transfer, der mehr als 10⁸ Lichteinheiten entsprach. Behandlung des Virus mit 0.01 % oder mit 0.1 % Formaldehyd resultierten in einer geringfügigen Abnahme der Gentransfer-Aktivität (ca. 10fache Abnahme bei 0.1 %). Obwohl die Behandlung mit 1 % Formaldehyd einen auffälligen Verlust der Gentransfer-Aktivität bewirkt, konnten 90 µl des Virus noch immer eine Genexpression entsprechend 10⁴ Lichteinheiten hervorrufen. Bei der Behandlung mit 0.1% Formaldehyd war eine Abnahme des Virustiters auf 10⁵ PFU ("Piaque forming units") mit einer Abnahme der Luciferase-Aktivität um nur 10 % gekoppelt. Das Ergebnis der durchgeführten Versuche ist in Fig. 12A dargestellt.

### c) Inaktivierung von Adenovirus mit langwelligem UV/8-Methoxypsoralen

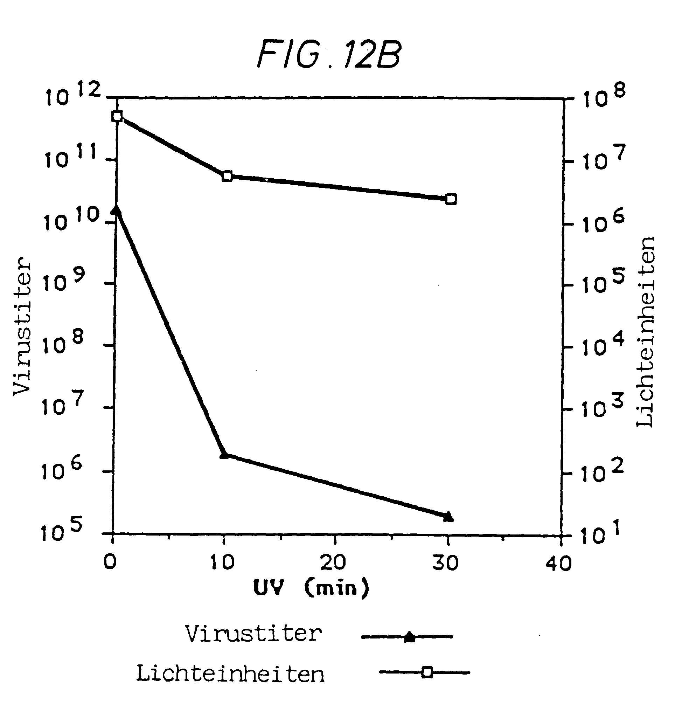

Aliquots von gereinigter Viruspräparation wurden auf eine Konzentration von 0.33 µg/ml 8-Methoxypsoralen (Stammkonzentration 33 µg/ml 8-Methoxypsoralen gelöst in DMSO) gebracht und einer 365 nm UV-Lichtquelle (UVP Modell TL-33), auf Eis in einem Abstand von 4 cm vom Lampenfilter, ausgesetzt. Die Dauer der Belichtung betrug 15 bis 30 min, wie aus Fig. 12B ersichtlich. Die Virusproben wurden dann über eine Sephadex G-25 Säule (Pharmacia, PD-10) equilibriert mit HBS + 40 % Glyzerin geschickt und bei -70°C gelagert. Die Viruspräparationen wurden einerseits auf ihre Fähigkeit, den Gentransfer mittels pCMVL/hTfpL-Komplexen in HeLa-Zellen zu verstärken, untersucht (dargestellt als Lichteinheiten, linke Achse in Fig. 12B), andererseits auf ihre Fähigkeit, in 293-Zellen zu replizieren (Virustiter, linke Achse in Fig. 12B).

### Beispiel 9

### Transfektion von NIH3T3-Zellen mit Moloney-Virus

In diesem und in den folgenden Beispielen, die die Verstärkung der Internalisierung von Transferrin-Polylysin-DNA-Komplexen mittels Retroviren zeigen, wurden, sofern nicht anders angegeben, die folgenden Materialien und Methoden verwendet:
Transferrin-Polylysinl90-Konjugate und Konjugat-DNA-Komplexe wurden analog den vorangegangenen Beispielen hergestellt, mit dem Unterschied, daß die Komplexbildungsreaktion in einem Volumen von 500 µl mM NaCl, 20 mM HEPES, pH 7.4, durchgeführt wurde.

NIH3T3-Zellen wurden in DMEM-Medium mit einem Zusatz von 10 % FCS, 100 I.E./ml Penicillin, 100 µg/ml Streptomycin und 2 mM Glutamin gezüchtet. Für die Transfektionen wurden 5 - 7 x 10⁵ Zellen pro T25-Flasche 18 bis 24 h vor der Transfektion ausplattiert. Unmittelbar vor der Transfektion wurden die Zellen in frisches Medium gegeben und die verschiedenen für die Transfektion verwendeten Komponenten in der folgenden Reihenfolge zugegeben:
Chloroquin (100 µM, wo angegeben), Polylysin-Transferrin-DNA-Komplex, Retrovirus-Präparat. Die Zellen wurden daraufhin 4 h lang bei 37°C inkubiert, dann wurden das Medium gewechselt und die Zellen 24 h später geerntet. Extrakte wurden hergestellt, indem drei Gefrier/Auftauzyklen angewendet wurden; Aliquots des Extraktes, standardisiert auf Proteingehalt, wurden auf Luciferase-Aktivität untersucht, wie für die vorangegangenen Beispiele angegeben.

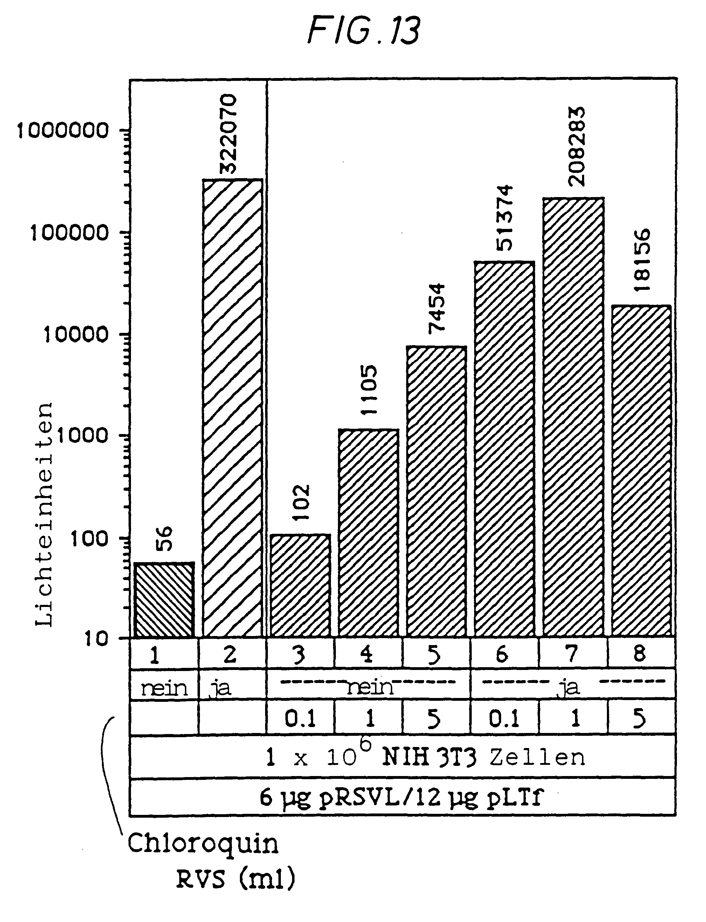

Unter den angegebenen Bedingungen wurden Transfektionen von 10⁶ NIH3T3-Zellen mit TfpL-DNA-Komplexen in Gegenwart von 100 µM Chloroquin oder ohne Chloroquin durchgeführt, wie in Fig. 13 angegeben. Es wurde festgestellt, daß ohne Chloroquin die Werte für die Luciferase-Aktivität nur Background-Niveau erreichten (Spalte 1), während in Gegenwart von Chloroquin eine hohe Expression des pRSVL-Reportergens gemessen werden konnte (Spalte 2). Steigende Mengen des Moloney-Leukämie-Virus, die gleichzeitig mit den DNA-Komplexen den Zellen beigegeben wurden, konnten eine zunehmende Erhöhung der Luciferase-Genexpression bewirken. (Die in Fig. 13 angegebenen Mengen sind ml.)

### Beispiel 10

### Untersuchung, ob die Steigerung des Gentransfers auf das Retrovirus zurückzuführen ist

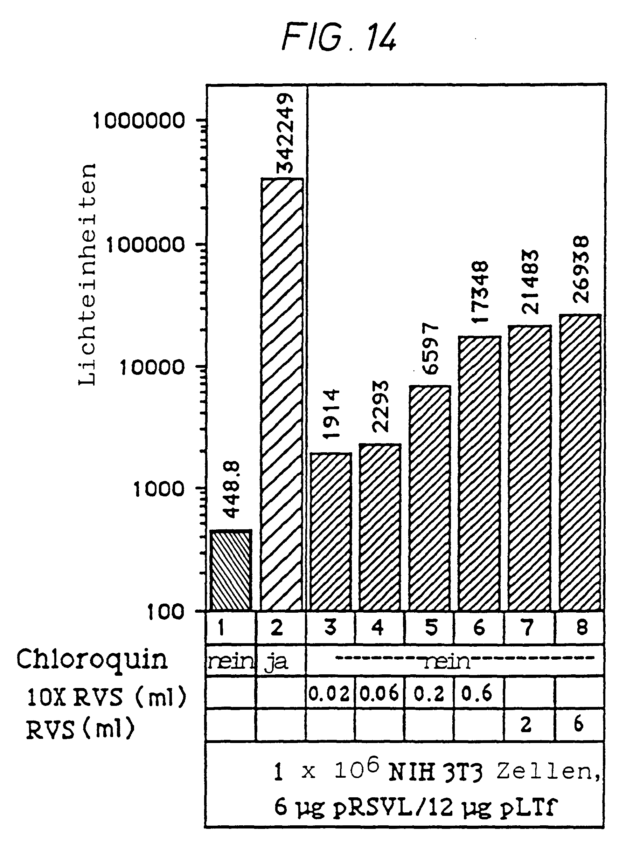

Das in Beispiel 9 verwendete Viruspräparat war ein roher, unfraktionierter Überstand von Retrovirus exprimierenden Zellen. Um den Beweis dafür zu erhalten, daß die Steigerung des DNA-Imports, die mit diesem Viruspräparat erhalten wurde, tatsächlich auf das Virus zurückzuführen war, wurde der Überstand der oben beschriebenen Dialyse/Konzentrationsreinigung unterworfen, wobei der Retrovirus-Überstand (in der Fig. mit RVS angegeben) um einen Faktor 10 konzentriert wurde. Falls das Retrovirus für die Verstärkung verantwortlich ist, sollte die im Membranrückstand gefundene Aktivität, abgesehen von einer etwaigen Inaktivierung des äußerst labilen Retrovirus während des Konzentrationsschrittes, ungefähr das 10fache des ursprünglichen Überstandes betragen. Wie im vorangegangenen Beispiel wurden 10⁶ NIH3T3-Zellen unter den in Fig. 14 angegebenen Bedingungen transfiziert. Aus Fig. 14 ist ersichtlich, daß der den Gentransfer verstärkende Effekt im Membranrückstand vorhanden ist (es wurden 20 - 600 µl verwendet, Spuren 3-6. Außerdem zeigte sich, daß 200 und 600 µl des 10fach konzentrierten Präparats ca. halb so aktiv sind, wie 2 oder 6 ml des ursprünglichen, nicht konzentrierten Retrovirus-Präparats (Spuren 7 und 8).

Parallel dazu wurden Versuche mit humanen K562-Zellen durchgeführt, die keinen Rezeptor für das ecotrope Mäuse-Retrovirus aufweisen. Wie erwartet, wurde keine Verstärkung der Genexpression erzielt.

### Beispiel 11

### Beim Gentransfereffekt von Moloneyvirus spielen Wechselwirkungen zwischen Transferrin und seinem Rezeptor eine Rolle

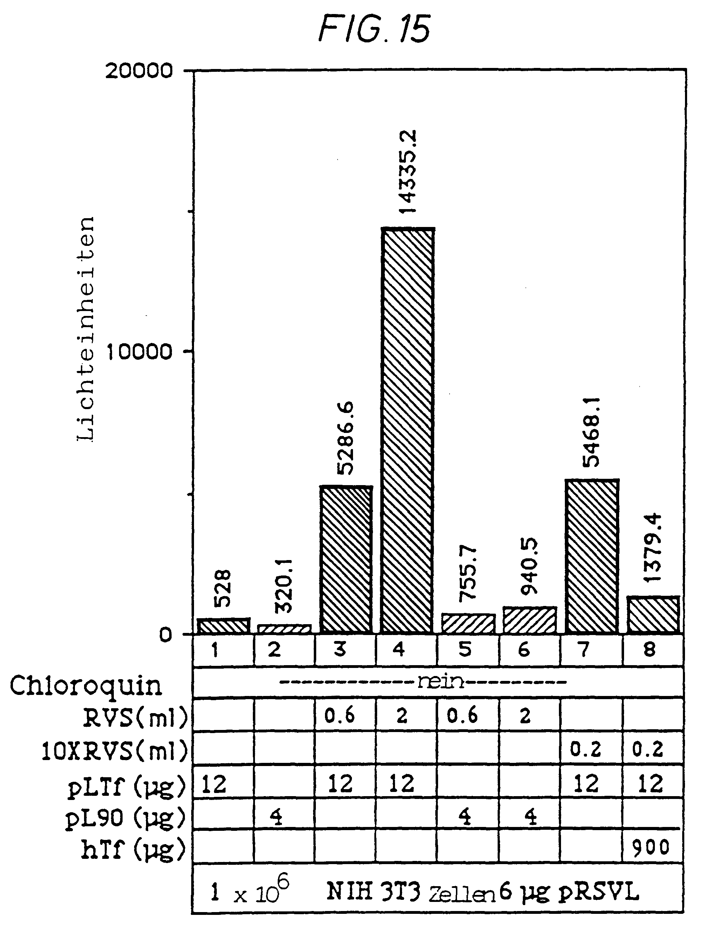

Um die Möglichkeit auszuschließen, daß der Transfer von TfpL/pRSVL-Komplexen in die Zellen auf unspezifische Bindung von Polylysin an das Retrovirus zurückzuführen ist, und um den Eintrittsmechanismus weiter aufzuklären, wurde das Retrovirus auf seine Fähigkeit untersucht, Plasmid-DNA in die Zelle zu befördern, die nur mit Polylysin komplexiert ist. Die verwendete Polylysinmenge entspricht dem früher ermittelten Optimum, das vollständige Kondensation der Plasmid-DNA bewirkt und ist ähnlich der Polylysinmenge, die mit dem Polylysin-Transferrin-Konjugat zur Anwendung kommt (Wagner et al., 1991a). Die Versuche, deren Ergebnis in Fig. 15 dargestellt ist, ergaben, daß das Reportergen bei Fehlen von Chloroquin weder in Form von TfpL-pRSVL-Komplexen noch in Form von pL-pRSVL-Komplexen exprimiert wird (Spalten 1 und 2). In Gegenwart des Retrovirus hingegen wurde die als TfpL-Komplex applizierte Reporter-DNA exprimiert, nicht jedoch in Form des pL-DNA-Komplexes (vgl. die Spalten 3 und 4 mit den Spalten 5 und 6). Weiters ergaben die durchgeführten Versuche, daß die Gegenwart von überschüssigem freiem Transferrin eine Abnahme des durch das Retrovirus erleichterten DNA-Imports bewirkte (Spalten 7 und 8). Auch diese Ergebnisse stützen die Vorstellung, daß Wechselwirkungen zwischen Transferrin und seinem Rezeptor für die Verstärkung der DNA-Aufnahme, die durch das Retrovirus bewirkt wird, eine wesentliche Rolle spielt.

### Beispiel 12

### Einfluß des pH-Werts auf den Gentransfereffekt von Retroviren

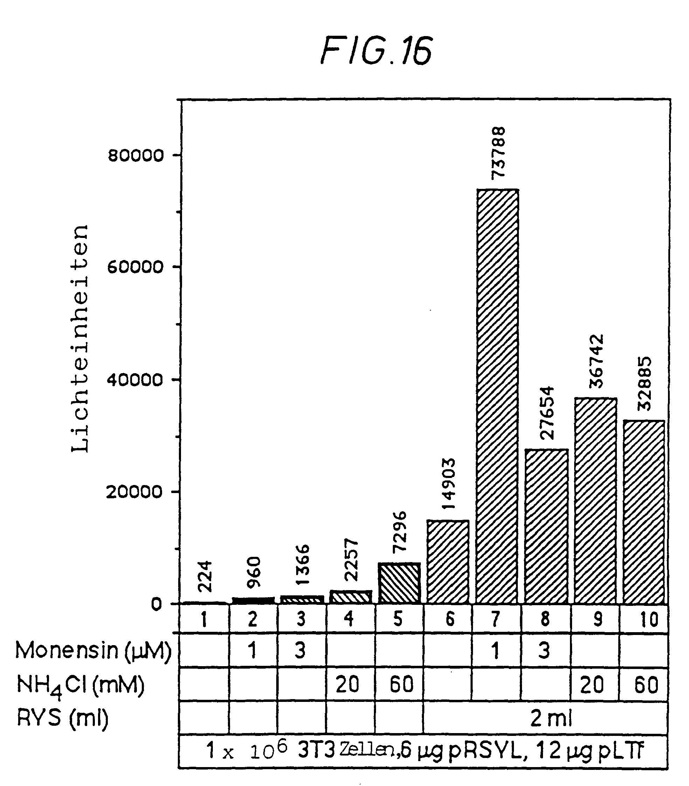

Die im Rahmen dieses Beispiels durchgeführten Experimente wurden durchgeführt, um den Einfluß des pH-Werts auf die Fähigkeit von Retroviren, den Gentransfer zu verstärken, zu untersuchen. Die Transfektionsversuche wurden wie in den vorangegangenen Beispielen vorgenommen. Um festzustellen, ob ein niedriger pH-Wert für den Gentransfer-Effekt wesentlich ist, wurden die beiden gut charakterisierten Inhibitoren der endosomalen pH-Wert-Senkung, Monensin und Ammoniumchlorid, verwendet. Es wurde von der Überlegung ausgegangen, daß diese beiden Substanzen dann den Gentransfer beeinträchtigen würden, wenn das Retrovirus für den Gentransfer-Effekt den niedrigeren pH-Wert des Endosoms benötigt. Wenn hingegen andere Mechanismen für diesen Effekt zum Tragen kommen, nämlich die direkte Fusion auf der Zytoplasmaoberfläche, ähnlich wie beim HIV-Eintrittsmechanismus, dann sollten diese Substanzen entweder keinen negativen Effekt, sondern eventuell sogar einen verstärkenden Effekt haben, wenn sie die Route der TfpL-DNA-Komplexe ändern. Die experimentellen Ergebnisse, die in Fig. 16 dargestellt sind, stützen eher letztere Hypothese. Es wurde die Wirkung der beiden Substanzen auf den TfpL-DNA-Transfer untersucht und es wurde gefunden, daß keine der beiden Substanzen Chloroquin funktionell ersetzen kann. Wenngleich eine geringe Zunahme der Luciferase-Genexpression bei höheren Ammoniumchlorid-Konzentrationen festgestellt wurde (Spalten 1-5). Das Retrovirus allein zeigt die schon in den vorangegangenen Beispielen beobachtete leichte Verstärkung des DNA-Transports (Spur 6). Eine starke Zunahme wurde beobachtet, wenn das Retrovirus in Gegenwart von 1 µM Monensin angewendet wurde (Spur 7). Ein weniger starker Effekt wurde bei einer höheren Monensin-Konzentration (Spur 8) sowie in Gegenwart von Ammoniumchlorid beobachtet (Spuren 9 und 10).

### Beispiel 13

### Verstärkung des durch Transferrin-Konjugate erzielten Gentransfers durch das N-terminale endosomolytische Peptid des Influenza-Hämagglutinins HA2

### a) Synthese des Peptids

Das Peptid der Sequenz (SEQ ID NO:1) Gly-Leu-Phe-Glu-Ala-Ile-Ala-Gly-Phe-Ile-Glu-Asn-Gly-Trp-Glu-Gly-Met-Ile-Asp-Gly-Gly-Gly-Cys wurde mit Hilfe der Fmoc(Fluorenylmethoxycarbonyl)-Methode (Atherton et al., 1979) synthetisiert, wobei ein Applied Biosystems 431A Peptidsythesizer verwendet wurde. Die Seitenketten-Schutzgruppen waren t-Butyl für Cys, Glu und Asp und Trityl für Asn. Nach der Kopplungsreaktion wurde ein Ninhydrintest durchgeführt, der einen Kopplungsgrad von > 98 % für jeden Schritt zeigte. Beginnend mit A-19 wurden Zweifachkopplungen durchgeführt. Die N-terminale Fmoc-Gruppe wurde mit 20 % Piperidin in NMP (N-Methylpyrrolidon) von einem Teil des Peptidharzes entfernt. Daraufhin wurden die Fmoc-geschützten und die ungeschützten Fraktionen mit DCM (Dichlormethan) gewaschen und unter Hochvakuum getrocknet. Die Ausbeuten betrugen 293.6 mg Fmoc-freies Peptidharz bzw. 366.5 mg Fmoc-geschütztem Peptidharz. 111.1 mg des Fmoc-freien Peptidharzes wurden 1.5 h lang einer Trifluoressigsäure-Spaltung unterworfen, wobei eine Mischung von 10 ml TFA, 0.75 g Phenol, 300 µl EDT (Ethandithiol) 250 µl Et-S-Me (Ethylmethylsulfid) und 500 µl Wasser verwendet wurde. Das Peptid wurde vom Harz durch eine Glassinternutsche filtriert. Das Harz wurde mit DCM gewaschen und dem Filtrat beigegeben. Das Filtrat wurde auf ca. 2 ml eingeengt und dann tropfenweise unter Rühren zu 40 ml Äther gegeben. Der Peptidniederschlag wurde abzentrifugiert und der Ätherüberstand verworfen. Der Niederschlag wurde 3 x mit 40 ml Äther gewaschen und im Hochvakuum getrocknet. Die erhaltenen 58 mg Rohprodukt wurden in 3.5 ml 20 mM NH₄HCO₃, enthaltend 300 µl 25 % NH₃/l, gelöst. Die Lösung wurde unter Verwendung desselben Puffers auf einer vorgepackten Sephadex G-25-Säule (Pharmacia PD-10) gelfiltriert. Das gesamte Material wurde auf eine Mono Q-Säule (Pharmacia 100 x 14 mm) aufgetragen (Gradient: 0-10 min 100 % A, 10-100 min 0-100 % B. A: 20 mM NH₄HCO₃ + 300 µl NH₃/l. B: A + 3 M NaCl. Messung bei 280 mM, Trp-Fluoreszenznachweis bei 354 nm. Flußrate 1 ml/min). Das Produkt eluiert mit 1 M NaCl. Die Hauptfraktion der Mono Q-Säule wurde durch Reverse Phase HPLC unter Verwendung einer BIORAD-Hi-Pore RP-304-Säule (250 x 10 ml) weiter gereinigt (Gradient: 50-100 % Puffer B in 12.5 min, 12.5-25 min 100 % B. A: 20 mM NH₄HCO₃ + 300 µl NH3/l, B: A in 98 % Methanol. Flußrate: 3 ml/min. Messung bei 237 nm). Das Produkt eluiert bei 100 % B. Die Produktfraktion wurden auf einer Speedvac eingedampft, in Puffer A wieder gelöst und abschließend lyophilisiert. Die Ausbeute betrug 8.4 mg des HPLC-gereinigten Produkts in der Cysteingeschützten Form (das Peptid wurde mit "P16" bezeichnet)). Um Peptid 1 in der freien Mercaptoform zu erhalten, wurde die t-Butyl-geschützte Substanz mit Thioanisol/Ethanedithiol/ Trifluoressigsäure/Trifluoromethansulfonsäure (2/1/40/3; Trifluoromethansulfonsäure wurde in dem angegebenen Verhältnis nach den anderen Komponenten beigegeben) 30 min lang bei Raumtemperatur behandelt. Das Peptid wurde durch Ätherfällung und anschließende Gelfiltration (Sephadex G-25) mit dem oben angegebenen Puffer A unter Argonatmosphäre isoliert.

### b) Kopplung des Influenzapeptids mit Polylysin

### b1) Direkte Bindung über SPDP (Succinimidylpyridyldithiopropionat)

19.8 mg Polylysin(pL)300-Hydrobromid (Sigma) wurden auf einer Sephadex G-25-Säule (Pharmacia PD-10) in Natriumacetat pH 5 gelfiltriert, um die niedermolekularen Fraktionen zu entfernen. Aufgrund des Ninhydrintests betrug die pL-Konzentration nach der Gelfiltration 3.16 mg/ml. Der pH-Wert der Lösung wurde mit 1 M NaOH auf 7 - 8 eingestellt. Zu 2.5 ml der pL-Lösung (7.9 mg pL = 0.13 µmol) wurden 0.64 µmol SPDP (Pharmacia: 40 mM Lösung in absolutem EtOH) gegeben. Dies entspricht einem molaren Verhältnis von SPDP:pL von 5:1. Die Mischung wurde über Nacht reagieren gelassen und in 20 mM NH₄HCO₃ pH 8.2 auf einer G25-Säule gelfiltriert. Nach Reduktion eines Aliquots des Filtrats mit DTT (Dithiothreitol) zeigte die Messung von Thiopyridon, daß die Reaktion vollständig abgelaufen war. 0.3 µmol pL-SPDP (bezogen auf µmol SPDP) in 2.212 ml wurden mit 0.35 µmol Peptid in der Thiolform reagieren gelassen. Ein weißer Niederschlag, der beim Mischen von Peptid und pL erschien, wurde gelöst, indem die Lösung auf 2 M Guanidinhydrochlorid eingestellt wurde, wobei die Reaktion über Nacht stattfand. Photometrische Messung von Thiopyridon in der Reaktionsmischung bestätigte erneut die Vollständigkeit der Reaktion. Daraufhin wurde die Mischung 2 x gegen 2 l 20 mM HEPES/0.5 M Guanidinhydrochlorid dialysiert. Die erhaltene Lösung wurde auf eine Mono S-Säule (0.7 x 6 cm, Pharmacia) aufgetragen (Gradient: 0-20 min 100 % A, 20-140 min 0-100 % B. A: 20 mM HEPES pH 7.3/0.5 M Guanidinhydrochlorid, B: 20 mM HEPES pH 7.3/3 M Guanidinhydrochlorid, 0.3 ml/min. Nachweis bei 280 nm und Fluoreszenznachweis bei 354 nm, Anregung bei 280 nm). Die Produktfraktion, die mit 1.5 M Guanidinhydrochlorid eluierte, wurde gegen 2 x 2 l HBS dialysiert. Die anschließende Bestimmung der pL-Konzentration durch den Ninhydrintest zeigte eine Konzentration von ca. 1.14 mg/ml. Die Peptidmenge in der Lösung des Konjugats wurde aus seiner Absorption bei 280 nm berechnet; dies ergab ein molares Verhältnis von Peptid:pL von 4:1.

### b2) Bindung über einen Polyethylenglykol-Linker

14.6 mg pL 300 Hydrobromid (Sigma) wurden gelfiltriert, wie unter b1) angegeben. Aufgrund des Ninhydrintests betrug die pL-Konzentration nach der Gelfiltration 4.93 mg/ml. Der pH-Wert der Lösung wurde mit 1 M NaOH auf 7 -8 eingestellt. Zu 2.7 ml pL-Lösung (13.3 mg pL = 0.22 µmol) wurden 4.33 µmol SPDP (Pharmacia; 30 mM Lösung in absolutem EtOH) gegeben. Dies entspricht einem molaren Verhältnis von SPDP:pL von 20:1. Nach 1.5 h wurde die Reaktionsmischung auf einer Sephadex G-25-Säule in 0.1 M Natriumacetat/3 M Guanidinhydrochlorid gelfiltriert. Nach Reduktion eines Aliquots des Filtrats mit DTT wurde eine Bestimmung von Thiopyridon durchgeführt, die einen Gehalt von 3.62 µmol SPDP in der Produktfraktion zeigte. Das SPDP-modifizierte pL wurde durch Zugabe von 79 mg DTT zur Lösung reduziert. Nach 2 h Reduktion wurde die Lösung erneut auf G25 unter den angegebenen Bedingungen filtriert. Die Thiolbestimmung mittels Ellmann-Test zeigte eine Thiolkonzentration von 3.15 µmol in 2.224 ml.

17.62 mg = 5 µmol POE (Polyoxyethylen-bis(6-aminohexyl), Sigma) wurden in 500 µl 20 mM NaHCO₃/3 M Guanidinhydrochlorid pH 7-8 gelöst und mit 13.8 mg EMCS (ε-Maleimidocapronsäure-N-hydroxysuccinimidester) (Sigma) (= 44.7 µmol), gelöst in 300 µl DMF (Dimethylformamid), reagieren gelassen. Nach 30 min wurde die Lösung auf G25 gelfiltriert (20 mM NaHCO₃/3 M Guanidinhydrochlorid). Die photometrische Bestimmung der Maleimido-Gruppe bei 300 nm zeigte eine Konzentration von 6.36 µmol reagiertem EMCS in 2 ml Lösung.

Zu 1.049 ml dieser Lösung (entsprechend 3.34 µmol EMCS) wurden 1.39 µmol des Peptids in Thiolform (in 2.5 ml 20 mM NaHCO₃/3 M Guanidinhydrochlorid) tropfenweise bei intensiver Durchmischung mittels Vortex in einem Argonstrom beigegeben. Nach 15 min waren mit dem Ellmann-Test keine freien Thiolgruppen mehr nachweisbar.

Die Lösung des reduzierten SPDP-modifizierten pL wurde durch Zusatz von 1 M NaOH auf einen pH-Wert von 7 - 8 gebracht. 1.373 ml dieser Lösung wurden bei intensiver Durchmischung mittels Vortex der obigen Reaktionsmischung beigegeben. Dies ergab ein molares Verhältnis von Peptid-SH:POE-EMCS:pL-SH von 1:2.4:1.4 (bezogen auf EMCS bzw. SH). Nach 2.5 h Reaktion waren mit dem Ellmann-Test keine freien Thiolgruppen mehr nachweisbar. Das Material wurde über Nacht gegen 2 l 20 mM HEPES pH 7.3/0.6 M NaCl dialysiert und anschließend auf eine Mono S-Säule aufgetragen (Gradient: 0-20 min 22 % A, 20-150 min 22-100 % B. A: 20 mM HEPES pH 7.3, B: A + 3 M NaCl. Flußrate 0.3 ml/min. Die Messung wurde bei 280 nm und die Fluoreszenzmessung bei 354 nm durchgeführt). Das Produkt, das mit 1.5 - 1.6 M NaCl eluierte, wurde gegen 2 l HBS dialysiert. Die Bestimmung der pL-Konzentration mittels Ninhydrintest und die photometrische Bestimmung der Peptidkonzentration bei 280 nm ergab ein errechnetes Peptid:pL-Verhältnis von 12:1 bei einer pL-Konzentration von 0.49 mg/ml in einem Gesamtvolumen von 4.5 ml.

### c) Liposomenpräparation

Mittels der REV-Methode ("reverse-phase evaporation") wurden Liposomen hergestellt (Szoka und Papahadjopoulos, 1978; Straubinger und Papahadjopoulos, 1983): Wässrige Phase 10 mM HEPES pH 7.3, 100 mM Calcein 150 mM NaCl; organische Phase: eine Lösung von 300 µmol L-α-Lezithin (aus Eidotter, hauptsächlich Palmitoyloleoylphosphatidylcholin; Avanti Polar Lipids) in 260 µl Chloroform wurden unter Verwendung eines Rotationsverdampfers eingedampft. Das Material wurde anschließend im Hochvakuum getrocknet und daraufhin wieder in 3 ml Diethyläther gelöst. 1 ml der wässrigen Phase wurde gründlich mit der Ätherphase mittels Vortex gemischt und 5 min lang bei 0°C in einem Sonicator (Badtyp) ultraschallbehandelt. Nach 30 min auf Eis wurde das Material nochmals 10 min lang ultraschallbehandelt. Die resultierende stabile Emulsion wurde langsam auf einem Rotationsverdampfer eingedampft. Nach Entfernung des Diethyläthers bei 100 mbar wurden 0.75 ml der wässrigen Phase hinzugefügt. Restliche Spuren von Äther wurden durch weitere Verdampfung bei 50 mbar während 30 min entfernt. Die erhaltene Emulsion (1.7 ml) wurde bei 500 Upm zentrifugiert und anschließend durch eine Nucleopore Polykarbonatmembran (0.1 µm) extrudiert, was ein Endvolumen von 0.7 ml Liposomenlösung ergab. Die Liposomen wurden vom nicht-inkorporierten Material durch Gelfiltration (Sephadex G-50 medium, Pharmacia; 23 ml Gel-Volumen, 10 mM HEPES pH 7.3/150 mM NaCl) getrennt. Es wurden sechs Fraktionen von 500 µl gesammelt. Lipidphosphor wurde nach der Methode von Bartlett, 1959, mit 2 mM bestimmt.

### d) Liposomen Leakage Assay

Die Freisetzung des Liposomeninhalts ("Leakage") wurde anhand des Austritts des eingeschlossenen Calceins und der daraus resultierenden Verdünnung, die eine Aufhebung der Selbstauslöschung der Fluoreszenz bewirkt (Bondeson et al., 1984), gemessen. Die Calceinfluoreszenz wurde mit einem Kontron SMF 25 Spektralfluorimeter (Anregung bei 490 nm, Emission bei 515 nm) gemessen. Zu diesem Zweck wurden 100 µl-Aliquots der obigen Liposomenlösung 100fach mit 0.1 M Natriumacetat oder 10 mM HEPES/150 mM NaCl Puffer mit dem entsprechenden pH-Wert (4.3, 4.5, 5.0, 6.0, 7.3) verdünnt, um ein Volumen von 1 ml zu erhalten. Zu diesen Lösungen wurden 2.5 µg des Peptids (t-Butyl-geschützte Form; 1 µg/µl Lösung in HBS) in Küvetten unter Mischen mit einem sanften Argonstrom (Endkonzentration 400 nM Peptid) gegeben. Die Calceinfluoreszenz wurde zu verschiedenen Zeitpunkten nach Beigabe des Peptids gemessen. Die Werte für 100 % Leakage wurden durch Zugabe von 2 µl Triton X-100 (Fluka) bestimmt.

Dieselbe Vorgangsweise wurde angewendet, um die Calceinfluoreszenz nach Beigabe von Peptid-pL-Konjugaten zur Liposomenlösung zu messen. 2.5 µg des Konjugats (1 µg/µl, Konzentration bezogen auf die pL-Menge allein) wurden 1 ml Liposomenlösung beigegeben (Endkonzentration 20 nM modifiziertes Peptid). Analog wurden 2.5 µg Peptid-Polylsin-Konjugat nach Inkubation mit 5 µg DNA (15 min) dem Leakage-Assay unterzogen.

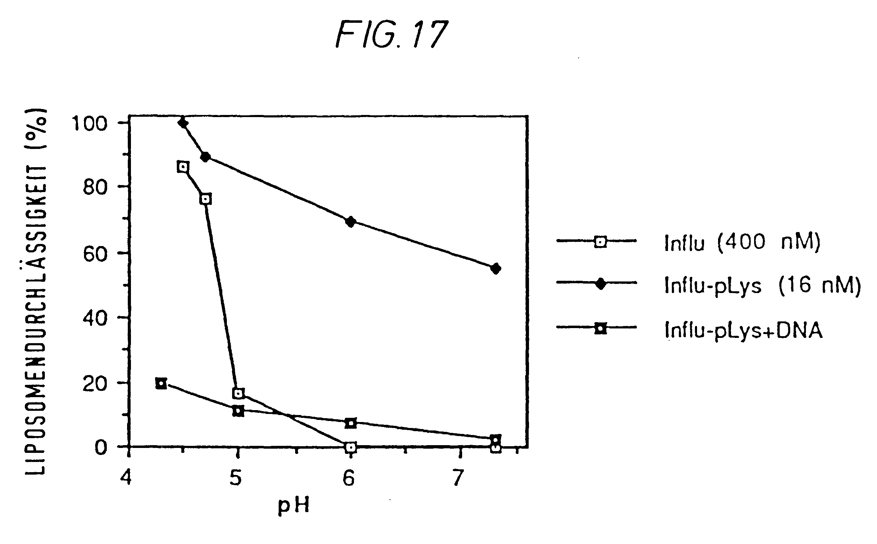

Es wurde gefunden, daß das Peptid nur im sauren Bereich die Freisetzung des Liposomeninhalts bewirkt (Fig. 17). Das Peptidkonjugat war bei wesentlich niedrigerem pH-Wert aktiv, wobei auch bei neutralem pH-Wert eine starke Aktivität gefunden wurde, die bei Senken des pH-Werts noch verstärkt wird.
Die Komplexierung des Konjugats mit DNA eliminierte die Aktivität bei neutralem pH-Wert, während bei saurem pH-Wert eine deutliche Aktivität vorhanden war.

### e) Transfektion von K562-Zellen

K562-Zellen wurden in Suspension in RPMI 1640-Medium (Gibco BRL plus 2 g Natriumbicarbonat/l) plus 10 % FCS, 100 Einheiten/ml Penicillin, 100 µl/µl Streptomycin und 2 mM Glutamin bis zu einer Dichte von 500.000 Zellen/ml gezüchtet. 12 bis 20 h vor der Transfektion wurden die Zellen in frisches Medium gegeben, das 50 µM Desferrioxamin enthielt (diese Maßnahme wurde getroffen, um eine Erhöhung der Transferrinrezeptor-Zahl zu erreichen). Am Morgen der Transfektion wurden die Zellen gesammelt, in frischem Medium, enthaltend 10 % FCS plus 50 µM Desferrioxamin suspendiert (250.000 Zellen/ml) und je 2 ml in eine Schale mit 24 Vertiefungen gegeben.

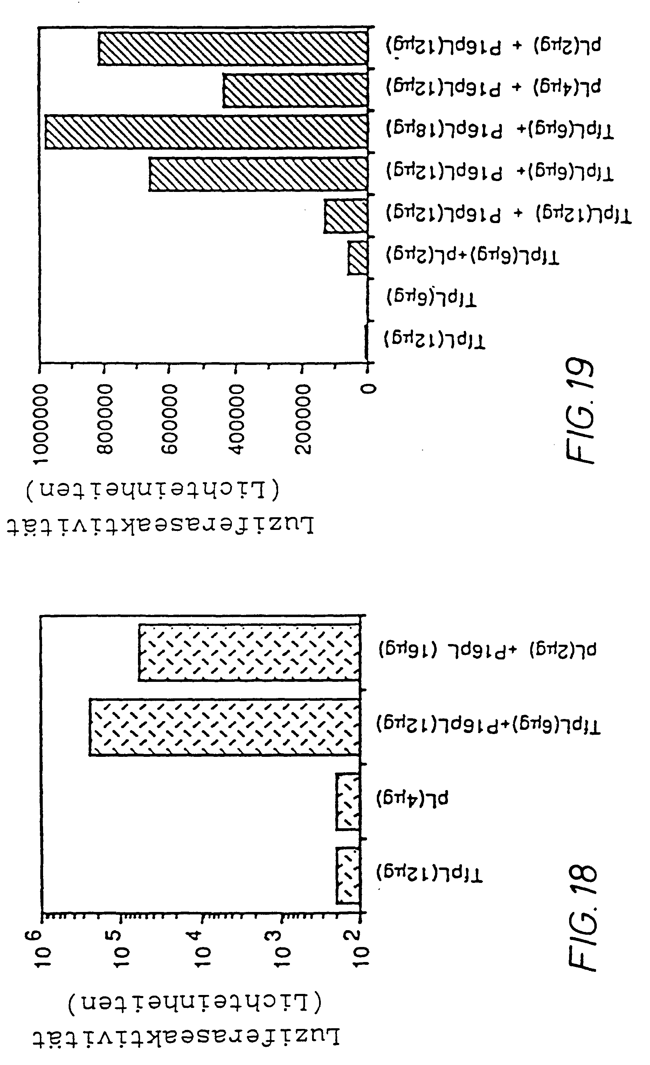

6 µg pCMVL-DNA in 160 µl HBS wurden mit den in Fig. 18 angegebenen Mengen TfpL-Konjugat oder mit pL300 in 160 µl HBS gemischt, nach 15 min wurden die angegebenen Mengen Influenzapeptid-pL-Konjugat ("P16pL") zugegeben, nach weiteren 15 min wurde die Mischung den K562-Zellen beigegeben. Die Zellen wurden bei 37°C 24 h lang inkubiert und dann für den Luciferase-Assay geerntet. Die Luciferaseaktivität wurde bestimmt, wie in den vorangegangenen Beispielen angegeben. Die in Fig. 18 angegebenen Werte stellen die Gesamtluciferaseaktivität der transfizierten Zellen dar.

### f) Transfektion von HeLa-Zellen

HeLa-Zellen wurden in 6 cm Kulturschalen gezüchtet, wie unter "Zellen und Medien" angegeben. Die Transfektionen wurden bei einer Dichte von 300.000 Zellen/Platte durchgeführt. Vor der Transfektion wurden die Zellen mit 1 ml frischem Medium, enthaltend 2 % FCS, inkubiert.

6 µg pCMVL-DNA in 160 µl HBS wurden mit den in Fig. 19 angegebenen Mengen TfpL-Konjugat oder mit pL300 oder einer Mischung von beiden in 160 µl HBS gemischt. Nach 15 min wurden die angegebenen Mengen InfluenzapeptidpL-Konjugate ("P16pL") zugegeben und nach weiteren 15 min die Mischung den Zellen beigegeben. Die Zellen wurden 2 h lang bei 37°C inkubiert, dann wurden 2.5 ml frisches Medium mit einem Zusatz von 10 % FCS zugegeben. Die Zellen wurden bei 37°C 24 h lang inkubiert und dann für den Luciferase-Assay geerntet. Die Luciferaseaktivität wurde bestimmt, wie in den vorangegangenen Beispielen angegeben. Die in Fig. 19 angegebenen Werte stellen die Gesamtluciferaseaktivität der transfizierten Zellen dar.

### Beispiel 14

### Verstärkung des durch Transferrin-Konjugate bewirkten Gentransfers mit Hilfe eines weiteren N-terminalen endosomolytischen Influenza-Hämagglutinin-HA2-Peptids

Das Peptid der Sequenz (SEQ ID NO:2) Gly-Leu-Phe-Gly-Ala-Ile-Ala-Gly-Phe-Ile-Glu-Asn-Gly-Trp-Glu-Gly-Met-Ile-Asp-Gly-Gly-Gly-Cys der Bezeichnung P41) wurde in analoger Weise synthetisiert wie das in Beispiel 13 a) beschriebene Peptid. Die Kopplung des Peptids an Polylysin (pL300) wurde wie in Beispiel 13 b1) durch Bindung über SPDP durchgeführt. Dabei wurden Konjugate mit einem molaren Verhältnis Peptid:Polylysin von 4:1 erhalten.

### b) Transfektion von HeLa Zellen mit Influenzapeptid-Konjugaten

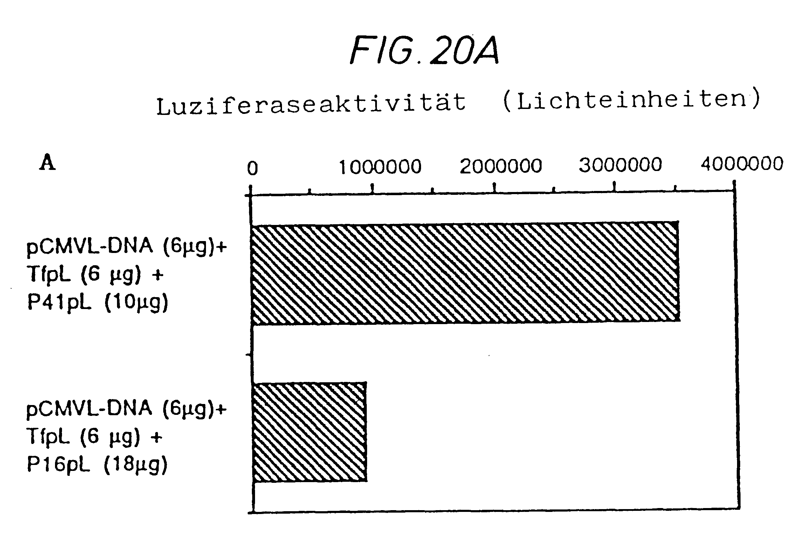

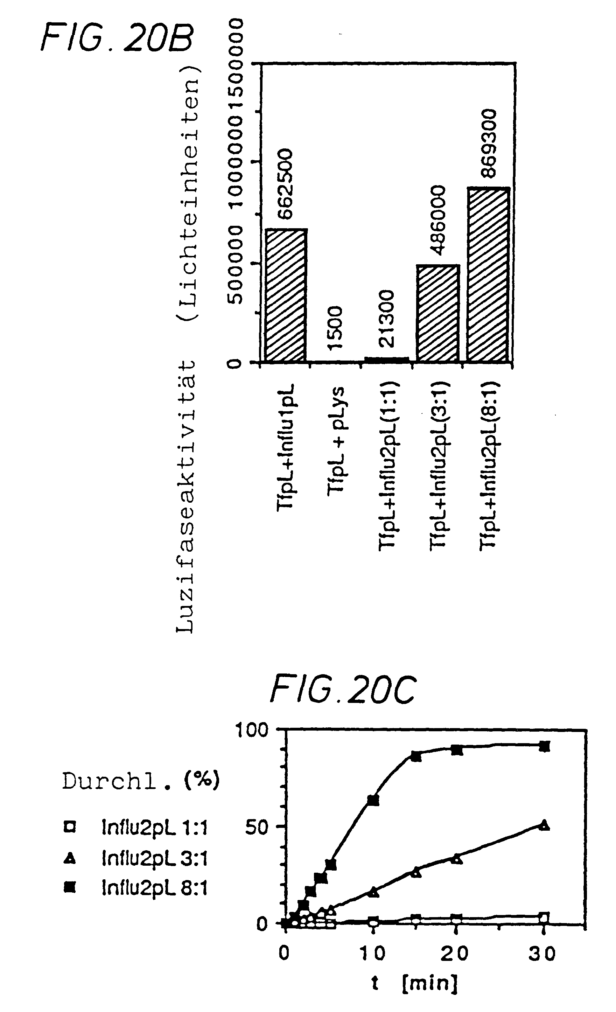

HeLa Zellen wurden, wie angegeben, in 6 cm Schalen gezüchtet, die Transfektionen wurden bei einer Dichte von 300 000 Zellen/Schale durchgeführt. Vor der Transfektion wurden die Zellen mit 1,5 ml frischem Medium, enthaltend 2 % FCS inkubiert. 6 µg pCMVL-DNA in 160 µl HBS (150 mM NaCl, 20 mM HEPES pH 7,3) wurden mit 6 µg des Konjugats TfpL190B in 160 µl HBS, nach 15 min wurden 10 µg Influenzapeptid-Polylysinkonjugat P41pL oder, zum Vergleich, 18 µg Influenzapeptid-Polylysinkonjugat P16pL (siehe Beispiel 13) zugefügt (Fig. 20); die angegebenen Mengen für die beiden Peptidkonjugate waren daraufhin getestet worden, daß sie die für die Verstärkung des Gentransfers optimalen Mengen darstellen. Nach weiteren 15 min wurde die Mischung den Zellen beigegeben. Nach 24 h wurden die Zellen für den Luciferasetest geerntet. Die in Fig. 20 gezeigten Werte stellen die Gesamtluciferaseaktivität der transfizierten Zellen dar.
Der Vergleich der Experimente mit den beiden Peptidkonjugaten zeigt eine mehr als 3,5fach höhere Verstärkung des Gentransfers durch das Peptidkonjugat P41pL.

### c) Transfektion von BNL CL.2 Zellen mit Influenzapeptidkonjugaten

BNL CL.2 Zellen wurden gezüchtet, wie in Beispiel 6 beschrieben. Das Influenzapeptid P41 wurde mit Polylysin300 bei molaren Verhältnissen Peptid:Polylysin von 1:1, 3:1 und 8:1 konjugiert. Komplexe aus 6 µg pCMVL-DNA und 20 µg der Konjugate wurden den Zellen beigegeben. Zum Vergleich wurden 20 µg pL300 oder 20 µg P16-Polylysin-Konjugate, hergestellt wie in Beispiel 13 beschrieben, verwendet. Die Zellen wurden bei 37°C 4 h lang inkubiert, dann wurden 2 ml Medium, enthaltend 18 % FCS, zugegeben. Nach 24 h wurden die Zellen für den Luciferasetest, dessen Ergebnisse in Fig. 20B dargestellt sind, geerntet. Im Liposomen Leakage Assay (Fig. 20C), der wie in Beispiel 13 durchgeführt wurde, stieg die Aktivität der Konjugate (entsprechend 2,5 µg Polylysin, pH Wert 5), mit ihrem Gehalt an Peptid (in der Fig. ist P41 als "influ2" bezeichnet).

### Beispiel 15

### Transfektion von HeLa-Zellen mit einem β-Galaktosidase-Reportergenkonstrukt und in situ Nachweis der β-Galaktosidase-Expression

### a) Züchtung und Transfektion der Zellen

Für die Transfektion wurden HeLa-Zellen in DMEM-Medium, enthaltend 5 % FCS, Penicillin, Streptomycin und Glutamin, wie in den vorangegangenen Beispielen angegeben, in 3 cm Kulturschalen auf Deckgläsern gezüchtet (3 x 10⁴ Zellen/Schale). Für die Transfektion wurden 6 µg des β-Galaktosidase-Reportergenkonstrukts (pCMV-β-gal) in 160 µl HBS mit 12 µg TfpL190B in 160 µl HBS komplexiert und 30 min bei Raumtemperatur inkubiert.

In einem weiteren Experiment wurden 6 µg pCMV-β-gal in 160 µl HBS mit 6 µg TfpL190B in 80 µl HBS 15 min bei Raumtemperatur inkubiert. Danach wurden 12 µg des in Beispiel 13 hergestellten Influenzapeptid-Konjugats (P16pL) in 80 µl HBS zugegeben und die Mischung weitere 15 min inkubiert. Diese DNA-Polykation-Komplexe wurden dann mit 1 ml DMEM plus 2 % FCS, Antibiotika und Glutamin, wie oben angegeben, gemischt. Um den Effekt von Chloroquin und Adenovirus auf die Leistungsfähigkeit der Transfektion zu zeigen, wurde in weiteren Experimenten Chloroquin bei einer Endkonzentration von 100 µM oder 50 µl der Adenovirus-Stammlösung dl312C noch zusätzlich dem Medium, das die DNA-Polykation-Komplexe enthält, beigegeben.

Für die Transfektionen wurde das ursprüngliche Kulturmedium von den Zellen entfernt und 1 ml Medium, enthaltend die DNA-Komplexe mit oder ohne Chloroquin oder Virus, zugegeben. Nach einer Inkubationszeit von 2 h bei 37°C wurde 1 ml DMEM, enthaltend 10 % FCS, Antibiotika und Glutamin, den Zellen zugesetzt und die Inkubation für weitere 2 h fortgesetzt. Dann wurde das gesamte Medium entfernt, und die Zellen wurden in 3 ml frischem DMEM plus 10 % FCS, Antibiotika und Glutamin, gezüchtet.

### b) β-Galaktosidase-Assay

48 h nach der Transfektion wurde das Medium entfernt, die Zellen wurden 1 x mit phosphatgepufferter Kochsalzlösung (PBS) gewaschen und mit 0.5 % Glutardialdehyd in PBS 5 min lang bei Raumtemperatur fixiert. Dann wurde das Fixiermittel entfernt und die Zellen 1 x mit PBS gewaschen. Anschließend wurde mit der Färbelösung (10 mM Phosphatpuffer pH 7.0, 150 mM NaCl, 1 mM MgCl₂, 3.3 mM K₄Fe(CN)₆3H₂O, 3.3 mM K₃Fe(CN)₆ und 0.2 % 5-Brom-4-chlor-3-indolyl-β-galaktopyranosid) bei 37 °C 20 min bis 3 h inkubiert (Lim und Chae, 1989). Danach wurden die Deckgläser in PBS, Wasser und 96 % Ethanol gespült, getrocknet und in Mowiol auf Objektträger eingedeckt. Zur Analyse wurde ein Zeiss Axiophot Mikroskop verwendet.

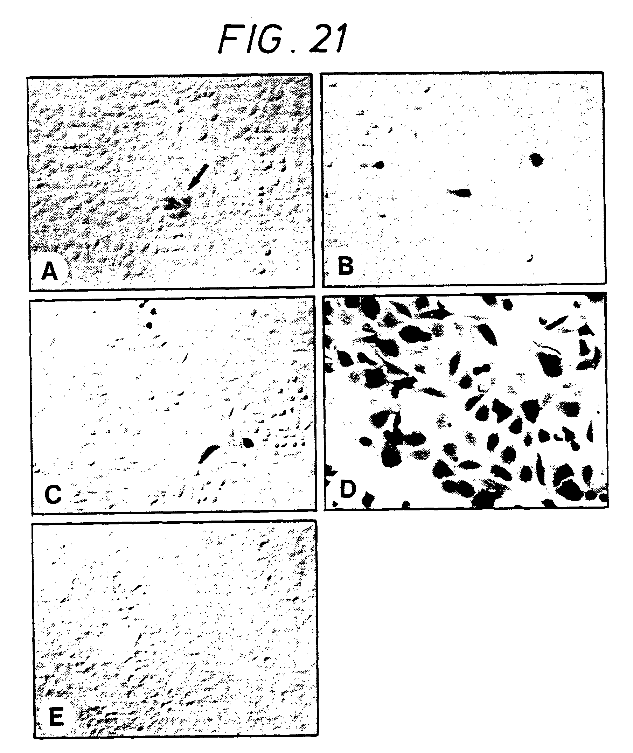

Fig. 21 zeigt Abbildungen der mikroskopischen Vergrößerungen (112 x). A: HeLa-Zellen, transfiziert mit 6 µg pCMV-β-gal, komplexiert mit 12 µg TfpL190B. Die Färbereaktion für β-Galaktosidase wurde während 3 h durchgeführt. Die Fig. zeigt, daß sehr wenige Zellen (55 Zellen; die Gruppe der gefärbten Zellen ist mit einem Pfeil angezeigt) das β-Galaktosidasegen exprimieren. B: HeLa-Zellen, transfiziert mit 6 µg pCMV-β-gal, komplexiert mit 6 µg TfpL190B und 12 µg P16pL. Färbereaktion: 3 h. Wenige Zellen (250 Zellen) exprimieren das β-Galaktosidasegen. Die Reaktion der Zellen ist jedoch stärker als in A. C: HeLa-Zellen, transfiziert mit 6 µg pCMV-β-gal, komplexiert mit 6 µg TfpL190B und 12 µg P16pL in Gegenwart von 100 µM Chloroquin. Färbereaktion: 3 h. Viele Gruppen von Zellen zeigen eine stark positive Reaktion (mehr als 1.000 Zellen). D: HeLa-Zellen, transfiziert mit 6 µg pCMV-β-gal, komplexiert mit 12 µg TfpL190B in Gegenwart von Adenovirus dl312. Färbereaktion: 20 min. Beinahe alle Zellen (mehr als 90 %) zeigen eine positive Reaktion. E: Nicht transfizierte HeLa-Zellen (Kontrolle für die Spezifität der β-Galaktosidasereaktion). Färbereaktion: 3 h.

### Beispiel 16

### Transfektion von HeLa-Zellen mit einem 48 kb Cosmid in Gegenwart von freiem Adenovirus

### a) Herstellung eines Cosmids, enthaltend die für Luciferase kodierende Sequenz

Ein 3,0 kb SalI-Fragment, enthaltend eine einzige für P. pyralis Luciferase kodierende Sequenz unter der Kontrolle des RSV-Promotors, wurde vom Plasmid p22RSVLuca und in die einzige SalI-Stelle des Cosmidklons C1-7a1 ligiert, um Concatamere zu bilden. (C1-7a1 enthält ein 37 kb humanes genomisches DNA Sau3A-Fragment (Teilverdau), kodierend für keine offensichtlichen Gene, kloniert in die BamHI-Stelle des Cosmidvektors pW15 (Stratagene)). Das Ligationsreaktionsprodukt wurde darauf in vitro verpackt und ein Aliquot der erhaltenen Phagenpartikel in E.coli MN544 infiziert und auf LB amp Platten plattiert. Die Rekombinanten wurden mittels Koloniehybridisierung unter Verwendung des 3,0 kb SalI-Fragments (³²-P-markiert durch randomisiertes Priming) als Hybridisierungsssonde gescreent und eine Anzahl von Positiven durch Restriktionskartierung analysiert. Ein Cosmid-Konstrukt (CosLuc), enthaltend eine einzige Kopie des SalI-Inserts, wurde gezüchtet und auf einem Caesiumgradienten gereinigt (Gesamtgröße: 48 kb). Ein kleines Kontroll-Cosmid pWELuc (12 kb) wurde hergestellt durch Verdau von CosLuc mit Not I, Religieren, Transformieren von Bakterien und Isolieren des richtigen Plasmids. Dies ergab ein 12 kb DNA-Molekül, dem ein Teil des humanen DNA-Inserts und ein Teil des Polylinkers von CosLuc fehlte. Das Plasmid pSPNeoLuc (8 kb) ist das in Beispiel 5 beschriebene Plasmid, das ein RSV-Luciferase-Genfragment (ein Apal/Pvul-Fragment von pRSVL, kloniert in die Cla1-Stelle des pUCµ-Lokus).

### b) Transport des Cosmids in HeLa-Zellen

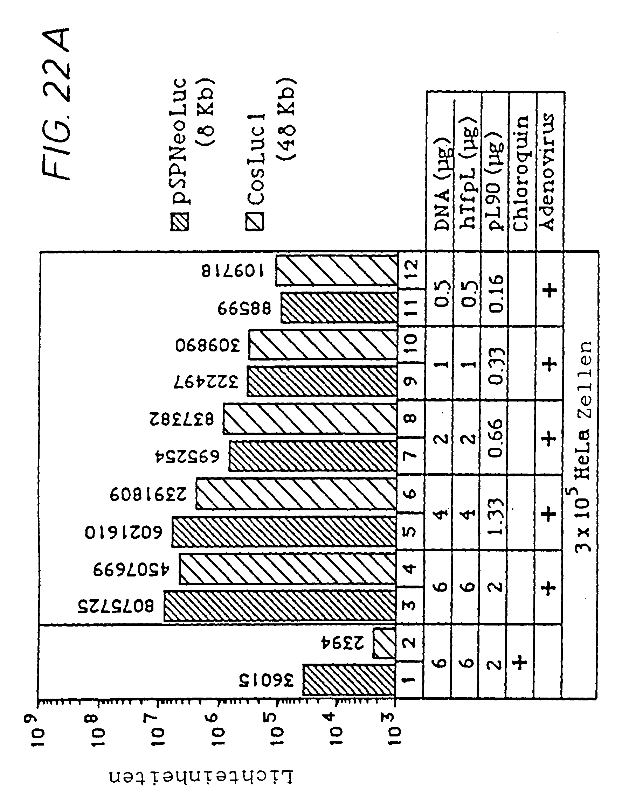

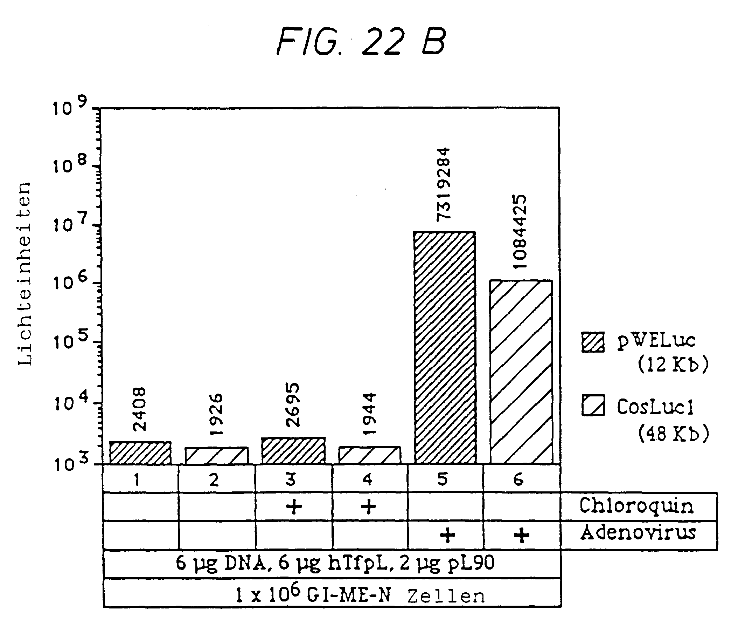

HeLa-Zellen (3 x 10⁴ Zellen pro 6 cm Schale), bedeckt mit 1 ml DMEM + 2 % FCS wurden mit TfpL/DNA-Komplexen, hergestellt wie in der Einleitung zu den Beispielen beschrieben, enthaltend die angegebenen Mengen hTfpL, freiem Polylysin und DNA, inkubiert. Zusätzlich enthielten die Inkubationsmischungen entweder 100 µM Chloroquin (Spalten 1 und 2) oder 10 µl Adenovirus dl312, enthaltend 5 x 10¹¹ Partikel pro ml (Spalten 3-12). Nach einer 2 stündigen Inkubation bei 37°C wurden jeder Schale 4 ml DMEM + 10 % FCS beigegeben. 24 Stunden später wurden die Zellen geerntet und die Luciferaseaktivität gemessen. Die Ergebnisse sind in Fig. 22A gezeigt.

### c) Transport des Cosmids in Neuroblastomzellen

Zellen einer Neuroblastom-Zellinie der Bezeichnung GI-ME-N (Donti et al., 1998) (1 x 10⁶ Zellen pro 6 cm Schale), bedeckt mit 1 ml DMEM + 2 % FCS wurden mit TfpL/DNA-Komplexen, hergestellt wie beschrieben, enthaltend die angegebenen Mengen von hTfpL, freiem Polylysin und DNA. Zusätzlich enthielten die Inkubationsmischungen entweder 100 µM Chloroquin (Spalten 3 und 4) oder 10 µl Adenovirus d1312, enthaltend 5 x 10¹¹ Partikel pro ml (Spalten 5 und 6). Nach einer 2 stündigen Inkubation bei 37°C wurden jeder Schale 4 ml DMEM + 10 % FCS beigegeben. 24 Stunden später wurden die Zellen geerntet und die Luciferaseaktivität gemessen. Die Ergebnisse sind in Fig. 22B gezeigt.

### Beispiel 17

### Gentransfer mittels chemisch gekoppelten Adenovirus-Polylysin-Konjugaten

### a) Herstellung von Adenovirus-Polylysin-Konjugaten mittels chemischer Kopplung

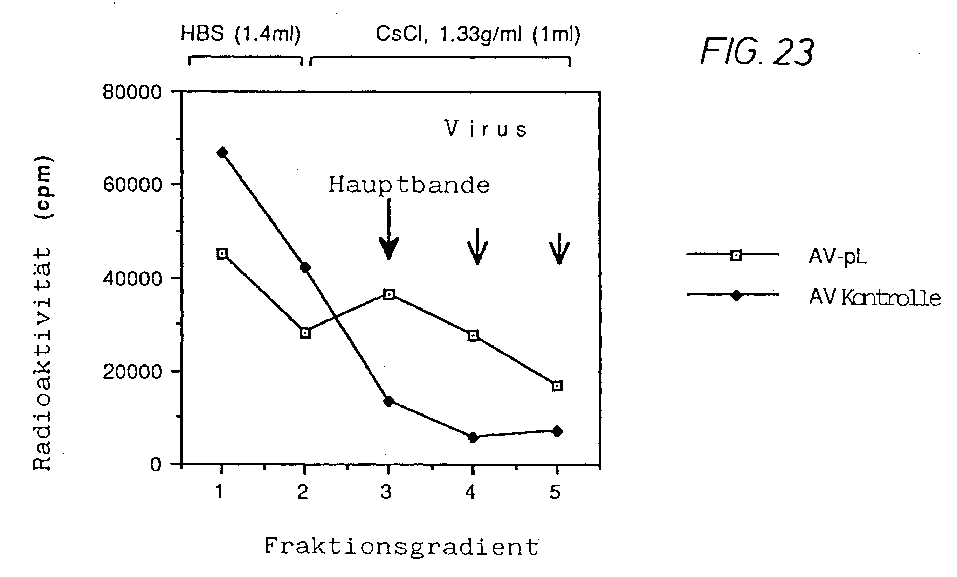

2.35 ml einer gelfiltrierten (Sephadex G-25 PD10, Pharmacia) Lösung von Adenovirus d1312 (ca. 10¹¹ Partikel) in 150 mM NaCl / 25 mM HEPES, pH 7.9 / 10 % Glycerin, wurde mit 10 µl (10 nmol) einer 1 mM Lösung von SPDP (Pharmacia) versetzt. Nach 3.5 h bei Raumtemperatur wurde der modifizierte Virus durch Gelfiltration (wie oben) vom überschüssigen Reagens abgetrennt. Die Lösung (2.5 ml) wurde mit Argon gespült und unter Sauerstoffausschluss und Argon mit 42 µl einer Lösung von FITC-markiertem Polylysin (1 nmol), modifiziert mit 2.3 nmol Mercaptopropionat-Gruppen (hergestellt wie in EP 388 758 beschrieben), reagieren gelassen. Nach 18 h bei Raumtemperatur wurde die Hälfte der Lösung in ein Zentrifugenröhrchen überführt, mit 1 ml einer Cesiumchlorid-Lösung (Dichte 1.33 mg/ml) vorsichtig unterschichtet und 2 h bei 35000 rpm (SW60 Rotor) bei Raumtemperatur zentrifugiert. Die Virusbande wurde als 200 µl Cesiumchlorid-Fraktion gesammelt und mit HBS / 50% Glycerin auf 1 ml verdünnt.
Ein DNA-Bindungsassay wurde mit 300 µl des modifizierten Virus durchgeführt: die Viruslösung wurde mit 1 ml HBS verdünnt und mit 100 µl Lösung einer ³⁵S-markierten DNA (15 ng pRSVL, hergestellt durch Nick-Translation) versetzt. Zur Kontrolle wurde das Experiment mit gleicher Menge unmodifiziertem Virus dl312 parallel durchgeführt. Nach 30 min wurden die Proben in Zentrifugenröhrchen überführt, mit 1 ml einer Cesiumchlorid-Lösung (Dichte 1.33 mg/ml) vorsichtig unterschichtet und 2 h bei 35000 rpm (SW60 Rotor) bei Raumtemperatur zentrifugiert. Der Gradient wurde je in 5 Fraktionen geteilt; Fraktion 1, 1 ml; Fraktion 2, 0.6 ml; Fraktion 3-5, je 200 µl. Die Radioaktivität von je 200 µl der Fraktionen wurde bestimmt und ist in Fig. 23 wiedergegeben. Dabei findet man in den Virushältigen Fraktionen (3-5), hauptsächlich Fraktion 3, eine deutlich höhere Radioaktivität als im Kontrollexperiment; dies ist auf spezifische Assoziation des Polylysin-modifizierten Adenovirus mit der markierten DNA zurückzuführen.

### b) Transfektion von K562-Zellen

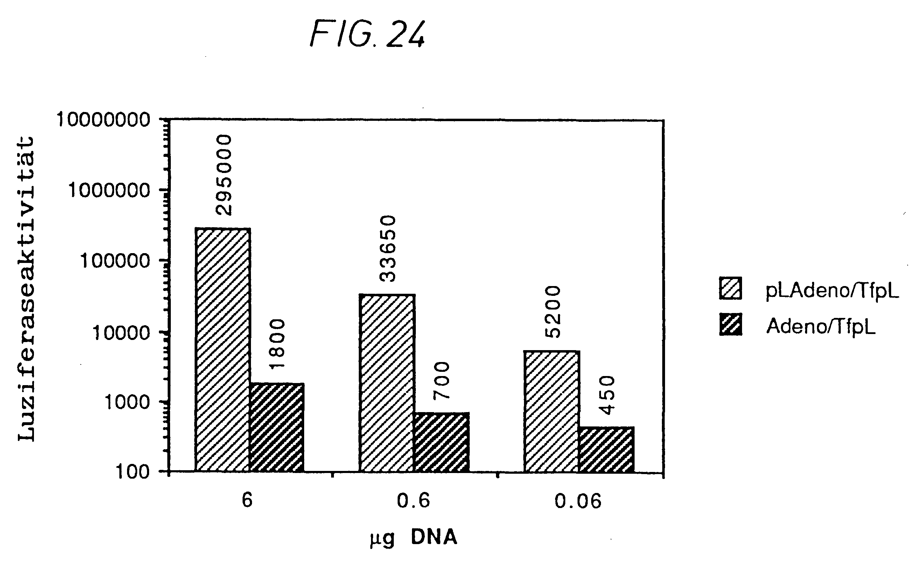

K562-Zellen (ATCC CCL 243) wurden in Suspension in RPMI 1640-Medium (Gibco BRL plus 2 g Natriumbicarbonat/l) plus 10 % FCS, 100 Einheiten/ml Penicillin, 100 µl/µl Streptomycin und 2 mM Glutamin bis zu einer Dichte von 500.000 Zellen/ml gezüchtet. 12 bis 20 h vor der Transfektion wurden die Zellen in frisches Medium gegeben, das 50 µM Desferrioxamin enthielt (diese Maßnahme wurde getroffen, um eine Erhöhung der Transferrinrezeptor-Zahl zu erreichen). Am Morgen der Transfektion wurden die Zellen gesammelt, in frischem Medium, enthaltend 10 % FCS plus 50 µM Desferrioxamin, suspendiert (250.000 Zellen/ml) und je 2 ml in eine Schale mit 24 Vertiefungen gegeben.
Angegebene Mengen an pCMVL-DNA (6, 0.6, 0.06 µg) in 100 µl HBS wurden mit 50 µl Polylysin-Adenovirus (pLAdeno) bzw. entsprechenden Mengen (35 µl) Kontroll-Adenovirus d1312 gemischt. Nach 20 min wurden jeweils entsprechende Mengen (12, 1.2, 0.12 µg) TfpL190B-Konjugat in 150 µl HBS zugegeben. Nach weiteren 20 min wurde die Mischung den K562-Zellen beigegeben. Die Zellen wurden bei 37°C 24 h lang inkubiert und dann für den Luciferase-Assay geerntet. Die Luciferaseaktivität wurde bestimmt, wie in den vorangegangenen Beispielen angegeben. Die in Fig. 24 angegebenen Werte stellen die Gesamtluciferaseaktivität der transfizierten Zellen dar.

### c) Transfektion von HeLa-Zellen

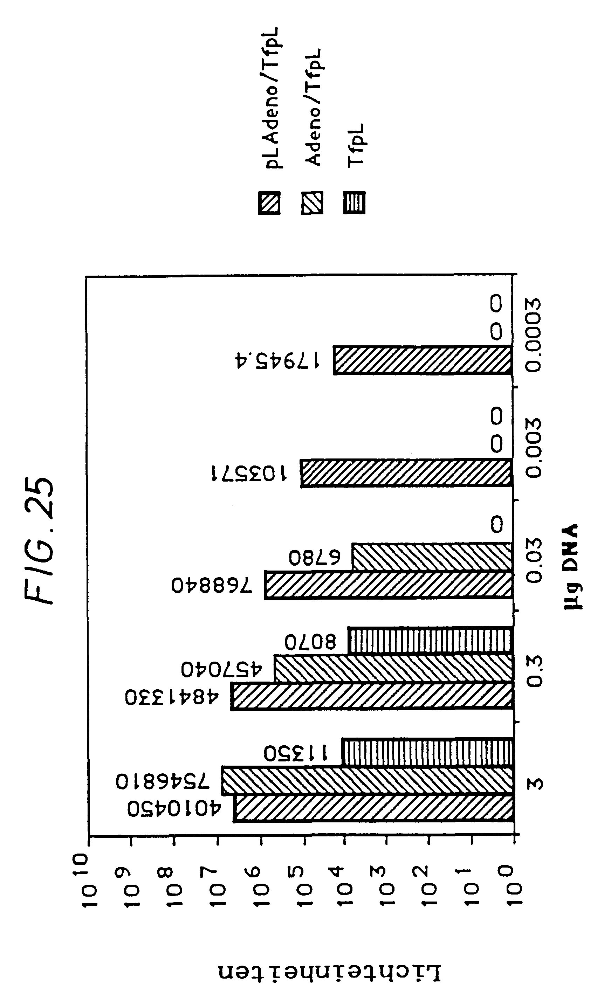

Eine Methode zur Überprüfung der Aktivität eines Polylysin-Viruskonjugats ist die Überprüfung des Konjugats auf seine Fähigkeit, sehr kleine DNA-Mengen (weniger als µg) zu transportieren. Es wurde eine erhöhte DNA-Transferkapazität erwartet, wenn das Adenovirus direkt an die Polylysin-kondensierte DNA gebunden ist, weil die Internalisierungsfaktoren (Transferrin und Adenovirus-Faserprotein) direkt mit der zu transportierenden DNA assoziiert sind. Um die Richtigkeit dieser Annahme zu überprüfen, wurde eine konstante Menge des Polylysin-Adenovirus-Konjugats (2.5 µl, ca. 5x10⁷ Viruspartikel) mit verschiedenen Mengen (3 µg bis 0.0003 µg) von Reporterplasmid in 475 µl HBS komplexiert. Nach 15 min Inkubation bei Raumtemperatur wurde eine der Masse an DNA entsprechende Menge von Transferrin-Polylysin jeder Probe beigegeben (diese Menge an TfpL wurde gewählt, weil sie vollständiges "packaging" (Elektroneutralität) von 50 % der Plasmid-DNA gewährleistet und gleichzeitig Bindungs-Freiraum für das Virus-Polylysin-Konjugat gewährleistet. Nach Zugabe von TfpL wurden die Mischungen 15 min lang inkubiert, dann wurde jede Mischung in eine 6 cm Kulturschale, enthaltend 300.000 HeLa-Zellen in 1 ml DMEM/2 % FCS, gegeben. Darauf wurden die Zellen 1.5 h lang bei 37°C inkubiert, danach wurden 4 ml DMEM/10 % FCS zugegeben. Parallel dazu wurden äquivalente Mengen DNA mit einem zweifachen Massenüberschuß TfpL (die Menge für vollständige DNA-Kondensation) komplexiert und für den Gentransfer in HeLa-Zellen verwendet (einmal allein, einmal in Gegenwart von 25 µl der nicht-Polylysin-gekoppelten Adenovirus d1312-Präparation). Nach 24 h wurden die Zellen geerntet, Extrakte hergestellt und Aliquots auf Luciferaseaktivität untersucht. Das Ergebnis dieser Versuche ist in Fig. 25 dargestellt: Bei Fehlen von Adenovirus ist bei einer DNA-Menge unter 0.3 µg keine Luciferaseaktivität nachweisbar. Sowohl Polylysingekoppeltes als auch nicht-gekoppeltes Adenovirus funktionierten bei großen DNA-Mengen (3 µg und 0.3 µg) gut. Mit dem nicht-gekoppelten Adenovirus wurde jedoch ein beinahe 100facher Aktivitätsabfall bei 0.03 µg und eine vernachlässigbare Aktivität unter dieser DNA-Menge beobachtet. Im Gegensatz dazu behält das Polylysingekoppelte Virus seine Gentransfer-Kapazität sowohl bei 0.003 als auch bei 0.0003 µg DNA. Diese Menge an DNA entspricht ca. 100 DNA-Molekülen/Zelle und ca. 1 Viruspartikel/DNA-Molekül.

### Beispiel 18

### Gentransfer mittels enzymatisch an Polylysin gekoppelte Adenoviren

### a) Enzymreaktion

2 ml der Adenovirus-Präparation (Stamm dl312; 5x10¹⁰ PFU/ml) wurden auf eine Sephadex G-25 Gelfiltrationsäule (Pharmacia), die mit 25 ml Reaktionspuffer (0.1 M Tris-HCl; pH 8.0, 2 mM DTT, 30 % Glycerin) equilibriert wurde, aufgetragen. Die Elution erfolgte mit 3.5 ml Reaktionspuffer. Der Reaktionsansatz zur enzymatischen Kopplung besteht aus 1150 µl der Viruselutionfraktion, 0.5 nmol Meerschweinchenleber-Transglutaminase (TG) (Sigma), 2 nmol beziehungsweise 20 nmol Polylysin290, 10 mM CaCl₂ und Reaktionspuffer in einem Endvolumen von 1500 µl. Die Reaktion wurde bei 37° C über 1 Stunde durchgeführt und danach durch Zugabe von 30 µl 0.5M EDTA gestoppt. Zur Kontrolle der Spezifität der Kopplung wurden auch Reaktionsansätze ohne Transglutaminase durchgeführt. Nicht inkorporiertes Polylysin wurde von den Viren durch Zentrifugation über einen CsCl-Gradienten (Dichte 1.33g/ml; 170000 x g, 2 Stunden) abgetrennt. Die Fraktion, die die Viren beinhaltet wurde abgezogen und mit gleichem Volumen Glycerin versetzt und in flüssigem Stickstoff eingefroren und bei -70° C aufgehoben.

### b) Demonstration der Bindung von Polylysin an Adenoviren

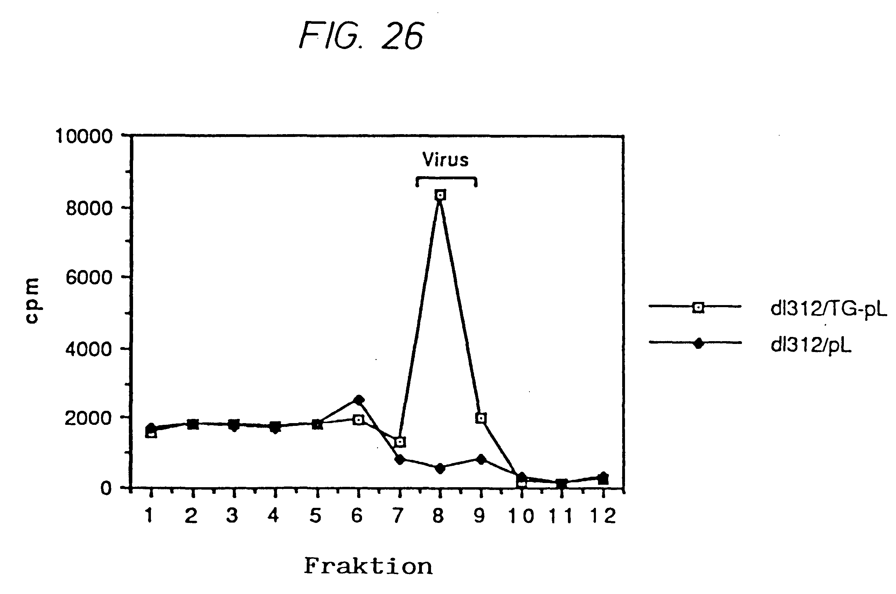

Die Reaktion wurde mit Polylysin, das mit 125j mittels Bolton-Hunter Reagenz (Amersham) markiert wurde, wie oben beschrieben durchgeführt. Nach der CsCl-Gradientenzentrifugation wurde die Virusfraktion abgezogen und über einen weiteren CsCl-Gradienten getrennt. Danach wurde der Gradient fraktioniert und die Radioaktivität in allen Fraktionen mit einem Szintillationszähler bestimmt. Wie in Fig. 26 dargestellt, zeigte sich dabei, daß bei dem Reaktionsansatz mit TG (dl312/TG-pL) radioaktives Polylysin in der Virusfraktion (Virus) angereichert war. Im Kontrollansatz ohne TG (dl312/pL) fand sich keine Anreicherung von radioaktivem Polylysin in der Virusfraktion.

### c) Testung der Polylysin-modifizierten Adenovirusfraktionen bezüglich ihres Effektes auf die Transfektionseffizienz

### i) Zellen und Medien

Zur Transfektion wurden 5x10⁵ Zellen (Maushepatozyten; ATCC No. : TIB 73) in DMEM mit 10 % hitzeinaktiviertem fötalem Kälberserum (FCS), 2 mM Glutamin, 100 I.E./ml Penicillin und 100 µg/ml Streptomycin in 6 cm Kulturschalen ausgesät.

### ii) Bildung der Virus-DNA-Transferrin-Komplexe

50 µl der jeweiligen Polylysin-modifizierten Virusfraktion wurden mit 6 µg des DNA-Plasmids pCMVL in 10 µ HBS gemischt und 20 min bei Raumtemperatur inkubiert. Danach wurde dem Gemisch 8 µg Mäuse-Transferrin-Polylysin290B (mTfpL) zugesetzt und für weitere 10 min inkubiert.

### iii) Transfektion der Maushepatozyten

Die Virus-DNA-Transferrin-Komplexe wurden mit 1.5 ml Medium (DMEM mit 2 % FCS, 2 mM Glutamin und Antibiotika) gemischt und den Zellen, nachdem das alte Medium entfernt wurde, zugesetzt. Nach einer Inkubation von 2 h bei 37° C wurde den Zellen 2 ml von DMEM mit 10 % FCS, Glutamin und Antibiotika zugegeben. Nach einer weiteren Kultivierungsphase von 2Stunden wurde das gesamte Medium entfernt und den Zellen 4 ml von frischem DMEM mit 10 % FCS, Glutamin und Antibiotika zugesetzt.

### iv) Bestimmung der Luciferaseexpression

24 Stunden nach der Transfektion wurden die Zellen geerntet und der Luciferase-Assay, wie oben beschrieben, durchgeführt.

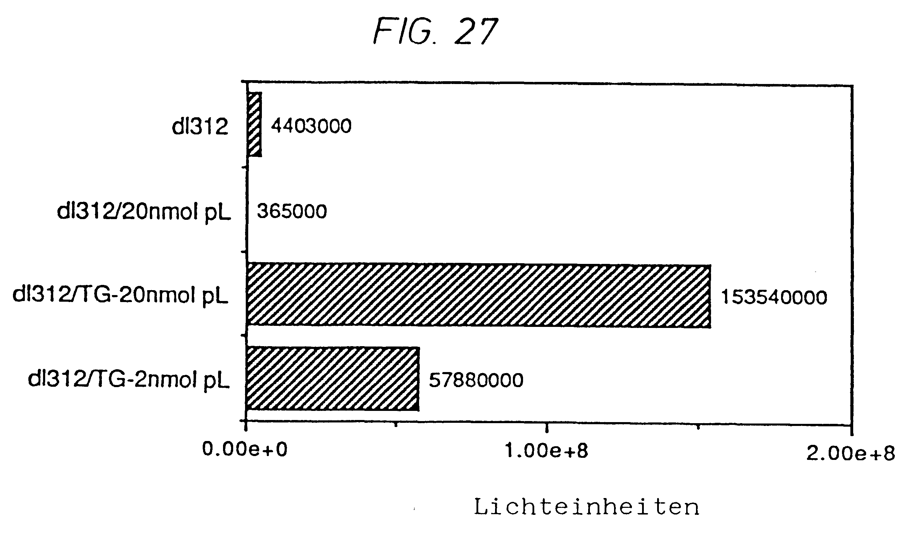

Wie aus Fig. 27 ersichtlich, ergab die Viruspräparation, bei der die Adenoviren mit TG und 20 nmol Polylysin behandelt wurden (dl312/TG-20 nmol pL), die stärkste Expression (153540000 Lichteinheiten). Die Viruspräparation mit TG und 2 nmol Polylysin (dl312/TG-2 nmol pL) war etwas weniger aktiv (57880000 Lichteinheiten). Die Kontrollfraktion, bei der die Adenoviren mit 20 nmol Polylysin, jedoch ohne TG, behandelt wurden, war nahezu um den Faktor 500 weniger wirksam. Zum Vergleich wurden weiters zur Transfektion Komplexe verwendet mit der Ausgangspräparation von Adenoviren, die weder mit TG noch mit Polylysin behandelt wurden (dl312). Diese Präparation ergab 4403000 Lichteinheiten.

### d) Erhöhung der Transfektionseffizienz durch Polylysin-modifizierte Adenoviren im Vergleich zu nicht modifizierten Adenoviren, insbesondere bei niedrigen DNA-Mengen

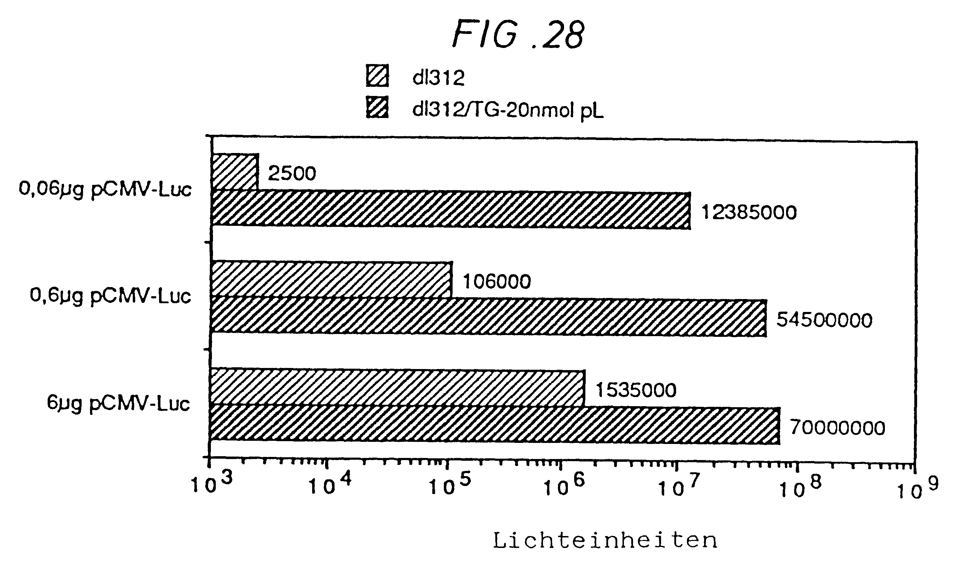

Die Transfektion wurde wie in Beispiel 3c) beschrieben durchgeführt, wobei zur Komplexbildung 50 µl der Adenovirusfraktion dl312/TG-20 nmol pL und 6 µg pCMVL/8 µg mTfpL, 0.6 µg pCMV-Luc/0.8 µg mTfpL oder 0.06 µg PCMVL/0.08 µg mTfpL verwendet wurden. Zum Vergleich wurden auch Transfektionen durchgeführt mit 6 µg, 0.6 µg, 0.06 µg pCMVL/mTfpL-Komplexen und nicht modifizierten Adenoviren (dl312). Es zeigte sich, daß die Komplexe mit Polylysin-modifizierten Adenoviren auch bei niedrigen DNA-Mengen noch hohe Expressionswerte ergaben, während bei nicht modifizierten Adenoviren die Expression stark vermindert war (Fig. 28).

### Beispiel 19

### Gentransfer mit Konjugaten, in denen die Bindung zwischen Adenovirus und Polylysin über eine Biotin-Streptavidin-Brücke erfolgt

### a) Biotinylierung von Adenovirus dl312

2.4 ml einer gelfiltrierten (Sephadex G-25 PD10, Pharmacia) Lösung von Adenovirus d1312 (ca. 10¹¹ Partikel) in 150 mM NaCl / 5 mM HEPES, pH 7.9 / 10 % Glycerin , wurde mit 10 µl (10 nmol) einer 1 mM Lösung von NHS-LC-Biotin (Pierce 21335) versetzt. Nach 3 h bei Raumtemperatur wurde der Biotin-modifizierte Virus durch Gelfiltration (wie oben) vom überschüssigen Reagens abgetrennt. Die Lösung wurde durch Zugabe von Glycerin auf eine Glycerinkonzentration von 40 % gebracht (Gesamtvolumen 3.2 ml) und bei -25°C gelagert. Die Biotinylierung des Virus konnte in einem qualitativem Nachweis nach Tropfen von verschiedenen Verdünnungen auf Cellulose-Nitrat Membran nachgewiesen werden: nach Trocknen bei 80°C / 2 h im Vakuumtrockenschrank, Blocken mit BSA, Inkubieren mit Streptavidin-konjugierter Alkalischer Phosphatase (BRL), Waschen und 1 h Inkubation mit der Entwicklungslösung NBT / X-Phosphat (Nitroblau-Tetrazolium Salz/ 5-Brom-4-chlor-3-indolylphosphat, Toluidin-Salz ; Boehringer Mannheim) wurde eine positive Farbreaktion gefunden.

### b) Herstellung von Streptavidin-Polylysin-Konjugaten

Die Kopplung von Streptavidin mit Polylysin wurde nach der von Wagner et al., 1990, und in der EP-A1 388 758 beschriebenen Methode durchgeführt.

79 nmol (4.7 mg) Streptavidin in 1 ml 200 mM HEPES pH 7.9 und 300 mM NaCl wurden mit einer 15 mM ethanolischen Lösung von SPDP (236 nmol) behandelt. Nach 1.5 h bei Raumtemperatur wurde das modifizierte Protein über eine Sephadex G-25 Säule gelfiltriert, wobei 75 nmol Streptavidin, modifiziert mit 196 nmol Dithiopyridinlinker, erhalten wurde. Das modifizierte Protein wurde mit 3-Mercaptopropionat-modifiziertem Polylysin (75 nmol, durchschnittliche Kettenlänge 290 Lysinmonomere, modifiziert mit 190 nmol Mercaptopropionat-Linker) in 2.6 ml 100 mM HEPES pH 7.9, 150 mM NaCl unter Argonatmosphäre reagieren gelassen. Konjugate wurden mittels Kationenaustauschchromatographie auf einer Mono S HR5-Säule (Pharmacia) isoliert. (Gradient: 20 - 100 % Puffer. Puffer A: 50 mM HEPES pH 7.9; Puffer B: Puffer A plus 3 M Natriumchlorid. Die Produktfraktion eluierte bei einer Salzkonzentration zwischen 1.2 M und 1.7 M. Dialyse gegen HBS (20 mM HEPES pH 7.3, 150 mM NaCl) ergab ein Konjugat, bestehend aus 45 nmol Streptavidin und 53 nmol Polylysin.

### c) Transfektion von HeLa-Zellen

HeLa-Zellen wurden in 6 cm Kulturschalen gezüchtet, wie in Beispiel 1 angegeben. Die Transfektionen wurden bei einer Dichte von 300.000 Zellen/Platte durchgeführt. Vor der Transfektion wurden die Zellen mit 1 ml frischem Medium, enthaltend 2 % FCS, inkubiert.

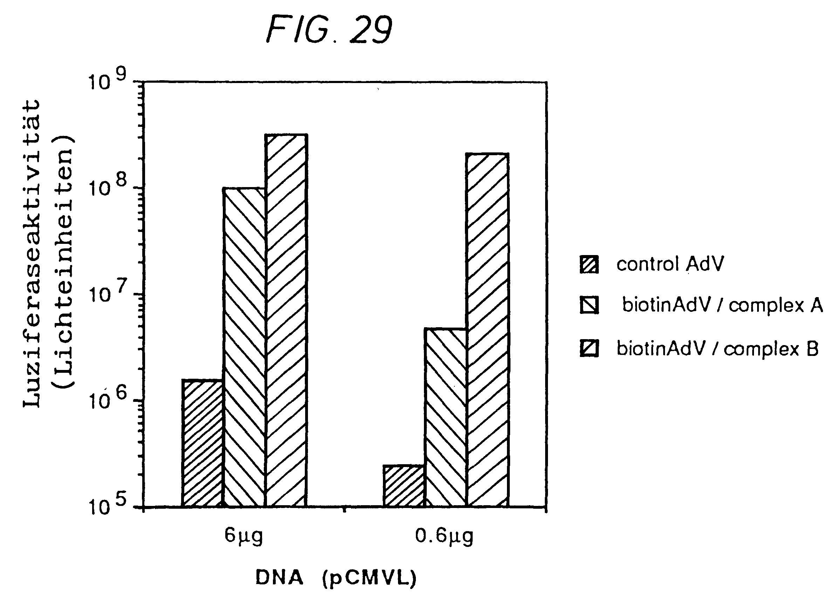

6 µg pCMVL-DNA in 100 µl HBS wurden mit 0.8 µg Streptavidin-Polylysin in 170 µl HBS gemischt. Nach 20 min wurden 3 µl Polylysin pL300 in 170 µl HBS zugemischt. Nach weiteren 20 min wurden 65 µl biotinyliertes Adenovirus oder als Kontrolle entsprechende Mengen an Adenovirus dl312 (30 µl, Ausgangsvirus für die Modifizierung) zugegeben. Die Komplexmischungen ("biotinAdV/complex A" bzw. "control AdV", siehe Fig. 29) wurden weitere 20 min stehengelassen.

Eine alternative Komplexbildung wurde durchgeführt, indem 65 µl biotinyliertes Adenovirus zuerst mit 0.8 µg Streptavidin-Polylysin in 50 µl HBS gemischt wurden, nach 20 min 6 µg pCMVL-DNA in 170 µl HBS zugegeben wurden, und nach weiteren 20 min 3 µl Polylysin pL300 in 200 µl HBS zugemischt wurden. (Komplexmischung "biotinAdV/complex B").

0.6 µg pCMVL-DNA in 67 µl HBS wurden mit 0.3 µg Streptavidin-Polylysin in 33 µl HBS gemischt. Nach 20 min wurden 65 µl biotinyliertes Adenovirus oder als Kontrolle entsprechende Mengen an Adenovirus dl312 (30 µl, Ausgangsvirus für die Modifizierung) zugegeben.Die Komplexmischungen ("biotinAdV/complex A" bzw. "control AdV", siehe Fig. 29) wurden weitere 20 min stehengelassen und dann mit HBS auf 500 µl verdünnt. Die alternative Komplexbildung wurde durchgeführt, indem 65 µl biotinylierter Adenovirus zuerst mit 0.3 µg Streptavidin-Polylysin in 50 µl HBS gemischt wurden, nach 20 min 0.6 µg pCMVL-DNA in 50 µl HBS zugegeben wurden. Die Komplexmischung ("biotinAdV/complex B") wurden weitere 20 min stehengelassen und dann mit HBS auf 500 µl verdünnt.

Die Mischungen wurden den Zellen beigegeben. Die Zellen wurden 2 h lang bei 37°C inkubiert, dann wurden 2.5 ml frisches Medium mit einem Zusatz von 10 % FCS zugegeben. Die Zellen wurden 24 h lang bei 37°C inkubiert und dann für den Luciferase-Assay geerntet. Die Luciferaseaktivität wurde bestimmt, wie in den vorangegangenen Beispielen angegeben. Die in Fig. 29 angegebenen Werte stellen die Gesamtluciferaseaktivität der transfizierten Zellen dar.

Parallel dazu wurden Transfektionen von HeLa-Zellen durchgeführt, wobei als Virus-Komponente des Konjugats ein biotinyliertes Virus verwendet wurde, das mittels Psoralen/UV-Behandlung inaktiviert worden war. Die Inaktivierung wurde wie folgt vorgenommen: Je 200 µl biotinylierte Viruspräparation wurden in zwei Vertiefungen einer 1.6 cm Gewebskulturplatte gegeben. Zu jeder Probe wurden 2 µl (33 mg/ml) 8-Methoxypsoralen (in DMSO) gegeben, die Schale auf Eis gestellt und 10 min lang mit einer UV-Lampe (365 nm; UVP TL-33 Lampe) bestrahlt, wobei der Abstand der Probe vom Filter 4 cm betrug. Nach der Bestrahlung wurden die zwei Proben vereinigt und gelfiltriert (G50, Nick-Säule, Pharmacia), wobei die Säule mit 40 % Glycerin in HBS vorequilibriert worden war. Aliquots von je 75 µl wurden mit 0.8 µg Streptavidin-Polylysin komplexiert und zur Transfektion von HeLa-Zellen, wie oben beschrieben, verwendet.

Mittels zytopathischem Endpunkt-Assay wurde festgestellt, daß der Virustiter durch die Inaktivierung um einen Faktor von mehr als 10⁴ abnahm, während die Transferkapazität bei hohen Konzentrationen um weniger als 50 % und bei niedrigen Konzentrationen um einen Faktor 5 abnahm.

### d) Transfektion von K562-Zellen

K562-Zellen wurden in Suspension in RPMI 1640-Medium (Gibco BRL, plus 2 g Natriumbikarbonat pro Liter) + 10 % FCS, 100 Einheiten/ml Penicillin, 100 µg/ml Streptomycin und 2 mM Glutamin bis zu einer Zelldichte von 500.000 Zellen/ml gezüchtet. 16 h vor der Transfektion wurden die Zellen in frisches Medium, enthaltend 50 µM Desferrioxamin (Sigma) gegeben. Am Morgen nach der Transfektion wurden die Zellen zu 250.000 Zellen/ml in frisches Medium, enthaltend 10 % FCS + 50 uM Desferrioxamin, aufgenommen und 2 ml pro Vertiefung in eine Platte mit 24 Vertiefungen gegeben.

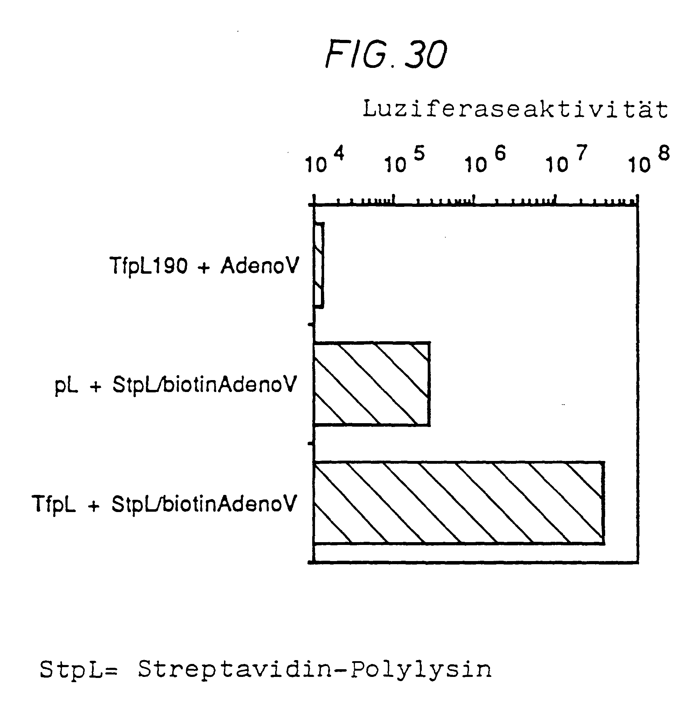

Es wurden drei verschiedene Arten von DNA-Komplexen hergestellt:
a) Eine Lösung von 6 µg pCMVL-DNA in 160 µl HBS (150 mM NaCl, 20 mM HEPES pH 7.3) wurde mit 12 µg des Konjugats TfpL190B in 160 µl HBS gemischt, nach 30 min wurden 20 µl Adenovirus dl312-Präparation hinzugefügt und die Mischung den Zellen beigegeben.
b) Eine Lösung von 800 ng Streptavidin-Polylysin in 160 µl HBS wurde mit 20 µl biotinyliertem Adenovirus, hergestellt wie in a), gemischt, nach 30 min wurde eine Lösung von 6 µg pCMVL-DNA in 160 µl HBS zugegeben und nach weiteren 30 min die Lösung mit 10 µg des Konjugats TfpL190B in 160 µl HBS gemischt. Nach 30 min wurde die Mischung den Zellen beigegeben.
c) Die DNA-Komplexe wurden hergestellt wie in b), mit dem Unterschied, daß statt TfpL190B eine Lösung von 3.5 µg Poly(L)lysin p(Lys)290 zugegeben wurde. Die Zellen wurden 24 h lang bei 37°C inkubiert und dann für den Luciferaseassay geerntet. Die in Fig. 30 dargestellten Werte stellen die Gesamtluciferaseaktivität der transfizierten Zellen dar.

### Beispiel 20

### Gentransfer in primäre Knochenmarkszellen

### a) Isolierung von Knochenmarkszellen

Primäre Knochenmarkszellen wurden aus Mäusen geerntet, indem Kulturmedium (IMDM, enthaltend 10 % FCS, 5 x 10⁻ 5_{M} β-Mercaptoethanol, 1 % IL-3 konditioniertes Medium und Antibiotika) mit einer Injektionsnadel (0.4 mm oder 0.5 mm Durchmesser), verbunden mit einer 1 ml Ampulle, durch isolierte Oberschenkel- und Schienbeinknochen gespült wurde. Die Zellen wurden dann 1 x in Kulturmedium durch Zentrifugieren bei 100 x g 8 min lang gewaschen. Daraufhin wurden die Zellen bei einer Konzentration von 10⁷ Zellen/ml resuspendiert und in T25-Kulturflaschen ausgesät. Nach 4 h wurden die nichthaftenden Zellen in eine neue T25-Kulturflasche überführt und über Nacht in Gegenwart von 50 µM Desferrioxamin gezüchtet.

### b) Bildung von Adenovirus-Transferrin-Polylysin/DNA-Komplexen

Für die Komplexbildung wurden 50 µl biotinyliertes Adenovirus mit 400 ng Streptavidin-modifiziertem Polylysin in 20 µl HBS 20 min lang inkubiert. Dann wurden 20 µl HBS, enthaltend 6 µg pCMVL beigegeben. Nach einer Inkubationszeit von 20 min wurden 7 µg Maustransferrin-Polylysin-Konjugat (mTfpL) in 160 µl HBS beigegeben und die ganze Mischung weitere 20 min inkubiert.

### c) Transfektion

Für die Transfektion wurden die Knochenmarkszellen durch 8 minütiges Zentrifugieren bei 100 x g aus dem Kulturmedium gewonnen. Der Zellniederschlag wurde in 3 ml Kulturmedium, enthaltend 2 % FCS und 250 µl der Adenovirus-Transferrin-Polylysin/DNA-Komplexe, aufgenommen und in einer neuen T25-Flasche 3 h lang bei 37°C gezüchtet. Dann wurden 3 ml und nach 2 h weitere 6 ml Kulturmedium, enthaltend 10 % FCS beigegeben.

### d) Bestimmung der Luciferaseexpression

48 h nach der Transfektion wurden die Zellen geerntet und wie in den anderen Beispielen auf Luciferaseexpression untersucht. Die Transfektion führte zu einer Luciferaseaktivität entsprechend 310 x 10³ Lichteinheiten/100 µg Gesamtzellprotein.

### Beispiel 21

### Transfektion von Neuroblastomzellen mit einem 48 kb Cosmid in Gegenwart von freiem Adenovirus und von Adenovirus-Polylysin-Konjugaten

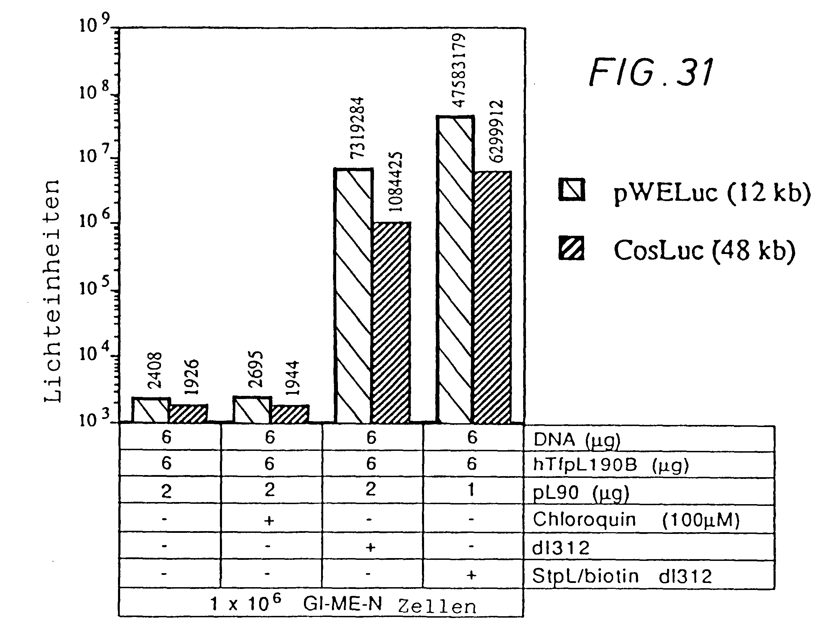

Zellen der Zellinie der Bezeichnung GI-ME-N wurden, wie in Beispiel 16 beschrieben, mit dem 48 kb Cosmid mit den angegebenen Mengen von hTfpL, freiem Polylysin und DNA transfiziert. Zusätzlich enthielten die Inkubationsmischungen entweder 100 µM Chloroquin (Spalten 3 und 4) oder 10 µl Adenovirus dl312, enthaltend 5 x 10¹¹ Partikel pro ml (Spalten 5 und 6). Die letzten beiden Proben (Spalten 7 und 8, StpL/Biotin) enthielten 15 µl biotinyliertes Adenovirus dl312 (1 x 10¹¹ Partikel), 30 min inkubiert mit Streptavidin-Polylysin (0.8 µg, hergestellt wie in Beispiel 19) in 150 µl HBS. Dann wurden 6 µg DNA in 150 µl HBS zur Probe gegeben, 30 min bei Raumtemperatur stehengelassen, worauf 150 µl HBS, enthaltend 6 µg hTfpL + 1 µg freies pL zugegeben wurden. Nach weiteren 30 min Inkubation bei Raumtemperatur wurde die Mischung den Zellen beigegeben. Nach einer 2 stündigen Inkubation bei 37°C wurden jeder Schale 4 ml DMEM + 10 % FCS beigegeben. 24 Stunden später wurden die Zellen geerntet und die Luciferaseaktivität gemessen. Die Ergebnisse sind in Fig. 31 gezeigt.

### Beispiel 22

### Gentransfer in primäre Atemwegs-Epithelzellen

Vorversuche im Hinblick auf eine genetische Korrektur der Zystischen Fibrose hatten gezeigt, daß immortalisierte Zellinien, abgeleitet vom Atemwegsepithel der erfindungsgemäßen Gentransfermethode zugänglich sind. Um die Möglichkeit auszuschließen, daß dieses Phänomen auf Veränderungen des Atemwegsepithels zurückzuführen ist, die durch die Immortalisierung induziert sind, wurden die Versuche mit Transferrin-Polylysin-Konjugaten auch in primären Atemwegsepithelzellen (1°AE) durchgeführt.

1°AE-Zellen wurden, wie von Yankaskas et al., 1987, beschrieben, aus Nasenpolypenproben vom Patienten erhalten. Die Gewebe wurden in steriler Kochsalzlösung gewaschen, dann in Joklik's Minimum Essential Medium (MEM) plus Antibiotika (Penicillin 50 E/ml, Streptomycin 50 µg/ml, Gentamicin 40 µg/ml) bei 4°C ins Labor transportiert. Knorpel- und überschüssiges submuköses Gewebe wurden freigelegt und die epithelialen Schichten in Proteaselösung (Sigma, Typ 14, 0.1 mg/dl) in MEM bei 4°C 16 bis 48 h inkubiert. Es wurde 10 % FBS (fötales Rinderserum) zur Neutralisierung der Protease beigegeben und die Zellen durch leichtes Bewegen abgelöst. Die erhaltene Suspension wurde durch ein 10 µm Nylongitter filtriert, um die Zelltrümmer zu entfernen, zentrifugiert (150 x g 5 min) und in F12 + 10 % FBS gewaschen.

Die Zellen wurden daraufhin mit Transferrin-Polylysin-Konjugaten (hTfpL) und einem für Luciferase kodierenden Plasmid (pRSVL) als Reportergen behandelt. Bei dieser Untersuchung zeigten die primären Zellen nicht die Empfänglichkeit für diese Komplexe wie die entsprechenden immortalisierten Zellinien (Untergrund = 429 Lichteinheiten; mit Zusatz von Konjugaten : 543 Lichteinheiten), was darauf hinwies, daß die 1°AE-Zellen relativ arm an Transferrin-Rezeptoren sind.

Um einen alternativen Rezeptor auf den Zellen zu benutzen, wurden biotinylierte Adenoviren (vgl. Beispiel 19) verwendet. Die mit diesem Konjugat behandelten Zellen zeigten eine signifikant stärkere Expression als Untergrundwerte (Zusatz von Konjugaten 2585753 ± 453585 Lichteinheiten). Außerdem erwiesen sich auch primäre Atemwegsepithelzellen von anderen Spezies als dieser Methode des Gentransfers zugänglich (Maus = 3230244 ± 343153; Affe = 53498880 ± 869481 Lichteinheiten).

### Beispiel 23

### Gentransfer in Hepatozyten und in Blutzellen

In den Experimenten dieses Beispiels wurden folgende Materialien und Methoden verwendet:
Transfektion von Gewebekulturzellen: Zellen der Zellinie BNL CL.2 wurden gezüchtet, wie in Beispiel 6 beschrieben. HeLa-Zellen und Hepatozyten wurden in 6 cm Petrischalen gezüchtet. Die Transfektion wurde bei einer Zelldichte von ca. 3 x 10⁵ Zellen pro Schale durchgeführt. Vor der Transfektion wurde das Standardkulturmedium durch 1 ml frisches Medium mit 2 % FCS ersetzt.

Bildung binärer Komplexe: Biotinylierte Adenoviren (ca. 10⁹ PFUs, siehe Beispiel 19 a) und 19 b)) wurden mit 800 ng streptavidyniliertem Polylysin in 50 µl HBS reagieren gelassen. Nach 30 min bei Raumtemperatur wurden 6 µg pCMVL-DNA in 170 µl HBS zugegeben, 30 min inkubiert und daraufhin 3 µg pL300 in 200 µl HBS zugegeben und nach weiteren 30 min die Lösung für die Transfektionsversuche verwendet.
Bildung ternärer Komplexe: Biotinylierte Adenoviren (ca. 10⁹ PFUs)) wurden mit 800 ng streptavidyniliertem Polylysin in 50 µl HBS reagieren gelassen. Nach 30 min bei Raumtemperatur wurden 6 µg pCMVL-DNA in 170 µl HBS zugegeben, 30 min inkubiert und daraufhin 10 µg TfpL190B in 200 µl HBS zugegeben und nach weiteren 30 min die Lösung für die Transfektionsversuche verwendet.

β-Galaktosidase-Assay: BNL CL.2-Zellen wurden auf Deckgläser ausgesät und 24 h die Zellen mit dem Reporterplasmid pCMV-β gal (Lim und Chae, 1989) transfiziert. 48 h später wurde der β-Galaktosidase-Test durchgeführt wie in Beispiel 15 angegeben.

### a) Die Verbindung zwischen DNA-Kondensaten und Adenovirus verstärkt die Luciferase-Reportergen-Expression in starkem Ausmaß

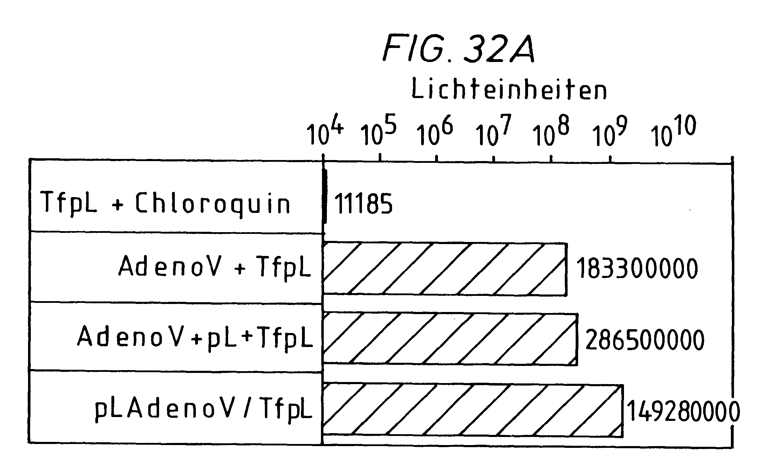

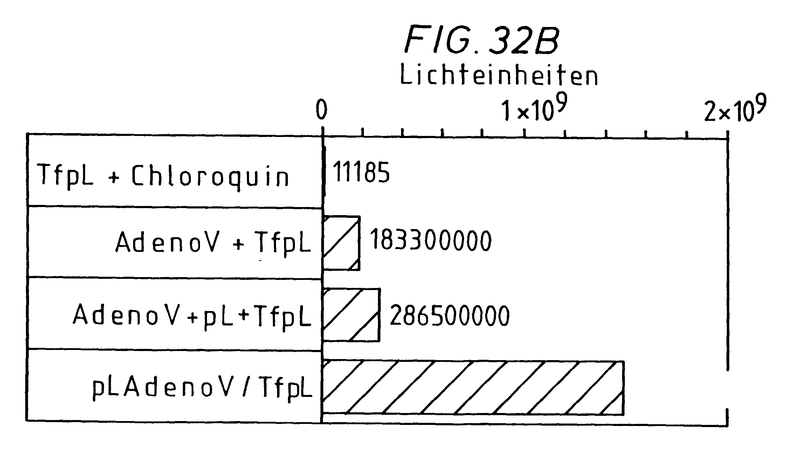

Das Ergebnis des Transfers von DNA in Hepatozyten mittels binären und ternären DNA-Komplexen ist in Fig. 32 dargestellt: Der Gentransfereffekt wurde in Gegenwart von freiem Adenovirus verstärkt. Die Spalten pLAdenoV/TfpL zeigen die Ergebnisse von Transfektionen mit Adenovirus, das mittels Transglutaminase mit Polylysin konjugiert worden und dann mit DNA reagieren gelassen wurde, die einen Teil der negativen Ladungen neutralisierte. Später wurde Transferrin-Polylysin beigegeben, das die restlichen negativen Ladungen neutralisierte. Auf diese Weise wurde ein ternärer Komplex Adenovirus-Polylysin/Transferrin-Polylysin/DNA gebildet. Wie aus der Fig. ersichtlich, wurde ein außerordentlich hoher Wert von 1.5x10⁹ Lichteinheiten erhalten (entsprechend ca. 5000 Lichteinheiten pro Zelle). Für den in Spalte Adenov+pL+TfpL dargestellten Versuch wurden Adenovirus und Polylysin gemischt wie für die Behandlung mit Transglutaminase. Um die Spezifität der durch Transglutaminase bewirkte Bindung von Polylysin an das Virus zu zeigen, wurde jedoch das Enzym weggelassen. Dann wurde die Viruspräparation mit derselben Menge DNA und TfpL komplexiert wie in pLAdeno/TfpL. In diesem Fall war die Transfektion mäßig wie mit AdenoV+TfpL, weil in beiden Experimenten die gemeinsame Lokalisierung von Virus und DNA/Transferrin-Polylysin-Komplex ein stochastischer Vorgang war, im Gegensatz zu dem in Spalte pLAdenoV/TfpL gezeigten Experiment, bei dem die gemeinsame Lokalisierung durch die Verbindung von Virus und DNA in dem ternären Komplex ein hohes Maß an Transferrinfektion (Transfektion mit Transferrin) liefern.

### b) Die Transfektion von K562-Zellen zeigt die endosomolytischen Eigenschaften von Adenovirus

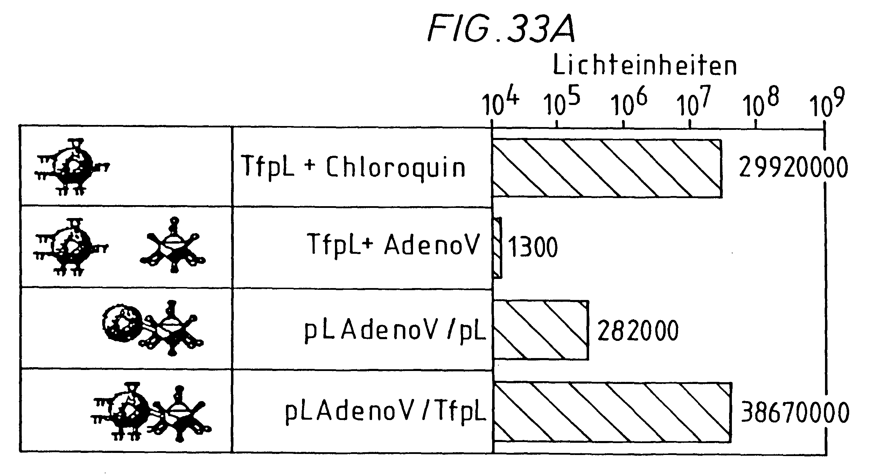

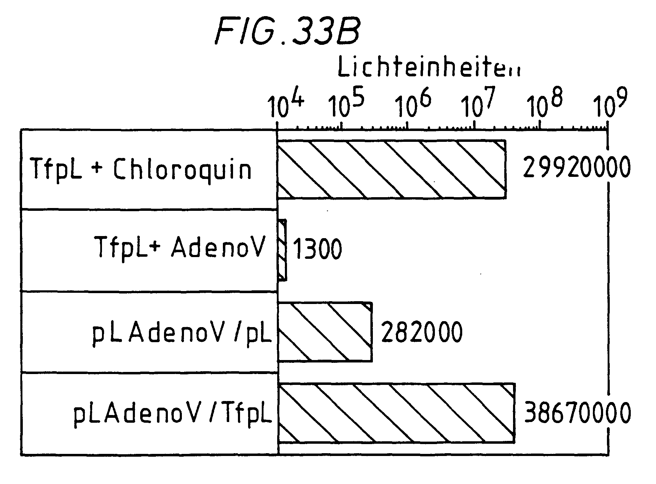

Zellen der humanen Erythroleukämie-Zellinie K562 enthalten ca. 150 000 Transferrin-Rezeptoren (Klausner et al., 1983 b). In Gegenwart von Chloroquin können diese Zellen auch in Abwesenheit von Adenovirus in starkem Ausmaß mit TfpL/Reporter-DNA-Komplexen transfiziert werden (Fig. 33, TfpL), wie bereits von Cotten et al., 1990, berichtet wurde. Dieselben Komplexe liefern in Gegenwart von freiem Adenovirus, aber in Abwesenheit von Chloroquin, relativ schwache Reportergenexpression (AdenoV/TfpL), vermutlich weil K562 Zellen wie andere Blutzellen (Silver et al., 1988; Horvath et al., 1988) eine geringe Zahl an Adenovirusrezeptoren aufweisen. Wenn das Adenovirus über eine Biotin/Streptavidin-Brücke an Polylysin gebunden wird und die Reporter-DNA durch Zusatz von mehr Polylysin zur Gänze kondensiert wird, um den binären Komplex zu komplettieren (pLAdenoV/pL), erreicht die durch Adenovirus unterstützte Transfektion mittlere Werte, vermutlich weil die wenigen Adenovirus-Rezeptoren effizient genützt werden. Wenn jedoch die mit Adenovirus-Polylysin komplexierte DNA zur Gänze kondensiert und durch Zusatz von Transferrin-Polylysin unter Bildung eines ternären Komplexes (pLAdenoV/TfpL) neutralisiert wird und die zahlreichen zellulären Transferrinrezeptoren ins Spiel kommen, wird die Transfektionseffizienz - sowohl dank der effizienten Transferrinbindung als auch den endosomolytischen Eigenschaften des Virus - um mindestens zwei weitere Größenordnungen gesteigert (Fig. 33).

### c) Ternäre DNA-Komplexe führen zur Expression des Reportergens in beinahe 100 % der Hepatozyten

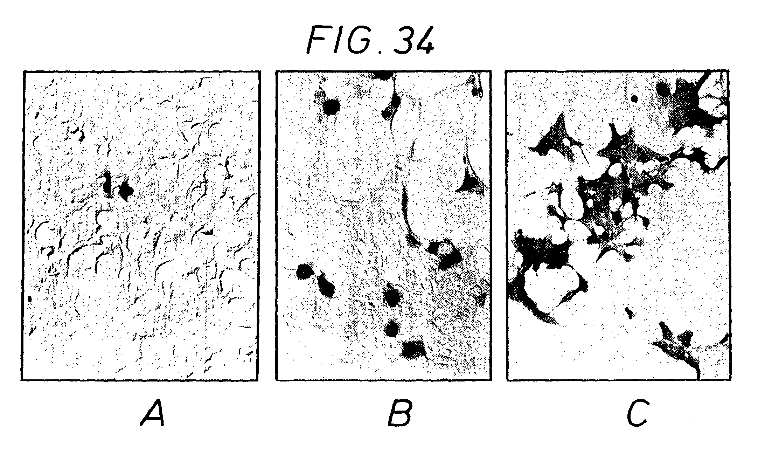

Um die Effizienz des Transportsystems in Maushepatozyten BNL CL.2 zu testen, wurden die Zellen mit dem β-Galaktosidase-Reportergen transfiziert. Fig. 34 zeigt den β-Galaktosidase-Assay nach a) Transferrinfektion in Gegenwart von Chloroquin, b) Transferrinfektion in Gegenwart von freiem Adenovirus dl312 und c) Transfektion mit ternären Adenovirus (dl312)-Polylysin-Transferrin-DNA-Komplexen. In Abwesenheit von Adenovirus exprimieren nach Standardtransferrinfektion nur wenige Zellen das Reportergen. Der Prozentsatz der Transfektion beträgt weniger als 0.1 %. Wenn Chloroquin mitverwendet wird, wird der Prozentsatz auf ca. 0.2 % gesteigert (Fig. 34A). Mit freiem Adenovirus exprimieren ca. 5-10 % der Zellen das Reportergen (Fig. 34B), während die ternären Komplexe mit Transglutaminase-modifiziertem Virus zur Expression in den meisten, wenn nicht allen Zellen führen (Fig. 34C). Da die ternären Komplexe bei hoher Verdünnung verwendet werden können, entsteht der bei hohen Dosen von freiem (inaktiviertem) Adenovirus beobachtete toxische Effekt für gewöhnlich nicht. Es ist jedoch zu beachten, daß bei Verwendung der ternären Komplexe in hoher Konzentration, um 100 % der Gewebekulturzellen zu erreichen, ein ähnlicher toxischer Effekt bemerkbar wird. Die toxischen Wirkungen können ihre Ursache in restlicher viraler Genaktivität oder in den endosomolytischen Eigenschaften des zugesetzten Virus haben, oder sie sind einfach eine Folge der sehr hohen Expression des transfizierten Gens.

### d) Die Expression eines transfizierten Reportergens ist transient, hält aber in nicht-teilenden Hepatozyten wochenlang an

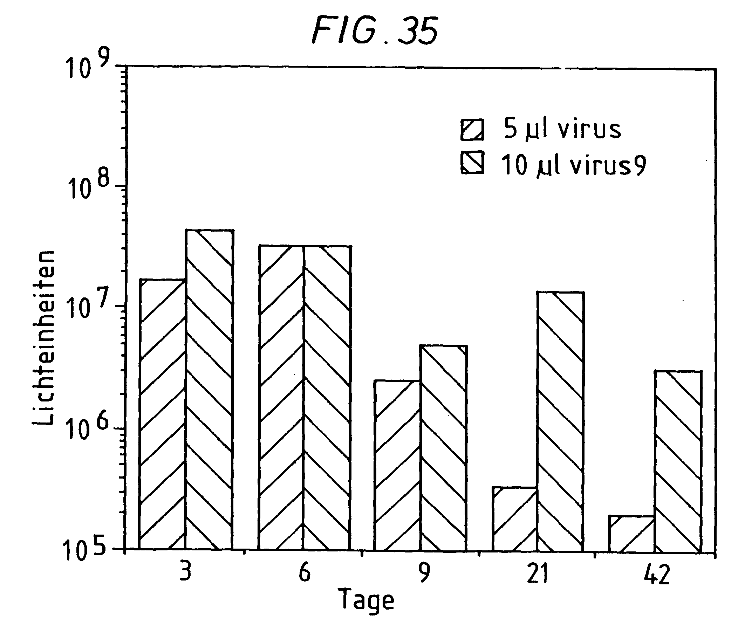

Ternäre Transportkomplexe (pLAdenoV/TfpL) wurden mit Polylysin-Adenovirus und mit modifiziertem Adenovirus, das durch Reaktion mit Psoralen weiter inaktiviert worden war, hergestellt. Eine zu 2/3 konfluente Hepatozyten-Zellkultur wurde wie in dem in Fig. 34B dargestellten Experiment mit dem Luciferase-Reportergenplasmid pCMVL transfiziert und die Luciferaseaktivität zu verschiedenen Zeitpunkten gemessen. Wie aus Fig. 35 ersichtlich ist, war die Luciferaseaktivität nach 3 Tagen, als die Hepatozytenzellkultur konfluent wurde und die Zellen aufhörten, sich zu teilen, am höchsten. Die Expression des Reportergens hielt in der sich nicht teilenden Zellkultur an, ohne daß eine Selektion auf die Erhaltung des Gens angewendet wurde und dauerte mindestens 6 Wochen lang an, insbesondere wenn Psoralen-inaktiviertes Adenovirus für die Bildung der ternären Komplexe verwendet wurde.

### Beispiel 24

### Verwendung des Hühner-Adenovirus CELO zur Verstärkung des DNA-Transports in humane Zellen

In diesem Beispiel wurde das Hühner-Adenovirus CELO auf seine Eignung zur Verstärkung des DNA-Transfers in humane HeLa-Zellen analog zu den vorangegangenen Beispielen, die das humane Adenovirus Typ 5 verwenden, getestet.
Es wurde das Hühner-Adenovirus CELO (Stamm Phelps, Serotyp FAV-1,7, Hühnernierenzellpassage) verwendet. Das Virus (2 ml) wurde durch eine PD-10 Gelfiltrationssäule, equilibriert mit 20 mM HEPES pH 7.3, 150 mM NaCl (HBS) + 10 % Glyzerin, und 2 ml des Eluats wurden mit 20 µl 1 mM NHS-LC-Biotin (Pierce) 3 h lang bei Raumtemperatur reagieren gelassen. Das biotinylierte Virus wurde daraufhin gegen 3 x 300 ml HBS + 40 % Glyzerin bei 4°C dialysiert und in Aliquots bei -70°C gelagert.

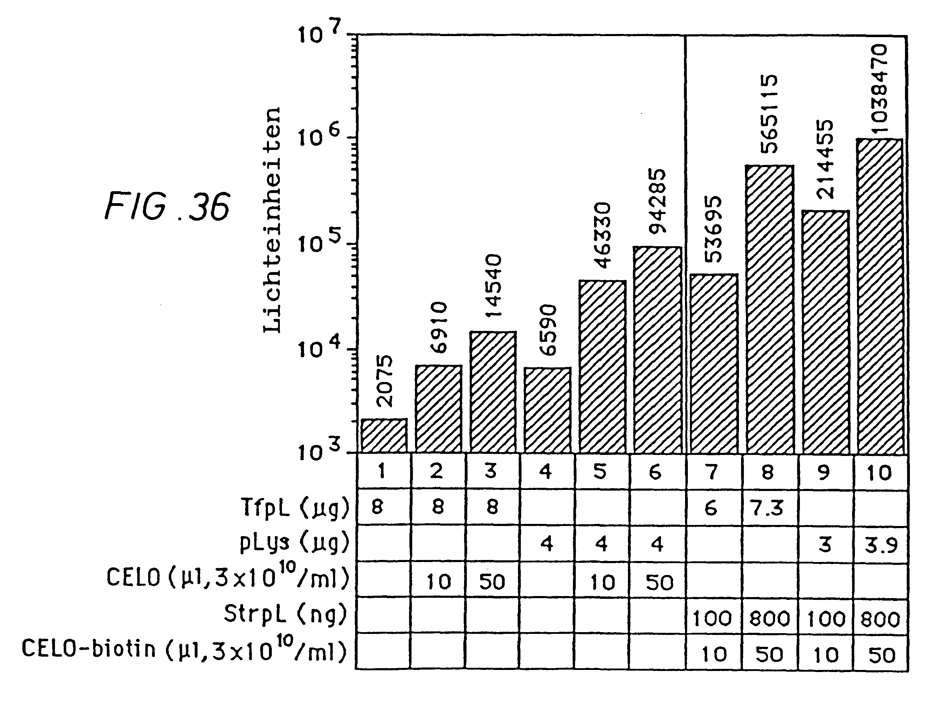

HeLa-Zellen (5 x 10⁵ Zellen pro 6 cm Schale) wurden in 2 ml DMEM + 2 % FCS mit 6 µg des Plasmids pCMVL, komplexiert mit Polylysin(pLys)- oder Transferrin-Polylysin(TfpL)-Mischungen in 500 µl HBS inkubiert (die Komplexe wurden 30 min bei Raumtemperatur vorinkubiert). Die Proben wurden daraufhin in Gegenwart der in Fig. 36 angegebenen Virusmengen zu den Zellen gegeben. Bei den Proben, die biotinyliertes CELO-Virus enthielten, wurde die angegebene Menge an Virus mit der angegebenen Menge von Streptavidin-Polylysin (StrpL) in 200 µl HBS 30 min lang bei Raumtemperatur vorinkubiert, bevor 6 µg des Plasmids pCMVL in 100 µl HBS beigegeben wurden. Nach einer 30 minütigen Inkubation bei Raumtemperatur wurde die angegebene Menge an TfpL-Material bei 37°C zu den Zellen gegeben. 2 h später wurden 5 ml DMEM + 10 % FCS den Zellen beigegeben und 24 h später die Zellen geerntet und für den Luciferaseassay vorbereitet.
Wie sich aus Fig. 36 ergibt, verstärkte das CELO-Virus in freier Form den DNA-Transport in HeLa-Zellen (Spalten 1-6). Wenn jedoch das CELO-Virus mit Biotin modifiziert und in einem Komplex mit Streptavidin enthalten war, zeigte sich, daß das Virus sowohl mit als auch ohne zusätzliches Transferrin-Polylysin den DNA-Transfer in einem mit mittels humanem Adenovirus d1312 erreichtem vergleichbaren Ausmaß verstärkt wird. Die spezielle Linie von HeLa-Zellen zeigt eine hohe Bindungskapazität für Polylysin/DNA-Komplexe in Abwesenheit von Transferrin (vgl. die Luciferaseaktivität der Proben 1 und 4 in Fig. 36). Es ist daher der Einschluß des CELO-Virus in einem Polylysin-DNA-Komplex ausreichend, die Aufnahme des Komplexes zu bewirken.

### Beispiel 25

### Transfektion von Myoblasten

### a) Transfektion von Myoblasten und Myotuben mit DNA/Transferrin-Polylysin-Komplexen in Gegenwart von freiem Adenovirus und in Gegenwart von Biotin/Streptavidin-gekoppeltem Adenovirus

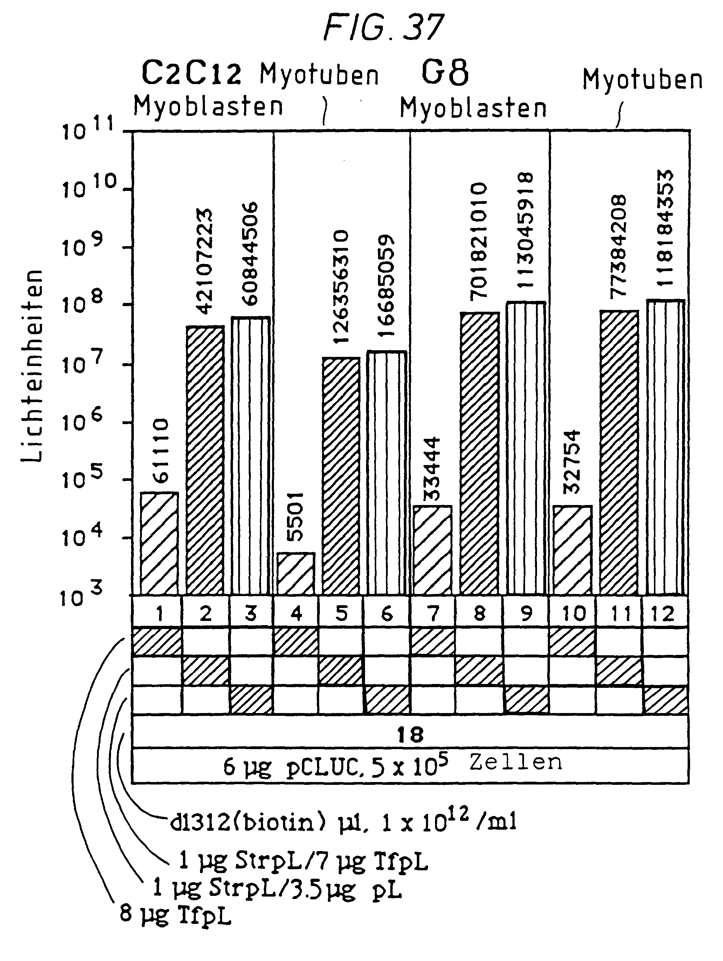

C2C12 Myoblasten (Blau et al., 1985; ATCC Nr.: CRL 1772) und G8-Myoblasten (ATCC Nr.: CRL 1456) wurden in "high glucose DMEM" + 10 % FCS Myoblastenkulturen wurden bei Subkonfluenz mit ca. 5 x10⁵ Zellen pro 6 cm Schale transfiziert. Myotubenkulturen wurden hergestellt, indem Myoblasten in 6 cm Schalen ausplattiert wurden (ca. 5 x 10⁵ Zellen pro Schale) und das Medium gegen "high glucose DMEM" + 2 % Pferdeserum ausgetauscht wurde, als die Zellen Konfluenz erreichten (Barr und Leiden, 1991; Dhawan et al., 1991). Die Transfektionen der Myotuben wurden 5 bis 7 Tage später durchgeführt. Die Transfektionskomplexe wurden hergestellt, wie in Beispiel 19 beschrieben, wobei die angegebenen Mengen an TfpL, StrpL und biotinyliertes Adenovirus dl312 verwendet wurden. Die Zellen wurden 20 h nach der Transfektion geerntet und die Luciferaseaktivität gemessen. Die in Fig. 37 angegebene Luciferaseaktivität bezieht sich auf die gesamte Zellprobe. Es zeigte sich, daß sowohl Myoblasten- als auch Myotubenkulturen mit hoher Effizienz transfiziert werden konnten. Nach Differenzierung zu Myotuben fiel die Transfektionseffizienz weniger als 1 log (C2C12) ab oder zeigte keine signifikante Abnahme (G8). Die Bildung von Myotuben ereignete sich bei der G8-Zellinie bei einer geringeren Frequenz, was zum Teil für das Fehlen einer nachweisbaren Abnahme der Leistungsfähigkeit in der differenzierten Kultur verantwortlich sein könnte. Die Rolle der Wechselwirkung zwischen Transferrin und dem Transferrinrezeptor im DNA-Transport in diesen Zelltyp war nicht wesentlich. In allen vier Zellpräparationen fand nur schwacher DNA-Transport unter Verwendung von TfpL/DNA-Komplexen in Gegenwart von freiem Adenovirus d1312 statt (Spalten 1, 4, 7, 10). Bei Verwendung des gekoppelten Virus wurde die Leistungsfähigkeit der Transfektion verstärkt (Spalten 2, 3, 5, 6, 8, 9, 11, 12). Beim Vergleich des mit Kombinations-Komplexen, enthaltend nur Virus und Polylysin/StrpL, erreichten Gentransfers mit den Ergebnissen, die mit Komplexen erhalten wurden, die Transferrin-Polylysin miteinschließen, ergab sich eine Zunahme der Leistungsfähigkeit um weniger als 1 log (vgl. z.B. Spur 2, kein Transferrin, mit Spur 3, Transferrin). Die geringe Transfektion mit freiem Virus und die starke Transfektion mit gekoppelten Viruskomplexen sowohl in Gegenwart als auch in Abwesenheit von Transferrin-Polylysin lassen vermuten, daß das Adenovirus bei diesen Zellen als Ligand dient und daß ohne Kopplung das freie Virus in die Zellen zwar eindringen mag, jedoch der TfpL/DNA-Komplex nicht in produktivem Ausmaß eintritt (die in diesem Beispiel verwendete pCMVL-DNA ist in der Figur mit pCLuc bezeichnet).

### b) Histochemische Analyse der Transfektionshäufigkeit in Myotuben

C2C12 Myotubenkulturen (wie die Myoblasten in 6 cm Schalen ausgesät, 5 x 10⁵ Zellen, differenziert in Myotuben) wurden hergestellt, wie in a) beschrieben. Bei den Proben mit freiem Virus wurde pCMVβ-gal DNA (6 µg) mit 8 µg TfpL in Gegenwart von 500 µl HBS komplexiert und den Zellen in Gegenwart von 18 µl Adenovirus dl312 (1 x 10¹² Viruspartikel pro ml) in 2 ml DMEM/2 % FCS beigegeben. Die Proben mit gekoppeltem Virus wurden mit pCMVLacz DNA ( 6 µg), komplexiert mit 7 µg TfpL und 800 ng StrpL + 18 µl biotinyliertem Adenovirus dl312 (1 x 10¹² Viren pro ml) in 500 µl HBS, hergestellt und den Zellen in 2 ml DMEM/2 % FCS beigegeben. Nach einer Inkubation von 24 h wurden die Zellen, wie in Beispiel 15 beschrieben, zur Bestimmung der β-Galaktosidaseaktivität gefärbt.

Die β-Galaktosidase-Färbemuster waren im Einklang mit den Ergebnissen der Transfektionen, bei denen Luciferase als Reportergenprodukt (siehe a)) verwendet wurde. In Myotubenkulturen wurde sehr niedrige Genexpression erhalten, wenn freies Virus verwendet wurde, während die Verbindung von Virus und DNA ein hohes Genexpressionsniveau ergibt. Das Vorhandensein von blaugefärbten vielkernigen Tubuli zeigte den erfolgreichen Transfer eines Gens in diese differenzierten Zellen in Gegenwart von freiem Adenovirus an.

### c) Transfer von DNA in primäre Maus-Myoblasten- und Maus-Myotubenkulturen

Die großen Skelettmuskel von den beiden Hinterbeinen einer 4 Wochen alten männlichen C57Bl/6 Maus wurden steril in PBS isoliert und in ca. 5 mm Stücke zerkleinert. Das Gewebe wurde in 20 ml PBS suspendiert, ca. 2 min absetzen gelassen und der Überstand abgesaugt. Dieses Waschen wurde 3 mal wiederholt. Das Gewebe wurde daraufhin mit 3.5 ml PBS plus 0.5 ml Trypsin/EDTA, 0.5 ml 1 % (Gew./Vol.) Kollagenase, Typ 2 Sigma und 0.5 ml 1 % BSA (Fraktion V in 4 mM CaCl₂) gemischt und bei 37°C 30 min lang unter häufigem vorsichtigen Rühren inkubieren gelassen. Am Ende der 30 minütigen Inkubation wurde das restliche Gewebe absetzen gelassen, der Überstand entfernt und mit 5 ml DMEM + 20 % FCS gemischt. Die Inkubation mit Protease wurde 3 - 4 mal wiederholt, bis das Gewebe vollständig dispergiert war. Die Zellsuspension wurde daraufhin durch ein Zellsieb (Falcon) geschickt, um Aggregate und Gewebsfragmente zu entfernen, und bei 500 x g 15 min lang zentrifugiert. Das Zellpellet wurde in 10 ml DMEM + 20 % FCS resuspendiert und die Fibroblasten entfernt, indem die Zellen 60 min lang auf einer unbedeckten Gewebekulturschale mit 15 cm Durchmesser plattiert wurden. Die nicht haftenden Zellen wurden dann vorsichtig entfernt und auf fünf Laminin-beschichtete 10 cm Gewebekulturschalen mit 15 ml DMEM + 20 % FCS pro Schale plattiert. Nach Erreichen der Konfluenz (ca. 1 Woche später) wurden die Zellen trypsiniert und zu ca. 1 x 10⁶ Zellen pro Schale auf Laminin-beschichtete 6 cm Schalen replattiert. Um Myotubenkulturen zu bilden, wurde ca. 5 Tage später (als die Zellen Konfluenz erreicht hatten) das Medium gegen DMEM + 2 % Pferdeserum gewechselt, und eine Woche später wurden Transfektionen durchgeführt. Die Transfektion der Myoblastenkulturen wurde in 6 cm Schalen bei ca. 80 % Konfluenz vorgenommen. Die Laminin-beschichteten Zellkulturplatten wurden wie folgt hergestellt:
Zellkulturschalen wurden mit 0.025 mg/ml Polylysin (MG 30.000 - 70.000, Sigma) in sterilem Wasser 30 min lang bei Raumtemperatur beschichtet. Die Platten wurden 3 x mit sterilem Wasser gespült und luftgetrocknet. Dann wurden die Platten mit 8 µg/ml Laminin (EHS, Sigma) in Wasser über Nacht bei Raumtemperatur beschichtet. Die Platten wurden dann 3 x vor dem Aussäen der Zellen gewaschen.

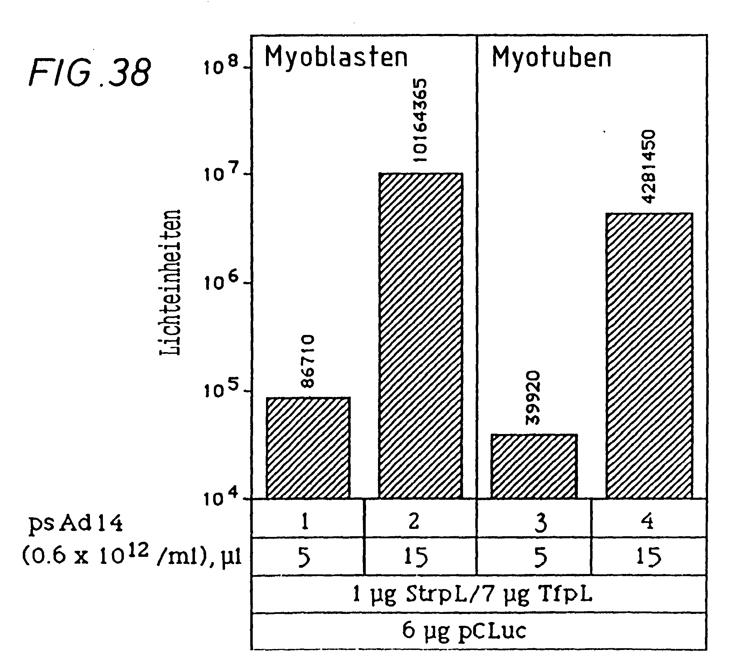

Die für die Transfektion verwendeten DNA-Komplexe wurden hergestellt, indem die angegebene Menge Psoralen/UV-inaktiviertes biotinyliertes Adenovirus d1312 (hergestellt wie in Beispiel 19) in 150 µl HBS verdünnt, 1 µg StrpL in 150 µl HBS zugegeben und anschließend 30 min bei Raumtemperatur inkubiert wurde. Daraufhin wurde jeder Probe HBS (100 µl), enthaltend 6 µg pCMVL (in der Fig. pCLuc bezeichnet), beigegeben und weitere 30 min bei Raumtemperatur inkubiert. Abschließend wurden jeder Probe 7 µg TfpL in 100 µl HBS beigegeben, 30 min bei Raumtemperatur inkubiert und dann entweder den Myoblasten- oder Myotubenkulturen in 6 cm Schalen, enthaltend 2 ml DMEM + 2 % FCS, zugegeben. Nach einer Stunde Inkubation wurde das Medium durch 5 ml DMEM + 20 % FCS (Myoblasten) bzw. DMEM + 2 % Pferdeserum (Myotuben) ersetzt und die Zellen 48 h später für die Luciferaseanalyse geerntet. Die Luciferaseaktivität der gesamten Zellprobe ist in Fig. 38 dargestellt.

### Beispiel 26

Verbesserung des Transfers mittels CELO-Virus in Myoblasten unter Verwendung eines Lectin-Liganden

### a) Vergleichende Analyse von Adenovirus dl312 und CELO-Virus in HeLa-Zellen und C2C12-Myoblasten

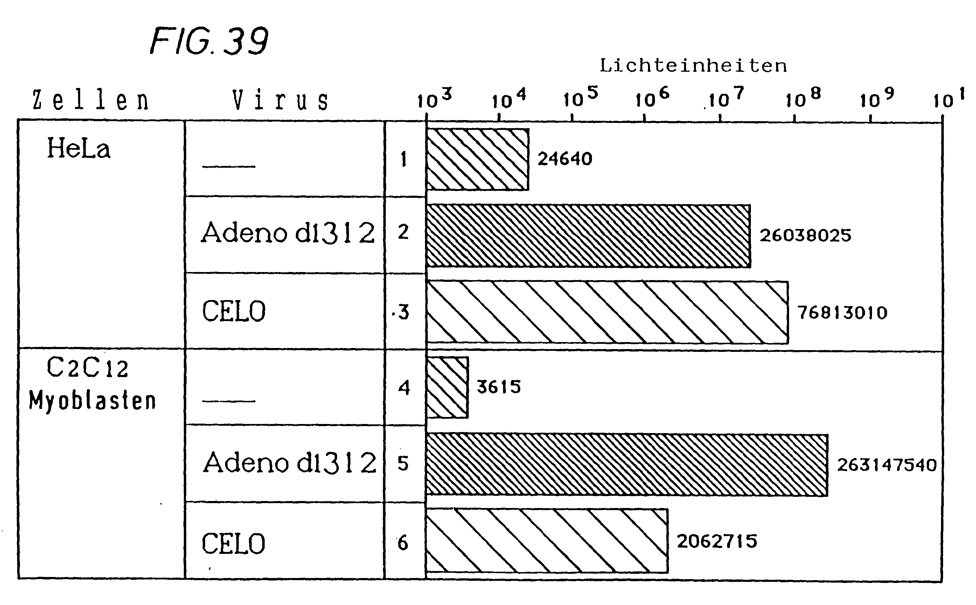

Proben von entweder HeLa-Zellen oder C2C12-Myoblasten (5 x 10⁵ Zellen pro 6 cm Schale) wurden mit 6 µg pCMVL (in der Fig. mit pCLuc bezeichnet), komplexiert mit 1 µg StrpL/7 µg TfpL plus 5 µl biotinyliertes Adenovirus d1312 (siehe Beispiel 19, 1 x 10¹² Partikel pro ml), oder 18 µl biotinyliertes CELO-Virus (siehe Beispiel 24, 0.3 x 10¹² Partikel pro ml) transfiziert. Nach 20 stündiger Inkubation wurden die Zellen geerntet und für die Luciferaseaktivitätsmessung vorbereitet. Fig. 39 zeigt die erhaltene Luciferaseaktivität von jeder gesamten Zellprobe.

Die Transfektion in HeLa-Zellen wurde unter Verwendung von human-Adenovirus dl312/StrpL/TfpL/DNA-Komplexen, die in die Zellen entweder über den Adenovirus-Rezeptor oder den Transferrin-Rezeptor eintreten können, oder von CELO-Virus/StrpL/TfpL/DNA-Komplexen, die über den Transferrin-Rezeptor eintreten können, mit vergleichbarer Effizienz durchgeführt. Während jedoch der Transfer von DNA in C2C12-Myoblasten mit Adenovirus dl312-Komplexen effizient durchgeführt werden konnte, funktionierten Komplexe mit CELO-Viren in diesen Zellen schlecht. Frühere Beispiele haben gezeigt, daß der Transferrin-Rezeptor beim Transport von Kombinations-Komplexen in diese Zellen nur eine geringe Rolle spielt; vermutlich ist der Adenovirus-Rezeptor die Haupteintrittsstelle. Die schwache Aktivität des CELO-Virus in Myoblasten dürfte also auf eine schwache Bindung sowohl des CELO-Virus als auch von Transferrin an die C2C12-Myoblasten zurückzuführen sein.

### b) Verbesserung der Transfektion von C2C12-Myoblasten mit CELO-Virus unter Verwendung von Weizenkeimagglutinin als Ligand

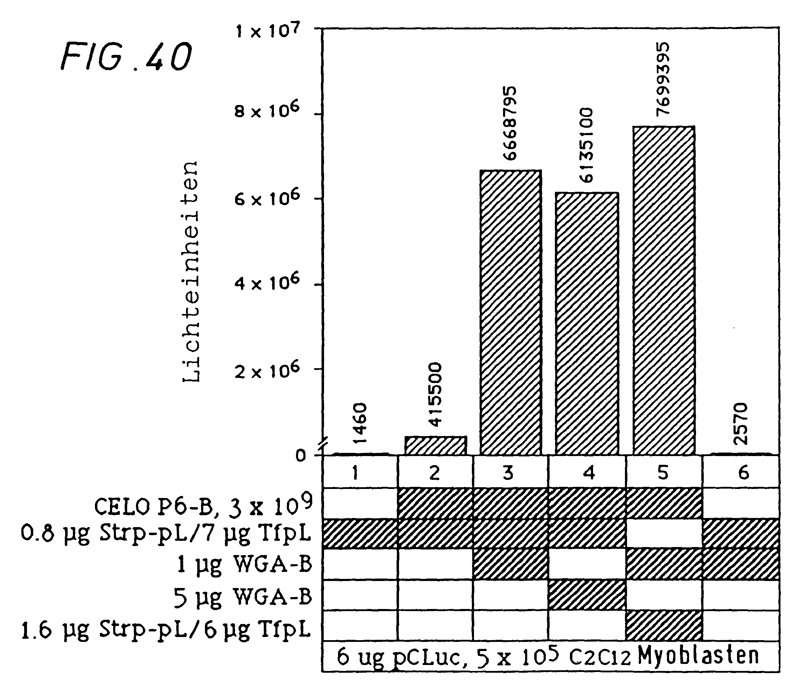

Aufgrund des schwachen Transports, der in a) erhalten wurde, wurde ein neuer Ligand als Ersatz für Transferrin ausgewählt, nämlich biotinyliertes Weizenkeimagglutinin (2 - 4 Mol Biotin pro Mol Protein; Boehringer Mannheim). Biotinyliertes CELO-Virus wurde hergestellt, wie bereits beschrieben. Komplexe, enthaltend 6 µg pCMVL plus die angegebenen Mengen StrpL, TfpL, biotinyliertes Weizenkeinagglutinin (WGA-B) und CELO-Virus wurden wie folgt hergestellt: Virus und WGA wurden zusammen in 150 µl HBS verdünnt. StrpL wurde ebenso in 150 µl HBS verdünnt, die beiden Lösungen wurden gemischt und 30 min bei Raumtemperatur inkubiert. Die DNA, verdünnt in 100 µl HBS, wurde der StrpL/Virus/WGA-Lösung zugegeben, gefolgt von einer weiteren 30 minütigen Inkubation bei Raumtemperatur. Abschließend wurde TfpL in 100 µl HBS der Mischung hinzugefügt und die Probe erneut 30 min bei Raumtemperatur inkubiert. Die Komplexe wurden den C2C12-Myoblasten (5 x 10⁵ Zellen pro 6 cm Schale) in 2 ml DMEM + 2 % FCS beigegeben. Eine Stunde später wurden 5 ml DMEM + 10 % FCS den Zellen hinzugefügt und 20 h später die Zellen für die Luciferaseaktivitätsmessung präpariert. Die Aktivität (Lichteinheiten) in jeder Gesamtzellprobe ist in Fig. 40 dargestellt (die in diesem Beispiel verwendete DNA war pCMVL, in der Fig. pCLuc bezeichnet).

In Abwesenheit von Virus wurde sehr schwacher DNA-Transfer sowohl mit als auch ohne WGA erhalten (Spalten 1, 6). Mäßiger Transfer wurde mit gekoppeltem CELO-Virus erhalten (Spur 2), es wurde jedoch eine 16fache Zunahme erhalten, wenn WGA-B im Komplex enthalten war. Die Erhöhung der WGA-Menge im Komplex (von 1 µg auf 5 µg) ergab eine leichte Abnahme im Transfer (vgl. Spalten 3 und 4), während die Erhöhung des Gehaltes an StrpL im Komplex (von 1 µg auf 2 µg) den Transfer leicht steigerte. Diese Ergebnisse zeigen deutlich, daß WGA-B als Ligand den Transport von DNA in C2C12-Zellen mittels CELO-Viren verstärkt.

### d) Expression von Faktor VIII in C2C12-Myoblastenund Myotubenkulturen

Die Myoblasten- und Myotubenkulturen wurden hergestellt, wie oben beschrieben. Transfektionen wurden unter Verwendung von 6 µg eines Plasmids, enthaltend eine full length Faktor VIII cDNA (Wood et al., 1984; Eaton et al., 1986) und komplexiert gemäß der üblichen Komplexbildungsvorschrift mit 5 oder 15 µl biotinyliertem Adenovirus (wie angegeben) plus 0.5 oder 1 µg StrpL und 7 oder 6 µg TfpL, durchgeführt.

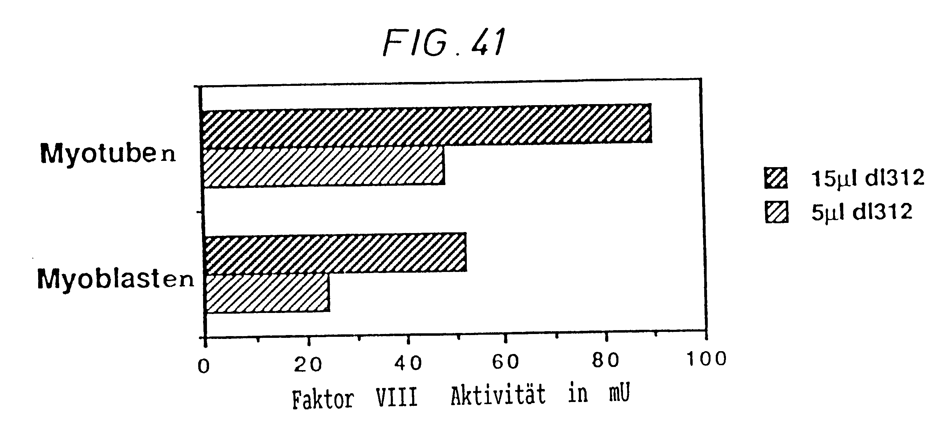

Die DNA/Viruskomplexe wurden auf die Zellen in 2 % FCS/DMEM aufgebracht. Nach einer 4 stündigen Inkubation bei 37°C wurden 3 ml frisches DMEM + 10 % FCS jeder Schale beigegeben. 18 h später wurde das Medium geerntet und unter Verwendung eines COATEST (KABI, Pharmacia) Testsystems mit einem internationalen Standard als Referenz auf das Vorhandensein von Faktor VIII untersucht. Die Faktor VIII Mengen wurden als mUnits, gebildet in 24 h pro 1 x 10⁶ Zellen, aufgetragen (Fig. 41).

### Beispiel 27

### Verwendung von Adenovirus-Protein für den DNA-Transport

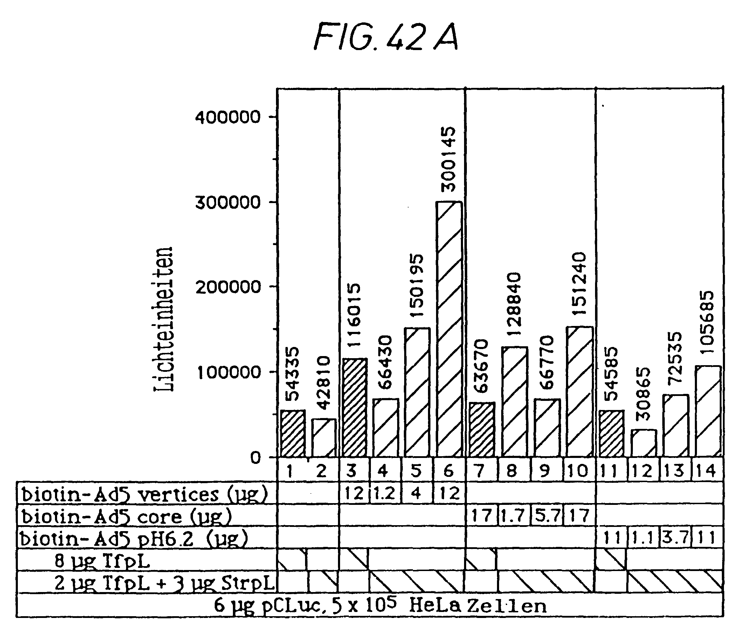

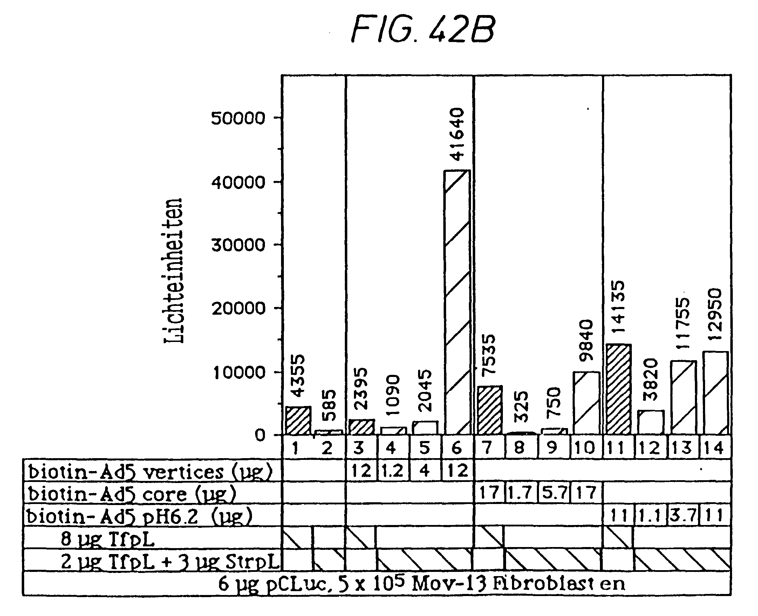

Adenovirus (Wildtyp 300) wurde in HeLa-Zellen gezüchtet, gereinigt und, wie für Adenovirus dl312 beschrieben, biotinyliert. 1.2 ml des Virus wurde gegen 3 x 300 ml 5 mM MES (2-(N-Morpholino)ethansulfonsäure), 1 mM EDTA pH 6.25, 4°C, 18 h lang dialysiert. Das Material wurde daraufhin 30 min lang bei 27 K in einem SW60-Rotor zentrifugiert. Der Überstand wurde vorsichtig entfernt, das Pellet in HBS/40 % Glyzerin resuspendiert. HEPES, pH 7.4 und NaCl wurden dem Überstand zu 20 mM und 150 mM zugegeben und sowohl die Pellet- (enthaltend die viralen Core-Proteine und die Hauptmenge des Hexonkapsids; in Fig. 42 "core") als auch die Überstandfraktionen (enthaltend die Vertices; in Fig. 42 "vertices") auf ihre DNA-Transportaktivität in Mov13 Mausfibroblasten (Strauss und Jaenisch, 1992) oder HeLa-Zellen untersucht.

Die Komplexbildung mit DNA wurde wie folgt durchgeführt: die angegebenen Mengen jeder Fraktion, aufgebrochenes Virus vor dem Zentrifugieren oder intaktes Virus (ausgedrückt als µg Protein, wie mit einem Bradford-Assay bestimmt) wurden in 300 µl HBS verdünnt. Streptavidin-Polylysin (3 µg in 50 µl HBS) wurde daraufhin zugegeben, gefolgt von 30 minütiger Inkubation bei Raumtemperatur. 6 µg pCMVL, in der Fig. "pCLuc" bezeichnet, wurden in 100 µl HBS verdünnt und für eine 30 minütige Inkubation zur ersten Lösung hinzugefügt. Abschließend wurden 2 µg TfpL in 100 µl HBS beigegeben, gefolgt von einer weiteren 30 minütigen Inkubation. In den Proben, die nur mit TfpL hergestellt wurden, wurden 8 µg TfpL in 170 µl HBS mit 6 µg pCMVL in 330 µl HBS 30 min bei Raumtemperatur gemischt. Die angegebenen Mengen Virusprotein wurden in 300 µl HBS verdünnt und dann zu den TfpL/DNA-Komplexen gegeben. Alle Proben wurden dann für eine Stunde zu 5 x 10⁵ Zellen in eine 6 cm Schale, enthaltend 2 ml DMEM/10 % FCS (entweder HeLa-Zellen oder Mov13-Fibroblasten), gegeben. Dann wurden 5 ml frisches Medium, enthaltend 10 % FCS, zugegeben und die Zellen 20 h später für die Luciferaseaktivität präpariert. Die erhaltene Luciferaseaktivität (in Lichteinheiten) ist in Fig. 42 sowohl für HeLa-Zellen (Tafel A) oder Mov13-Fibroblasten (Tafel B) dargestellt.

Bei beiden Zelltypen fand eine dosisabhängige Zunahme der DNA-Transferaktivität in Verbindung mit der Vertexfraktion (Proben 4-6 in beiden Tafeln) statt. Wenn dieselbe Menge an biotinyliertem Virusprotein in TfpL/DNA-Komplexen ohne Streptavidin-Polylysin enthalten war, wurde ein DNA-Transport nahe Untergrundwerten (Probe 3 auf jeder Tafel) beobachtet.

### Beispiel 28

### Verstärkter Gentransfer bei Verwendung ternärer DNA-Komplexe, enthaltend ein Galaktose-Ligendenkonjugat

### a) Ternäre Komplexe, enthaltend ein Influenzapeptid-Konjugat

Die Beispiele 13 und 14 haben gezeigt, daß Polylysinkonjugierte Peptide, die Sequenzen, abgeleitet vom N-Terminus der Influenzavirus-Hämagglutinin HA-2-Untereinheit, enthalten, in DNA/Transferrin-Polylysin-Komplexen den Gentransfer mittels Transferrin-Polylysin wesentlich steigern.

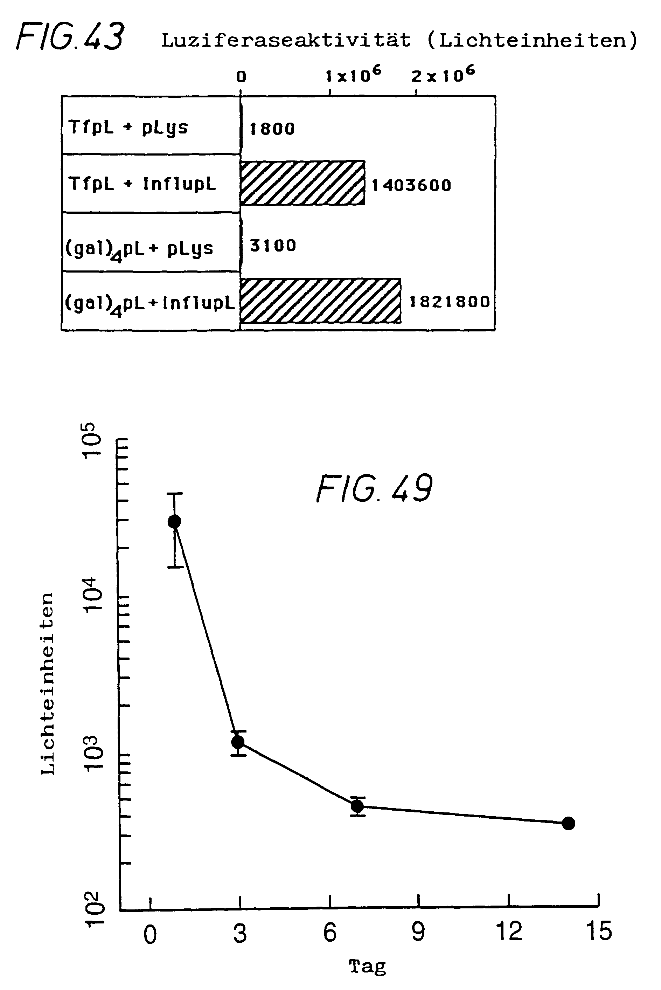

Es wurden ähnliche DNA-Kombinations-Komplexe, enthaltend das tetra-antennäre Galaktose-Ligand-Polylysin-Konjugat und das Polylysin-modifizierte Influenzapeptid (hergestellt wie in Beispiel 6 bzw. 13), hergestellt, indem das Liganden-Polylysin-Konjugat zur Plasmid-DNA pCMVL gegeben wurde, um die Hälfte der DNA-Ladung zu neutralisieren, und der Rest der Ladung verwendet wurde, um die Komplexe mit Influenzapeptid-Polylysin-Konjugat zu beladen. Der Transport dieser DNA-Komplexe, die den synthetischen Liganden (gal)4 enthielten, zu BNL CL.2 Hepatozyten (die Transfektionen wurden durchgeführt, wie in Beispiel 6g) beschrieben) ergab eine Luciferasegenexpression (Fig. 43), die signifikant höher war als die mit Transferrin als Ligand erhaltene Expression. Die Expression war mehr als 500 mal höher als die Expression, die in Kontrollexperimenten mit DNA-Komplexen ohne Influenzapeptide, aber mit derselben Menge an Polylysin (Fig. 43) erhalten wurde. Die mit den DNA-Kombinations-Komplexen erhaltene Aktivität war auch ca. 30 mal höher als mit DNA/(gal)4pL-Komplexen, mit denen die Zellen in Gegenwart von Chloroquin inkubiert wurden.

### b) Ternäre Komplexe, enthaltend Adenovirus-Konjugat

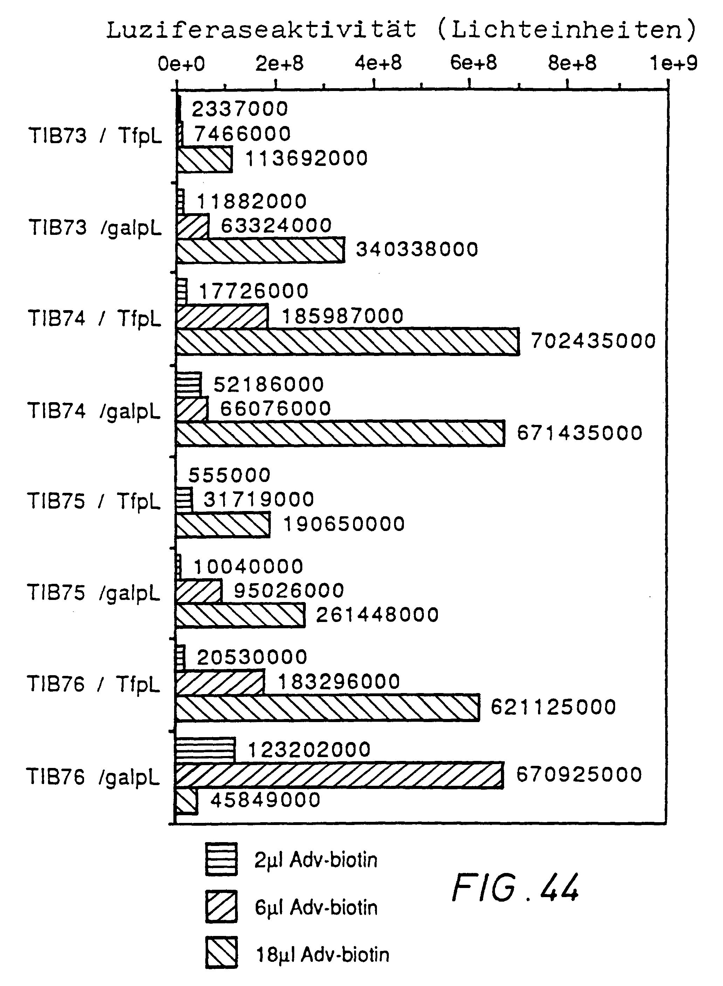

Die Komplexe wurden wie folgt hergestellt:
biotinyliertes Adenovirus d1312 (hergestellt wie in Beispiel 19; 2 µl, 6 µl oder 18 µl; 10¹² Partikel pro ml) in 50 µl HBS wurden mit Streptavidin-Polylysin (100 ng, 160 ng oder 480 ng) in 100 µl HBS gemischt. Nach einer Inkubation von 30 min wurde eine Lösung von 6 µg pCMVL in 200 µl HBS und nach weiteren 30 min eine Lösung von 3.8 µg (gal)4pL (hergestellt wie in Beispiel 6) oder 7 µg TfpL in 150 µl HBS zugegeben. Die DNA-Komplexlösungen wurden zu je 300.000 Zellen (ATCC TIB73, ATCC TIB74, ATCC TIB75, ATCC TIB76), gezüchtet in 6 cm Schalen in "high glucose DMEM" + 2 % FCS, gegeben. Die folgenden Zellkulturverfahrensschritte und die Luciferaseassays wurden durchgeführt, wie in den vorangegangenen Beispielen beschrieben, es wurden die in Fig. 44 gezeigten Genexpressionswerte (nach 24 h) erhalten.

### Beispiel 29

### Gentransfer in B-lymphoblastoide B-Zellen

Human-Ig- und anti-human-Ig-Polylysin-Konjugate wurden wie folgt hergestellt, wobei die Kopplung analog literaturbekannten Methoden durch Einführung von Disulfidbrücken nach Modifizierung mit Succinimidyl-Pyridyldithio-propionat (SPDP, Jung et al., 1981) durchgeführt wurde:

### a) Herstellung von anti-human-Ig-Polylysin 300-Konjugaten

Eine Lösung von 2 mg Ziegen-anti-human-Ig (Southern Biotechnology Associates, Inc., Birmingham, AL, USA) in HBS (150 M NaCl, 20 mM HEPES, pH 7.8) wurde mit 14 µl 5 mM ethanolischer Lösung von SPDP (Pharmacia) versetzt. Nach 10 h bei Raumtemperatur wurde über eine Gelsäule Sephadex G 25 filtriert (Eluens 100 mM HEPES-Puffer pH 7.3),- wobei 1.3 mg anti-human-Ig, modifiziert mit 30 nMol Pyridyldithiopropionatresten, erhalten wurden. Poly(L)lysin 300 (durchschnittlicher Polymerisationsgrad von 300 Lysinresten (Sigma)), wurde analog mit SPDP modifiziert und durch Behandlung mit Dithiothreitol und nachfolgender Gelfiltration in die mit freien Mercaptogruppen modifizierte Form gebracht. Eine Lösung von 12 nMol Polylysin 300, modifiziert mit 29 nMol Mercaptogruppen, in 0.3 ml HBS wurde unter Sauerstoffausschluß mit oben angeführtem modifiziertem anti-human-Ig gemischt und über Nacht bei Raumtemperatur stehen gelassen. Das Reaktionsgemisch wurde durch Zugabe von 5 M NaCl auf einen Gehalt von 0.6 M NaCl gebracht. Die Isolierung der Konjugate erfolgte durch Ionenaustauschchromatographie (Pharmacia, Mono S HR 5/5); nach Dialyse gegen 25 mM HEPES pH 7.3 wurden entsprechende Konjugate, bestehend aus 0.33 mg anti-human-Ig, modifiziert mit 4 nMol Polylysin 300 erhalten (molares Verhältnis 1:2).

### b) Herstellung von human-IgG-Polylysin 300-Konjugaten

Eine Lösung von 19.5 mg (122 nMol) Antikörper (Sigma I-4506) in 2 ml HBS wurde mit 39 µl 10 mM ethanolischer Lösung von Succinimidyl-Pyridyldithio-propionat (SPDP, Pharmacia) versetzt. Nach 2.5 h bei Raumtemperatur wurde über eine Gelsäule Sephadex G 25 filtriert (Eluens 100 mM HEPES-Puffer pH 7.9), wobei 19 mg (119 nMol) human-IgG, modifiziert mit 252 nMol Pyridyldithiopropionatresten, erhalten wurden. Poly(L)lysin 300 (durchschnittlicher Polymerisationsgrad von 300 Lysinresten; Sigma) wurde analog mit SPDP modifiziert und durch Behandlung mit Dithiothreitol und nachfolgender Gelfiltration in die mit freien Mercaptogruppen modifizierte Form gebracht. Eine Lösung von 119 nMol Polylysin 300, modifiziert mit 282 nMol Mercaptogruppen, in 1 ml HBS wurde unter Sauerstoffausschluß mit oben angeführtem modifiziertem human-IgG gemischt und über Nacht bei Raumtemperatur stehen gelassen. Das Reaktionsgemisch wurde durch Zugabe von 5 M NaCl auf einen Gehalt von ca. 0.6 M NaCl gebracht. Die Isolierung der Konjugate erfolgte durch Ionenaustauschchromatographie (Mono S, Pharmacia, 50 mM HEPES pH 7.3, Salzgradient 0.6 M bis 3 M NaCl); nach Dialyse gegen HBS pH 7.3 wurden entsprechende Konjugate, bestehend aus 9 mg (57 nMol) Antikörper, modifiziert mit 90 nMol Polylysin 300 erhalten (molares Verhältnis 1:1.6).

### c) Komplexbildung und Transfektion

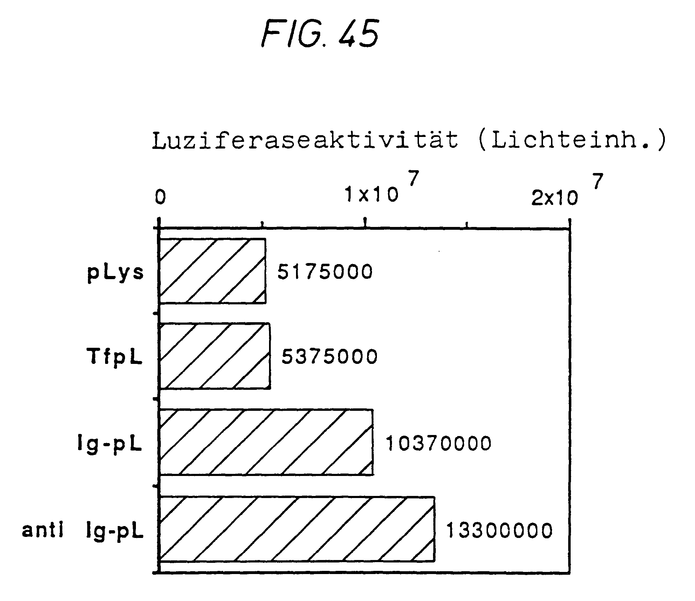

Die Komplexe wurden wie folgt hergestellt:
biotinyliertes Adenovirus dl312 (30 µl; 10¹² Partikel pro ml) in 50 µl HBS wurden mit Streptavidin-Polylysin (800 ng) in 100 µl HBS gemischt; nach 30 minütiger Inkubation wurde eine Lösung von 9 µg pCMVL in 200 µl HBS beigegeben. Nach weiteren 30 min wurde eine Lösung von 5.1 µg Polylysin (pLys450), 10.2 µg TfpL, 12 µg human-IgG-Polylysin-Konjugat oder 10 µg anti-human-Ig-Polylysin-Konjugat in 150 µl HBS zugegeben. Die DNA-Komplexe wurden zu je 10⁶ B-lymphoblastoiden Zellen gegeben (die Zellen wurden aus humanen peripheren mononuklearen Blutzellen durch Immortalisieren mit Epstein Barr Virus, wie von Walls und Crawford, 1989, beschrieben, erhalten und in Platten mit 24 Vertiefungen in 1 ml RPMI 1640 + 2 % FCS gezüchtet). Der weitere Vorgang der Zellkultur und die Luciferaseassays wurden durchgeführt wie für die vorangegangenen Beispiele beschrieben. Die erhaltenen Genexpressionswerte (Luciferaseaktivität in Lichteinheiten) ist in Fig. 45 dargestellt.

### Beispiel 30

### DNA-Transport mit Transferrin-Polylysin in Gegenwart von freiem und konjugiertem Rhinovirus

### a) Rhinovirus HRV-2 Präparationen

Rhinovirus HRV-2 wurde hergestellt und gereinigt, wie von Skern et al., 1984, beschrieben.

Eine 400 µl Lösung von Rhinovirus (ca. 30 µg) in HBS (150 mM NaCl/5 mM HEPES pH 7.9)/10 % Glyzerin wurde mit 10 nMol NHS-LC-Biotin (Pierce 21335) behandelt. Nach 3 stündiger Inkubation bei Raumtemperatur wurde das Virus durch ausgedehnte Dialyse gegen HBS/40 % Glyzerin bei 4°C von nicht eingebautem Biotin getrennt.

Lichtempfindliches Rhinovirus, hergestellt durch Züchtung des Virus in Gegenwart von Acridinorange, wurde, wie von Madshus et al., 1984, beschrieben, inaktiviert.

### b) Herstellung von DNA-Komplexen und Transfektionen

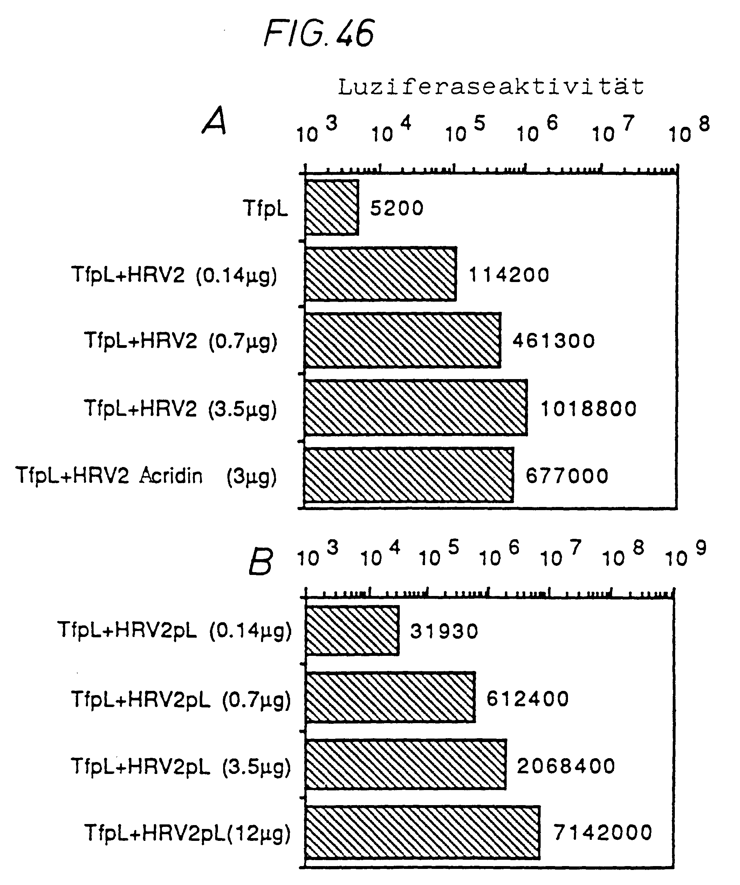

i) Transferrin-Polylysin/DNA-Komplexe wurden hergestellt, indem eine Lösung von 6 µg Plasmid-DNA pCMVL in 330 µl HBS (150 mM NaCl, 20 mM HEPES pH 7.3) mit einer Lösung von 8 µg TfpL290 in 170 µl HBS gemischt wurde. Die DNA-Komplexe wurden mit 1.5 ml Medium (DMEM + 2 % FCS) und mit 0.14 µg bis 3.5 µg Rhinovirus HRV-2 (oder inaktiviertem HRV-2) gemischt. Die Mischung wurde auf NIH 3T3-Zellen aufgebracht (300.000 Zellen pro 6 cm Schale). 4 h später wurde das Transfektionsmedium durch 4 ml frisches Medium (DMEM + 10 % FCS) ersetzt. Die Zellen wurden nach 24 h geerntet und auf Luciferaseaktivität untersucht, wie früher beschrieben (Fig. 46A).
ii) DNA-Kombinations-Komplexe, enthaltend Transferrin-Polylysin- und Rhinovirus-Polylysin-Konjugate wurden wie folgt hergestellt: eine 100 µl Lösung von biotinyliertem Rhinovirus HRV-2 (3.5 µg) in HBS wurde mit 1 µg StrpL in 100 µl HBS gemischt (die anderen Viruskonzentrationen wurden mit entsprechenden Anteilen gemischt). Nach 30 min bei Raumtemperatur wurde die Lösung mit 6 µg Plasmid-DNA in 150 µl HBS gemischt, weitere 30 min bei Raumtemperatur inkubiert und darauf mit 6 µg TfpL290 in 150 µl HBS gemischt. Die DNA-Komplexe wurden mit 1.5 ml Medium (DMEM + 2 % FCS) gemischt und den NIH 3T3-Zellen (300.000 Zellen pro 6 cm Schale) beigegeben. Die weitere Behandlung der Kulturen und der Luciferaseassay wurden durchgeführt, wie in i) beschrieben (Fig. 46B).

### Beispiel 31

### Transfektion von HeLa-Zellen mit Kombinations-Komplexen, die ionisch gebundenes Adenovirus enthalten

Komplexbildung A): Die DNA-Komplexe wurden hergestellt, indem zuerst 30 µl Adenovirus d1312 (ca. 10⁹ PFUs) mit 1 µg Polylysin pLys450 (durchschnittliche Kettenlänge 450 Monomere) in 170 µl HBS gemischt und daraufhin nach 30 min bei Raumtemperatur mit 6 µg pCMVL-DNA in 170 µl HBS versetzt wurden. Nach einer Inkubation von weiteren 30 min Dauer wurden die Komplexe mit 9 µg TfpL190 in 170 µl HBS gemischt. Ein Aliquot der Komplexmischung (10 % = 50 µl Lösung, 600 ng DNA; oder 1 % = 5 µl Lösung, 60 ng DNA) wurde in 1.5 ml DMEM + 2 % FCS verdünnt und 300.000 HeLa-Zellen beigegeben. Nach 4 h wurden 2 ml DMEM + 20 % FCS beigegeben. Zellernte nach 24 h und Luciferaseassay wurden wie üblich durchgeführt. Die Luciferaseaktivität entsprechend dem Gesamtextrakt betrug 29,115.000 Lichteinheiten (im Fall von 600 ng DNA) und 1,090.000 Lichteinheiten (im Fall von 60 ng DNA).

Komplexbildung B) (Kontrollexperiment): Die DNA-Komplexe wurden hergestellt, indem zuerst 6 µg pCMVL-DNA in 170 µl HBS mit 1 µg Polylysin pLys450 in 170 µl HBS und, nach 30 min bei Raumtemperatur, anschließend mit 9 µg TfpL190 in 170 µl HBS gemischt wurde. Nach einer Inkubation für weitere 30 min Dauer wurden die Komplexe mit 30 µl Adenovirus d1312 (ca. 10⁹ PFUs) versetzt. Ein Aliquot der Komplexmischung (10 % = 50 µl Lösung, 600 ng DNA; oder 1 % = 5 µl Lösung, 60 ng DNA) wurde in 1.5 ml DMEM + 2 % FCS verdünnt und 300.000 HeLa-Zellen beigegeben. Nach 4 h wurden 2 ml DMEM + 20 % FCS beigegeben. Zellernte nach 24 h und Luciferaseassay wurden wie üblich durchgeführt. Die Luciferaseaktivität entsprechend dem Gesamtextrakt betrug 405.000 Lichteinheiten (im Fall von 600 ng DNA) und 200 Lichteinheiten (im Fall von 60 ng DNA).

### Beispiel 32

### Lokale Verabreichung von DNA/Adenovirus/Transferrin-Polylysin-Komplexen in die Leber von Ratten

### a) Direkte Injektion

Die DNA-Komplexe wurden hergestellt, wie in Beispiel 19 beschrieben. Sie enthielten 200 µl Adenovirus dl312, 6.4 µg Streptavidin-Polylysin, 48 µg pCMVL und 48 µg TfpL290 in einem Gesamtvolumen von 2.000 µl HBS. Eine männliche Sprague-Dawley Ratte (Körpergewicht 240 g) wurde mit Avertin anästhesiert und eine 4 cm lange Laparotomie durchgeführt. Die Injektion der Komplexe erfolgte über eine Zeitspanne von 2 min in den linken seitlichen Leberlappen. Danach wurde die Laparotomiewunde schichtweise verschlossen. Die Ratte wurde 48 h nach Injektion der Komplexe in Ätheranästhesie getötet und die Luciferaseexpression in verschiedenen Proben der Leber gemessen. Es zeigte sich eine Luciferaseaktivität von 5.615 Lichteinheiten/mg Proteingehalt des Leberhomogenates im Bereich der Injektionsstelle; die Gesamtaktivität betrug 370.600 Lichteinheiten. In den von der Injektionsstelle entfernten Leberanteilen ließ sich keine Luciferaseaktivität messen.

### b) Verabreichung der Komplexe über das Gallengangsystem in die Leber

Die Komplexe wurden wie folgt hergestellt: 200 µl biotinyliertes Adenovirus dl312, verdünnt mit 200 µl HBS, wurden mit 6.4 µg Streptavidin-modifiziertem Polylysin in 400 µl HBS 30 min lang bei Raumtemperatur inkubiert. Dann wurden 48 µg pCMVL in 800 µl HBS beigegeben. Nach 30 min Inkubation wurden 48 µg TfpL in 900 µl HBS zugegeben. Für die Anwendung der Komplexe wurden männliche Sprague-Dawley Ratten (250 g Körpergewicht) mit Avertin anästhesiert und der Bauch mit einem Medianschnitt geöffnet. Der Darm wurde auf die linke Seite des Körpers gelegt und eine 27 G Nadel, die mit einem Schlauch und einer 1 ml Spritze verbunden war, in den Gallengang eingeführt. Die Injektion der Komplexe dauerte 4 min. Dann wurde die Nadel aus dem Gallengang zurückgezogen und die Injektionsstelle mit einem Fibrinkleber (Immuno) versiegelt. Die Bauchwunde wurde mit Nähten und Metallklammern verschlossen. Nach 30 h wurde die Ratte getötet und Proben von verschiedenen Leberlappen auf Luciferaseexpression untersucht. Der Spitzenwert der Luciferaseaktivität betrug 19.000 Lichteinheiten pro mg Protein; die berechnete Gesamtexpression in der ganzen Leber lag im Bereich von 2.7 x 10⁶ Lichteinheiten.

### Beispiel 33

### Verabreichung von DNA/Adenovirus/TfpL-Komplexen in die gestaute Schwanzvene von Mäusen

Die Komplexe wurden gebildet, wie in Beispiel 19 beschrieben. Sie bestanden aus 45 µl Adenovirus dl312, 0.8 µg Streptavidin-Polylysin, 6 µg pCMVL und 24 µg mTfpL290 in einem Gesamtvolumen von 150 µl HBS. Die Komplexe wurden in die Schwanzvene einer männlichen C3H/He Maus (Alter: 2 Monate) unter Anästhesie mit Avertin injiziert. Unmittelbar am Ende der Injektion wurde die Schwanzvene am proximalen und distalen Schwanzende komprimiert, so daß die Komplexlösung während der Zeit der Komprimierung (25 min) in dem injizierten Schwanzvenenabschnitt zurückgehalten wurde und nicht mit dem Blut ausgeschwemmt werden konnte. 48 h nach der Injektion wurde die Maus durch zervikale Dislokation getötet und die injizierte Schwanzvene präpariert. Die Expression von Luciferase wurde im Homogenat des Schwanzvenenabschnittes gemessen. Es ergab sich dabei eine Luciferaseaktivität von 2.600 Lichteinheiten/3 cm Schwanzvene.

### Beispiel 34

### Transfektion von primären humanen Melanomzellen

### a) Transfektion mit Adenovirus-Polylysin/Transferrin-Polylysin-Kombinations-Komplexen

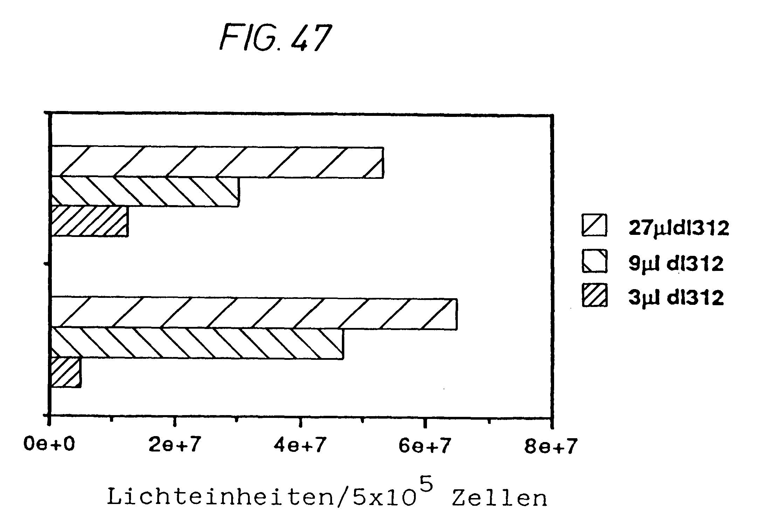

Primäre Melanomzellen wurden aus einem chirurgisch entfernten Melanom eines Patienten isoliert. Dazu wurde der Tumor mechanisch in RPMI 1640 Zellkulturmedium mit 5 % FCS 2mM Glutamin und Antibiotikazusatz zerteilt und die Gewebsfragmente durch ein Stahlsieb gedrückt. Danach wurden die Tumorzellen mehrfach durch Zentrifugation und Resuspension gewaschen und in T25 Zellkulturflaschen ausgesät. 24 h nach der Isolierung der Tumorzellen erfolgte die Transfektion mit ternären Komplexen bestehend aus: 3 µl, 9 µl oder 27 µl biotinylierten Adenovirus dl312 (1 x 10¹²/ml), 0.5 µg Streptavidin-Polylysin, 6 µg pCMVL und 7 µg TfpL290 (im Ansatz mit 27 µl Adenovirus: 1 µg Streptavidin-Polylysin und 6 µg TfpL290) in einem Gesamtvolumen von 500 µl HBS. 36 h nach der Transfektion wurden die Zellen geerntet und die Luciferaseaktivität (Lichteinheiten) gemessen (siehe Fig. 47; der obere Balken zeigt die Ergebnisse mit den Zellen in Suspension, der untere mit den anhaftenden Zellen).

### b) Transfektion mit Adenovirus-Polylysin/Low Density Lipoprotein-Polylysin-Kombinations-Komplexen

### i) Herstellung der LDL-Polylysin-Konjugate

Eine Lösung von 10 mg (14.3 nmol) LDL (Low Density Lipoprotein, Sigma, L-2139, Molekulargewicht 3,500.000, Partikeldurchmesser ca. 26 nm) in 2 ml HBS wurde mit 143 µl einer 10 mM ethanolischen Lösung von SPDP (1.43 µmol; Pharmacia) versetzt und 2 h bei Raumtemperatur reagieren gelassen. Gelfiltration über eine Sephadex G25 Säule (14 x 140 mm) mit HBS ergab 3.2 ml einer Lösung von ca. 10 mg LDL, modifiziert mit 0.70 µmol Pyridyldithiopropionatresten. Die Lösung wurde mit 1.2 ml 5 M NaCl versetzt, um bei der darauffolgenden Zugabe von Polylysin die sonst auftretende Präzipitation zu verhindern. Poly(L)lysin mit durchschnittlicher Kettenlänge von 300 Monomeren wurde wie beschrieben mit SPDP modifiziert und durch Behandlung mit Dithiothreitol und darauffolgende Gelfiltration in die mit Mercaptogruppen modifizierte Form gebracht. Die oben beschriebene modifizierte LDL-Lösung wurde mit der Lösung von 0.33 µmol Polylysin 300, modifiziert mit 0.76 µmol Mercaptogruppen, in 4 ml 2 M NaCl, 0.5 M HEPES, pH 7.9, unter Argon gemischt und 48 h bei Raumtemperatur stehengelassen. Die Reaktionslösung wurde mit sterilem Wasser auf 32 ml verdünnt (NaCl-Konzentration ca. 0.5 M) und durch Ionenaustauschchromatographie (Biorad Macroprep S, 10 x 100 mm, 20 mM HEPES pH 7.3, Gradient von 0.2 - 3 M NaCl) aufgetrennt. Die Produktfraktionen eluierten bei einer Salzkonzentration von 1.8 M - 2.2 M und wurden gepoolt. Nach Dialyse gegen HBS wurden Konjugate, bestehend aus 2.35 mg (3.4 nmol) LDL modifiziert mit 190 nmol Polylysin (entspricht 7.5 mg der freien Polylysin-Basenform) erhalten. Dies entspricht einer durchschnittlichen Modifizierung der LDL-Partikel mit ca. 55 Polylysinketten.

### ii) Komplexbildung und Transfektion:

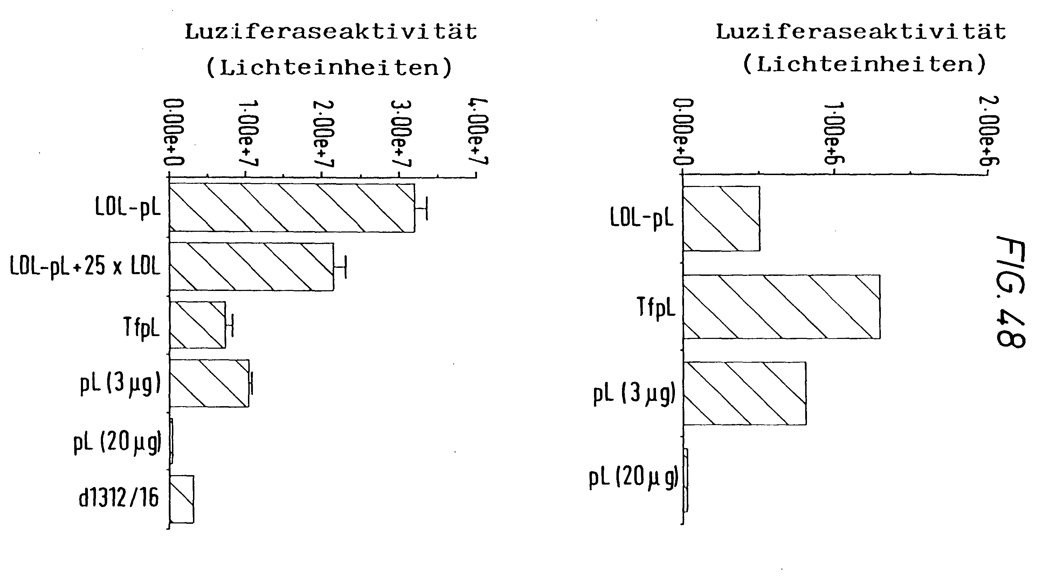

18 µl biotinyliertes Adenovirus dl312 Präparation wurden mit HBS auf 100 µl Volumen verdünnt. 1.2 µg Streptavidin-Polylysin wurden mit HBS auf 100 µl Volumen eingestellt und mit den 100 ml Adenoviruspräparation gemischt. Nach 30 min wurden 150 µl HBS mit 6 µg pCMVL dazugemischt. Nach weiteren 30 min wurden 300 µl HBS mit 4 µg LDLpL (Polylysingehalt 20 µg) dazugegeben. Diese hergestellte Lösung wurde mit 1.5 ml DMEM (10 % PCS) gemischt und zu 3 x 10⁵ primären Melanomzellen gegeben (hergestellt wie unter a) beschrieben und als HMM1 und HMM4 bezeichnet), die sich in einer Zellkulturschale mit 6 mm Durchmesser befanden. Die weiteren Zellkulturarbeiten und Luziferasemessungen wurden durchgeführt, wie unter a) beschrieben. Die Genexpression ist Fig. 48 zu entnehmen; A) zeigt die Ergebnisse mit den Melanomzellen HMM1; sämtliche Versuche wurden mit AdpL-Konjugaten durchgeführt, die in der Fig. nicht gesondert angeführt sind. B) zeigt die Versuche mit den Melanomzellen HMM4; sämtliche Versuche wurden mit AdpL-Konjugaten durchgeführt, diese sind nur in der letzten Spalte gesondert angeführt (die Bezeichnung dl312/16 bezieht sich auf die spezielle Viruspräparation). In dem in Spalte 2 dargestellten Versuch wurde LDL in 25fachem Überschuß zugesetzt, was aufgrund der Kompetition um den LDL-Rezeptor in einer verringerten Gentransfereffizienz resultierte.

### Beispiel 35

### Transfektion von primären Humanfibroblasten

Humane Hautbiopsien wurden in eine 6 cm Petrischale, enthaltend DMEM, 2 mM Glutamin, 20 % FCS und Antibiotika gegeben. Dann wurden die Biopsien mit einem chirurgischen Messer gründlich zerkleinert und in Gegenwart von 3 ml Medium 5 Tage lang gezüchtet. Darauf wurden die Zellen mit frischem Medium (vgl. oben) gewaschen und weitere 7 Tage gezüchtet. Nach dieser Zeitdauer wurden die Zellen trypsinisiert und in neue Petrischalen subkultiviert. Als die Zellen beinahe konfluent waren, wurden sie wieder trypsinisiert und gefroren bis zur Transfektion gelagert. Für die Transfektion wurden die Zellen aufgetaut, in 6 cm Petrischalen ausgesät und in DMEM, enthaltend 2 mM Glutamin, 10 % FCS und Antibiotika gezüchtet. Die Konjugate für die Transfektion wurden wie folgt hergestellt: 3 µl, 10 µl, 20 µl und 30 µl biotinyliertes Adenovirus dl312 wurden mit 0.1 µg, 0.3 µg, 0.5 µg und 0.8 µg Polylysin-modifiziertem Streptavidin in 150 µl HBS 30 min bei Raumtemperatur inkubiert. Dann wurden 6 µg des Plasmids pCMVβ-gal in 170 µl HBS beigegeben und die Mischung weitere 30 min inkubiert. Im letzten Schritt wurden 7.8 µg TfpL für die Konjugate mit 3 µl dl312, 7 µg TfpL für 10 µl dl312 und 6 µg TfpL für die Konjugate mit 20 µl und mit 30 µl dl312 in 170 µg HBS zugegeben. Nach einer Inkubationsdauer von 30 min wurden die Konjugate auf die Zellen in 2 ml DMEM, enthaltend 2 mM Glutamin, 2 % FCS und Antibiotika, aufgebracht und die Zellen 4 h lang bei 37°C inkubiert. Dann wurde das Medium entfernt und die Kultur bei 37°C mit DMEM, enthaltend 2 mM Glutamin, 10 % FCS und Antibiotika, fortgesetzt. Nach 48 h wurde die Expression von β-Galaktosidase bestimmt, wie in den vorangegangenen Beispielen beschrieben.

Bei der Transfektion mit 3 µl dl312 zeigten 14 % der Zellen β-Galaktosidaseproduktion, bei 10 µl dl312 wurden 32 % positive Zellen erhalten, mit 20 µl dl312 waren 39 % und mit 30 µl dl312 waren 64 % der Zellen positiv.

### Beispiel 36

### Gentransfer in Atemwegsepithelzellen von Ratten in vivo mit Hilfe von Kombinations-Komplexen

### a) Herstellung von TfpL/AdpL-Kombinations-Komplexen

Humantransferrin-Polylysin-DNA-Komplexe (hTfpL) wurden durch Kombination von 8 µg Transferrin-Polylysin (Serva Biochemical) in 150 µl HBS (150 mM NaCl/20 mM HEPES pH 7.3) mit 6 µg pCMVL-DNA in 350 µl HBS hergestellt und 30 min bei Raumtemperatur inkubiert. Adenovirus-Polylysin-Konjugate wurden hergestellt, wie in Beispiel 19 c) beschrieben; es wurde Psoralen/UV-inaktiviertes Adenovirus verwendet. Die Kombinations-Komplexe hTfpL/AdpL wurden gemäß der Vorschrift in Beispiel 23 c) hergestellt.

### b) Verabreichung der Komplexe in vivo über die intratracheale Route

Für diese Versuche wurde die Ratte Sigmodon hispidus ("Cotton Rat") verwendet, von der sich gezeigt hat, daß sie ein geeignetes Tiermodell für humane adenovirale Lungenerkrankungen darstellt (Pacini et al., 1984). Die Tiere wurden mit Methoxyfluran anästhesiert. Nach einem Vertikalschnitt in die ventrale Seite des Halses wurde die Luftröhre stumpf präpariert. Die Komplexe (250 bis 300 µl; 3 µg Plasmid-DNA) wurden unter Sicht direkt in die Luftröhre der in einem Winkel von 45° schräggelegten Tiere injiziert. Zu den in Fig. 49 angegebenen Zeitpunkten nach der Injektion wurden die Tiere durch CO₂ getötet und die Luftröhre und die Lunge en bloc nach in situ Spülung mit kalter Phosphatgepufferter Kochsalzlösung (PBS) geerntet. Für den Luziferasetest wurde das Lungengewebe in Extraktionspuffer homogenisiert, die Lysate auf Gesamtproteingehalt standardisiert und die Luziferasegenexpression gemessen wie beschrieben. Die angegebenen Lichteinheiten beziehen sich auf 1.250 µg Gesamtprotein, erhalten aus den Lungenlysaten. Die Experimente wurden je 3 - 4 x durchgeführt, die Ergebnisse sind als Mittelwerte ± SEM dargestellt.

### Beispiel 37

### Gentransfer unter Verwendung nicht-viraler endosomolytischer Mittel

### a) Synthese von membranaufbrechenden Peptiden

### i) Peptidsynthese:

Die Peptide wurden auf einem automatischen Syntheziser (ABI 431A) mittels der Festphasenmethode unter Verwendung von p-Alkoxybenylalkoholharz (0.97 mMol/g) als feste Phase und Fmoc-geschützten Aminosäuren. Die Carboxy-terminale Aminosäure wurde über das symmetrische Anhydrid an das Harz gebunden. Die folgenden Aminosäuren wurden mittels der 1-Hydroxybenzotriazol-Dicyclohexylcarbodiimid-Methode gekoppelt. Es wurden folgende Seitenketten-Schutzgruppen verwendet: (Trt)Asn, (Trt)Cys [(*t*-Bu)Cys im Fall von EALA and GLF], (*t*-Bu)Glu, (Trt)His, (*t*-Bu)Ser.

Die Peptide wiesen die folgenden Sequenzen auf:

EALA:    (SEQ ID NO:5) Trp Glu Ala Ala Leu Ala Glu Ala
Leu Ala Glu Ala Leu Ala Glu His Leu Ala Glu Ala Leu Ala
Glu Ala Leu Glu Ala Leu Ala Ala Gly Gly Ser Cys

GLF (SEQ ID NO:6) Gly Leu Phe Gly Ala Leu Ala Glu
Ala Leu Ala Glu Ala Leu Ala Glu His Leu Ala Glu Ala Leu
Ala Glu Ala Leu Glu Ala Leu Ala Ala Gly Gly Ser Cys

GLF-II    (SEQ ID NO:7) Gly Leu Phe Gly Ala Leu Ala Glu
Ala Leu Ala Glu Ala Leu Ala Glu  Ala Leu Ala Glu Ala
Leu Ala Glu Ala Leu Glu Ala Leu Ala Ala Gly Gly Ser Cys

GLF-delta (SEQ ID NO:8) Gly Leu Phe Glu Leu Ala Glu Ala
Leu Ala Glu Ala Leu Ala Glu Ala Leu Ala Glu Ala Leu Ala
Glu Ala Leu Glu Ala Leu Ala Ala Gly Gly Ser Cys

EALA-Inf (SEQ ID NO:9) Gly Leu Phe Gly Ala Ile Ala Gly
Phe Ile Glu Asn Gly Trp Glu Gly Leu Ala Glu Ala Leu Ala
Glu Ala Leu Glu Ala Leu Ala Ala Gly Gly Ser Cys

EALA-P50 (SEQ ID NO:10) Gly Leu Phe Glu Ala Ile Glu Gly
Phe Ile Glu Asn Gly Trp Glu Gly Leu Ala Glu Ala Leu Ala
Glu Ala Leu Glu Ala Leu Ala Ala Gly Gly Ser Cys

P50        (SEQ ID NO:11) Gly Leu Phe Glu Ala Ile Glu Gly
Phe Ile Glu Asn Gly Trp Glu Gly Met Ile Asp Gly Gly Gly
Cys

Die Peptide wurden vom Harz gespalten und die Seitenketten-Schutzgruppen, mit Ausnahme von (t-Bu)Cys, durch Behandlung von 100 mg Peptid-beladener Festphase mit 3 ml einer Mischung Phenol/Ethandithiol/Thioanisol/ Wasser/Trifluoressigsäure 0.75:0.25:0.5:0.5:10 während 1.5 h bei Raumtemperatur entfernt. Die rohen Peptide wurden in Äther ausgefällt und 2 x gewaschen. Die S-t-Bu geschützten Peptide EALA und GLF wurden in einem kleinen Volumen 1 M Triethylammoniumbicarbonat (TEAB) pH 8 gelöst, auf 100 mM TEAB verdünnt und weiter durch Reverse Phase HPLC auf einer Nucleosil 500-5C4 Säule gereinigt (0.1 % TFA/Acetonitril-Gradient), beide Peptide eluierten bei ca. 50 % Acentonitril. Die freie Cys-SH-Form der Peptide wurde erhalten, indem von den Trt-Cys-Peptiden die Schutzgruppen genauso entfernt wurden, wie oben beschrieben. Die rohen Peptide (5 mg) wurden in 100 µl 100 mM TEAB, pH 8, enthaltend 1 µl β-Mercaptoethanol, gelöst und durch Gelfiltration (Sephadex G-25, 100 mM TEAB, 0.5 mM EDTA) und Gefriertrocknen oder Ionenaustauschchromatographie (Mono Q Pharmacia, 20 mM HEPES, pH 7.3, Gradient 0 bis 3 M NaCl; das Peptid eluiert bei 1.5 M NaCl) gereinigt.

### ii) Modifikation mit N-(hydroxyethyl)maleimid

Die C-terminale SH-Gruppe der Peptide GLF-delta, GLF-II, EALA-Inf, EALA-P50, P50 wurde nach der Gelfiltration (Sephadex G-25, 20 mM HEPES, pH 7.3, 0.5 mM EDTA) von der freien SH-Form durch Reaktion mit einem 1.3- bis 10-fachen molaren Überschuß von N-(Hydroxyethyl)maleimid (1 h Raumtemperatur) blockiert. Überschüssiges Maleimid wurde durch Gelfiltration (Sephadex G-25, 100 mM TEAB, pH 8) entfernt und die Peptide (GLF-delta-mal, GLF-II-mal, EALA-Inf-mal, EALA-P50-mal, P50-mal) wurden nach Gefriertrocknen als Triethylammoniumsalz erhalten.

### iii) Modifikation mit 2,2'-Dithiobispyridin

Die freien SH-Peptide wurden mit 10 Äquivalenten von 2,2'-Dithiobispyridin (20 mM HEPES, pH 7.9, 0.5 mM EDTA) über Nacht bei Raumtemperatur reagieren gelassen. Überschüssiges Reagens wurde durch Gelfiltration (Sephadex G-25, 100 mM TEAB, pH 8) oder Ionenaustauschchromatographie (Mono Q Pharmacia, 20 mM HEPES, pH 7.3, Gradient 0 bis 3 M NaCl; das Peptid eluiert bei 1.5 M NaCl) entfernt, um die (2-Pyridylthio)-Cys-Peptide (GLF-delta-SSPy, GLF-II-SSPy, EALA-Inf-SSPy, EALA-P50-SSPy, P50-SSPy) zu erhalten.

### iv) Dimerisierung der Peptide

Das Homodimer von P50 (P50 dim) wurde hergestellt, indem äquimolare Mengen von P50-Cys-(2-Pyridylthio) und P50-Cys-SH in 20 mM HEPES, pH 7.3, 3 Tage lang bei Raumtemperatur reagieren gelassen wurden. Die Reaktionsmischung wurde auf einer Mono Q Säule getrennt (HR-5/5 Pharmacia, 20 mM HEPES pH 7.3, Gradient 0.09 M bis 3 M NaCl; das P50-Dimere eluierte bei 1.1 M NaCl). Das Heterodimer GLF-SS-P50 wurde analog durch Reaktion des Peptids P50 (freie Mercaptoform) mit pyridylthiomodifiziertem Peptid GLF hergestellt.

### b) Liposomen Leakage Assay

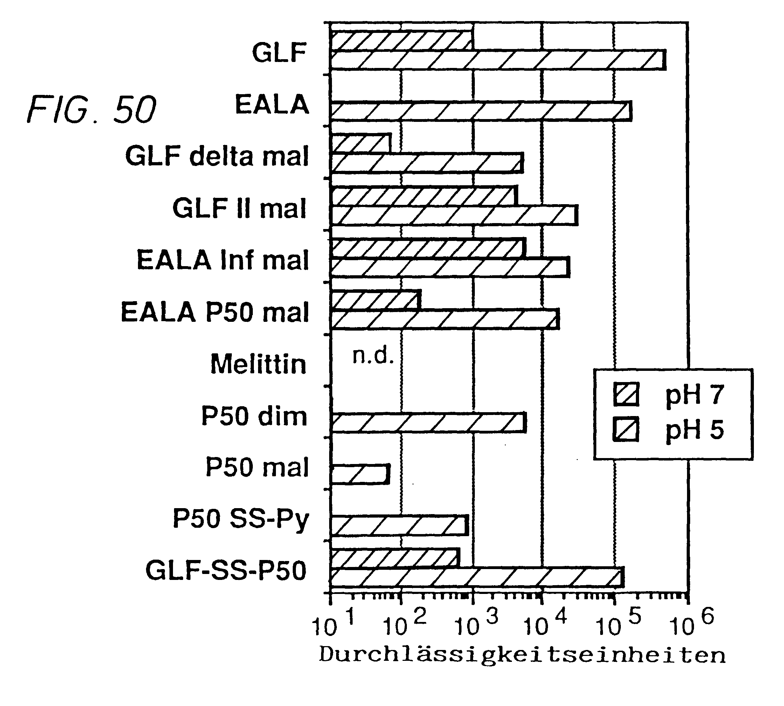

Die Fähigkeit der synthetischen Peptide, Liposomen aufzubrechen, wurde anhand des Austritts von Fluoreszenzfarbstoff aus Liposomen, die mit einer selbstauslöschenden Konzentration von Calcein beladen sind, gemessen. Die Liposomen wurden mittels der REV-Methode ("reverse phase evaporation") (Szoka und Papahadjopoulos, 1978) mit einer wässrigen Phase von 100 mM Calcein, 375 mM Na⁺, 50 mM NaCl, pH 7.3 hergestellt und durch ein 100 nm Polycarbonatfilter (MacDonald et al., 1991) extrudiert, um eine einheitliche Größenverteilung zu erhalten. Die Liposomen wurden durch Gelfiltration auf Sephadex G-25 mit einem iso-osmotischen Puffer (200 mM NaCl, 25 mM HEPES, pH 7.3) von nicht-eingebautem Material getrennt. Für den Leakage Assay bei verschiedenen pH-Werten wurde die Stammlösung (6 µl/ml) in 2 x Assaypuffer (400 mM NaCl, 40 mM Na-Citrat) verdünnt. Ein Aliquot von 100 µl wurde zu 80 µl einer Serienverdünnung des Peptids in Wasser in einer Mikrotiterplatte mit 96 Vertiefungen gegeben (Endkonzentration an Lipid: 25 µM) und bei 600 nm (Anregung bei 490 nm) auf einem Mikrotiterplatten-Fluoreszenzphotomer nach 30 min Inkubation bei Raumtemperatur auf Calceinfluoreszenz untersucht. Der Wert für 100 % Leakage wurde durch Zugabe von 1 µl einer 10 %igen Triton X-100 Lösung erhalten. Die Leakage-Einheiten wurden als Reziprokwert der Peptidkonzentration berechnet, bei der 50 % Leakage beobachtet wurde (d.h. das Volumen in µl Liposomenlösung, die zu 50 % pro µg Peptid lysiert wird). Die Werte unter 20 Einheiten wurden extrapoliert. Die Ergebnisse des Liposomen Leakage Assays sind in Fig. 50 dargestellt. GLF und EALA zeigten die höchste pH-spezifische Aktivität.

### c) Erythrozyten-Lyse-Assay

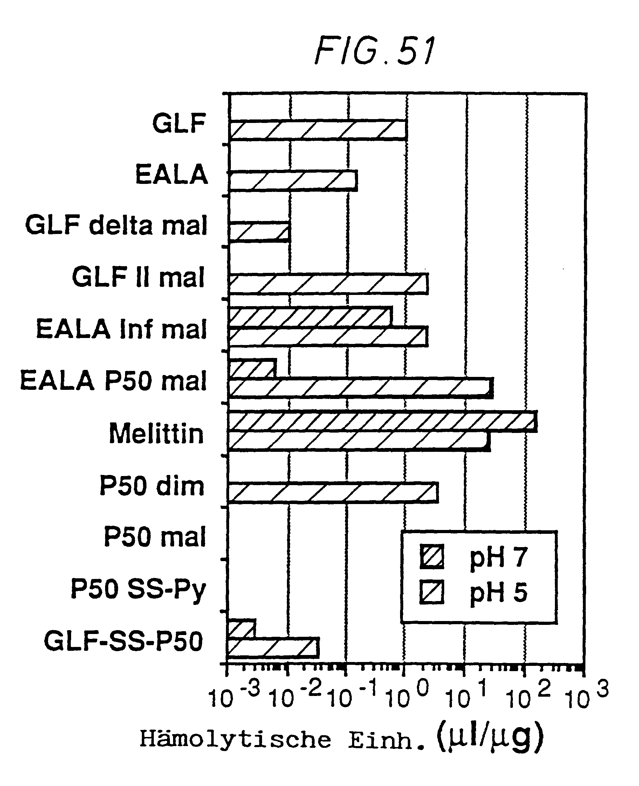

Frische Humanerythrozyten wurden mehrere Male mit HBS gewaschen und in 2 x Assaypuffer von geeignetem pH-Wert (300 mM NaCl, 30 mM Na-Citrat) bei einer Konzentration von 6.6 x 10⁷/ml resuspendiert. Ein Aliquot von 75 µl wurde zu 75 µl einer Serienverdünnung des Peptids in Wasser in einer Mikrotiterplatte mit 96 Vertiefungen (Konus-Typ) gegeben und 1 h lang bei 37°C unter konstantem Schütteln inkubiert. Nach Entfernung der nicht-lysierten Erythrozyten durch Zentrifugieren (1000 rcf, 5 min) wurden 100 µl des Überstandes in eine neue Mikrotiterplatte überführt und die Hämoglobinabsorption bei 450 nm (Untergrundkorrektur bei 750 nm) bestimmt. 100 % Lyse wurde durch Zugabe von 1 µl einer 10 %igen Triton X-100 Lösung vor dem Zentrifugieren bestimmt. Die hämolytischen Einheiten wurden als Reziprokwert der Peptidkonzentration berechnet, bei der 50 % Leakage beobachtet wurde (d.h. das Volumen in µl der Erythrozytenlösung, die zu 50 % pro µg Peptid lysiert wird). Die Werte unter 3 hämolytischen Einheiten wurden extrapoliert. Die Werte sind in Fig. 51 wiedergegeben. Daraus ist ersichtlich, daß P50 dim und EALA-P50 die höchste pH-spezifische Aktivität hinsichtlich der Lyse von Zellen und/oder der Freisetzung von größeren Molekülen wie Hämoglobin aufwiesen. Die P50-Monomeren P50mal und P50 SS-Py hatten niedrigere Aktivität. Melittin zeigte die höchste Aktivität, die jedoch nicht spezifisch für sauren pH-Wert war.

### d) Herstellung von DNA-Kombinationskomplexen

Die DNA-Komplexe wurden hergestellt, indem zuerst 6 µg pCMVL-DNA in 150 µl HBS mit 4 µg TfpL290 in 150 µl HBS gemischt und anschließend nach 30 min bei Raumtemperatur mit 4 bis 20 µg Poly(L)lysin290 in 100 µl HBS versetzt wurden. Nach weiteren 30 min Inkubation bei Raumtemperatur wurden 0.3 bis 30 µg Peptid in 100 µl HBS zugegeben und weitere 30 min inkubiert. Die optimale Menge von endosomolytischen Mittel wurde in Vortitrationen bestimmt, wobei die erhaltene Leistungsfähigkeit des Gentransfers gemessen wurde (siehe Tab. 3: Gentransfer in BNL CL.2-Zellen). Es zeigte sich, daß die gleichzeitige Zugabe von Polylysin und endosomolytischem Mittel ebenso wie die Verwendung größerer Volumen für die Komplexherstellung (1.5 ml Endvolumen) vergleichbare (oder bessere) Transfektionseffizienz ergab. In diesen Versuchen zeigte sich auch, daß auch die nicht-peptidischen amphipathischen Substanzen Desoxycholsäure und Ölsäure den Transfer von DNA verstärken.

### e) Transfektion von Zellen

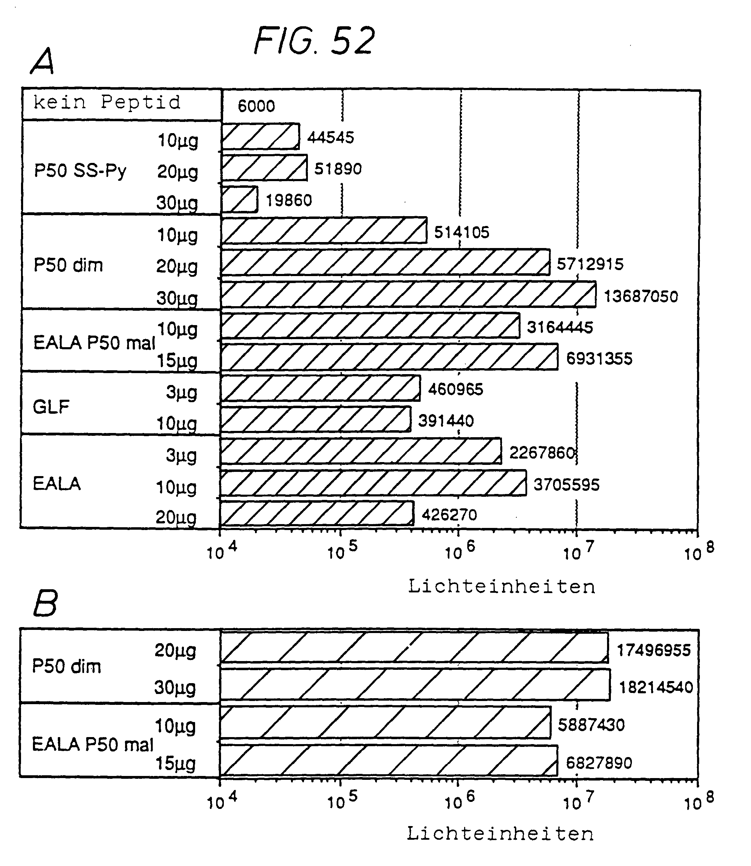

Adhärente Zellinien (BNL CL.2 Hepatozyten bzw. NIH 3T3-Zellen) wurden 1 bis 2 Tage vor der Transfektion in 6 cm Schalen gezüchtet (DMEM Medium mit 10 % FCS; 300.000 Zellen/Schale). Das Medium wurde entfernt und 1.5 ml DMEM + 2 % FCS und 500 µl DNA-Komplexe zugegeben. Alternativ wurden 0.5 ml DMEM + 6 % FCS und 1.5 ml DNA-Komplexe verwendet. Nach 4 h Inkubation wurden 2 µl DMEM (18 % FCS) zugegeben. (Es wurde festgestellt, daß alternativ das Medium durch 4 ml DMEM mit 10 % FCS ersetzt werden kann.) Die Zellernte und die Luciferaseassays wurden 24 h nach der Transfektion, wie beschrieben, vorgenommen. Die gezeigten Werte für die Lichteinheiten stellen die Gesamtluciferaseaktivität der transfizierten Zellen dar. Die Transfektion von BNL CL.2 Hepatozyten ist in Fig. 52 dargestellt:
Fig. 52A: Die DNA-Komplexe wurden hergestellt, indem zuerst 6 µg pCMVL-DNA in 250 µl HBS mit 4 µg TfpL290 in 250 µl HBS gemischt und anschließend nach 30 min bei Raumtemperatur mit 20 µg Poly(L)lysin290 in 750 µl HBS versetzt wurden. Nach weiteren 30 min Inkubation bei Raumtemperatur wurden die angegebenen Mengen an Peptiden in 250 µl HBS zugegeben. Nach einer Inkubation von weiteren 30 min wurden die Komplexe mit 0.5 ml DMEM + 6 % FCS versetzt und 450.000 Zellen beigegeben.
Fig. 52B: Die DNA-Komplexe wurden hergestellt, indem zuerst 6 µg pCMVL-DNA in 500 µl HBS mit 4 µg TfpL290 in 250 µl HBS gemischt und 30 min bei Raumtemperatur stehengelassen wurde. Eine 500 µl Lösung von 20 µg Poly(L)lysin290 in HBS wurde mit den angegebenen Mengen an Peptiden in 250 µl HBS versetzt und sofort zu der TfpL/DNA-Mischung gegeben. Nach weiteren 30 min Inkubation bei Raumtemperatur wurden die Komplexe mit 0.5 ml DMEM + 6 % FCS versetzt und zu 450.000 Zellen gegeben. Die Zellernte 24 h nach der Transfektion und die Luciferaseassays wurden durchgeführt wie beschrieben.

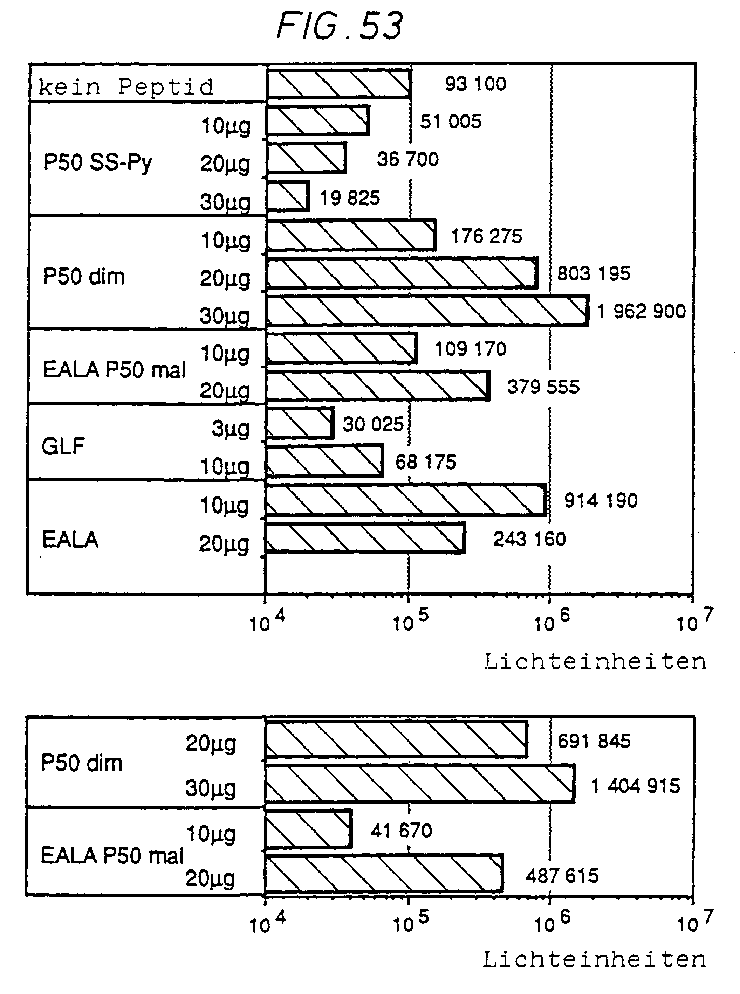

Die mit NIH 3T3-Zellen durchgeführten Experimente sind in Fig. 53 dargestellt. Die Herstellung der Komplexe A) und B) war dieselbe, wie für die Transfektion von BNL CL.2-Zellen.

In den Zellkulturexperimenten zeigten die Peptide P50 dim und EALA-P50 die höchste Aktivität, EALA und GLF hatten mittlere Aktivität, während P50-Monomere und Melittin niedrige Aktivität hatten.

### Beispiel 38

### Gentransfer unter Verwendung eines synthetischen nichtviralen Peptids mit einer Oligolysin-Verlängerung am C-Terminus

Ein Peptid der Sequenz (SEQ ID NO:4) Met Ala Gln Asp Ile Ile Ser Thr Ile Gly Asp Leu Val Lys Trp Ile Ile Asp Thr Val Asn Lys Phe Thr Lys Lys Lys Lys Lys Lys Lys Lys Lys Lys Lys Lys wurde nach der im vorigen Beispiel beschriebenen Methode synthetisiert und gereinigt. Dieses Peptid ist vom Staphylococcus aureus δ-toxin (SEQ ID NO:3) Met Ala Gln Asp Ile Ile Ser Thr Ile Gly Asp Leu Val Lys Trp Ile Ile Asp Thr Val Asn Lys Phe Thr Lys Lys abgleitet, von dem bekannt ist, daß es Spezifität für das Aufbrechen von Membranen bei saurem pH-Wert aufweist, abgeleitet, indem dieses Peptid um 10 zusätzliche Lysine verlängert wurde.

Die DNA-Komplexe wurden hergestellt, indem zuerst 6 µg pCMVL-DNA in 170 µl HBS mit 4 µg TfpL290 in 170 µl HBS gemischt und anschließend nach 30 min bei Raumtemperatur mit ca. 3 µg Peptid in 170 µl HBS versetzt wurden. Nach weiteren 30 min Inkubation bei Raumtemperatur wurden die Komplexe mit 1.5 ml DMEM + 2 % FCS gemischt und 450.000 BNL CL.2 Hepatozyten beigegeben. Nach 2 h wurden 2 ml DMEM + 20 % FCS zugegeben. Die Zellernte 24 h nach der Transfektion und die Messung der Luciferaseaktivität wurden wie in den vorangegangenen Beispielen durchgeführt. Die Luciferaseaktivität entsprechend dem Gesamtextrakt betrug 481.000 Lichteinheiten.

### Beispiel 39

### Transfektion von Hepatozyten in Gegenwart von Melittin-Peptiden mit einem Oligolysinschwanz am C-Terminus

Peptide der Sequenzen (Richtung: N- zu C-Terminus) (SEQ ID NO:12) Gly Ile Gly Ala Val Leu Lys Val Leu Thr Thr Gly Leu Pro Ala Leu Ile Ser Trp Ile Lys Arg Lys Arg Gln Gln Lys Lys Lys Lys Lys Lys Lys Lys Lys Lys (bezeichnet als mel 1)
und (SEQ ID NO:13) Gly Ile Gly Ala Val Leu Glu Val Leu Glu Thr Gly Leu Pro Ala Leu Ile Ser Trp Ile Lys Arg Lys Arg Gln Gln Lys Lys Lys Lys Lys Lys Lys Lys Lys Lys (saure Mutante, bezeichnet als mel 2) wurden synthetisiert, wie in Beispiel 38 beschrieben.

Die DNA-Komplexe wurden hergestellt, indem zuerst 6 µg pCMVL-DNA in 170 µl HBS mit 4 µg TfpL290 in 170 µl HBS gemischt und anschließend nach 30 min bei Raumtemperatur mit ca. 3 µg Peptid mel1 oder 5 µg mel2 in 170 µl HBS versetzt wurde. Nach einer Inkubation von weiteren 30 min wurden die Komplexe mit 1.5 ml DMEM + 2 % FCS gemischt und 450.000 BNL CL.2 Zellen, die gezüchtet wurden wie in Beispiel 38, beigegeben. Die Zellernte 24 h nach der Transfektion und der Luciferasetest wurden wie beschrieben durchgeführt. Die Luciferaseaktivität, bezogen auf den Gesamtextrakt, betrug 9.200 Lichteinheiten (im Fall von mel1) und 9.400 Lichteinheiten (im Falle von mel2).

### Beispiel 40

### Expression von Interferon alpha in HeLa-Zellen

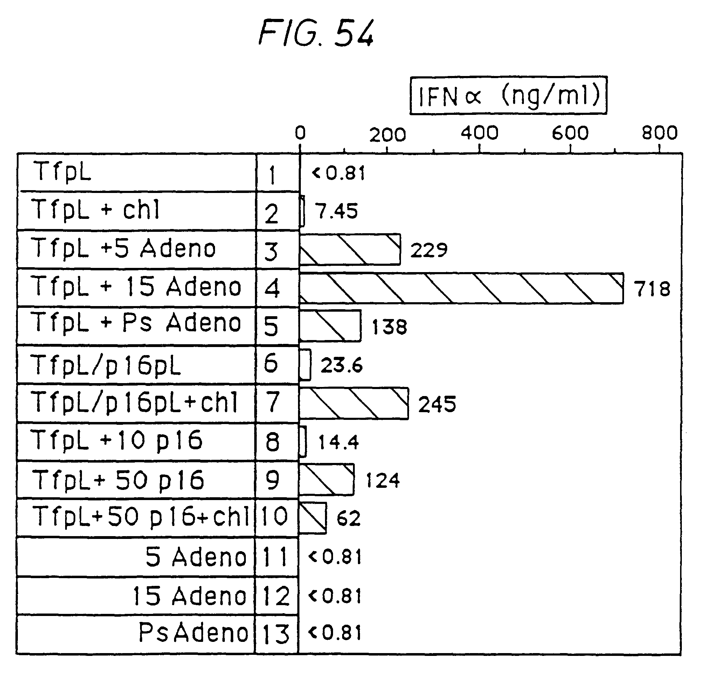

HeLa-Zellen (5 x 10⁵ Zellen pro 6 cm Schale) wurden mit dem Plasmid pAD-CMV1-IFN transfiziert, das für humanes Interferon alpha2c unter der Kontrolle der CMV Enhancer/Promoter-Sequenz kodiert (das Plasmid ist beschrieben in DE 40 21 917 A. pAD-CMV1-IFN wurde erhalten, indem das HindIII-XbaI IFN-α2c-Insert in pAD-CMV1 rekloniert wurde). Proben von 6 µg DNA in 330 µl HBS wurden mit 8 µg TfpL in 330 µl HBS versetzt und 30 min lang bei Raumtemperatur stehengelassen. Die Proben 6-10 enthielten nur 4 µg TfpL, und nach den ersten 30 min Inkubation wurde den Proben 6 und 7 je ein Aliquot von P16pL (20 µg) in 160 µl HBS und den Proben 8, 9 und 10 ein Aliquot von pLys 290 (20 µg) beigegeben. Nach weiteren 30 min Inkubation wurden Aliquots von 160 µl HBS, enthaltend 10 µl (Probe 8) oder 50 µl (Probe 9 und 10) freies P16, zugegeben (zur Synthese von P16 und P16pL siehe Beispiel 13). Nach weiteren 30 min Inkubation wurden die Proben auf HeLa-Zellen in 2 ml DMEM/2 % FCS in Gegenwart der folgenden zusätzlichen Komponenten aufgebracht: Die Proben 2, 7 und 10 enthielten 100 µM Chloroquin, die Proben 3 und 4 enthielten 5 und 15 µl Adenovirus dl312 (1 x 10¹² Partikel/ml), Probe 5 enthielt 15 µl desselben, Psoralen-inaktivierten Virus. (Als Kontrolle für die Stimulierung von endogener Interferon-Produktion durch Adenovirus wurden die Proben 11, 12 und 13 mit Aliquots von Virus wie in den Beispielen 3, 4 und 5 behandelt.) 2 h nach der Transfektion wurden 5 ml frisches Medium DMEM + 10 % FCS den Zellen beigegeben. 48 h nach der Transfektion wurde das Medium entfernt und mit 2 ml frischem DMEM + 10 % FCS ersetzt. Dieses Medium wurde 72 h nach der Transfektion geerntet und ein ELISA-Test zur Bestimmung von IFN-α durchgeführt, wie in DE 40 21 917 A beschrieben. Die IFN-α Mengen (in ng/ml) sind in Fig. 54 dargestellt.

In Übereinstimmung mit den früher gemachten Beobachtungen, die bei Verwendung von Luciferase- oder β-Galaktosidase-Reportergenen gemacht wurden, funktionierte TfpL nur schwach beim Transfer des IFN-Gens in diese Zellen. Die Gegenwart von Chloroquin erzeugte ein nachweisbares Signal (ca. 7 ng/ml, Probe 2), Adenovirus d1312 stimulierte hingegen den DNA-Transfer in dosisabhängiger Weise (Proben 3 und 4). Die Behandlung dieser Zellen mit vergleichbaren Mengen von Virus in Abwesenheit von IFN-DNA-Komplexen ergab kein nachweisbares IFN-Signal (Proben 11 und 12). Die Transfektion mit dem synthetischen, vom Influenzapeptid abgeleiteten endosomolytischen Peptid P16 (siehe Beispiel 13) als Konjugat (Proben 6 und 7) oder als ionisch an die Oberfläche von TfpL/DNA-Komplexen gebundenes Peptid (Proben 8, 9 und 10, zur Bindung der Peptide siehe Beispiel 37) erzeugte nachweisbare Interferonproduktion, die mit dem Peptidkonjugat in Gegenwart von Chloroquin (Probe 7) verstärkt wurde.

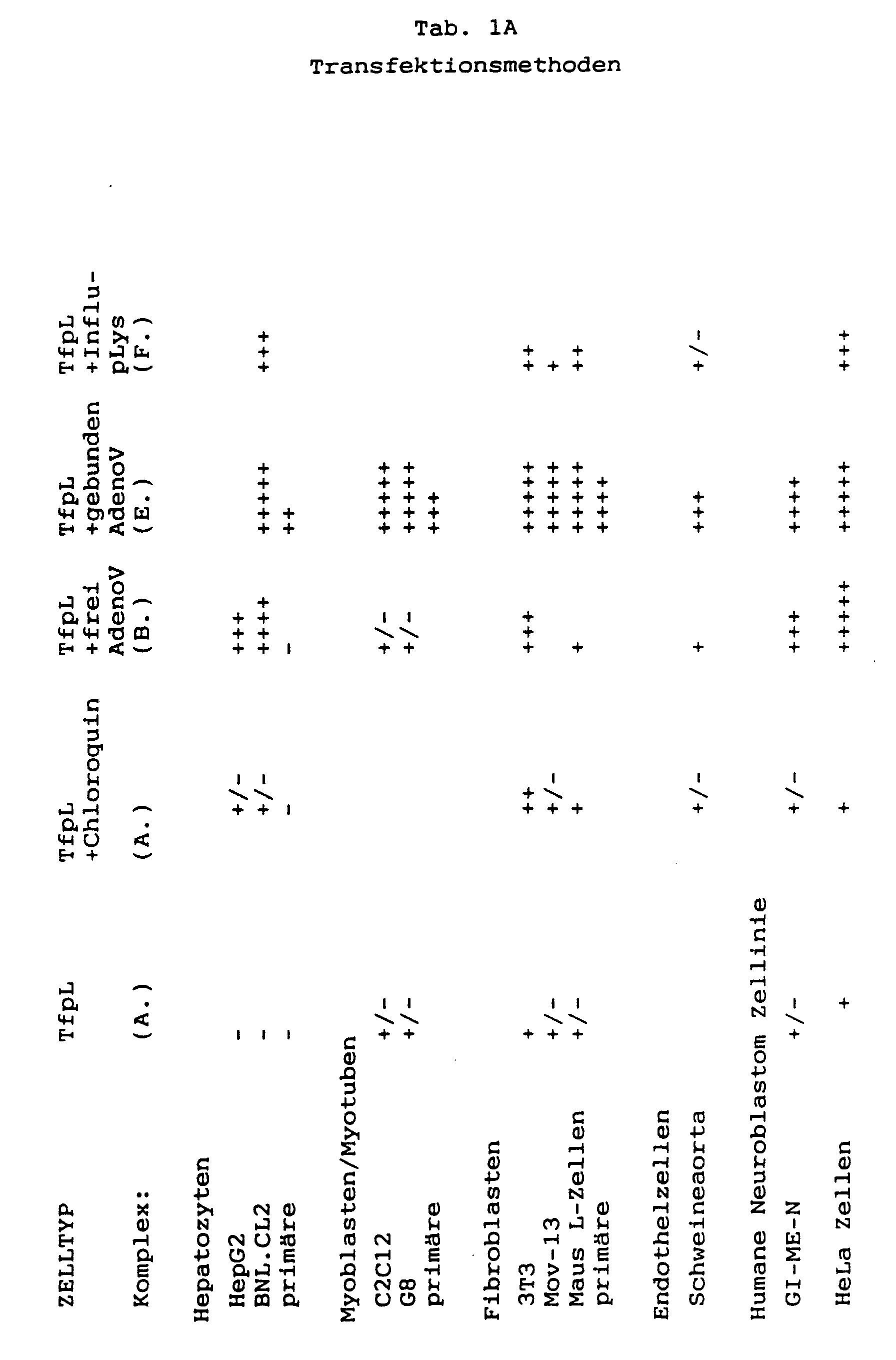

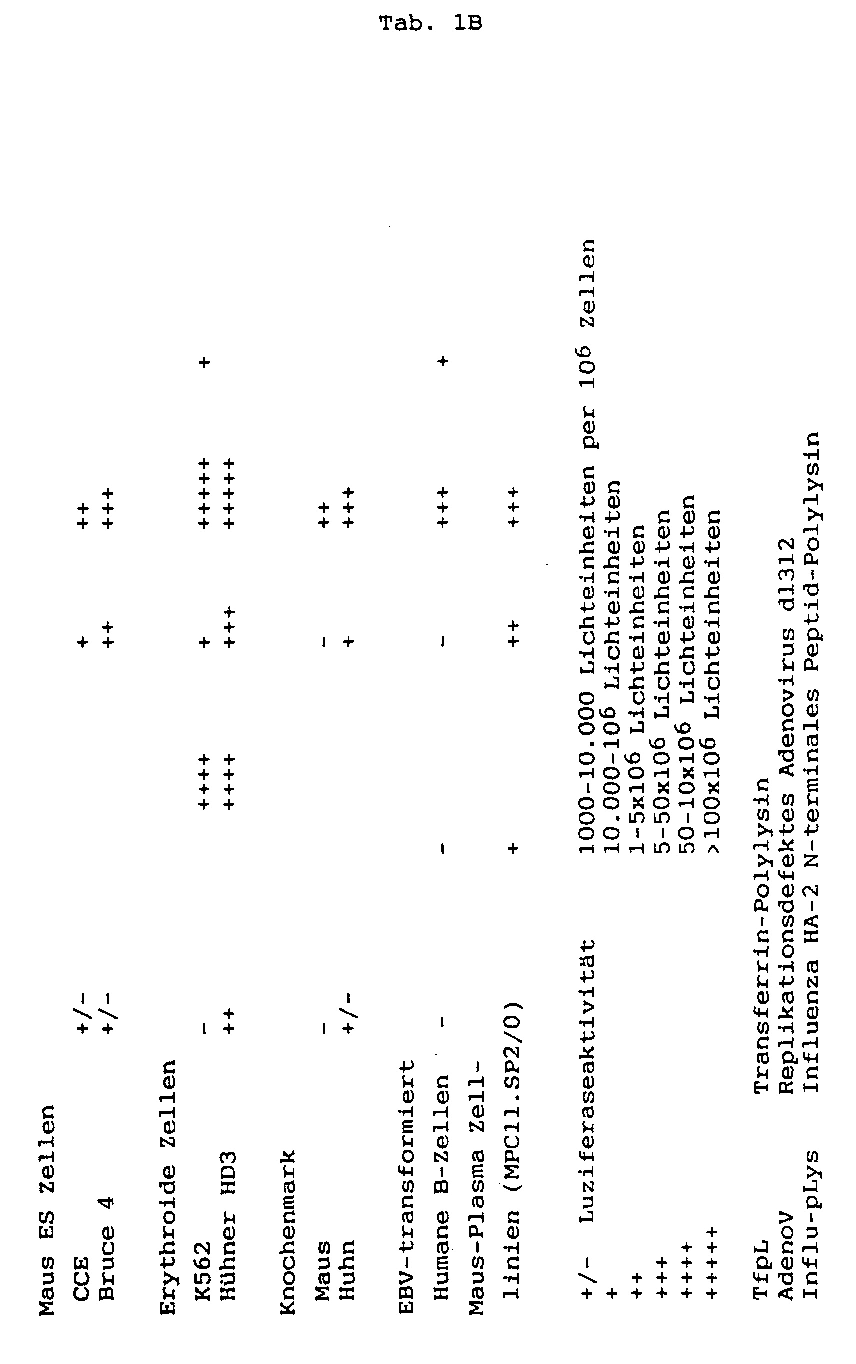

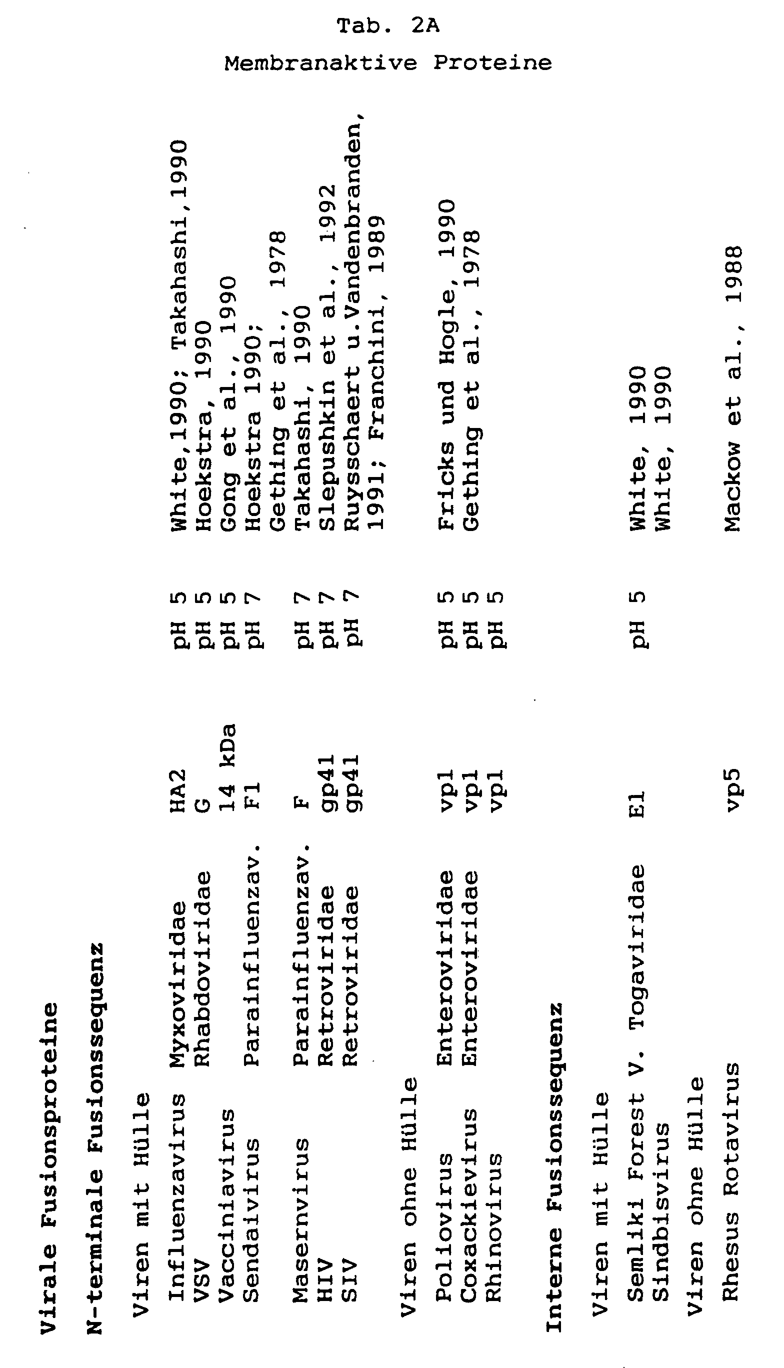

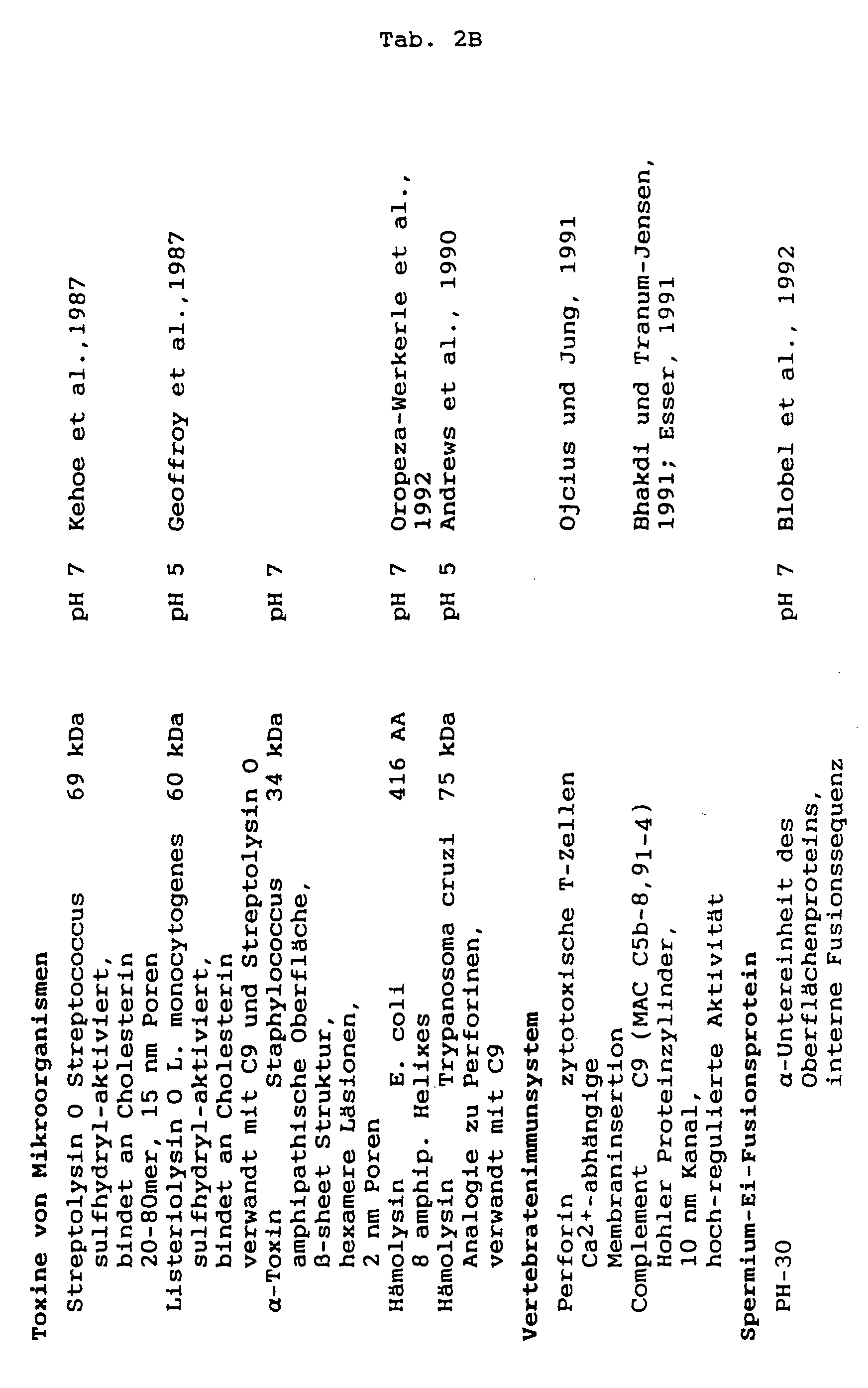

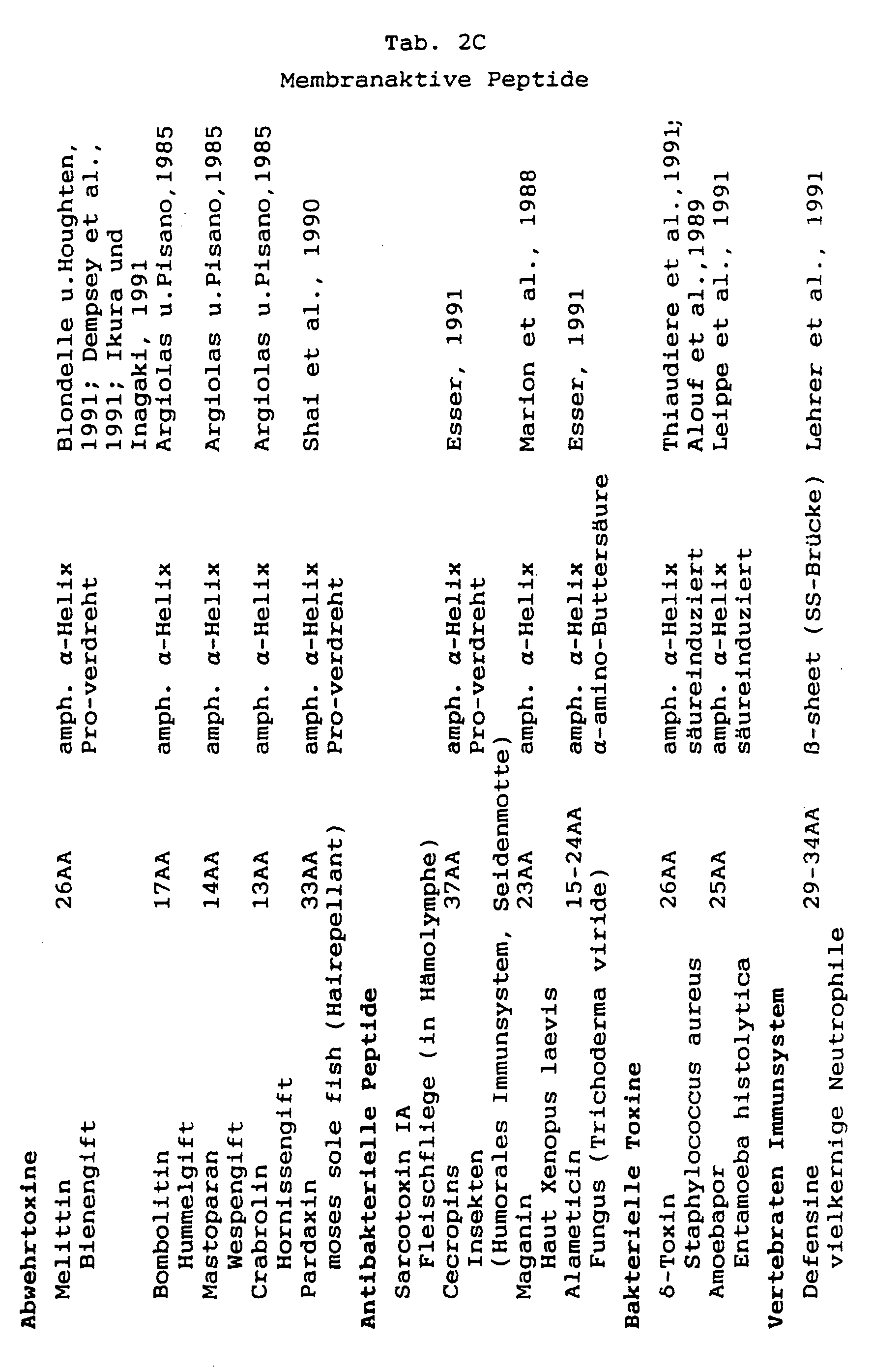

**Tab. 3**

| Transfektion von BNL CL.2 Zellen (6 µg pCMV-L DNA, 4 µg TfpL290) | | | | | | | | |
|---|---|---|---|---|---|---|---|---|
| **pLys** | | 0 µg | 0.3 µg | 1 µg | 3 µg | 10 µg | 20 µg | 30 µg |
| 0 µg | P50 dim | 160 | 330 | 540 | 300 | | | |
| | GLF | | 490 | 290 | 340 | | | |
| | Melittin | | 0 | 0 | 0 | 70 | | 425 |
| 4 µg | P50 dim | 3 100 | | 200 | 430 | 180 | 410 | |
| | GLF | | 670 | 600 | 170 | | | |
| | EALA | | 3 140 | 150 | 560 | | | |
| 10 µg | P50 dim | 5 700 | | | | 760 | 1 330 | 3 424 800 |
| | GLF | | 1 950 | 16 600 | 217 000 | 215 000 | 1 980 | |
| | EALA | | 2 120 | 16 800 | 179 300 | 181 700 | 76 360 | |
| 20 µg | P50 dim | 3 200 | | | 23 300 | 185 100 | 7 054 800 | 9 344 000 |
| | GLF | | | | 418 400 | 320 600 | 294 200 | |
| | EALA | | | | 191 000 | 181 000 | 273 600 | |
| | Mellittin | | | | 6 545 | | | |
| | Desoxy- | | | 6 730 | 34 700 | 16 000 | | |
| | cholic acid | | | | | | | |
| | Oleic acid | | | 12 200 | 11 900 | 4 100 | | |

### Literatur:

Abrahamson, D.R. et al., 1981, J. Cell Biol., 91, 270-280.

Akopian, T.A. et al., 1991, Nucl. Acids Res. 19, 424.

Alouf, J.E., Dufourcq J., Siffert O., Thiaudiere E. und Geoffroy Ch., 1989, Eur. J. Biochem. 183, 381-390.

Anderson, P. et al., 1982, J. Biol. Chem., 257, 11301-11304.

Andrews, N.W., Abrams C.K., Slatin S.L. und Griffiths G., 1990, Cell 61, 1277-87.

Ansardi, D.C. et al., 1991, J. Virology 65, 2088-2092.

Argiolas, A. und Pisano J.J., 1985, J. Biol. Chem. 260, 1437-1444.

Armentano, D., Yu, S., Kantoff, P., von Rüden, T., Anderson, W.F. und Gilboa, E., 1987, J. Virol. 61, 1647-1650.

Armentano, D. et al., 1990, Proc. Natl. Acad. Sci. USA, 87, 6141-6145.

Asada-Kubota, M. et al., 1983, Exp. Pathol., 23, 95-101.

Ascoli, M. et al., 1978, J. Biol. Chem., 253, 7832-7838.

Ashwell, G. et al., 1982, Annu. Rev. Biochem., 51, 531-554.

Atherton, E., Gait, M.J., Sheppard, R.C. und Williams, B.J., 1979, Bioorg. Chem. 8, 351.

Barr, E. und Leiden, J., 1991, Science 254, 1507-1509.

Bartlett, G.R., 1959, J. Biol. Chem. 234, 466.

Berkner, K.L., 1988, BioTechniques 6, 616-629.

Berns, K.I., 1990, Virology, 2nd Edition, Ed. by Fields, B.N., Knipe, D.M. et al., Raven Press Ltd., New York, 1743-1759.

Bhakdi, S. und Tranum-Jensen J., 1991, Immunology today 12, 318-320.

Blau, H. et al., 1985, Science 230, 758-766.

Blobel, C.P., Wolfsberg T.G., Turuck C.W., Myles D.G., Primakoff P. und White J.M., 1992, Nature 356, 248-252.

Blondelle, S.E. und Houghten R.A., 1991, Biochemistry 30, 4671-4678.

Bondeson, J., Wijkander, J. und Sundler, R., 1984, Biochim. Biophys. Acta 777, 21-27.

Boulanger, P.A. und Puvion, F., 1973, Eur. J. Biochem. 39, 37-42.

Bragg, R.R. et al., 1991, Onderstepoort J. Vet. Res. 58, 309-310.

Carpenter, G., 1984, Cell, 37, 357-358.

Chardonnet, Y. und Dales, S., 1970, Viology 40, 462-477.

Cheng, S-Y. et al., 1980, Proc. Natl. Acad. Sci. USA, 77, 3425-3429.

Ciliberto, G., Dente, L., Cortese, R., 1985, Cell 41, 531-540.

Clarke, D.D., Mycek, M.J., Neidle, A. und Waelsch, H., 1959, Arch. Biochem. Biophys. 79, 338-354.

Collis, P., Antoniou, M. und Grosveld, F., 1990, EMBO J. 9, 233-240.

Cotten, M., Laengle-Rouault, F., Kirlappos., H., Wagner, E., Mechtler, K., Zenke, M., Beug, H., und Birnstiel, M.L., 1990, Proc.Natl.Acad.Sci. USA 87, 4033-4037.

Davidson, D. und Hassell, J.A., 1987, J. Virol. 61, 1226-1239.

Davis, B.D. und Dulbecco, R., 1980, Microbiology, 3rd Edition, Ed. by Davis, B.D. *et al*., Harper & Row, Sterilization and Disinfection, 1263-1274.

Defer, C., Belin, M., Caillet-Boudin, M. und Boulanger, P., 1990, J. Virol. 64, 3661-3673.

Dempsey, C.E., Bazzo R., Harvey T.S., Syperek I., Boheim G. und Campbell I.D., 1991, FEBS Lett. 281, 240-244.

De Wet, J., Wood, K., DeLuca, M., Helinski, D. und Subramani, S., 1987, Mol. Cell. Biol. 7, 725-737.

Dhawan, J. et al., 1991, Science 254, 1509-1512.

Donti, E. et al., 1988, Cancer Genet. Cytogenet. 30, 225-231.

Dulbecco, R., 1980, Microbiology, 3rd Edition, Ed. by Davis, B.D. *et al*., Harper & Row, The Nature of Viruses, 853-884.

Eaton, D.L. et al., 1986, Biochemistry 25, 8343-8347.

Esser, A.F., 1991, Immunology today 12, 316-318.

Fields, B.N. und Knipe, D.M., 1990, Virology, 2nd edition, Raven Press, Ltd., New York.

FitzGerald, D., Padmanabhan, R., Pastan, I. und Willingham, M., 1983, Cell 32, 607-617.

Folk, J.E., 1985, Methods Enzymol. 113, 358-375.

Franchini, G., 1989, Science 244, 694-697.

Fricks, C.E. und Hogle J.M., 1990, J. Virol. 64, 1934-1945.

Fujiwara, *et al.,* 1981, J. Immunol. Meth. 45, 195.

Geoffroy, C., Gaillard J.-L., Alouf J.E. und Berche P., 1987, Infection and Immunity 55, 1641-1646.

Gething, M.J., White J.M. und Waterfield M.D., 1978, Proc. Natl. Acad. Sci. USA 75, 2737-2740.

Ginsberg, H.S., 1980, Microbiology, 3rd Edition, Ed. by Davis, B.D. *et al*., Harper & Row, Picornaviruses, 1095-1117.

Goldmacher, V.S., Blättler, W.A., Lambert, J.M., McIntyre, G. und Steward, J., 1989, Molecular Pharmacology 36, 818-822.

Goldstein, J.L. et al., 1982, Clin. Res., 30, 417-426.

Goldstein, J.L. et al., 1979, Proc. Natl Acad. Sci. USA, 76, 333-337.

Goldstein, L.J. et al., 1980, Nature 285, 66

Gong, S., Lai C. und Esteban M., 1990, Virology 178, 81-91.

Green, M. et al., 1989, Cell 58, 215-223.

Hearst, J. E. und Thiry, L., 1977, Nucl. Acids Res. 4, 1339-1347.

Heldin, C-H. et al., 1982, J. Biol. Chem., 257, 4216-4221.

Hèlène, C. und Toulme J.-J., 1990, Biochem. Biophys. Acta 1049, 99-125.

Herskowitz, I., 1987, Nature 329, 219.

Hizuka, N. et al., 1981, J. Biol. Chem., 256, 4591-4597.

Hoekstra, D., 1990, J. Bioenerg. Biomembr. 22, 121-155.

Holland, J.J., 1990, Virology,2nd Edition, Ed. by Fields, B.N., Knipe, D.M. *et al.,* Raven Press Ltd., New York, Defective Viral Genomes, 151-165.

Horvath, J. et al., 1988, J. Virol. 62, 341-345.

Horwitz, M.S., 1990, Virology, 2nd Edition, Ed. by Fields, B.N., Knipe, D.M. *et al.,* Raven Press Ltd., New York, Adenoviridae and their replication, 1679-1721.

Hosang, M. et al., 1987, EMBO J., 6, 1197-1202.

Huang, A.S., 1987, The Molecular Basis of Viral Replication, Ed. by Bercoff, R.P., Plenum Press New York and London, The Role of Defective Interfering (DI) Particles in Viral Infection, 191-194.

Ikura, T., Go N. und Inagaki F., 1991, Proteins 9, 81-89.

Imamura, K. et al., 1987, J. Immunol., 139, 2989-2992.

Iwanij, V., 1977, Eur. J. Biochem. 80, 359-368.

Jones, N. und Shenk, T., 1979, Proc.Natl.Acad.Sci. USA 76, 3665-3669.

Jung, *et al.,* 1981, Biochem. Res. Commun. 101, 599.

Kaplan, *J.* et al., 1979, *J.* Biol. Chem., 254, 7323-7328.

Kasid, A., Morecki, S., Aebersold, P., Cornetta, K., Culver, K., Freeman, S., Director, E., Lotze, M.T., Blaese, R.M., Anderson, W.F. und Rosenberg, S.A., 1990, Proc.Natl.Acad.Sci. USA 87, 473-477.

Kehoe, M.A., Miller L., Walker J.A. und Boulnois G.J., 1987, Infect. Immun. 55, 3228-3232.

Keller, G., Paige, C., Gilboa, E. und Wagner, E.F., 1985, Nature 318, 149-154.

Khang, C. und Nagaraji. K.V., 1989, Am. J. Vet. Res. 50, 1466-1470.

Klausner, R.D. et al., 1983, J. Biol. Chem., 258, 4715-4724.

Klausner, R.D. et al., 1983, Proc. Natl. Acad. Sci. USA 80, 2263-2266.

Kuhn, L.C. et al., 1982, Trends Biochem. Sci., 7, 299-302.

Kurachi, K., Davie, E.W., 1982, Proc. Natl. Acad. Sci USA 79, 6461-6464.

Laver, W.G. et al., 1971, Virology 45, 598-614.

Lehrer, R.I., Ganz T. und Selsted M.E., 1991, Cell 64, 229-230.

Leippe, M., Ebel S., Schoenberger O.L., Horstmann R.D. und Müller-Eberhard H.J., 1991, Proc. Natl. Acad. Sci. USA 88, 7659-7663.

Lim, K. und Chae, C.B., 1989, BioTechniques 7, 576-579.

Luthmann, H. und Magnusson, G., 1983, Nucl. Acids Res. 11, 1295-1308.

MacDonald, R.C. et al., 1991, Biochim. Biophys. Acta 1061, 297-303.

Macgregor, G. und Caskey, C.T., 1989, Nucl. Acids Res. 17, 2365.

Mackow, E.R., Shaw R.D., Matsui S.M., Vo P.T., Dang M.N. und Greenberg H.B., 1988, Proc. Natl. Acad. Sci. USA 85, 645-649.

Madshus, I.H., Olsnes, S. und Sandvig, K., 1984, Virology 139, 346-357.

Malim, M. et al., 1989, Cell 58, 205-214.

Maniatis, T., Fritsch, E.F. und Sambrook, J. (1982) Molecular Cloning A Laboratory Manual. Cold Spring Harbor Laboratory, 474.

Marion, D., Zasloff M. und Bax A., 1988, FEB 227, 21.

Marshall, S., 1985, J Biol. Chem., 250, 4133-4144.

Massague, J. et al., 1986, J. Cell. Physiol., 128, 216-222.

McClure, M.O., Sommerfelt, M.A., Marsh, M. und Weiss, R.A., 1990, J. General Virol. 71, 767-773.

Mellman, I.S. et al., 1984, J. Cell Biol., 98, 1170-1177.

Mizel, S.B. et al., 1987, 1987, J. Immunol., 138, 2906-2912.

Ojcius, D.M. und Young J.D., 1991, TIBS 16, 225-229.

Oropeza-Werkerle, R.L., Muller S., Briand J.P., Benz R., Schmid A. und Goebel W., 1992, Mol. Microbiol. 6, 115-121.

Otero, M.J. und Carrasco, L., 1987, Virology 160, 75-80.

Pacini, D.L. et al., 1984, J. Infect. Dis. 150, 92-97.

Parente, R.A. et al., 1990, Biochemistry 29, 8720-8728.

Patek, P.Q., Collins, J.L. und Cohn, M. 1978, Nature 276, 510-511.

Ponder, J.P. et al., Proc. Natl. Acad. Sci. USA, 1991, 88, 1217-1221.

Posner, B.I. et al., 1982, J. Cell Biol., 93, 560-567.

Precious, B. und Russell, W.C., 1985, Virology, ed. Mahy, B.W.J., IRL Press, Oxford, Washington, DC, 193-205.

Rafalski, M., Lear, J.D. und DeGrado, W.F., 1990, Biochemistry 29, 7917-7922.

Reece, R.L. et al, 1987, Aust. Vet. J. 64, 365-367.

Riordan, J.R. et al., 1989, Science, 245, 1066-1073.

Rosenberg, St.A. et al., 1992, Human Gene Therapy 3, 75-90.

Ruysschaert, J.-M. und Vandenbranden M., 1991, Biochem. Biophys. Res. Commun. 175, 872-879.

Sambrook, J. et al. (Eds.), 1989, Molecular Cloning, 2nd Edition, Vol. 3, 16.39-16.40.

Schalch, D.S. et al., 1986, Endocrinology, 118, 1590-1597.

Sennett, C. et al., 1981, Annu. Rev. Biochem., 50, 1053-1086.

Seth, P., FitzGerald, D., Ginsberg, H., Willingham, M. und Pastan, I., 1984, Mol. Cell. Biol. 4, 1528-1533.

Severne, Y., Wieland, S., Schaffner, W. und Rusconi, S., 1988, EMBO J. 7, 2503-2508.

Shai, Y., Bach D. und Yanovsky A., 1990, JBS 265, 20202-20209.

Sharon, N., 1987, Cell Separation: Methods and Selected Applications, Vol. 5, Academic Press Inc., pp.13-44.

Silver, L. et al., 1988, Virology 165, 377-387.

Sipe, D.M. et al., 1991, J. Biol. Chem. 256, 3469-3474.

Skern, T. et al., 1984, Virology 136, 125-132.

Slepushkin, V.A., Andreev S.M., Siderova M.V., Melikyan G.B., Grigoriev V.B., Chumakov V.M., Grinfeldt A.E., Manukyan R.A. und Karamov E.V., 1992, AIDS Res. Human Retroviruses 8, 9-18.

Sly, W. et al., 1982, J. Cell Biochem., 18, 67-85.

Smith, K.A. et al., 1985, Proc. Natl. Acad. Sci. USA, 82, 864-867.

Stahl, P.D. et al., 1978, Proc. Natl. Acad. Sci. USA, 75, 1399-1403.

Straubinger, R.M. und Papahadjopoulos, D., 1983, Meth. Enzymol. 101, 512-527.

Strauss, W. und Jaenisch, R., 1992, EMBO J. 11, 417-422.

Subbarao, N.K. et al., 1987, Biochemistry 26, 2964-2972.

Sullenger, B.A. et al., 1990, Cell 63, 601-608.

Svensson, U., 1985, J.Virol., 55, 442-449.

Szoka, F. und Papahadjopoulos, D., 1978, Proc.Natl.Acad.Sci. USA 75, 4194-4198.

Takahashi, S., 1990, Biochemistry 29, 6257-6264.

Takayama, K.M. und Inouye, M., 1990, Critical reviews in Biochem. and Mol.Biol. 25, 155-184.

Takese, K. et al., 1990, Nippon Juigaki Zasshi 52, 207-215.

Thiaudiere, E., Siffert O., Talbot J.-C., Bolard J., Alouf J.E. und Dufourcq J., 1991, Eur. J. Biochem. 195, 203-213.

Trono, D. et al., 1989, Cell 59, 113-120.

Uchida, Y., Tsukada, U. und Sugimori, T., 1977, J. Biochem. 82, 1425-1433.

Urakawa, T., et al., 1989, J. Gen. Virol. 70, 1453-1463.

Valerio, D., Mclvor, R.S., Williams, S.R., Duyvesteyn, M.G.C., Van Ormondt, H., Van der Eb, A.J., Martin, D.W. Jr, 1984, Gene, 31, 147-153.

van Oostrum, J. und Burnett, R.M., 1985, J. Virol. 56, 439-448.

Wagner, E., Zenke, M., Cotten, M., Beug, H. und Birnstiel, M.L., 1990, Proc.Natl.Acad.Sci. USA 87, 3410-3414.

Wagner, E., Cotten, M., Foisner, R. und Birnstiel, M.L., 1991a, Proc.Natl.Acad.Sci. USA 88, 4255-4259.

Wagner, E., Cotten, M., Mechtler, K., Kirlappos, H. und Birnstiel, M.L., 1991b, Bioconjugate Chemistry 2, 226-231.

Walker, F. et al., 1987, J. Cell Physiol., 130, 255-261.

Walker, R.D. et al., 1989, Proc.Natl.Acad.Sci. USA 86, 9514-9518.

Walls, E.V. und Crawford, D.H., 1989, Lymphocytes, a practical approach, G.G.B. Klaus, Ed., IRL press Oxford, 149-162

Watson, J.D., et al., 1987, Mol. Biol. of the Gene, Benjamin/Cummings Publ. Company Inc., 676-677.

Wharton, S.A., Martin, S.R., Ruigrok, R.W.H., Skehel, J.J. und Wiley, D.C., 1988, J. Gen. Virol. 69, 1847-1857.

White, J.M., 1990, Annu. Rev. Physiol 52, 675-697

Wilchek, M. *et al.,* 1988, Anal. Biochem. 171, 1.

Wilson, J.M., Danos, O., Grossman, M., Raulet, D.H. und Mulligan, R.C., 1990, Proc.Natl.Acad.Sci. USA 87, 439-443.

Willumsen, N.J., Davis, C.W. und Boucher, R.C., 1989, Am. J. Physiol. 256 (Cell Physiol. 25), 1033-1044.

Wood, W.I., Capon, D.J., Simonsen, C.C., Eaton, D.L., Gitschier, J., Keyt, B., Seeburg, P.H., Smith, D.H., Hollinshead, P., Wion, K.L., Delwart, E., Tuddenham, E.G.D., Vehar, G.A., Lawn, R.M., 1984, Nature, 312, 330-337.

Wu, R., Yankaskas, J.R., Cheng, E., Knowles, M.R. und Boucher, R., 1985, Am.Rev.Respir.Dis. 132, 311-320.

Wu, G.Y. und Wu, C.H., 1987, J. Biol. Chem. 262, 4429-4432.

Wu, G.Y. und Wu, C.H., 1988, J. Biol. Chem. 263, 14621-14624.

Yankaskas, J.R., Stutts, M.J., Cotton, C.U., Knowles, M.R., Gatzy, J.T. und Boucher, R.C., 1987, Genetics and Epithelial Cell Dysfunction in Cystic Fibrosis, Alan R. Liss, Inc., pp. 139-149.

Yankaskas, J.R., Haizlip, J.E., Conrad, M., Koval, D., Schlegel, R. und Boucher, R.C., 1991, Am. Rev. Respir. Dis. 143, A139.

Zamecnik, P.C., Goodchild, J., Taguchi, Y. und Sarin, P.S., 1986, Proc.Natl.Acad.Sci. USA 83, 4143-4146.

Zatloukal, K., Denk, H., Lackinger, E. und Rainer, I., 1989, Lab. Invest. 61, 603-608.

Zenke, M., Steinlein, P., Wagner, E., Cotten, M., Beug, H. und Birnstiel, M.L., 1990, Proc.Natl.Acad.Sci. USA 87, 3655-3659.

Zon, G., 1988, Pharmaceut. Research 5, No.9, 539-549.

Remington's Pharmaceutical Sciences, 1980, Mack Publ. Co., Easton, PA, Osol (ed.).

Trends Pharmacol. Sci. 10, 1989, 458-462.

### SEQUENZLISTE

(ii) TITEL DER ERINDUNG: Zusammensetzung für das Einbringen von Nukleinsäure-Komplexen in höhere eukaryotische Zellen
(iii) ANZAHL DER SEQUENZEN: 13
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: Patentln Release #1.0, Version #1.25 (EPO)

(2) INFORMATION FÜR SEQ ID NO:1:
   (i) SEQUENZCHARAKTERISIERUNG:
      (A) LÄNGE: 23 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:1: 
Gly Leu Phe Glu Ala Ile Ala Gly Phe Ile Glu Asn Gly Trp
1               5                   10
Glu Gly Met Ile Asp Gly Gly Gly Cys
15                  20
(2) INFORMATION FÜR SEQ ID NO:2:
   (i) SEQUENZCHARAKTERISIERUNG:
      (A) LÄNGE: 23 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:2: 
Gly Leu Phe Gly Ala Ile Ala Gly Phe Ile Glu Asn Gly Trp
1               5                   10
Glu Gly Met Ile Asp Gly Gly Gly Cys
15                  20
(2) INFORMATION FÜR SEQ ID NO:3:
   (i) SEQUENZCHARAKTERISIERUNG:
      (A) LÄNGE: 26 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:3: 
Met Ala Gln Asp Ile Ile Ser Thr Ile Gly Asp Leu Val Lys
1               5                   10
Trp Ile Ile Asp Thr Val Asn Lys Phe Thr Lys Lys
15                  20                  25
(2) INFORMATION FÜR SEQ ID NO:4:
   (i) SEQUENZCHARAKTERISIERUNG:
      (A) LÄNGE: 36 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:4: 
Met Ala Gln Asp Ile Ile Ser Thr Ile Gly Asp Leu Val Lys
1               5                   10
Trp Ile Ile Asp Thr Val Asn Lys Phe Thr Lys Lys Lys Lys
15                  20                  25
Lys Lys Lys Lys Lys Lys Lys Lys
    30                  35
(2) INFORMATION FÜR SEQ ID NO:5:
   (i) SEQUENZCHARAKTERISIERUNG:
      (A) LÄNGE: 34 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:5: 
Trp Glu Ala Ala Leu Ala Glu Ala Leu Ala Glu Ala Leu Ala
1               5                   10
Glu His Leu Ala Glu Ala Leu Ala Glu Ala Leu Glu Ala Leu
15                  20                  25
Ala Ala Gly Gly Ser Cys
    30
(2) INFORMATION FÜR SEQ ID NO:6:
   (i) SEQUENZCHARAKTERISIERUNG:
      (A) LÄNGE: 35 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:6: 
Gly Leu Phe Gly Ala Leu Ala Glu Ala Leu Ala Glu Ala Leu
1               5                   10
Ala Glu His Leu Ala Glu Ala Leu Ala Glu Ala Leu Glu Ala
15                  20                  25
Leu Ala Ala Gly Gly Ser Cys
    30                  35
(2) INFORMATION FÜR SEQ ID NO:7:
   (i) SEQUENZCHARAKTERISIERUNG:
      (A) LÄNGE: 35 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:7: 
Gly Leu Phe Gly Ala Leu Ala Glu Ala Leu Ala Glu Ala Leu
1               5                   10
Ala Glu Ala Leu Ala Glu Ala Leu Ala Glu Ala Leu Glu Ala
15                  20                  25
Leu Ala Ala Gly Gly Ser Cys
    30                  35
(2) INFORMATION FÜR SEQ ID NO:8:
   (i) SEQUENZCHARAKTERISIERUNG:
      (A) LÄNGE: 34 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:8: 
Gly Leu Phe Glu Leu Ala Glu Ala Leu Ala Glu Ala Leu Ala
1               5                   10
Glu Ala Leu Ala Glu Ala Leu Ala Glu Ala Leu Glu Ala Leu
15                  20                  25
Ala Ala Gly Gly Ser Cys
    30
(2) INFORMATION FÜR SEQ ID NO:9:
   (i) SEQUENZCHARAKTERISIERUNG:
      (A) LÄNGE: 34 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:9: 
Gly Leu Phe Gly Ala Ile Ala Gly Phe Ile Glu Asn Gly Trp
1               5                   10
Glu Gly Leu Ala Glu Ala Leu Ala Glu Ala Leu Glu Ala Leu
15                  20                  25
Ala Ala Gly Gly Ser Cys
    30
(2) INFORMATION FÜR SEQ ID NO:10:
   (i) SEQUENZCHARAKTERISIERUNG:
      (A) LÄNGE: 34 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:10: 
Gly Leu Phe Glu Ala Ile Glu Gly Phe Ile Glu Asn Gly Trp
1               5                   10
Glu Gly Leu Ala Glu Ala Leu Ala Glu Ala Leu Glu Ala Leu
15                  20                  25
Ala Ala Gly Gly Ser Cys
    30
(2) INFORMATION FÜR SEQ ID NO:11:
   (i) SEQUENZCHARAKTERISIERUNG:
      (A) LÄNGE: 23 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:11: 
Gly Leu Phe Glu Ala Ile Glu Gly Phe Ile Glu Asn Gly Trp
1               5                   10
Glu Gly Met Ile Asp Gly Gly Gly Cys
15                  20
(2) INFORMATION FÜR SEQ ID NO:12:
   (i) SEQUENZCHARAKTERISIERUNG:
      (A) LÄNGE: 36 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:12: 
Gly Ile Gly Ala Val Leu Lys Val Leu Thr Thr Gly Leu Pro
1               5                   10
Ala Leu Ile Ser Trp Ile Lys Arg Lys Arg Gln Gln Lys Lys
15                  20                  25
Lys Lys Lys Lys Lys Lys Lys Lys
    30                  35
(2) INFORMATION FÜR SEQ ID NO:13:
   (i) SEQUENZCHARAKTERISIERUNG:
      (A) LÄNGE: 36 Aminosäuren
      (B) TYP: Aminosäure
      (C) STRANGFORM: Einzelstrang
      (D) TOPOLOGIE: beide
   (ii) ART DES MOLEKÜLS: Peptid
   (xi) SEQUENZBESCHREIBUNG: SEQ ID NO:13:

Gly Ile Gly Ala Val Leu Glu Val Leu Glu Thr Gly Leu Pro
1               5                   10
Ala Leu Ile Ser Trp Ile Lys Arg Lys Arg Gln Gln Lys Lys
15                  20                  25
Lys Lys Lys Lys Lys Lys Lys Lys
    30                  35

## Patentansprüche

1. Zusammensetzung für die Transfektion von höheren eukaryotischen Zellen, enthaltend einen Komplex, in dem Nukleinsäure mit Nukleinsäure-affiner Substanz, gegebenenfalls konjugiert mit einem Internalisierungsfaktor für die Zellen, komplexiert ist, **dadurch gekennzeichnet, daß** die Zusammensetzung außerdem ein endosomolytisches Mittel enthält, wobei
a) das endosomolytische Mittel als Bestandteil des Komplexes vorliegt, indem es über eine ihm eigene Nukleinsäure-Bindungsdomäne oder über eine Nukleinsäure-affine Substanz, an die es gebunden ist, mit der Nukleinsäure komplexiert ist, und wobei der Komplex gegebenenfalls außerdem einen Internalisierungsfaktor enthält, der über eine Nukleinsäure-affine Substanz, mit der er konjugiert ist, ebenfalls mit der Nukleinsäure komplexiert ist, oder wobei
b) als endosomolytisches Mittel ein Virus oder eine Viruskomponente, das bzw. die die Fähigkeit hat, *per se* in die zu transfizierende Zelle einzudringen, außerhalb des Komplexes vorliegt, in dem Nukleinsäure-affine Substanz, die gegebenenfalls einem Internalisierungsfaktor konjugiert ist, mit der Nukleinsäure komplexiert ist.

2. Zusammensetzung nach Anspruch 1, **dadurch gekennzeichnet, daß** in einer Zusammensetzung gemäß
a) das endosomolytische Mittel kovalent an eine Nukleinsäure-affine Substanz gebunden ist.

3. Zusammensetzung nach Anspruch 1, **dadurch gekennzeichnet, daß** in einer Zusammensetzung gemäß a) das endosomolytische Mittel nicht-kovalent an die Nukleinsäure-affine Substanz gebunden ist.

4. Zusammensetzung nach Anspruch 3, **dadurch gekennzeichnet, daß** die Bindung über eine Biotin-Streptavidin-Brücke erfolgt.

5. Zusammensetzung nach Anspruch 3, **dadurch gekennzeichnet, daß** die Bindung ionisch erfolgt.

6. Zusammensetzung nach Anspruch 1, **dadurch gekennzeichnet, daß** in einer Zusammensetzung gemäß a) das endosomolytische Mittel ein Virus oder eine Viruskomponente ist.

7. Zusammensetzung nach Anspruch 1 oder 6, **dadurch gekennzeichnet, daß** in einer Zusammensetzung gemäß a) oder b) das endosomolytische Mittel ein Adenovirus ist.

8. Zusammensetzung nach Anspruch 7, **dadurch gekennzeichnet, daß** das Adenovirus eine Mutante ist.

9. Zusammensetzung nach Anspruch 8, **dadurch gekennzeichnet, daß** das Adenovirus eine replikationsinkompetente Mutante ist.

10. Zusammensetzung nach Anspruch 9, **dadurch gekennzeichnet, daß** das Adenovirus eine oder mehrere Mutationen und/oder Deletionen in der ElA-Region aufweist.

11. Zusammensetzung nach Anspruch 7, **dadurch gekennzeichnet, daß** das Adenovirus inaktiviert ist.

12. Zusammensetzung nach Anspruch 11, **dadurch gekennzeichnet, daß** das Adenovirus durch kurzwelliges UV inaktiviert ist.

13. Zusammensetzung nach Anspruch 11, **dadurch gekennzeichnet, daß** das Adenovirus durch UV/Psoralen inaktiviert ist.

14. Zusammensetzung nach Anspruch 11, **dadurch gekennzeichnet, daß** das Adenovirus durch Formaldehyd inaktiviert ist.

15. Zusammensetzung nach Anspruch 6, **dadurch gekennzeichnet, daß** die Viruskomponente aus einem oder mehreren Adenovirusproteinen besteht.

16. Zusammensetzung nach Anspruch 1 oder 6, **dadurch gekennzeichnet, daß** in einer Zusammensetzung gemäß a) oder b) das endosomolytische Mittel ein Picornavirus ist.

17. Zusammensetzung nach Anspruch 16, **dadurch gekennzeichnet, daß** das Picornavirus ein, gegebenenfalls inaktiviertes, Rhinovirus ist.

18. Zusammensetzung nach Anspruch 1, **dadurch gekennzeichnet, daß** das endosomolytische Mittel in einer Zusammensetzung gemäß a) ein Virus ist, das für eine Spezies verschieden vom Menschen infektiös ist.

19. Zusammensetzung nach Anspruch 18, **dadurch gekennzeichnet, daß** das Virus ein Adenovirus ist.

20. Zusammensetzung nach Anspruch 19, **dadurch gekennzeichnet, daß** das Adenovirus ein Vogel-Adenovirus ist.

21. Zusammensetzung nach Anspruch 20, **dadurch gekennzeichnet, daß** das Adenovirus das Chick Embryo Lethal Orphan Virus ist.

22. Zusammensetzung nach Anspruch 1, **dadurch gekennzeichnet, daß** das endosomolytische Mittel in einer Zusammensetzung gemäß a) ein, gegebenenfalls modifiziertes, endosomolytisches virales Peptid ist.

23. Zusammensetzung nach Anspruch 22, **dadurch gekennzeichnet, daß** das endosomolytische Peptid ein Influenza-Hämagglutinin HA2-Peptid ist.

24. Zusammensetzung nach Anspruch 23, **dadurch gekennzeichnet, daß** das Peptid die Sequenz Gly-Leu-Phe-Glu-Ala-Ile-Ala-Gly-Phe-Ile-Glu-Asn-Gly-Trp-Glu-Gly-Met-Ile-Asp-Gly-Gly-Gly-Cys hat.

25. Zusammensetzung nach Anspruch 23, **dadurch gekennzeichnet, daß** das Peptid die Sequenz Gly-Leu-Phe-Gly-Ala-Ile-Ala-Gly-Phe-Ile-Glu-Asn-Gly-Trp-Glu-Gly-Met-Ile-Asp-Gly-Gly-Gly-Cys hat.

26. Zusammensetzung nach Anspruch 1, **dadurch gekennzeichnet, daß** das endosomolytische Mittel in einer Zusammensetzung gemäß a) ein nicht-virales, gegebenenfalls modifiziertes, natürliches oder synthetisches Peptid ist, das die Fähigkeit des Aufbrechens von Zellmembranen, bestimmbar mittels Liposomendurchlässigkeitstest und/oder Erythrozytenlysetest, aufweist.

27. Zusammensetzung nach Anspruch 26, **dadurch gekennzeichnet, daß** das Peptid die Sequenz Gly Leu Phe Glu Ala Ile Glu Gly Phe Ile Glu Asn Gly Trp Glu Gly Met Ile Asp Gly Gly Gly Cys aufweist.

28. Zusammensetzung nach Anspruch 26, **dadurch gekennzeichnet, daß** das Peptid ein Homo- oder Heterodimer des in Anspruch 27 definierten Peptids ist.

29. Zusammensetzung nach Anspruch 28, **dadurch gekennzeichnet, daß** das Peptid ein Homodimer ist.

30. Zusammensetzung nach Anspruch 28, **dadurch gekennzeichnet, daß** das Peptid ein Heterodimer ist, das die Sequenz Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser Cys aufweist.

31. Zusammensetzung nach Anspruch 26, **dadurch gekennzeichnet, daß** das Peptid die Sequenz Trp Glu Ala Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys aufweist.

32. Zusammensetzung nach Anspruch 26, **dadurch gekennzeichnet, daß** das Peptid die Sequenz Gly Leu Phe Gly Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys aufweist.

33. Zusammensetzung nach Anspruch 22 oder 26, **dadurch gekennzeichnet, daß** das Peptid eine Nukleinsäure-Bindungsdomäne aufweist.

34. Zusammensetzung nach Anspruch 33, **dadurch gekennzeichnet, daß** das Peptid eine Oligolysinverlängerung aufweist.

35. Zusammensetzung nach Anspruch 1, **dadurch gekennzeichnet, daß** der Internalisierungsfaktor Transferrin ist.

36. Zusammensetzung nach Anspruch 1, **dadurch gekennzeichnet, daß** der Internalisierungsfaktor ein Ligand für T-Zellen ist.

37. Zusammensetzung nach Anspruch 1, **dadurch gekennzeichnet, daß** der Internalisierungsfaktor ein Ligand für B-Zellen ist.

38. Zusammensetzung nach Anspruch 37, **dadurch gekennzeichnet, daß** der Internalisierungsfaktor ein Immunglobulin ist.

39. Zusammensetzung nach Anspruch 37, **dadurch gekennzeichnet, daß** der Ligand ein anti-Immunglobulin ist.

40. Zusammensetzung nach Anspruch 1, **dadurch gekennzeichnet, daß** der Internalisierungsfaktor ein Ligand für Hepatozyten ist.

41. Zusammensetzung nach Anspruch 40, **dadurch gekennzeichnet, daß** der Internalisierungsfaktor ein Ligand für den Asialoglykoproteinrezeptor ist.

42. Zusammensetzung nach Anspruch 41, **dadurch gekennzeichnet, daß** der Internalisierungsfaktor ein Tetragalaktose-Polylysin ist.

43. Zusammensetzung nach Anspruch 1, **dadurch gekennzeichnet, daß** der Internalisierungsfaktor ein Lectin ist.

44. Zusammensetzung nach Anspruch 1, **dadurch gekennzeichnet, daß** der Internalisierungsfaktor ein Low Density Lipoprotein ist.

45. Komplex als Bestandteil einer Zusammensetzung nach einem der Ansprüche 1 bis 44, **dadurch gekennzeichnet, daß** er eine oder mehrere Nukleinsäuren enthält, mit denen ein endosomolytisches Mittel über eine ihm eigene Nukleinsäure-Bindungsdomäne oder über eine Nukleinsäure-affine Substanz, an die es gebunden ist, komplexiert ist und daß er gegebenenfalls einen Internalisierungsfaktor enthält, der über eine Nukleinsäure-affine Substanz, mit der er konjugiert ist, ebenfalls mit der Nukleinsäure komplexiert ist.

46. Komplex nach Anspruch 45, **dadurch gekennzeichnet, daß** er eine therapeutisch aktive Nukleinsäure enthält.

47. Komplex nach Anspruch 46, **dadurch gekennzeichnet, daß** die Nukleinsäure aus einem oder mehreren gentherapeutisch wirksamen DNA-Molekülen besteht.

48. Komplex nach Anspruch 47, **dadurch gekennzeichnet, daß** das DNA-Molekül für ein Zytokin kodiert.

49. Komplex nach Anspruch 47, **dadurch gekennzeichnet, daß** das DNA-Molekül für Faktor VIII kodiert.

50. Komplex nach Anspruch 46, **dadurch gekennzeichnet, daß** die Nukleinsäure eine Nukleotidsequenz enthält, von der RNA-Moleküle transkribiert werden, die spezifisch Zellfunktionen inhibieren.

51. Komplex nach Anspruch 45, **dadurch gekennzeichnet, daß** die Nukleinsäure-affine Substanz ein organisches Polykation ist.

52. Komplex nach Anspruch 51, **dadurch gekennzeichnet, daß** das Polykation Polylysin ist.

53. Komplex nach Anspruch 51, **dadurch gekennzeichnet, daß** das Polykation Polyethylenimin ist.

54. Komplex nach Anspruch 45, **dadurch gekennzeichnet, daß** er einen Internalisierungsfaktor enthält, der an dieselbe Nukleinsäure-affine Substanz gebunden ist wie das endosomolytische Mittel.

55. Komplex nach Anspruch 54, **dadurch gekennzeichnet, daß** die Nukleinsäure-affine Substanz Polylysin ist.

56. Komplex nach Anspruch 55, **dadurch gekennzeichnet, daß** er außerdem nicht-kovalent gebundenes Polylysin enthält.

57. Konjugat als Bestandteil eines Komplexes nach einem der Ansprüche 45 bis 56, **dadurch gekennzeichnet, daß** das Konjugat aus einem endosomolytischen Mittel und einer Nukleinsäure-affinen Substanz besteht.

58. Konjugat nach Anspruch 57, **dadurch gekennzeichnet, daß** das endosomolytische Mittel kovalent an eine Nukleinsäure-affine Substanz gebunden ist.

59. Konjugat nach Anspruch 57, **dadurch gekennzeichnet, daß** das endosomolytische Mittel nicht-kovalent an eine Nukleinsäure-affine Substanz gebunden ist.

60. Konjugat nach Anspruch 59, **dadurch gekennzeichnet, daß** die Bindung über eine Biotin-Streptavidin-Brücke erfolgt.

61. Konjugat nach Anspruch 59, **dadurch gekennzeichnet, daß** die Bindung ionisch erfolgt.

62. Konjugat nach Anspruch 57, **dadurch gekennzeichnet, daß** das endosomolytische Mittel ein Virus oder eine Viruskomponente ist.

63. Konjugat nach Anspruch 62, **dadurch gekennzeichnet, daß** das endosomolytische Mittel ein Adenovirus ist.

64. Konjugat nach Anspruch 63, **dadurch gekennzeichnet, daß** das Adenovirus eine Mutante ist.

65. Konjugat nach Anspruch 64, **dadurch gekennzeichnet, daß** das Adenovirus eine replikationsinkompetente Mutante ist.

66. Konjugat nach Anspruch 65, **dadurch gekennzeichnet, daß** das Adenovirus eine oder mehr Mutationen und/oder Deletionen in der ElA-Region aufweist.

67. Konjugat nach Anspruch 63, **dadurch gekennzeichnet, daß** das Adenovirus inaktiviert ist.

68. Konjugat nach Anspruch 67, **dadurch gekennzeichnet, daß** das Adenovirus durch kurzwelliges UV inaktiviert ist.

69. Konjugat nach Anspruch 67, **dadurch gekennzeichnet, daß** das Adenovirus durch UV/Psoralen inaktiviert ist.

70. Konjugat nach Anspruch 67, **dadurch gekennzeichnet, daß** Adenovirus durch Formaldehyd inaktiviert ist.

71. Konjugat nach Anspruch 62, **dadurch gekennzeichnet, daß** die Viruskomponente aus einem oder mehreren Adenovirusproteinen besteht.

72. Konjugat nach Anspruch 62, **dadurch gekennzeichnet, daß** das Virus ein Picornavirus ist.

73. Konjugat nach Anspruch 72, **dadurch gekennzeichnet, daß** das Virus ein, gegebenenfalls inaktiviertes, Rhinovirus ist.

74. Konjugat nach Anspruch 57, **dadurch gekennzeichnet, daß** das endosomolytische Mittel nicht *per se* die Fähigkeit aufweist, in die zu transfizierende Zelle einzudringen.

75. Konjugat nach Anspruch 74, **dadurch gekennzeichnet, daß** das endosomolytische Mittel ein Virus oder eine Viruskomponente ist, das bzw. die kein Internalisierungsfaktor für eine humane Zelle ist.

76. Konjugat nach Anspruch 75, **dadurch gekennzeichnet, daß** das endosomolytische Mittel ein Virus ist, das für eine Spezies verschieden vom Menschen infektiös ist.

77. Konjugat nach Anspruch 76, **dadurch gekennzeichnet, daß** das Virus ein Adenovirus ist.

78. Konjugat nach Anspruch 77, **dadurch gekennzeichnet, daß** das Adenovirus ein Vogeladenovirus ist.

79. Konjugat nach Anspruch 78, **dadurch gekennzeichnet, daß** das Adenovirus das Chick Embryo Lethal Orphan Virus ist.

80. Konjugat nach Anspruch 74, **dadurch gekennzeichnet, daß** das endosomolytische Mittel ein gegebenenfalls modifiziertes endosomolytisches virales Peptid mit der in Anspruch 26 definierten Eigenschaft ist.

81. Konjugat nach Anspruch 80, **dadurch gekennzeichnet, daß** das endosomolytische Peptid ein Influenza-Hämagglutinin HA2-Peptid ist.

82. Konjugat nach Anspruch 81, **dadurch gekennzeichnet, daß** das Peptid die Sequenz Gly-Leu-Phe-Glu-Ala-Ile-Ala-Gly-Phe-Ile-Glu-Asn-Gly-Trp-Glu-Gly-Met-Ile-Asp-Gly-Gly-Gly-Cys hat.

83. Konjugat nach Anspruch 81, **dadurch gekennzeichnet, daß** das Peptid die Sequenz Gly-Leu-Phe-Gly-Ala-Ile-Ala-Gly-Phe-Ile-Glu-Asn-Gly-Trp-Glu-Gly-Met-Ile-Asp-Gly-Gly-Gly-Cys hat.

84. Konjugat nach Anspruch 74, **dadurch gekennzeichnet, daß** das endosomolytische Mittel ein in Anspruch 26 definiertes Peptid ist.

85. Konjugat nach Anspruch 84, **dadurch gekennzeichnet, daß** das Peptid die Sequenz Gly Leu Phe Glu Ala Ile Glu Gly Phe Ile Glu Asn Gly Trp Glu Gly Met Ile Asp Gly Gly Gly Cys aufweist.

86. Konjugat nach Anspruch 84, **dadurch gekennzeichnet, daß** das Peptid ein Homo- oder Heterodimer des in Anspruch 85 definierten Peptids ist.

87. Konjugat nach Anspruch 86, **dadurch gekennzeichnet, daß** das Peptid ein Homodimer ist.

88. Konjugat nach Anspruch 86, **dadurch gekennzeichnet, daß** das Peptid ein Heterodimer ist, das die Sequenz Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser Cys aufweist.

89. Konjugat nach Anspruch 84, **dadurch gekennzeichnet, daß** das Peptid die Sequenz Trp Glu Ala Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys aufweist.

90. Konjugat nach Anspruch 84, **dadurch gekennzeichnet, daß** das Peptid die Sequenz Gly Leu Phe Gly Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys aufweist.

91. Konjugat nach Anspruch 84, **dadurch gekennzeichnet, daß** das Peptid eine Nukleinsäure-Bindungsdomäne aufweist.

92. Konjugat nach Anspruch 91, **dadurch gekennzeichnet, daß** das Peptid eine Oligolysinverlängerung auf weist.

93. Endosomolytisches Peptid, geeignet als Bestandteil der Zusammensetzung nach Anspruch 33, **dadurch gekennzeichnet, daß** es eine endosomolytische Domäne und eine künstlich eingeführte Nukleinsäure-Bindungsdomäne hat.

94. Peptid nach Anspruch 93, **dadurch gekennzeichnet, daß** die Nukleinsäure-Bindungsdomäne eine Oligolysinverlängerung ist.

95. Verfahren zur Herstellung eines Konjugats nach Anspruch 58, **dadurch gekennzeichnet, daß** ein Virus oder ein (poly)peptidisches endosomolytisches Mittel und ein Polyamin enzymatisch in Gegenwart einer Transglutaminase gekoppelt werden.

96. Verfahren zur Herstellung eines Konjugats nach Anspruch 58, **dadurch gekennzeichnet, daß** ein Virus oder ein (poly)peptidisches endosomolytisches Mittel und ein Polyamin chemisch gekoppelt werden.

97. Verfahren zur Herstellung eines Konjugats nach Anspruch 60, **dadurch gekennzeichnet, daß** ein Virus oder eine Viruskomponente mit Biotin modifiziert wird und daß das modifizierte Virus oder die modifizierte Viruskomponente an ein Streptavidingekoppeltes Polyamin gebunden wird.

98. Verfahren zum Einbringen von Nukleinsäure in höhere eukaryotische Zellen *in vitro oder ex vivo,* **dadurch gekennzeichnet, daß** die Zellen mit einer Zusammensetzung nach einem der Ansprüche 1 bis 44 behandelt werden.

99. Verfahren nach Anspruch 98, **dadurch gekennzeichnet, daß** die Zellen humane Zellen sind.

100. Verfahren nach Anspruch 99, **dadurch gekennzeichnet, daß** die Zellen Tumorzellen sind.

101. Verfahren nach Anspruch 99, **dadurch gekennzeichnet, daß** die Zellen Myoblasten sind.

102. Verfahren nach Anspruch 99, **dadurch gekennzeichnet, daß** die Zellen Fibroblasten sind.

103. Verfahren nach Anspruch 99, **dadurch gekennzeichnet, daß** die Zellen Hepatozyten sind.

104. Verfahren nach Anspruch 99, **dadurch gekennzeichnet, daß** die Zellen endotheliale Zellen sind.

105. Verfahren nach Anspruch 104, **dadurch gekennzeichnet, daß** die Zellen Atemwegszellen sind.

106. Verfahren nach einem der Ansprüche 99 bis 105, **dadurch gekennzeichnet, daß** die Nukleinsäure in der Zusammensetzung gentherapeutisch wirksam ist.

107. Verfahren nach den Ansprüchen 100 und 106, **dadurch gekennzeichnet, daß** die Tumorzellen mit der Zusammensetzung *ex vivo* behandelt werden und daß die DNA für eine oder mehrere immunmodulierende Substanzen, vorzugsweise Zytokine, kodiert.

108. Verfahren zur Herstellung eines heterologen Proteins in einer höheren eukaryotischen Zelle, **dadurch gekennzeichnet, daß** man die Zellen mit einer Zusammensetzung gemäß Anspruch 1 behandelt,
wobei die Nukleinsäure eine DNA-Sequenz enthält, die für das gewünschte Protein kodiert, die Zellen unter Bedingungen, die für die Expression des Proteins geeignet sind, kultiviert und das Protein isoliert.

109. Pharmazeutische Zubereitung, enthaltend als wirksamen Bestandteil eine Zusammensetzung nach einem der Ansprüche 1 bis 44, enthaltend eine therapeutisch aktive Nukleinsäure und einen pharmazeutisch annehmbaren Träger.

110. Transfektionskit, enthaltend eine Trägereinheit, in der sich zwei oder mehrere Behälter befinden, wobei ein erster Behälter eine Nukleinsäure-affine Substanz enthält, die gegebenenfalls mit einem Internalisierungsfaktor für eine höhere eukaryotische Zelle konjugiert ist und ein zweiter Behälter ein endosomolytisches Mittel enthält, welches ein Virus oder eine Viruskomponente, ist, das bzw. die die Fähigkeit hat, *per se* in die zu transfizierende Zelle einzudringen.

111. Transfektionskit, enthaltend eine Trägereinheit, in der sich zwei oder mehrere Behälter befinden, wobei ein erster Behälter eine Nukleinsäure-affine Substanz enthält, die mit einem Internalisierungsfaktor für eine höhere eukaryotische Zelle konjugiert ist und ein zweiter Behälter eine Nukleinsäure-affine Substanz enthält, die an ein endosomolytisches Mittel gebunden ist.

112. Transfektionskit nach Anspruch 111, **dadurch gekennzeichnet, daß** er in dem zweiten Behälter Transglutaminase-gekoppeltes Adenovirus-Polylysin-Konjugat enthält.

113. Transfektionskit, enthaltend eine Trägereinheit, in der sich zwei oder mehrere Behälter befinden, wobei ein erster Behälter ein Biotin-modifiziertes endosomolytisches Mittel und ein zweiter Behälter eine Streptavidin-modifizierte Nukleinsäure-affine Substanz enthält.

114. Transfektionskit nach Anspruch 113, **dadurch gekennzeichnet, daß** der erste Behälter biotinyliertes Adenovirus und ein zweiter Behälter Streptavidin-Polylysin enthält.

## Claims

1. Composition for the transfection of higher eukaryotic cells containing a complex in which nucleic acid is complexed with a substance having affinity for nucleic acid, optionally conjugated with an internalizing factor for the cells, **characterized in that** the composition also contains an endosomolytic agent, wherein
a) the endosomolytic agent is present as a component of the complex by being complexed with the nucleic acid via a nucleic acid binding domain peculiar to said agent or via a substance having affinity for nucleic acid to which it is bound, and wherein the complex optionally also contains an internalising factor which is also complexed with the nucleic acid via a substance having affinity for nucleic acid with which it is conjugated, or wherein
b) as the endosomolytic agent, a virus or a virus component which has the ability to penetrate *per se* into the cell which is to be transfected is present outside the complex in which a substance having affinity for nucleic acid, which is optionally conjugated with an internalising factor, is complexed with the nucleic acid.

2. Composition according to claim 1, **characterized in that** in a composition according to a) the endosomolytic agent is covalently bound to a substance having affinity for nucleic acid.

3. Composition according to claim 1, **characterized in that** in a composition according to a) the endosomolytic agent is non-covalently bound to a substance having affinity for nucleic acid.

4. Composition according to claim 3, **characterized in that** the binding is effected via a biotin-streptavidin bridge.

5. Composition according to claim 3, **characterized in that** the binding is effected ionically.

6. Composition according to claim 1, **characterized in that** in a composition according to a) the endosomolytic agent is a virus or a virus component.

7. Composition according to claim 1 or 6, **characterized in that** in a composition according to a) or b) the endosomolytic agent is an adenovirus.

8. Composition according to claim 7, **characterized in that** the adenovirus is a mutant.

9. Composition according to claim 8, **characterized in that** the adenovirus is a mutant incapable of replication.

10. Composition according to claim 9, **characterized in that** the adenovirus has one or more mutations and/or deletions in the E1A region.

11. Composition according to claim 7, **characterized in that** the adenovirus is inactivated.

12. Composition according to claim 11, **characterized in that** the adenovirus is inactivated by short wave UV.

13. Composition according to claim 11, **characterized in that** the adenovirus is inactivated by UV/psoralen.

14. Composition according to claim 11, **characterized in that** the adenovirus is inactivated by formaldehyde.

15. Composition according to claim 6, **characterized in that** the virus component consists of one or more adenovirus proteins.

16. Composition according to claim 1 or 6, **characterized in that** in a composition according to a) or b) the endosomolytic agent is a picornavirus.

17. Composition according to claim 16, **characterized in that** the picornavirus is an optionally inactivated rhinovirus.

18. Composition according to claim 1, **characterized in that** the endosomolytic agent in a composition according to a) is a virus which is infectious for a species other than man.

19. Composition according to claim 18, **characterized in that** the virus is an adenovirus.

20. Composition according to claim 19, **characterized in that** the adenovirus is an avian adenovirus.

21. Composition according to claim 20, **characterized in that** the adenovirus is the Chick Embryo Lethal Orphan virus.

22. Composition according to claim 1, **characterized in that** the endosomolytic agent in a composition according to a) is an optionally modified endosomolytic viral peptide.

23. Composition according to claim 22, **characterized in that** the endosomolytic peptide is an influenzahaemagglutinin HA2 peptide.

24. Composition according to claim 23, **characterized in that** the peptide has the sequence Gly-Leu-Phe-Glu-Ala-Ile-Ala-Gly-Phe-Ile-Glu-Asn-Gly-Trp-Glu-Gly-Met-Ile-Asp-Gly-Gly-Gly-Cys.

25. Composition according to claim 23, **characterized in that** the peptide has the sequence Gly-Leu-Phe-Gly-Ala-Ile-Ala-Gly-Phe-Ile-Glu-Asn-Gly-Trp-Glu-Gly-Met-Ile-Asp-Gly-Gly-Gly-Cys.

26. Composition according to claim 1, **characterized in that** the endosomolytic agent in a composition according to a) is a non-viral, optionally modified, natural or synthetic peptide which is capable of breaking open cell membranes, which can be detected by the liposome permeability test and/or erythrocyte lysing test.

27. Composition according to claim 26, **characterized in that** the peptide has the sequence Gly Leu Phe Glu Ala Ile Glu Gly Phe Ile Glu Asn Gly Trp Glu Gly Met Ile Asp Gly Gly Gly Cys.

28. Composition according to claim 26, **characterized in that** the peptide is a homo- or heterodimer of the peptide defined in claim 27.

29. Composition according to claim 28, **characterized in that** the peptide is a homodimer.

30. Composition according to claim 28, **characterized in that** the peptide is a heterodimer which contains the sequence Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser Cys.

31. Composition according to claim 26, **characterized in that** the peptide has the sequence Trp Glu Ala Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

32. Composition according to claim 26, **characterized in that** the peptide has the sequence Gly Leu Phe Gly Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

33. Composition according to claim 22 or 26, **characterized in that** the peptide has a nucleic acid binding domain.

34. Composition according to claim 33, **characterized in that** the peptide has an oligolysine extension.

35. Composition according to claim 1, **characterized in that** the internalizing factor is transferrin.

36. Composition according to claim 1, **characterized in that** the internalising factor is a ligand for T-cells.

37. Composition according to claim 1, **characterized in that** the internalising factor is a ligand for B-cells.

38. Composition according to claim 37, **characterized in that** the internalising factor is an immunoglobulin.

39. Composition according to claim 37, **characterized in that** the ligand is an anti-immunoglobulin.

40. Composition according to claim 1, **characterized in that** the internalizing factor is a ligand for hepatocytes.

41. Composition according to claim 40, **characterized in that** the internalizing factor is a ligand for the asialoglycoprotein receptor.

42. Composition according to claim 41, **characterized in that** the internalizing factor is a tetra-galactosepolylysine.

43. Composition according to claim 1, **characterized in that** the internalising factor is a lectin.

44. Composition according to claim 1, **characterized in that** the internalising factor is a low-density lipoprotein.

45. Complex as a constituent of a composition according to one of claims 1 to 44, **characterised in that** it contains one or more nucleic acids with which an endosomolytic agent is complexed via a nucleic acid binding domain peculiar to said agent or via a substance having affinity for nucleic acid, to which it is bound, and **in that** it optionally contains an internalizing factor which is also complexed with the nucleic acid via a substance having affinity for nucleic acid with which it is conjugated.

46. Complex according to claim 45, **characterized in that** it contains a therapeutically active nucleic acid.

47. Complex according to claim 46, **characterized in that** the nucleic acid consists of one or more DNA molecules which are active in gene therapy.

48. Complex according to claim 47, **characterized in that** the DNA molecule codes for a cytokine.

49. Complex according to claim 47, **characterized in that** the DNA molecule codes for Factor VIII.

50. Complex according to claim 46, **characterized in that** the nucleic acid contains a nucleotide sequence from which RNA molecules which specifically inhibit cell functions can be transcribed.

51. Complex according to claim 45, **characterized in that** the substance having affinity for nucleic acid is an organic polycation.

52. Complex according to claim 51, **characterized in that** the polycation is polylysine.

53. Complex according to claim 51, **characterized in that** the polycation is polyethyleneimine.

54. Complex according to claim 45, **characterized in that** it contains an internalising factor which is bound to the same substance having affinity for nucleic acid as the endosomolytic agent.

55. Complex according to claim 54, **characterized in that** the substance having affinity for nucleic acid is polylysine.

56. Complex according to claim 55, **characterized in that** it further comprises non-covalently bound polylysine.

57. Conjugate as a constituent of a complex according to one of claims 45 to 56, **characterized in that** the conjugate consists of an endosomolytic agent and a substance having affinity for nucleic acid.

58. Conjugate according to claim 57, **characterized in that** the endosomolytic agent is covalently bound to a substance having affinity for nucleic acid.

59. Conjugate according to claim 57, **characterized in that** the endosomolytic agent is non-covalently bound to a substance having affinity for nucleic acid.

60. Conjugate according to claim 59, **characterized in that** the binding is effected via a biotin-streptavidin bridge.

61. Conjugate according to claim 59, **characterized in that** the binding is effected ionically.

62. Conjugate according to claim 57, **characterized in that** the endosomolytic agent is a virus or a virus component.

63. Conjugate according to claim 62, **characterized in that** the endosomolytic agent is an adenovirus.

64. Conjugate according to claim 63, **characterized in that** the adenovirus is a mutant.

65. Conjugate according to claim 64, **characterized in that** the adenovirus is a mutant incapable of replication.

66. Conjugate according to claim 65, **characterized in that** the adenovirus has one or more mutations and/or deletions in the E1A region.

67. Conjugate according to claim 63, **characterized in that** the adenovirus is inactivated.

68. Conjugate according to claim 67, **characterized in that** the adenovirus is inactivated by short wave UV.

69. Conjugate according to claim 67, **characterized in that** the adenovirus is inactivated by UV/psoralen.

70. Conjugate according to claim 67, **characterized in that** the adenovirus is inactivated by formaldehyde.

71. Conjugate according to claim 62, **characterized in that** the virus component consists of one or more adenovirus proteins.

72. Conjugate according to claim 62, **characterized in that** the virus is a picornavirus.

73. Conjugate according to claim 72, **characterized in that** the virus is an optionally inactivated rhinovirus.

74. Conjugate according to claim 57, **characterized in that** the endosomolytic agent does not *per se* have the ability to penetrate into the cell which is to be transfected.

75. Conjugate according to claim 74, **characterized in that** the endosomolytic agent is a virus or a virus component which is not an internalising factor for a human cell.

76. Conjugate according to claim 75, **characterized in that** the endosomolytic agent is a virus which is infectious for a species other than man.

77. Conjugate according to claim 76, **characterized in that** the virus is an adenovirus.

78. Conjugate according to claim 77, **characterized in that** the adenovirus is an avian adenovirus.

79. Conjugate according to claim 78, **characterized in that** the adenovirus is the Chick Embryo Lethal Orphan virus.

80. Conjugate according to claim 74, **characterized in that** the endosomolytic agent is an optionally modified endosomolytic viral peptide having the property defined in claim 26.

81. Conjugate according to claim 80, **characterized in that** the endosomolytic peptide is an influenzahaemagglutinin HA2 peptide.

82. Conjugate according to claim 81, **characterized in that** the peptide has the sequence Gly-Leu-Phe-Glu-Ala-Ile-Ala-Gly-Phe-Ile-Glu-Asn-Gly-Trp-Glu-Gly-Met-Ile-Asp-Gly-Gly-Gly-Cys.

83. Conjugate according to claim 81, **characterized in that** the peptide has the sequence Gly-Leu-Phe-Gly-Ala-Ile-Ala-Gly-Phe-Ile-Glu-Asn-Gly-Trp-Glu-Gly-Met-Ile-Asp-Gly-Gly-Gly-Cys.

84. Conjugate according to claim 74, **characterized in that** the endosomolytic agent is a peptide as defined in claim 26.

85. Conjugate according to claim 84, **characterized in that** the peptide has the sequence Gly Leu Phe Glu Ala Ile Glu Gly Phe Ile Glu Asn Gly Trp Glu Gly Met Ile Asp Gly Gly Gly Cys.

86. Conjugate according to claim 84, **characterized in that** the peptide is a homo- or heterodimer of the peptide defined in claim 85.

87. Conjugate according to claim 86, **characterized in that** the peptide is a homodimer.

88. Conjugate according to claim 86, **characterized in that** the peptide is a heterodimer which contains the sequence Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser Cys.

89. Conjugate according to claim 84, **characterized in that** the peptide has the sequence Trp Glu Ala Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

90. Conjugate according to claim 84, **characterized in that** the peptide has the sequence Gly Leu Phe Gly Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

91. Conjugate according to claim 84, **characterized in that** the peptide has a nucleic acid binding domain.

92. Conjugate according to claim 91, **characterized in that** the peptide has an oligolysine extension.

93. Endosomolytic peptide suitable as a constituent of the composition according to claim 33, **characterized in that** it has an endosomolytic domain and an artificially introduced nucleic acid binding domain.

94. Peptide according to claim 93, **characterized in that** the nucleic acid binding domain is an oligolysine extension.

95. Process for preparing a conjugate according to claim 58, **characterized in that** a virus or a (poly)peptidic endosomolytic agent and a polyamine are enzymatically coupled in the presence of a transglutaminase.

96. Process for preparing a conjugate according to claim 58, **characterized in that** a virus or a (poly)peptidic endosomolytic agent and a polyamine are chemically coupled.

97. Process for preparing a conjugate according to claim 60, **characterised in that** a virus or a virus component is modified with biotin, and the modified virus or the modified virus component is bound to a streptavidin-coupled polyamine.

98. Process for introducing nucleic acid into higher eukaryotic cells *in vitro* or *ex vivo*, **characterized in that** the cells are treated with a composition according to any of the claims 1 to 44.

99. Process according to claim 98, **characterized in that** the cells are human cells.

100. Process according to claim 99, **characterized in that** the cells are tumour cells.

101. Process according to claim 99, **characterized in that** the cells are myoblasts.

102. Process according to claim 99, **characterized in that** the cells are fibroblasts.

103. Process according to claim 99, **characterized in that** the cells are hepatocytes.

104. Process according to claim 99, **characterized in that** the cells are endothelial cells.

105. Process according to claim 104, **characterized in that** the cells are respiratory tract cells.

106. Process according to one of claims 99 to 105, **characterized in that** the nucleic acid in the composition is active in gene therapy.

107. Process according to claims 100 and 106, **characterized in that** the tumour cells are treated with the composition *ex vivo* and that the DNA codes for one or more immune modulating substances, preferably cytokines.

108. Process for producing a heterologous protein in a higher eukaryotic cell, **characterized in that** the cells are treated with a composition according to claim 1, the nucleic acid containing a DNA sequence which codes for the desired protein, the cells are cultivated under conditions suitable for expression of the protein, and the protein is isolated.

109. Pharmaceutical preparation containing, as active ingredient, a composition according to one of claims 1 to 44, containing a therapeutically active nucleic acid and a pharmaceutically acceptable carrier.

110. Transfection kit containing a carrier unit in which there are two or more containers, a first container containing a substance having an affinity for nucleic acid which is optionally coupled to an internalising factor for a higher eukaryotic cell and a second container containing an endosomolytic agent which is a virus or a virus component which is capable of penetrating *per se* into the cell which is to be transfected.

111. Transfection kit containing a carrier unit in which there are two or more containers, a first container containing a substance having an affinity for nucleic acid which is conjugated with an internalising factor for a higher eukaryotic cell and a second container containing a substance having an affinity for nucleic acid which is bound to an endosomolytic agent.

112. Transfection kit according to claim 111, **characterized in that** it contains in the second container transglutaminase-coupled adenovirus-polylysine conjugate.

113. Transfection kit containing a carrier unit in which there are two or more containers, a first container containing a biotin-modified endosomolytic agent and a second container containing a streptavidin-modified substance having an affinity for nucleic acid.

114. Transfection kit according to claim 113, **characterized in that** the first container contains biotinylated adenovirus and a second container contains streptavidin-polylysine.

## Revendications

1. Composition pour la transfection de cellules eucaryotes supérieures, contenant un complexe dans lequel un acide nucléique est complexé avec une substance à affinité pour les acides nucléiques, éventuellement conjuguée avec un facteur d'internalisation pour les cellules, **caractérisée en ce que** la composition contient en outre un agent endosomolytique où
a) l'agent endosomolytique est sous forme de constituant du complexe en étant complexé avec l'acide nucléique par le biais d'un domaine de liaison d'acide nucléique qui lui est propre ou par le biais d'une substance à affinité pour les acides nucléiques à laquelle il est lié, et où le complexe contient éventuellement en outre un facteur d'internalisation qui est complexé aussi avec l'acide nucléique par le biais d'une substance à affinité pour les acides nucléiques avec laquelle il est conjugué,ou bien où
b) un virus ou un composant viral qui est capable de pénétrer par lui-même dans la cellule à transfecter, est présent en tant qu'agent endosomolytique à l'extérieur du complexe dans lequel une substance à affinité pour les acides nucléiques, qui est éventuellement conjuguée avec un facteur d'internalisation, est complexée avec l'acide nucléique.

2. Composition selon la revendication 1 **caractérisée en ce que**, dans une composition selon a), l'agent endosomolytique est lié de manière covalente à une substance à affinité pour les acides nucléiques.

3. Composition selon la revendication 1 **caractérisée en ce que**, dans une composition selon a), l'agent endosomolytique est lié de manière non covalente à la substance à affinité pour les acides nucléiques.

4. Composition selon la revendication 3 **caractérisée en ce que** la liaison a lieu par le biais d'un pont biotine-streptavidine.

5. Composition selon la revendication 3 **caractérisée en ce que** la liaison a lieu de manière ionique.

6. Composition selon la revendication 1 **caractérisée en ce que**, dans une composition selon a), l'agent endosomolytique est un virus ou un composant viral.

7. Composition selon la revendication 1 ou 6 **caractérisée en ce que**, dans une composition selon a) ou b), l'agent endosomolytique est un adénovirus.

8. Composition selon la revendication 7 **caractérisée en ce que** l'adénovirus est un mutant.

9. Composition selon la revendication 8 **caractérisée en ce que** l'adénovirus est un mutant incompétent pour la réplication.

10. Composition selon la revendication 9 **caractérisée en ce que** l'adénovirus comporte une ou plusieurs mutations et/ou délétions dans la région E1A.

11. Composition selon la revendication 7 **caractérisée en ce que** l'adénovirus est inactivé.

12. Composition selon la revendication 11 **caractérisée en ce que** l'adénovirus est inactivé par des UV de courte longueur d'onde.

13. Composition selon la revendication 11 **caractérisée en ce que** l'adénovirus est inactivé par UV/psoralène.

14. Composition selon la revendication 11 **caractérisée en ce que** l'adénovirus est inactivé par le formaldéhyde.

15. Composition selon la revendication 6 **caractérisée en ce que** le composant viral consiste en une ou plusieurs protéines adénovirales.

16. Composition selon la revendication 1 ou 6 **caractérisée en ce que**, dans une composition selon a) ou b), l'agent endosomolytique est un picornavirus.

17. Composition selon la revendication 16 **caractérisée en ce que** le picornavirus est un rhinovirus éventuellement inactivé.

18. Composition selon la revendication 1 **caractérisée en ce que** l'agent endosomolytique dans une composition selon a) est un virus qui est infectieux pour une espèce autre que l'homme.

19. Composition selon la revendication 18 **caractérisée en ce que** le virus est un adénovirus.

20. Composition selon la revendication 19 **caractérisée en ce que** l'adénovirus est un adénovirus d'oiseau.

21. Composition selon la revendication 20 **caractérisée en ce que** l'adénovirus est le chick embryo lethal orphan virus.

22. Composition selon la revendication 1 **caractérisée en ce que** l'agent endosomolytique dans une composition selon a) est un peptide viral endosomolytique éventuellement modifié.

23. Composition selon la revendication 22 **caractérisée en ce que** le peptide endosomolytique est un peptide d'hémagglutinine de la grippe HA2.

24. Composition selon la revendication 23 **caractérisée en ce que** le peptide a la séquence Gly-Leu-Phe-Glu-Ala-Ile-Ala-Gly-Phe-Ile-Glu-Asn-Gly-Trp-Glu-Gly-Met-Ile-Asp-Gly-Gly-Gly-Cys.

25. Composition selon la revendication 23 **caractérisée en ce que** le peptide a la séquence Gly-Leu-Phe-Gly-Ala-Ile-Ala-Gly-Phe-Ile-Glu-Asn-Gly-Trp-Glu-Gly-Met-Ile-Asp-Gly-Gly-Gly-Cys.

26. Composition selon la revendication 1 **caractérisée en ce que** l'agent endosomolytique dans une composition selon a) est un peptide non viral, éventuellement modifié, naturel ou synthétique qui présente la capacité de rompre les membranes cellulaires qui peut être déterminée par un test de perméabilité des liposomes et/ou par un test de lyse des érythrocytes.

27. Composition selon la revendication 26 **caractérisée en ce que** le peptide a la séquence Gly Leu Phe Glu Ala Ile Glu Gly Phe Ile Glu Asn Gly Trp Glu Gly Met Ile Asp Gly Gly Gly Cys.

28. Composition selon la revendication 26 **caractérisée en ce que** le peptide est un homo- ou hétérodimère du peptide défini dans la revendication 27.

29. Composition selon la revendication 28 **caractérisée en ce que** le peptide est un homodimère.

30. Composition selon la revendication 28 **caractérisée en ce que** le peptide est un hétérodimère qui présente la séquence Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser Cys.

31. Composition selon la revendication 26 **caractérisée en ce que** le peptide présente la séquence Trp Glu Ala Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

32. Composition selon la revendication 26 **caractérisée en ce que** le peptide présente la séquence Gly Leu Phe Gly Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

33. Composition selon la revendication 22 ou 26 **caractérisée en ce que** le peptide présente un domaine de liaison d'acide nucléique.

34. Composition selon la revendication 33 **caractérisée en ce que** le peptide présente un prolongement d'oligolysine.

35. Composition selon la revendication 1 **caractérisée en ce que** le facteur d'internalisation est la transferrine.

36. Composition selon la revendication 1 **caractérisée en ce que** le facteur d'internalisation est un ligand pour les lymphocytes T.

37. Composition selon la revendication 1 **caractérisée en ce que** le facteur d'internalisation est un ligand pour les lymphocytes B.

38. Composition selon la revendication 37 **caractérisée en ce que** le facteur d'internalisation est une immunoglobuline.

39. Composition selon la revendication 37 **caractérisée en ce que** le ligand est un anti-immunoglobuline.

40. Composition selon la revendication 1 **caractérisée en ce que** le facteur d'internalisation est un ligand pour les hépatocytes.

41. Composition selon la revendication 40 **caractérisée en ce que** le facteur d'internalisation est un ligand pour le récepteur d'asialoglycoprotéine.

42. Composition selon la revendication 41 **caractérisée en ce que** le facteur d'internalisation est une tétragalactose-polylysine.

43. Composition selon la revendication 1 **caractérisée en ce que** le facteur d'internalisation est une lectine.

44. Composition selon la revendication 1 **caractérisée en ce que** le facteur d'internalisation est une low density lipoprotein.

45. Complexe comme constituant d'une composition selon l'une des revendications 1 à 44 **caractérisé en ce qu'**il contient un ou plusieurs acides nucléiques avec lesquels est complexé un agent endosomolytique par le biais d'un domaine de liaison d'acide nucléique qui lui est propre ou par le biais d'une substance à affinité pour les acides nucléiques à laquelle il est lié, et **en ce qu'**il contient éventuellement un facteur d'internalisation qui est complexé de même avec l'acide nucléique par le biais d'une substance à affinité pour les acides nucléiques avec laquelle il est conjugué.

46. Complexe selon la revendication 45 **caractérisé en ce qu'**il contient un acide nucléique thérapeutiquement actif.

47. Complexe selon la revendication 46 **caractérisé en ce que** l'acide nucléique consiste en une ou plusieurs molécules d'ADN efficaces du point de vue de la thérapie génique.

48. Complexe selon la revendication 47 **caractérisé en ce que** la molécule d'ADN code une cytokine.

49. Complexe selon la revendication 47 **caractérisé en ce que** la molécule d'ADN code le facteur VIII.

50. Complexe selon la revendication 46 **caractérisé en ce que** l'acide nucléique contient une séquence nucléotidique à partir de laquelle sont transcrites des molécules d'ARN qui inhibent spécifiquement des fonctions cellulaires.

51. Complexe selon la revendication 45 **caractérisé en ce que** la substance à affinité pour les acides nucléiques est un polycation organique.

52. Complexe selon la revendication 51 **caractérisé en ce que** le polycation est la polylysine.

53. Complexe selon la revendication 51 **caractérisé en ce que** le polycation est la polyéthylèneimine.

54. Complexe selon la revendication 45 **caractérisé en ce qu'**il contient un facteur d'internalisation qui est lié à la même substance à affinité pour les acides nucléiques que l'agent endosomolytique.

55. Complexe selon la revendication 54 **caractérisé en ce que** la substance à affinité pour les acides nucléiques est la polylysine.

56. Complexe selon la revendication 55 **caractérisé en ce qu'**il contient en outre de la polylysine liée de manière non covalente.

57. Conjugué comme constituant d'un complexe selon l'une des revendications 45 à 56 **caractérisé en ce que** le conjugué consiste en un agent endosomolytique et en une substance à affinité pour les acides nucléiques.

58. Conjugué selon la revendication 57 **caractérisé en ce que** l'agent endosomolytique est lié de manière covalente à une substance à affinité pour les acides nucléiques.

59. Conjugué selon la revendication 57 **caractérisé en ce que** l'agent endosomolytique est lié de manière non covalente à une substance à affinité pour les acides nucléiques.

60. Conjugué selon la revendication 59 **caractérisé en ce que** la liaison a lieu par le biais d'un pont biotine-streptavidine.

61. Conjugué selon la revendication 59 **caractérisé en ce que** la liaison a lieu de manière ionique.

62. Conjugué selon la revendication 57 **caractérisé en ce que** l'agent endosomolytique est un virus ou un composant viral.

63. Conjugué selon la revendication 62 **caractérisé en ce que** l'agent endosomolytique est un adénovirus.

64. Conjugué selon la revendication 63 **caractérisé en ce que** l'adénovirus est un mutant.

65. Conjugué selon la revendication 64 **caractérisé en ce que** l'adénovirus est un mutant incompétent pour la réplication.

66. Conjugué selon la revendication 65 **caractérisé en ce que** l'adénovirus comporte une ou plusieurs mutations et/ou délétions dans la région E1A.

67. Conjugué selon la revendication 63 **caractérisé en ce que** l'adénovirus est inactivé.

68. Conjugué selon la revendication 67 **caractérisé en ce que** l'adénovirus est inactivé par des UV de courte longueur d'onde.

69. Conjugué selon la revendication 67 **caractérisé en ce que** l'adénovirus est inactivé par UV/psoralène.

70. Conjugué selon la revendication 67 **caractérisé en ce que** l'adénovirus est inactivé par le formaldéhyde.

71. Conjugué selon la revendication 62 **caractérisé en ce que** le composant viral consiste en une ou plusieurs protéines adénovirales.

72. Conjugué selon la revendication 62 **caractérisé en ce que** le virus est un picornavirus.

73. Conjugué selon la revendication 72 **caractérisé en ce que** le virus est un rhinovirus éventuellement inactivé.

74. Conjugué selon la revendication 57 **caractérisé en ce que** l'agent endosomolytique n'est pas par lui-même capable de pénétrer dans la cellule à transfecter.

75. Conjugué selon la revendication 74 **caractérisé en ce que** l'agent endosomolytique est un virus ou un composant viral qui n'est pas un facteur d'internalisation pour une cellule humaine.

76. Conjugué selon la revendication 75 **caractérisé en ce que** l'agent endosomolytique est un virus qui est infectieux pour une espèce différente de l'homme.

77. Conjugué selon la revendication 76 **caractérisé en ce que** le virus est un adénovirus.

78. Conjugué selon la revendication 77 **caractérisé en ce que** l'adénovirus est un adénovirus d'oiseau.

79. Conjugué selon la revendication 78 **caractérisé en ce que** l'adénovirus est le chick embryo lethal orphan virus.

80. Conjugué selon la revendication 74 **caractérisé en ce que** l'agent endosomolytique est un peptide viral endosomolytique éventuellement modifié ayant la propriété définie dans la revendication 26.

81. Conjugué selon la revendication 80 **caractérisé en ce que** le peptide endosomolytique est un peptide d'hémagglutinine de la grippe HA2.

82. Conjugué selon la revendication 81 **caractérisé en ce que** le peptide a la séquence Gly-Leu-Phe-Glu-Ala-Ile-Ala-Gly-Phe-Ile-Glu-Asn-Gly-Trp-Glu-Gly-Met-Ile-Asp-Gly-Gly-Gly-Cys.

83. Conjugué selon la revendication 81 **caractérisé en ce que** le peptide a la séquence Gly-Leu-Phe-Gly-Ala-Ile-Ala-Gly-Phe-Ile-Glu-Asn-Gly-Trp-Glu-Gly-Met-Ile-Asp-Gly-Gly-Gly-Cys.

84. Conjugué selon la revendication 74 **caractérisé en ce que** l'agent endosomolytique est un peptide défini dans la revendication 26.

85. Conjugué selon la revendication 84 **caractérisé en ce que** le peptide a la séquence Gly Leu Phe Glu Ala Ile Glu Gly Phe Ile Glu Asn Gly Trp Glu Gly Met Ile Asp Gly Gly Gly Cys.

86. Conjugué selon la revendication 84 **caractérisé en ce que** le peptide est un homo- ou hétérodimère du peptide défini dans la revendication 85.

87. Conjugué selon la revendication 86 **caractérisé en ce que** le peptide est un homodimère.

88. Conjugué selon la revendication 86 **caractérisé en ce que** le peptide est un hétérodimère qui présente la séquence Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser Cys.

89. Conjugué selon la revendication 84 **caractérisé en ce que** le peptide présente la séquence Trp Glu Ala Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

90. Conjugué selon la revendication 84 **caractérisé en ce que** le peptide présente la séquence Gly Leu Phe Gly Ala Leu Ala Glu Ala Leu Ala Glu Ala Leu Ala Glu His Leu Ala Glu Ala Leu Ala Glu Ala Leu Glu Ala Leu Ala Ala Gly Gly Ser Cys.

91. Conjugué selon la revendication 84 **caractérisé en ce que** le peptide présente un domaine de liaison d'acide nucléique.

92. Conjugué selon la revendication 91 **caractérisé en ce que** le peptide présente un prolongement d'oligolysine.

93. Peptide endosomolytique approprié comme constituant de la composition selon la revendication 33 **caractérisé en ce qu'**il a un domaine endosomolytique et un domaine de liaison d'acide nucléique introduit artificiellement.

94. Peptide selon la revendication 93 **caractérisé en ce que** le domaine de liaison d'acide nucléique est un prolongement d'oligolysine.

95. Procédé de préparation d'un conjugué selon la revendication 58 **caractérisé en ce qu'**un virus ou un agent endosomolytique (poly)peptidique et une polyamine sont couplés par voie enzymatique en présence d'une transglutaminase.

96. Procédé de préparation d'un conjugué selon la revendication 58 **caractérisé en ce qu'**un virus ou un agent endosomolytique (poly)peptidique et une polyamine sont couplés par voie chimique.

97. Procédé de préparation d'un conjugué selon la revendication 60 **caractérisé en ce qu'**un virus ou un composant viral est modifié avec la biotine et **en ce que** le virus modifié ou le composant viral modifié est lié à une polyamine couplée à la streptavidine.

98. Procédé pour introduire un acide nucléique dans des cellules eucaryotes supérieures in vitro ou ex vivo **caractérisé en ce que** les cellules sont traitées avec une composition selon l'une des revendications 1 à 44.

99. Procédé selon la revendication 98 **caractérisé en ce que** les cellules sont des cellules humaines.

100. Procédé selon la revendication 99 **caractérisé en ce que** les cellules sont des cellules tumorales.

101. Procédé selon la revendication 99 **caractérisé en ce que** les cellules sont des myoblastes.

102. Procédé selon la revendication 99 **caractérisé en ce que** les cellules sont des fibroblastes.

103. Procédé selon la revendication 99 **caractérisé en ce que** les cellules sont des hépatocytes.

104. Procédé selon la revendication 99 **caractérisé en ce que** les cellules sont des cellules endothéliales.

105. Procédé selon la revendication 104 **caractérisé en ce que** les cellules sont des cellules des voies respiratoires.

106. Procédé selon l'une des revendications 99 à 105 **caractérisé en ce que** l'acide nucléique dans la composition est efficace du point de vue de la thérapie génique.

107. Procédé selon les revendications 100 et 106 **caractérisé en ce que** les cellules tumorales sont traitées avec la composition ex vivo et **en ce que** l'ADN code une ou plusieurs substances immunomodulatrices, de préférence des cytokines.

108. Procédé de préparation d'une protéine hétérologue dans une cellule eucaryote supérieure **caractérisé en ce que** l'on traite les cellules avec une composition selon la revendication 1 où l'acide nucléique contient une séquence d'ADN qui code la protéine voulue, on cultive les cellules dans des conditions qui sont appropriées pour l'expression de la protéine et un isole la protéine.

109. Préparation pharmaceutique contenant comme constituant efficace une composition selon l'une des revendications 1 à 44 contenant un acide nucléique thérapeutiquement actif et un support pharmaceutiquement acceptable.

110. Kit de transfection contenant une unité de support dans laquelle se trouvent deux ou plusieurs récipients, où un premier récipient contient une substance à affinité pour les acides nucléiques, qui est éventuellement conjuguée avec un facteur d'internalisation pour une cellule eucaryote supérieure, et un second récipient contient un agent endosomolytique qui est un virus ou un composant viral qui est capable par lui-même de pénétrer dans la cellule à transfecter.

111. Kit de transfection contenant une unité de support dans laquelle se trouvent deux ou plusieurs récipients, où un premier récipient contient une substance à affinité pour les acides nucléiques, qui est conjuguée avec un facteur d'internalisation pour une cellule eucaryote supérieure, et un second récipient contient une substance à affinité pour les acides nucléiques qui est liée à un agent endosomolytique.

112. Kit de transfection selon la revendication 111 **caractérisé en ce qu'**il contient dans le second récipient un conjugué adénovirus-polylysine couplé à la transglutaminase.

113. Kit de transfection contenant une unité de support dans laquelle se trouvent deux ou plusieurs récipients, où un premier récipient contient un agent endosomolytique modifié avec la biotine et un second récipient contient une substance à affinité pour les acides nucléiques modifiée avec la streptavidine.

114. Kit de transfection selon la revendication 113 **caractérisé en ce que** le premier récipient contient un adénovirus biotiné et un second récipient contient de la streptavidine-polylysine.
